# EUROPEAN PATENT APPLICATION

(11) **EP 2 196 465 A1**
(43) Date of publication of application: **16.06.2010**
(21) Application number: 08382077.9
(22) Date of filing: 15.12.2008
(51) Int. Cl.: C07D 401/04, C07D 401/14, A61K 31/501, A61P 29/00, A61P 11/00

(54) **(3-oxo)pyridazin-4-ylurea derivatives as PDE4 inhibitors**

(71) Applicant: Almirall, S.A., 08022 Barcelona (ES)
(72) Inventor: Gracia Ferrer, Jordi, 08720, Vilafranca del Penedès, (Barcelona) (ES); Buil Albero, Maria Antonia, 08029, Barcelona (ES); Moreno Mollo, Inmaculada Montserrat, 25150, Artesa de Lleida (Lleida) (ES); Pages Santacana, Lluis Miquel, 08029, Barcelona (ES); Roberts, Richard Spurring, 08012, Barcelona (ES); Sevilla Gome, Sara, 08690, Santa Coloma de Cervelló (Barcelona) (ES); Taltavull Moll, Joan, 08015, Barcelona (ES)
(74) Representative: Elzaburu Marquez, Alberto

(57) **Abstract**

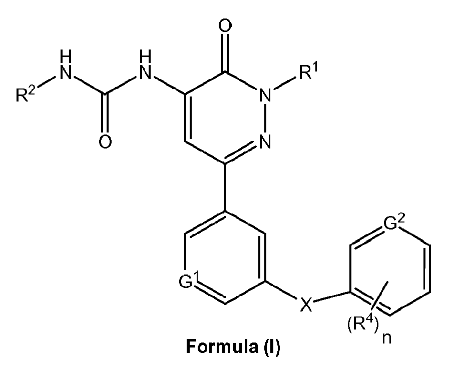

New (3-oxo)pyridazin-4-ylurea derivatives having the chemcial structure of formula (I) are disclosed; as well as process for their preparation, pharmaceutical compositions comprising them and their use in therapy as inhibitors of the phosphodiesterase IV (PDE4).

## Description

The present invention relates to new therapeutically useful (3-oxo)pyridazin-4-ylurea derivatives, to processes for their preparation and to pharmaceutical compositions comprising them. These compounds are potent and selective inhibitors of phosphodiesterase 4 (PDE4) and are thus useful in the treatment, prevention or suppression of pathological conditions, diseases and disorders known to be susceptible of being improved by inhibition of PDE4.

Phosphodiesterases (PDEs) comprise a superfamily of enzymes responsible for the hydrolysis and inactivation of the second messenger cyclic adenosine monophosphate (cAMP) and cyclic guanosine monophosphate (cGMP). Eleven different PDE families have been identified to date (PDE1 to PDE11) which differ in substrate preference, catalytic activity, sensitivity to endogenous activators and inhibitors, and encoding genes.

The PDE4 isoenzyme family exhibits a high affinity for cyclic AMP but has weak affinity for cyclic GMP. Increased cyclic AMP levels caused by PDE4 inhibition are associated with the suppression of cell activation in a wide range of inflammatory and immune cells, including lymphocytes, macrophages, basophils, neutrophils, and eosinophils. Moreover, PDE4 inhibition decreases the release of the cytokine Tumor Necrosis Factor (TNF ). The biology of PDE4 is described in several recent reviews, for example M. D. Houslay, P. Schafer, K. Y. Zhang, Drug Discov Today 2005, 10, 1503-19.

In view of these physiological effects, PDE4 inhibitors of varied chemical structures have been recently disclosed for the treatment or prevention of chronic and acute inflammatory diseases and other pathological conditions, diseases and disorders known to be susceptible to amelioration by inhibition of PDE4. See, for example, US 5449686, US 5710170, WO 98/45268, WO 99/06404, WO 01/57025, WO 01/57036, WO 01/46184, WO 97/05105, WO 96/40636, WO03/097613, US 5786354, US 5773467, US 5753666, US 5728712, US 5693659, US 5679696, US 5596013, US 5541219, US 5508300, US 5502072 or H. J. Dyke and J. G. Montana, Exp. Opin. Invest. Drugs 1999, 8, 1301-1325.

A few compounds having the capacity to selectively inhibit phosphodiesterase 4 are in active development. Examples of these compounds are roflumilast, GSK-256066, apremilast, tetomilast, rolipram, MK-0873 and oglemilast.

The international applications WO03097613 A1 and WO04058729 A1 describe (3-oxo)pyridazin-4-ylurea derivatives as potent and selective inhibitors of PDE4. We have now found that the compounds of formula (I) described in more detail below have surprising and particularly advantageous properties.

It is known that the clinical developement in human of early PDE4 inhibitors such as rolipram has been hampered by the appearance of side effects such as nausea and vomiting at therapeutic plasma levels (Curr. Pharm. Des. 2002, 8, 1255-96). The compounds described in the present invention are potent and selective PDE4 inhibitors with reduced emetic potential. This property makes them useful for the treatment or prevention of pathological conditions or diseases such as respiratory diseases, skin disease, inflammatory diseases, diseases of the central or peripheral nervous system and Cancer. Theses deseases include but are not limited to asthma, chronic obstructive pulmonary disease, allergic rhinitis, rheumatoid arthritis, multiple sclerosis, atopic dermatitis, psoriasis and inflammatory bowel disease.

The compounds of the present invention can also be used in combination with other drugs known to be effective in the treatment of these diseases. For example, they can be used in combination with bronchodilators such as β2-adrenergic agonists, antagonists of M3 muscarinic receptors or dual acting molecules combining β2 agonism with M3 antagonism, anti-allergics such as anti-histamines, mast cell stabilizers, CRTH antagonists, anti-inflammatory agents such as corticosteroids, LTD4 receptor antagonists, leukotriene synthesis inhibitors, COX inhibitors and PDE inhibitors,, immunosuppressive agents such as calcineurin inhibitors, cyclosporin A, rapamycin, T-cell receptor blockers, B cell receptor blockers, and/or antiinfectives such as antibiotics, antimycotics, antiviral agents.

Accordingly, the present invention provides novel compounds of formula (I): 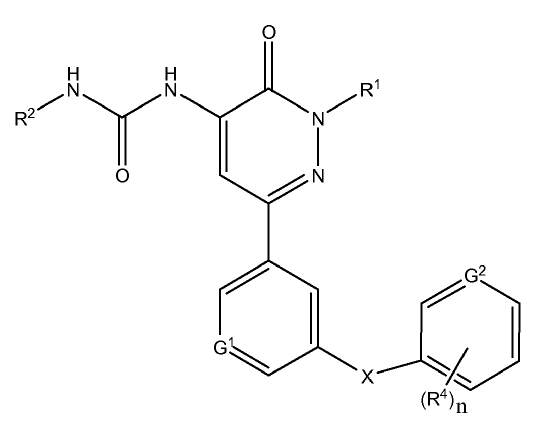 wherein:
- R¹ is selected from the group consisting of a -(C₁₋₄ alkyl)-(C₃₋₅ cycloalkyl) group and a C₁₋₄ alkyl group optionally substituted by one or more halogen atoms,
- R² is selected from the group consisting of a C₅₋₁₀ aryl group and a 5 to 10- membered heteroaryl group containing at least one heteroatom selected from N, S and O, wherein the aryl and the heteroaryl group are optionally substituted by one or more substituents selected from halogen atoms, C₁₋₄ alkyl groups and C₁₋₄.alkoxy groups,
- X is selected from the group consisting of a direct bond, -O-, -O-CH₂-, -S-, and - S(O)₂-,
- G¹ is selected from the group consisting of a nitrogen atom and a -CH= group,
- G² is selected from the group consisting of a nitrogen atom and a -CR³= group,
- R³ and R⁴ independently are selected from the group consisting of a hydrogen atom, a halogen atom, a hydroxy group, a cyano group, a linear or branched C₁₋₄ alkyl group, a linear or branched C₁₋₄ hydroxyalkyl group, a methylsulphonyl group, a sulphonyloxy group, a trifluoromethyl group, a trifluoromethoxy group, a cyclopropyloxy group, a formyl group, a -CH₂N(CH₃)₂ group, -O-(CH₂)₍₁₋₃₎-R^{b}, -O-(CH₂)₍₁₋₅₎NR^{a}R^{b}-, -(CH₂)₍₀₋₄₎-C(O)OR^{b}, -(CH₂)₍₀₋₄₎-C(O)NR^{a}R^{b}, -(CH₂)₍₀₋₂₎-CO-NH-(CH₂)₍₁₋₃₎-R^{b} and -(CH₂)₍₀₋₂₎-CO-NH-(CH₂)₍₁₋₈₎-NR^{a}R^{b} wherein
   ○ R^{a} represents a hydrogen atom or a C₁₋₄ alkyl group,
   ○ R^{b} represents a hydrogen atom, a C₁₋₄ alkyl group, a C₁₋₄ carboxyalkyl group, a C₁₋₂ hydroxyalkyl group, a carbamoyl group, a monocyclic or polycyclic C₃₋₁₀ cycloalkyl group, a monocyclic or polycyclic unsaturated non-aromatic C₃₋₁₀ carbocyclic group, a monocyclic or polycyclic C₅₋₁₀ aryl group, a monocyclic or polycyclic 5- to 10- membered heteroaryl group, or a monocyclic or polycyclic 5 to 10- membered heterocyclic group, which cycloalkyl, unsaturated non-aromatic carbocyclic, aryl, heteroaryl and heterocyclic groups are optionally substituted by one or more substituents selected from the group consisting of halogen atoms, hydroxy groups, benzyl groups, C₁₋₂ alkyl groups, C₁₋₆ hydroxyalkyl groups, C₁₋₄ dialkylamino groups or carboxy groups, or
   ○ R^{a} and R^{b} together with the nitrogen atom to which they are attached form a 4 to 6 membered saturated or unsaturated heterocyclic ring optionally containing one additional heteroatom selected from N and O, and optionally substituted with a C₁₋₄ alkyl group, a carbamoyl group or a C₁₋₂alkoxy(C₁₋₂)alkyl group;
- n is integer from 1 to 3
and the pharmaceutically acceptable salts or N-oxides thereof.

Further objectives of the present invention are to provide compounds of formula (I) for use in the treatment of the human or animal body, in particular in the treatment of a pathological condition or disease susceptible to amelioration by inhibition of phosphodiesterase IV, such as asthma, chronic obstructive pulmonary disease, allergic rhinitis, rheumatoid arthritis, multiple sclerosis, atopic dermatitis, psoriasis or inflammatory bowel disease. The invention is also directed to the use of compounds of formula (I) for the manufacture of a medicament for the treatment of these diseases and also to a method of treatment thereof

As used herein the term alkyl embraces optionally substituted, linear or branched hydrocarbon radicals having 1 to 8 carbon atoms, preferably 1 to 4 carbon atoms. Examples include methyl, ethyl, *n*-propyl, *i*-propyl, *n*-butyl, *sec*-butyl and *tert*-butyl radicals.

As used herein, the term hydroxyalkyl embraces linear or branched alkyl radicals having 1 to 8, preferably 1 to 4 carbon atoms, any one of which may be substituted with one or more hydroxyl radicals.

Examples of such radicals include hydroxymethyl, hydroxyethyl, hydroxypropyl, hydroxybutyl and 1,2-dihydroxypropyl.

As used herein, the term carboxyalkyl embraces linear or branched alkyl radicals having 1 to 8, preferably 1 to 4 carbon atoms, any one of which may be substituted with one or more carboxyl radicals.

Examples of such radicals include carboxymethyl, carboxyethyl, carboxypropyl and carboxybutyl.

As used herein, the term dialkylamino embraces radicals containing a trivalent nitrogen atoms with two optionally substituted, linear or branched alkyl radicals of 1 to 8, preferably 1 to 4 carbon atoms in each alkyl radical.

A dialkylamino group typically contains two alkyl groups, each of which is unsubstituted or substituted with 1, 2 or 3 substituents which may be the same or different. Typically and unless otherwise specified, the substituents on a dialkylamino group are themselves unsubstituted.

Preferred optionally substituted dialkylamino radicals include dimethylamino, diethylamino, methyl(ethyl)amino, di(n-propyl)amino, n-propyl(methyl)amino, n-propyl(ethyl)amino, di(i-propyl)amino, i-propyl(methyl)amino, i-propyl(ethyl)amino, di(n-butyl)amino, n-butyl(methyl)amino, n-butyl(ethyl)amino and n-butyl(i-propyl)amino

As used herein the term alkoxy embraces optionally substituted, linear or branched oxygen containing radicals each having 1 to 4 carbon atoms. Preferred substituents on the alkoxy groups are halogen atoms and hydroxy groups.

Examples include methoxy, ethoxy, *n*-propoxy, *i*-propoxy, *n*-butoxy, *sec*-butoxy and *tert-*butoxy radicals.

As used herein, the term cycloalkyl embraces optionally substituted saturated carbocyclic radicals and, unless otherwise specified, a cycloalkyl radical typically has from 3 to 10 carbon atoms, preferably from 3 to 4 carbon atoms.

Examples include cyclopropyl, cyclobutyl, cyclopentyl, cyclohexyl, cycloheptyl and adamantyl. When a cycloalkyl radical carries 2 or more substituents, the substituents may be the same or different.

As used herein, the term unsaturated non-aromatic carbocyclic group embraces optionally substituted unsaturated non-aromatic carbocyclic radicals and, unless otherwise specified, an unsaturated non-aromatic carbocyclic group typically has from 3 to 10 carbon atoms, preferably from 3 to 4 carbon atoms.

Examples include cyclopropenyl, cyclobutenyl, cyclopentenyl, cyclohexenyl and cycloheptenyl. When an unsaturated non-aromatic carbocyclic radical carries 2 or more substituents, the substituents may be the same or different.

As used herein, the term aryl radical embraces typically optionally substituent C₅-C₁₀, preferably C₆-C₁₀, monocyclic or polycyclic aryl radical such as phenyl, naphthyl, anthranyl and phenanthryl. Phenyl is preferred. A said optionally substituted aryl radical is typically unsubstituted or substituted with 1, 2 or 3 substituents which may be the same or different. Unless otherwise specified, the substituents on an aryl group are typically themselves unsubstituted.

As used herein, the term heteroaryl embraces typically a 5- to 10- membered ring system, comprising at least one heteroaromatic ring and containing at least one heteroatom selected from O, S and N. A heteroaryl radical may be a single ring (monocyclic) or two or more fused rings (polycyclic) wherein at least one ring contains a heteroatom.

As used herein, the term heterocyclic ring embraces typically a non-aromatic, saturated or unsaturated C₃-C₁₀ carbocyclic ring system, such as a 5, 6 or 7 membered radical, in which one or more, for example 1, 2, 3 or 4 of the carbon atoms preferably 1 or 2 of the carbon atoms are replaced by a heteroatom selected from N, O and S. Saturated heterocyclic ring are preferred.

As used herein, the term halogen atom embraces chlorine, fluorine, bromine or iodine atoms typically a fluorine, chlorine or bromine atom. The term halo when used as a prefix has the same meaning.

As used herein, some of the atoms, radicals, moieties, chains or cycles present in the general structures of the invention are "optionally substituted". This means that these atoms, radicals, moieties, chains or cycles can be either unsubstituted or substituted in any position by one or more, for example 1, 2, 3 or 4, substituents, whereby the hydrogen atoms bound to the unsubstituted atoms, radicals, moieties, chains or cycles are replaced by chemically acceptable atoms, radicals, moieties, chains or cycles. When two or more substituents are present, each substituent may be the same or different.

As used herein, the term pharmaceutically acceptable salt embraces salts with a pharmaceutically acceptable acid or base. Pharmaceutically acceptable acids include both inorganic acids, for example hydrochloric, sulphuric, phosphoric, diphosphoric, hydrobromic, hydroiodic and nitric acid and organic acids, for example citric, fumaric, maleic, malic, mandelic, ascorbic, oxalic, succinic, tartaric, benzoic, acetic, methanesulphonic, ethanesulphonic, benzenesulphonic, cyclohexylsulfamic (cyclamic) or p-toluenesulphonic acid. Pharmaceutically acceptable bases include alkali metal (e.g. sodium or potassium) and alkali earth metal (e.g. calcium or magnesium) hydroxides and organic bases, for example alkyl amines, arylalkyl amines and heterocyclic amines.

Other preferred salts according to the invention are quaternary ammonium compounds wherein an equivalent of an anion (X⁻) is associated with the positive charge of the N atom. X⁻ may be an anion of various mineral acids such as, for example, chloride, bromide, iodide, sulphate, nitrate, phosphate, or an anion of an organic acid such as, for example, acetate, maleate, fumarate, citrate, oxalate, succinate, tartrate, malate, mandelate, trifluoroacetate, methanesulphonate and *p*-toluenesulphonate. X⁻ is preferably an anion selected from chloride, bromide, iodide, sulphate, nitrate, acetate, maleate, oxalate, succinate or trifluoroacetate. More preferably X⁻ is chloride, bromide, trifluoroacetate or methanesulphonate.

Typically, R¹ represents a C₁₋₄ alkyl group optionally substituted with one or or more halogen atoms. Preferably, R¹ represents a methyl or ethyl group, more preferably an ethyl group.

Typically, R² represents a 5 to 10 membered N- containing heteroaryl group optionally substituted by one or more substituents selected from halogen atoms and C₁₋₄ alkyl groups. Preferably, R² is a pyridyl group optionally substituted by one or two substitutents selected from fluorine atoms, chlorine atoms and methyl groups. More preferably, R² is a pyridyl group substituted by two substitutents selected from chlorine atoms and methyl groups, being most preferably chlorine atoms.

When X represents a moiety -O-CH₂-, it is to be understood that preferably (a) the oxygen atom forms a bond to the left hand side of the molecule as depicted in formula (I), and (b) the CH₂ forms a bond to the right hand side of the molecule as depicted in formula (I).

Typically, X is selected from the group consisting of a direct bond, -O-, -S-, and -S(O)₂-. Preferably, X is a direct bond.

Typically, G¹ represents a -CH= group.

Typically G² represents a -CR³= group.

Typically, R³ and R⁴ independently are selected from the group consisting of hydrogen atom, fluorine atom, chlorine atom, hydroxy group, cyano group, a methyl group, a hydroxyethyl group, a trifluoromethoxy group, -O-(CH₂)₍₁₋₃₎-R^{b}, -O-(CH₂)₍₂₋₅₎NR^{a}R^{b}, -(CH₂)₍₀₋₂₎-C(O)OR^{b}, -(CH₂)₍₀₋₂₎-C(O)NR^{a}R^{b}, -(CH₂)₍₀₋₂₎-CO-NH-(CH₂)₍₁₋₃₎-R^{b} and -(CH₂)₍₀₋₂)-CO-NH-(CH₂)₍₂₋₇₎-NR^{a}R^{b}, wherein:
○ R^{b} represents a hydrogen atom, a C₁₋₄ alkyl group, a C₁₋₂ hydroxyalkyl group, a C₃₋₅ cycloalkyl group, a phenyl group, a pirrolidin-2-yl group, a piperidin-4-yl group, werein the cyclic groups optionally are substituted by one or two substituents selected from the group consisting of chlorine atoms, hydroxy groups, benzyl groups, C₁₋₂ alkyl groups, C₂₋₅ hydroxyalkyl groups and C₁₋₂ dialkylamino groups, or
○ R^{a} and R^{b} together with the nitrogen atom to which they are attached from a 5 to 6 membered saturated or non-saturated heterocyclic ring optionally containing one additional heteroatom selected from N and O, and optionally substituted with a C₁₋₂ alkyl group, a carbamoyl group or a C₁₋₂alkoxy(C₁₋₂)alkyl group,

Preferably, R³ and R⁴ independently are selected from the group consisting of hydrogen atom, fluorine atom, hydroxy group, cyano group, a trifluoromethoxy group, -O-(CH₂)₍₁₋₃₎-R^{b}, -O-(CH₂)₍₂₋₄₎NR^{a}R^{b}, -(CH₂)₍₀₋₁₎-C(O)OR^{b}, -(CH₂)₍₀₋₁₎-C(O)NR^{a}R^{b}, -(CH₂)₍₀₋₁₎-CO-NH-(CH₂)₍₁₋₃₎-R^{b} and -(CH₂)₍₀₋₂₎-CO-NH-(CH₂)₍₂₋₇₎-NR^{a}R^{b}, wherein:
○ R^{b} represents a hydrogen atom, a C₁₋₄ alkyl group, a C₁₋₂ hydroxyalkyl group, a C₃₋₅ cycloalkyl group, a phenyl group, a pirrolidin-2-yl group, a piperidin-4-yl group, werein the cyclic groups optionally are substituted by one or more substituents selected from the group consisting of halogen atoms, hydroxy groups, benzyl groups, C₁₋₂ alkyl groups, C₁₋₅ hydroxyalkyl groups, C₁₋₂ dialkylamino groups or carboxy groups, or
○ R^{a} and R^{b} together with the nitrogen atom to which they are attached from a 5 to 6 membered saturated or non-saturated heterocyclic ring optionally containing one additional heteroatom selected from N and O, and optionally substituted with a C₁₋₂ alkyl group, carbamoyl group or a C₁₋₂alkoxy(C₁₋₂)alkyl

More preferably, R³ and R⁴ independently are selected from the group consisting of -O-(CH₂)₍₁₋₂₎-R^{b}, -O-(CH₂)₍₂₋₅₎NR^{a}R^{b}, -C(O)OR^{b}, -C(O)NR^{a}R^{b}, -CO-NH-(CH₂)₍₁₋₃₎-R^{b} and -CO-NH-(CH₂)₍₂₋₄₎-NR^{a}R^{b}, wherein
○ R^{a} represents a hydrogen atom, a methyl group or an ethyl group,
○ R^{b} represents a hydrogen atom, a methyl group, a hydroxyethyl group, a cyclopropyl group, a phenyl group, a pirrolidin-2-yl group or a piperidin-4-yl group, werein the cyclic groups optionally are substituted by one substituent selected from the group consisting of hydroxy groups, methyl groups, benzyl groups and hydroxyethyl groups, or
○ R^{a} and R^{b} together with the nitrogen atom to which they are attached from a 5 or 6 membered saturated or non-saturated heterocyclic ring optionally containing one additional heteroatom selected from N and O, and optionally substituted with a methyl group, a carbamoyl group or a methoxyethyl group,
being most preferably, n has a value of 1

In a preferred embodiment of the present invention, R¹ represents an ethyl group, R² is a pyridyl group substituted by two chlorine atoms, X is a direct bond, G¹ represents a -CH= group, G² represents a -CR³= group, n has a value of 1, R³ and R⁴ independently are selected from the group consisting of -O-(CH₂)₍₁₋₂₎-R^{b}, -O-(CH₂)₍₂₋₅₎NR^{a}R^{b}, -C(O)OR^{b}, - C(O)NR^{a}R^{b}, -CO-NH-(CH₂)₍₁₋₃₎-R^{b} and -CO-NH-(CH₂)₍₂₋₄₎-NR^{a}R^{b}, wherein
○ R^{a} represents a hydrogen atom, a methyl group or an ethyl group,
○ R^{b} represents a hydrogen atom, a methyl group, a hydroxyethyl group, a cyclopropyl group, a phenyl group, a pirrolidin-2-yl group or a piperidin-4-yl group, werein the cyclic groups optionally are substituted by one substituent selected from the group consisting of hydroxy group, a methyl group, a benzyl group and a hydroxyethyl group, or
○ R^{a} and R^{b} together with the nitrogen atom to which they are attached from a 5 or 6 membered saturated or non-saturated heterocyclic ring optionally containing one additional heteroatom selected from N and O, and optionally substituted with a methyl group, a carbamoyl or a methoxyethyl group.

In a further preferred embodiment of the invention,
R¹ represents an C₁-C₂ alkyl group which is unsubstituted or substituted by 1 or 2 halogen atoms or a -(C₁₋₂ alkyl)-(cyclopropyl) group,
R² represents a pyridyl group which is unsubstituted or substituted by two substituents independently selected from halogen atoms and methyl groups,
X represents a direct bond, -O-, -O-CH₂-, -S-, or -S(O)₂-,
G¹ represents a nitrogen atom or a -CH= group,
G² represents a nitrogen atom or a -CR³= group,
R³ and R⁴ are independently selected from a hydrogen atom, a fluorine atom, a chlorine atom, hydroxy group, a cyano group, a methyl group, a linear C₁₋₃ hydroxyalkyl group, a methylsulphonyl group, a sulphonyloxy group, a trifluoromethyl group, a trifluoromethoxy group, a cyclopropyloxy group, a formyl group, a -CH₂N(CH₃)₂ group, -O-(CH₂)₍₁₋₃₎-R^{b}, -O-(CH₂)₍₂₋₅₎NR^{a}R^{b}, -(CH₂)₍₀₋₂)-C(O)OR^{b}, -(CH₂)₍₀₋₂₎-C(O)NR^{a}R^{b}, -(CH₂)₍₀₋₂₎-CO-NH-(CH₂)₍₁₋₂₎-R^{b} and -(CH₂)₍₀₋₂₎-CO-NH-(CH₂)₍₂₋₇₎-NR^{a}R^{b} wherein
   ○ R^{a} represents a hydrogen atom or a methyl or ethyl group,
   ○ R^{b} represents a hydrogen atom, a C₁₋₄ alkyl group, a -CH₂COOH group, a C₁₋₂ hydroxyalkyl group, a carbamoyl group, a pyrrolidin-2-yl group, a piperidin-4-yl group, an adamantyl group, a pyridinyl group, a phenyl group, which pyrrolidin-2-yl, a piperidin-4-yl, adamantyl, pyridinyl and phenyl groups are optionally substituted by one or two substituents selected from chlorine atoms, hydroxy groups, benzyl groups, methyl groups, hydroxyethyl groups and carboxy groups, or
   ○ R^{a} and R^{b} together with the nitrogen atom to which they are attached form a 5 or 6 membered saturated or unsaturated heterocyclic ring optionally containing one additional heteroatom selected from N and O, and optionally substituted with a methyl group; a carbamoyl group or a - CH₂OCH₃ group; and
n is 1 or 2.

Particular individual compounds of the invention include:
N-(3,5-Dichloropyridin-4-yl)-N'-[2-ethyl-6-(3'-hydroxybiphenyl-3-yl)-3-oxo-2,3-dihydropyridazin-4-yl]urea
N-(3,5-Dichloropyridin-4-yl)-N'-[2-ethyl-6-(4'-hydroxybiphenyl-3-yl)-3-oxo-2,3-dihydropyridazin-4-yl]urea
N-(3,5-Dichloropyridin-4-yl)-N'-[2-ethyl-6-(2'-hydroxybiphenyl-3-yl)-3-oxo-2,3-dihydropyridazin-4-yl]urea
N-(3,5-Dichloropyridin-4-yl)-N'-[2-ethyl-6-(5'-fluoro-2'-hydroxybiphenyl-3-yl)-3-oxo-2,3-dihydropyridazin-4-yl]urea
N-(3,5-Dichloropyridin-4-yl)-N'-[2-ethyl-6-(2'-fluoro-4'-hydroxybiphenyl-3-yl)-3-oxo-2,3-dihydropyridazin-4-yl]urea
N-(3,5-Dichloropyridin-4-yl)-N'-[2-ethyl-6-(3'-fluoro-4'-hydroxybiphenyl-3-yl)-3-oxo-2,3-dihydropyridazin-4-yl]urea
N-(3,5-Dichloropyridin-4-yl)-N'-[2-ethyl-6-(2'-hydroxy-5'-methylbiphenyl-3-yl)-3-oxo-2,3-dihydropyridazin-4-yl]urea
N-(3,5-Dichloropyridin-4-yl)-N'-[2-ethyl-6-(2'-fluoro-3'-hydroxybiphenyl-3-yl)-3-oxo-2,3-dihydropyridazin-4-yl]urea
N-(3,5-Dichloropyridin-4-yl)-N'-[2-ethyl-6-(4'-hydroxy-2'-methylbiphenyl-3-yl)-3-oxo-2,3-dihydropyridazin-4-yl]urea
N-(3,5-Dichloropyridin-4-yl)-N'-{2-ethyl-6-[4'-(hydroxymethyl)biphenyl-3-yl]-3-oxo-2,3-dihydropyridazin-4-yl}urea
N-(3,5-Dichloropyridin-4-yl)-N'-[2-ethyl-6-(4'-fluoro-2'-hydroxybiphenyl-3-yl)-3-oxo-2,3-dihydropyridazin-4-yl]urea
N-(6-Biphenyl-3-yl-2-ethyl-3-oxo-2,3-dihydropyridazin-4-yl)-N'-(3,5-dichloropyridin-4-yl)urea
N-(3,5-Dichloropyridin-4-yl)-N'-{2-ethyl-6-[4'-(1-hydroxy-1-methylethyl)biphenyl-3-yl]-3-oxo-2,3-dihydropyridazin-4-yl}urea
N-(3,5-Dichloropyridin-4-yl)-N'-{2-ethyl-6-[3'-(hydroxymethyl)biphenyl-3-yl]-3-oxo-2,3-dihydropyridazin-4-yl}urea
3'-[5-({[(3,5-Dichloropyridin-4-yl)amino]carbonyl}amino)-1-ethyl-6-oxo-1,6-dihydropyridazin-3-yl]-4-hydroxybiphenyl-3-carboxamide
3'-[5-({[(3,5-Dichloropyridin-4-yl)amino]carbonyl}amino)-1-ethyl-6-oxo-1,6-dihydropyridazin-3-yl]biphenyl-3-carboxamide
3'-[5-({[(3,5-Dichloropyridin-4-yl)amino]carbonyl}amino)-1-ethyl-6-oxo-1,6-dihydropyridazin-3-yl]-N-[2-(dimethylamino)ethyl]biphenyl-3-carboxamide
N-(3,5-Dichloropyridin-4-yl)-N'-{2-ethyl-6-[5-(3-hydroxyphenyl)pyridin-3-yl]-3-oxo-2,3-dihydropyridazin-4-yl}urea
2-{3'-[5-({[(3,5-Dichloropyridin-4-yl)amino]carbonyl}amino)-1-ethyl-6-oxo-1,6-dihydropyridazin-3-yl]biphenyl-3-yl}acetamide
3-{5-[5-({[(3,5-Dichloropyridin-4-yl)amino]carbonyl}amino)-1-ethyl-6-oxo-1,6-dihydropyridazin-3-yl]pyridin-3-yl}benzamide
N-Cyclopropyl-3'-[5-({[(3,5-dichloropyridin-4-yl)amino]carbonyl}amino)-1-ethyl-6-oxo-1,6-dihydropyridazin-3-yl]biphenyl-3-carboxamide
3'-[5-({[(3,5-Dichloropyridin-4-yl)amino]carbonyl}amino)-1-ethyl-6-oxo-1,6-dihydropyridazin-3-yl]-N-[3-(dimethylamino)propyl]biphenyl-3-carboxamide
3'-[5-({[(3,5-Dichloropyridin-4-yl)amino]carbonyl}amino)-1-ethyl-6-oxo-1,6-dihydropyridazin-3-yl]-6-hydroxybiphenyl-3-carboxamide
3'-[5-({[(3,5-Dichloropyridin-4-yl)amino]carbonyl}amino)-1-ethyl-6-oxo-1,6-dihydropyridazin-3-yl]-N-[2-(dimethylamino)ethyl]-3-hydroxybiphenyl-4-carboxamide
Ethyl 3'-[5-({[(3,5-dichloropyridin-4-yl)amino]carbonyl}amino)-1-ethyl-6-oxo-1,6-dihydropyridazin-3-yl]biphenyl-4-carboxylate
3'-[5-({[(3,5-Dichloropyridin-4-yl)amino]carbonyl}amino)-1-ethyl-6-oxo-1,6-dihydropyridazin-3-yl]biphenyl-4-carboxylic acid
Methyl 5-{3-[5-({[(3,5-dichloropyridin-4-yl)amino]carbonyl}amino)-1-ethyl-6-oxo-1,6-dihydropyridazin-3-yl]phenyl}nicotinate
5-{3-[5-({[(3,5-Dichloropyridin-4-yl)amino]carbonyl}amino)-1-ethyl-6-oxo-1,6-dihydropyridazin-3-yl]phenyl}nicotinic acid
Methyl 3'-[5-({[(3,5-dichloropyridin-4-yl)amino]carbonyl}amino)-1-ethyl-6-oxo-1,6-dihydropyridazin-3-yl]-4-fluorobiphenyl-3-carboxylate
3'-[5-({[(3,5-Dichloropyridin-4-yl)amino]carbonyl}amino)-1-ethyl-6-oxo-1,6-dihydropyridazin-3-yl]-4-fluorobiphenyl-3-carboxylic acid
Ethyl 3'-[5-({[(3,5-dichloropyridin-4-yl)amino]carbonyl}amino)-1-ethyl-6-oxo-1,6-dihydropyridazin-3-yl]biphenyl-2-carboxylate
3'-[5-({[(3,5-Dichloropyridin-4-yl)amino]carbonyl}amino)-1-ethyl-6-oxo-1,6-dihydropyridazin-3-yl]biphenyl-2-carboxylic acid
Methyl 3'-(5-(3-(3,5-dichloropyridin-4-yl)ureido)-1-ethyl-6-oxo-1,6-dihydropyridazin-3-yl)-6-fluorobiphenyl-3-carboxylate
3'-[5-({[(3,5-Dichloropyridin-4-yl)amino]carbonyl}amino)-1-ethyl-6-oxo-1,6-dihydropyridazin-3-yl]-6-fluorobiphenyl-3-carboxylic acid
Methyl 3'-(5-(3-(3,5-dichloropyridin-4-yl)ureido)-1-ethyl-6-oxo-1,6-dihydropyridazin-3-yl)-6-methoxybiphenyl-3-carboxylate
3'-[5-({[(3,5-Dichloropyridin-4-yl)amino]carbonyl}amino)-1-ethyl-6-oxo-1,6-dihydropyridazin-3-yl]-6-methoxybiphenyl-3-carboxylic acid
Methyl 3'-[5-({[(3,5-dichloropyridin-4-yl)amino]carbonyl}amino)-1-ethyl-6-oxo-1,6-dihydropyridazin-3-yl]-4-hydroxybiphenyl-3-carboxylate
3'-[5-({[(3,5-Dichloropyridin-4-yl)amino]carbonyl}amino)-1-ethyl-6-oxo-1,6-dihydropyridazin-3-yl]-4-hydroxybiphenyl-3-carboxylic acid
Ethyl {3'-[5-({[(3,5-dichloropyridin-4-yl)amino]carbonyl}amino)-1-ethyl-6-oxo-1,6-dihydropyridazin-3-yl]biphenyl-4-yl}acetate
{3'-[5-({[(3,5-Dichloropyridin-4-yl)amino]carbonyl}amino)-1-ethyl-6-oxo-1,6-dihydropyridazin-3-yl]biphenyl-4-yl}acetic acid
Methyl {3'-[5-({[(3,5-dichloropyridin-4-yl)amino]carbonyl}amino)-1-ethyl-6-oxo-1,6-dihydropyridazin-3-yl]biphenyl-3-yl}acetate
{3'-[5-({[(3,5-Dichloropyridin-4-yl)amino]carbonyl}amino)-1-ethyl-6-oxo-1,6-dihydropyridazin-3-yl]biphenyl-3-yl}acetic acid
Ethyl 4-{5-[5-({[(3,5-dichloropyridin-4-yl)amino]carbonyl}amino)-1-ethyl-6-oxo-1,6-dihydropyridazin-3-yl]pyridin-3-yl}benzoate
4-{5-[5-({[(3,5-Dichloropyridin-4-yl)amino]carbonyl}amino)-1-ethyl-6-oxo-1,6-dihydropyridazin-3-yl]pyridin-3-yl}benzoic acid
Methyl 3'-[5-({[(3,5-dichloropyridin-4-yl)amino]carbonyl}amino)-1-ethyl-6-oxo-1,6-dihydropyridazin-3-yl]-6-hydroxybiphenyl-3-carboxylate
3'-[5-({[(3,5-Dichloropyridin-4-yl)amino]carbonyl}amino)-1-ethyl-6-oxo-1,6-dihydropyridazin-3-yl]-6-hydroxybiphenyl-3-carboxylic acid
Ethyl 3'-[5-({[(3,5-dichloropyridin-4-yl)amino]carbonyl}amino)-1-ethyl-6-oxo-1,6-dihydropyridazin-3-yl]biphenyl-3-carboxylate
3'-[5-({[(3,5-Dichloropyridin-4-yl)amino]carbonyl}amino)-1-ethyl-6-oxo-1,6-dihydropyridazin-3-yl]biphenyl-3-carboxylic acid
3'-[5-({[(3,5-Dichloropyridin-4-yl)amino]carbonyl}amino)-1-ethyl-6-oxo-1,6-dihydropyridazin-3-yl]-N-(2-morpholin-4-ylethyl)biphenyl-3-carboxamide
3'-[5-({[(3,5-Dichloropyridin-4-yl)amino]carbonyl}amino)-1-ethyl-6-oxo-1,6-dihydropyridazin-3-yl]-N-[3-(dimethylamino)propyl]biphenyl-4-carboxamide
3'-[5-({[(3,5-Dichloropyridin-4-yl)amino]carbonyl}amino)-1-ethyl-6-oxo-1,6-dihydropyridazin-3-yl]-N-(2-piperidin-1-ylethyl)biphenyl-3-carboxamide
3'-[5-({[(3,5-Dichloropyridin-4-yl)amino]carbonyl}amino)-1-ethyl-6-oxo-1,6-dihydropyridazin-3-yl]-N-[2-(methylamino)ethyl]biphenyl-3-carboxamide
N-(3,5-Dichloropyridin-4-yl)-N'-{2-ethyl-6-[3-(5-methoxypyridin-3-yl)phenyl]-3-oxo-2,3-dihydropyridazin-4-yl}urea
N-(3,5-Dichloropyridin-4-yl)-N'-{2-ethyl-6-[3-(5-hydroxypyridin-3-yl)phenyl]-3-oxo-2,3-dihydropyridazin-4-yl}urea
N-(3,5-Dichloropyridin-4-yl)-N'-{2-ethyl-6-[3-(6-methoxypyridin-3-yl)phenyl]-3-oxo-2,3-dihydropyridazin-4-yl}urea
N-(3,5-Dichloropyridin-4-yl)-N'-{2-ethyl-6-[3-(6-hydroxypyridin-3-yl)phenyl]-3-oxo-2,3-dihydropyridazin-4-yl}urea
N-[6-(5'-Chloro-2'-methoxybiphenyl-3-yl)-2-ethyl-3-oxo-2,3-dihydropyridazin-4-yl]-N'-(3,5-dichloropyridin-4-yl)urea
N-[6-(5'-Chloro-2'-hydroxybiphenyl-3-yl)-2-ethyl-3-oxo-2,3-dihydropyridazin-4-yl]-N'-(3,5-dichloropyridin-4-yl)urea
N-(3,5-Dichloropyridin-4-yl)-N'-[2-ethyl-6-(2'-fluoro-5'-methoxybiphenyl-3-yl)-3-oxo-2,3-dihydropyridazin-4-yl]urea
N-(3,5-Dichloropyridin-4-yl)-N'-[2-ethyl-6-(2'-fluoro-5'-hydroxybiphenyl-3-yl)-3-oxo-2,3-dihydropyridazin-4-yl]urea
N-[6-(2'-Chloro-4'-methoxybiphenyl-3-yl)-2-ethyl-3-oxo-2,3-dihydropyridazin-4-yl]-N'-(3,5-dichloropyridin-4-yl)urea
N-[6-(2'-Chloro-4'-hydroxybiphenyl-3-yl)-2-ethyl-3-oxo-2,3-dihydropyridazin-4-yl]-N'-(3,5-dichloropyridin-4-yl)urea
N-[6-(3'-Chloro-4'-methoxybiphenyl-3-yl)-2-ethyl-3-oxo-2,3-dihydropyridazin-4-yl]-N'-(3,5-dichloropyridin-4-yl)urea
N-[6-(3'-Chloro-4'-hydroxybiphenyl-3-yl)-2-ethyl-3-oxo-2,3-dihydropyridazin-4-yl]-N'-(3,5-dichloropyridin-4-yl)urea
N-(3,5-Dichloropyridin-4-yl)-N'-{2-ethyl-3-oxo-6-[3'-(3-pyrrolidin-1-ylpropoxy)biphenyl-3-yl]-2,3-dihydropyridazin-4-yl}urea
N-(3,5-Dichloropyridin-4-yl)-N'-{2-ethyl-6-[4'-(2-hydroxyethyl)biphenyl-3-yl]-3-oxo-2,3-dihydropyridazin-4-yl}urea
N-(3,5-Dichloropyridin-4-yl)-N'-{2-ethyl-6-[3-(3-methoxyphenoxy)phenyl]-3-oxo-2,3-dihydropyridazin-4-yl}urea
N-(3,5-Dichloropyridin-4-yl)-N'-{2-ethyl-6-[3-(3-hydroxyphenoxy)phenyl]-3-oxo-2,3-dihydropyridazin-4-yl}urea
N-(3,5-Dichloropyridin-4-yl)-N'-{2-ethyl-6-[3-(2-methoxyphenoxy)phenyl]-3-oxo-2,3-dihydropyridazin-4-yl}urea
N-(3,5-Dichloropyridin-4-yl)-N'-{2-ethyl-6-[3-(2-hydroxyphenoxy)phenyl]-3-oxo-2,3-dihydropyridazin-4-yl}urea
N-(3,5-Dichloropyridin-4-yl)-N'-{2-ethyl-6-[3-(4-methoxyphenoxy)phenyl]-3-oxo-2,3-dihydropyridazin-4-yl}urea
N-(3,5-Dichloropyridin-4-yl)-N'-{2-ethyl-6-[3-(4-hydroxyphenoxy)phenyl]-3-oxo-2,3-dihydropyridazin-4-yl}urea
N-(3,5-Dichloropyridin-4-yl)-N'-(2-ethyl-6-{3-[(3-methoxyphenyl)thio]phenyl}-3-oxo-2,3-dihydropyridazin-4-yl)urea
N-(3,5-Dichloropyridin-4-yl)-N'-(2-ethyl-6-{3-[(3-hydroxyphenyl)thio]phenyl}-3-oxo-2,3-dihydropyridazin-4-yl)urea
N-(3,5-Dichloropyridin-4-yl)-N'-(2-ethyl-6-{3-[(2-methoxyphenyl)thio]phenyl}-3-oxo-2,3-dihydropyridazin-4-yl)urea
N-(3,5-Dichloropyridin-4-yl)-N'-(2-ethyl-6-{3-[(2-hydroxyphenyl)thio]phenyl}-3-oxo-2,3-dihydropyridazin-4-yl)urea
N-(3,5-Dichloropyridin-4-yl)-N'-(2-ethyl-6-{3-[(4-methoxyphenyl)thio]phenyl}-3-oxo-2,3-dihydropyridazin-4-yl)urea
N-(3,5-Dichloropyridin-4-yl)-N'-(2-ethyl-6-{3-[(4-hydroxyphenyl)thio]phenyl}-3-oxo-2,3-dihydropyridazin-4-yl)urea
N-(3,5-Dichloropyridin-4-yl)-N'-(2-ethyl-6-{3-[(3-methoxyphenyl)sulfonyl]phenyl}-3-oxo-2,3-dihydropyridazin-4-yl)urea
N-(3,5-Dichloropyridin-4-yl)-N'-(2-ethyl-6-{3-[(3-hydroxyphenyl)sulfonyl]phenyl}-3-oxo-2,3-dihydropyridazin-4-yl)urea
N-(3,5-Dichloropyridin-4-yl)-N'-(2-ethyl-6-{3-[(2-methoxyphenyl)sulfonyl]phenyl}-3-oxo-2,3-dihydropyridazin-4-yl)urea
N-(3,5-Dichloropyridin-4-yl)-N'-(2-ethyl-6-{3-[(2-hydroxyphenyl)sulfonyl]phenyl}-3-oxo-2,3-dihydropyridazin-4-yl)urea
N-(3,5-Dichloropyridin-4-yl)-N'-(2-ethyl-6-{3-[(4-methoxyphenyl)sulfonyl]phenyl}-3-oxo-2,3-dihydropyridazin-4-yl)urea
N-(3,5-Dichloropyridin-4-yl)-N'-(2-ethyl-6-{-[(4-hydroxyphenyl)sulfonyl]phenyl}-3-oxo-2,3-dihydropyridazin-4-yl)urea
N-{6-[3-(Benzyloxy)phenyl]-2-ethyl-3-oxo-2,3-dihydropyridazin-4-yl}-N'-(3,5-dichloropyridin-4-yl)urea
3'-[5-({[(3,5-Dichioropyridin-4-yl)amino]carbonyl}amino)-1-ethyl-6-oxo-1,6-dihydropyridazin-3-yl]biphenyl-3-yl hydrogen sulfate
N-(3,5-Dichloropyridin-4-yl)-N'-{2-ethyl-6-[3-(4-methoxypyridin-3-yl)phenyl]-3-oxo-2,3-dihydropyridazin-4-yl}urea
3'-[5-({[(3,5-Dichloropyridin-4-yl)amino]carbonyl}amino)-1-ethyl-6-oxo-1,6-dihydropyridazin-3-yl]-N-(2-hydroxyethyl)biphenyl-3-carboxamide
3'-[5-({[(3,5-Dichloropyridin-4-yl)amino]carbonyl}amino)-1-ethyl-6-oxo-1,6-dihydropyridazin-3-yl]-N-[2-(4-methylpiperazin-1-yl)ethyl]biphenyl-3-carboxamide
3'-[5-({[(3,5-Dichloropyridin-4-yl)amino]carbonyl}amino)-1-ethyl-6-oxo-1,6-dihydropyridazin-3-yl]-N-(pyridin-3-ylmethyl)biphenyl-3-carboxamide
N-Benzyl-3'-[5-({[(3,5-dichloropyridin-4-yl)amino]carbonyl}amino)-1-ethyl-6-oxo-1,6-dihydropyridazin-3-yl]biphenyl-3-carboxamide
3'-[5-({[(3,5-Dichloropyridin-4-yl)amino]carbonyl}amino)-1-ethyl-6-oxo-1,6-dihydropyridazin-3-yl]-N-(3-morpholin-4-ylpropyl)biphenyl-3-carboxamide
3'-[5-({[(3,5-Dichloropyridin-4-yl)amino]carbonyl}amino)-1-ethyl-6-oxo-1,6-dihydropyridazin-3-yl]-N-[3-(1H-imidazol-1-yl)propyl]biphenyl-3-carboxamide
N-1-Adamantyl-3'-[5-({[(3,5-dichloropyridin-4-yl)amino]carbonyl}amino)-1-ethyl-6-oxo-1,6-dihydropyridazin-3-yl]biphenyl-3-carboxamide
N-(3-Chlorobenzyl)-3'-[5-({[(3,5-dichioropyridin-4-yl)amino]carbonyl}amino)-1-ethyl-6-oxo-1,6-dihydropyridazin-3-yl]biphenyl-3-carboxamide
N-(1-Adamantylmethyl)-3'-[5-({[(3,5-dichloropyridin-4-yl)amino]carbonyl}amino)-1-ethyl-6-oxo-1,6-dihydropyridazin-3-yl]biphenyl-3-carboxamide
3'-[5-({[(3,5-Dichloropyridin-4-yl)amino]carbonyl}amino)-1-ethyl-6-oxo-1,6-dihydropyridazin-3-yl]-N-(2-pyrrolidin-1-ylethyl)biphenyl-3-carboxamide
3'-[5-({[(3,5-Dichloropyridin-4-yl)amino]carbonyl}amino)-1-ethyl-6-oxo-1,6-dihydropyridazin-3-yl]-N-(2-morpholin-4-ylethyl)biphenyl-4-carboxamide
N-(3,4-Dichlorobenzyl)-3'-[5-({[(3,5-dichloropyridin-4-yl)amino]carbonyl}amino)-1-ethyl-6-oxo-1,6-dihydropyridazin-3-yl]biphenyl-3-carboxamide
3'-[5-({[(3,5-Dichloropyridin-4-yl)amino]carbonyl}amino)-1-ethyl-6-oxo-1,6-dihydropyridazin-3-yl]-N-(2-phenylethyl)biphenyl-3-carboxamide
N-Cyclopentyl-3'-[5-({[(3,5-dichloropyridin-4-yl)amino]carbonyl}amino)-1-ethyl-6-oxo-1,6-dihydropyridazin-3-yl]biphenyl-3-carboxamide
3'-[5-({[(3,5-Dichloropyridin-4-yl)amino]carbonyl}amino)-1-ethyl-6-oxo-1,6-dihydropyridazin-3-yl]-N-(2-pyridin-3-ylethyl)biphenyl-3-carboxamide
3'-[5-({[(3,5-Dichloropyridin-4-yl)amino]carbonyl}amino)-1-ethyl-6-oxo-1,6-dihydropyridazin-3-yl]-N-[2-(1-methylpyrrolidin-2-yl)ethyl]biphenyl-3-carboxamide
N-(3,5-Dichloropyridin-4-yl)-N'-{2-ethyl-3-oxo-6-[3'-(pyrrolidin-1-ylcarbonyl)biphenyl-3-yl]-2,3-dihydropyridazin-4-yl}urea
3'-[5-({[(3,5-Dichloropyridin-4-yl)amino]carbonyl}amino)-1-ethyl-6-oxo-1,6-dihydropyridazin-3-yl]-N-(2-pyridin-2-ylethyl)biphenyl-3-carboxamide
N-(3,5-Dichloropyridin-4-yl)-N'-{2-ethyl-3-oxo-6-[3'-(piperidin-1-ylcarbonyl)biphenyl-3-yl]-2,3-dihydropyridazin-4-yl}urea
N-Cyclobutyl-3'-[5-({[(3,5-dichloropyridin-4-yl)amino]carbonyl}amino)-1-ethyl-6-oxo-1,6-dihydropyridazin-3-yl]biphenyl-3-carboxamide
3'-[5-({[(3,5-Dichloropyridin-4-yl)amino]carbonyl}amino)-1-ethyl-6-oxo-1,6-dihydropyridazin-3-yl]-N-[2-(dimethylamino)ethyl]biphenyl-4-carboxamide
Methyl 3'-[5-({[(3,5-dichloropyridin-4-yl)amino]carbonyl}amino)-1-ethyl-6-oxo-1,6-dihydropyridazin-3-yl]-3-hydroxybiphenyl-4-carboxylate
3'-[5-({[(3,5-Dichloropyridin-4-yl)amino]carbonyl}amino)-1-ethyl-6-oxo-1,6-dihydropyridazin-3-yl]-3-hydroxybiphenyl-4-carboxylic acid
Methyl 3'-[5-({[(3,5-dichloropyridin-4-yl)amino]carbonyl}amino)-1-ethyl-6-oxo-1,6-dihydropyridazin-3-yl]-5-hydroxybiphenyl-3-carboxylate
3'-[5-({[(3,5-Dichloropyridin-4-yl)amino]carbonyl}amino)-1-ethyl-6-oxo-1,6-dihydropyridazin-3-yl]-5-hydroxybiphenyl-3-carboxylic acid
N-Cyclopropyl-3'-[5-({[(3,5-dichloropyridin-4-yl)amino]carbonyl}amino)-1-ethyl-6-oxo-1,6-dihydropyridazin-3-yl]biphenyl-4-carboxamide
N-Cyclopropyl-3'-[5-({[(3,5-dichloro-1-oxidopyridin-4-yl)amino]carbonyl}amino)-1-ethyl-6-oxo-1,6-dihydropyridazin-3-yl]biphenyl-3-carboxamide
3'-[5-({[(3,5-Dichloropyridin-4-yl)amino]carbonyl}amino)-1-ethyl-6-oxo-1,6-dihydropyridazin-3-yl]-N-(2-piperidin-1-ylethyl)biphenyl-4-carboxamide
2-{3'-[5-({[(3,5-Dichloropyridin-4-yl)amino]carbonyl}amino)-1-ethyl-6-oxo-1,6-dihydropyridazin-3-yl]biphenyl-4-yl}-N-(2-morpholin-4-ylethyl)acetamide
2-{3'-[5-({[(3,5-Dichloropyridin-4-yl)amino]carbonyl}amino)-1-ethyl-6-oxo-1,6-dihydropyridazin-3-yl]biphenyl-4-yl}-N-(2-piperidin-1-ylethyl)acetamide
2-{3'-[5-({[(3,5-Dichloropyridin-4-yl)amino]carbonyl}amino)-1-ethyl-6-oxo-1,6-dihydropyridazin-3-yl]biphenyl-3-yl}-N-(2-piperidin-1-ylethyl)acetamide
2-{3'-[5-({[(3,5-Dichloropyridin-4-yl)amino]carbonyl}amino)-1-ethyl-6-oxo-1,6-dihydropyridazin-3-yl]biphenyl-3-yl}-N-(2-morpholin-4-ylethyl)acetamide
3'-[5-({[(3,5-Dichloropyridin-4-yl)amino]carbonyl}amino)-1-ethyl-6-oxo-1,6-dihydropyridazin-3-yl]-N-{7-[(2-hydroxyethyl)(methyl)amino]heptyl}biphenyl-3-carboxamide
3'-[5-({[(3,5-Dichloropyridin-4-yl)amino]carbonyl}amino)-1-ethyl-6-oxo-1,6-dihydropyridazin-3-yl]-N-{7-[(2-hydroxyethyl)(methyl)amino]heptyl}biphenyl-4-carboxamide
2-{3'-[5-({[(3,5-Dichloropyridin-4-yl)amino]carbonyl}amino)-1-ethyl-6-oxo-1,6-dihydropyridazin-3-yl]biphenyl-4-yl}-N-{7-[(2-hydroxyethyl)(methyl)amino] heptyl}acetamide
2-{3'-[5-({[(3,5-Dichloropyridin-4-yl)amino]carbonyl}amino)-1-ethyl-6-oxo-1,6-dihydropyridazin-3-yl]biphenyl-3-yl}-N-{7-[(2-hydroxyethyl)(methyl)amino] heptyl}acetamide
3'-[5-({[(3,5-Dichloropyridin-4-yl)amino]carbonyl}amino)-1-ethyl-6-oxo-1,6-dihydropyridazin-3-yl]-5-hydroxy-N-{7-[(2-hydroxyethyl)(methyl)amino]heptyl}biphenyl-3-carboxamide
N-Cyclopropyl-3'-[5-({[(3,5-dichloropyridin-4-yl)amino]carbonyl}amino)-1-ethyl-6-oxo-1,6-dihydropyridazin-3-yl]-5-hydroxybiphenyl-3-carboxamide
N-Cyclopropyl-2-{3'-[5-({[(3,5-dichloropyridin-4-yl)amino]carbonyl}amino)-1-ethyl-6-oxo-1,6-dihydropyridazin-3-yl]biphenyl-3-yl}acetamide
N-Cyclopropyl-2-{3'-[5-({[(3,5-dichloropyridin-4-yl)amino]carbonyl}amino)-1-ethyl-6-oxo-1,6-dihydropyridazin-3-yl]biphenyl-4-yl}acetamide
N-Cyclopropyl-3'-[5-({[(3,5-dichloropyridin-4-yl)amino]carbonyl}amino)-1-ethyl-6-oxo-1,6-dihydropyridazin-3-yl]-5-fluoro-6-methylbiphenyl-3-carboxamide
N-(Cyclopropylmethyl)-3'-[5-({[(3,5-dichioropyridin-4-yl)amino]carbonyl}amino)-1-ethyl-6-oxo-1,6-dihydropyridazin-3-yl]biphenyl-3-carboxamide
3'-[5-({[(3,5-Dichloropyridin-4-yl)amino]carbonyl}amino)-1-ethyl-6-oxo-1,6-dihydropyridazin-3-yl]-N-propylbiphenyl-3-carboxamide
3'-[5-({[(3,5-Dichloropyridin-4-yl)amino]carbonyl}amino)-1-ethyl-6-oxo-1,6-dihydropyridazin-3-yl]-N-ethylbiphenyl-3-carboxamide
N-Butyl-3'-[5-({[(3,5-dichloropyridin-4-yl)amino]carbonyl}amino)-1-ethyl-6-oxo-1,6-dihydropyridazin-3-yl]biphenyl-3-carboxamide
3'-[5-({[(3,5-Dichloropyridin-4-yl)amino]carbonyl}amino)-1-ethyl-6-oxo-1,6-dihydropyridazin-3-yl]-N-(3-hydroxybenzyl)biphenyl-3-carboxamide
3'-[5-({[(3,5-dichloropyridin-4-yl)amino]carbonyl}amino)-1-ethyl-6-oxo-1,6-dihydropyridazin-3-yl]-N-pyridin-3-ylbiphenyl-3-carboxamide
N-(3,5-Dichloropyridin-4-yl)-N'-(2-ethyl-6-{3'-[(4-methylpiperazin-1-yl)carbonyl]biphenyl-3-yl}-3-oxo-2,3-dihydropyridazin-4-yl)urea
N-Cyclopropyl-5-{3-[5-({[(3,5-dichloropyridin-4-yl)amino]carbonyl}amino)-1-ethyl-6-oxo-1,6-dihydropyridazin-3-yl]phenyl}nicotinamide
3'-[5-({[(3,5-Dichloropyridin-4-yl)amino]carbonyl}amino)-1-ethyl-6-oxo-1,6-dihydropyridazin-3-yl]-N-(4-hydroxybenzyl)biphenyl-3-carboxamide
N-(3,5-Dichloropyridin-4-yl)-N'-[2-ethyl-6-(3'-methoxybiphenyl-3-yl)-3-oxo-2,3-dihydropyridazin-4-yl]urea
3'-[5-({[(3,5-Dichloropyridin-4-yl)amino]carbonyl}amino)-1-ethyl-6-oxo-1,6-dihydropyridazin-3-yl]biphenyl-4-carboxamide
3'-[5-({[(3,5-Dichloropyridin-4-yl)amino]carbonyl}amino)-1-ethyl-6-oxo-1,6-dihydropyridazin-3-yl]-2-methylbiphenyl-4-carboxamide
N-(3,5-Dichloropyridin-4-yl)-N'-{2-ethyl-6-[2'-(methylsulfonyl)biphenyl-3-yl]-3-oxo-2,3-dihydropyridazin-4-yl}urea
N-(3,5-Dichloropyridin-4-yl)-N'-{2-ethyl-6-[3'-(methylsulfonyl)biphenyl-3-yl]-3-oxo-2,3-dihydropyridazin-4-yl}urea
N-(3,5-Dichloropyridin-4-yl)-N'-{2-ethyl-6-[4'-(methylsulfonyl)biphenyl-3-yl]-3-oxo-2,3-dihydropyridazin-4-yl}urea
N-(3, 5-Di m ethyl pyrid in-4-yl)-N'-[2-ethyl-6-(3'-hyd roxybiphenyl-3-yl)-3-oxo-2,3-dihydropyridazin-4-yl]urea
Methyl 5-[(cyclopropylamino)carbonyl]-3'-[5-({[(3,5-dichloropyridin-4-yl)amino]carbonyl}amino)-1-ethyl-6-oxo-1,6-dihydropyridazin-3-yl]biphenyl-3-carboxylate
5-[(Cyclopropylamino)carbonyl]-3'-[5-({[(3,5-dichloropyridin-4-yl)amino]carbonyl}amino)-1-ethyl-6-oxo-1,6-dihydropyridazin-3-yl]biphenyl-3-carboxylic acid
3-{3'-[5-({[(3,5-Dichloropyridin-4-yl)amino]carbonyl}amino)-1-ethyl-6-oxo-1,6-dihydropyridazin-3-yl]biphenyl-4-yl}propanoicacid
N-Cyclopropyl-3-{3'-[5-({[(3,5-dichloropyridin-4-yl)amino]carbonyl}amino)-1-ethyl-6-oxo-1,6-dihydropyridazin-3-yl]biphenyl-4-yl}propanamide
2-{3'-[5-({[(3,5-Dichloropyridin-4-yl)amino]carbonyl}amino)-1-ethyl-6-oxo-1,6-dihydropyridazin-3-yl]biphenyl-3-yl}-N-(3-morpholin-4-ylpropyl)acetamide
2-{3'-[5-({[(3,5-Dichloropyridin-4-yl)amino]carbonyl}amino)-1-ethyl-6-oxo-1,6-dihydropyridazin-3-yl]biphenyl-4-yl}-N-(3-morpholin-4-ylpropyl)acetamide
2-{3'-[5-({[(3,5-Dichloropyridin-4-yl)amino]carbonyl}amino)-1-ethyl-6-oxo-1,6-dihydropyridazin-3-yl]biphenyl-3-yl}-N-(3-hydroxybenzyl)acetamide
N,N'-Dicyclopropyl-3'-[5-({[(3,5-dichloropyridin-4-yl)amino]carbonyl}amino)-1-ethyl-6-oxo-1,6-dihydropyridazin-3-yl]biphenyl-3,5-dicarboxamide
N-Cyclopropyl-3'-[5-({[(3,5-dichloropyridin-4-yl)amino]carbonyl}amino)-1-ethyl-6-oxo-1,6-dihydropyridazin-3-yl]-N'-{2-[(2R)-1-methylpyrrolidin-2-yl]ethyl}biphenyl-3,5-dicarboxamide
N-Cyclopropyl-3'-[5-({[(3,5-dichloropyridin-4-yl)amino]carbonyl}amino)-1-ethyl-6-oxo-1,6-dihydropyridazin-3-yl]-N'-(2-piperidin-1-ylethyl)biphenyl-3,5-dicarboxamide
N-Cyclopropyl-3'-[5-({[(3,5-dichloropyridin-4-yl)amino]carbonyl}amino)-1-ethyl-6-oxo-1,6-dihydropyridazin-3-yl]-N'-{7-[(2-hydroxyethyl)(methyl)amino]heptyl}biphenyl-3,5-dicarboxamide
Methyl 3'-[5-({[(3,5-dichloropyridin-4-yl)amino]carbonyl}amino)-1-ethyl-6-oxo-1,6-dihydropyridazin-3-yl]-5-({[3-(dimethylamino)propyl]amino}carbonyl)biphenyl-3-carboxylate
Ethyl 3'-[5-({[(3,5-dichloropyridin-4-yl)amino]carbonyl}amino)-1-ethyl-6-oxo-1,6-dihydropyridazin-3-yl]-5-(2-hydroxyethoxy)biphenyl-3-carboxylate
3'-[5-({[(3,5-Dichloropyridin-4-yl)amino]carbonyl}amino)-1-ethyl-6-oxo-1,6-dihydropyridazin-3-yl]-5-(2-hydroxyethoxy)biphenyl-3-carboxylic acid
N-Cyclopropyl-3'-[5-({[(3,5-dichloropyridin-4-yl)amino]carbonyl}amino)-1-ethyl-6-oxo-1,6-dihydropyridazin-3-yl]-5-(2-hydroxyethoxy)biphenyl-3-carboxamide
3'-[5-({[(3,5-Dichloropyridin-4-yl)amino]carbonyl}amino)-1-ethyl-6-oxo-1,6-dihydropyridazin-3-yl]-N-(1-methylpiperidin-4-yl)biphenyl-3-carboxamide
2-{3'-[5-({[(3,5-Dichloropyridin-4-yl)amino]carbonyl}amino)-1-ethyl-6-oxo-1,6-dihydropyridazin-3-yl]biphenyl-4-yl}-N-(1-methylpiperidin-4-yl)acetamide
2-{3'-[5-({[(3,5-Dichloropyridin-4-yl)amino]carbonyl}amino)-1-ethyl-6-oxo-1,6-dihydropyridazin-3-yl]biphenyl-4-yl}-N-{2-[1-(dimethylamino)cyclopentyl]ethyl} acetamide
3'-[5-({[(3,5-Dichloropyridin-4-yl)amino]carbonyl}amino)-1-ethyl-6-oxo-1,6-dihydropyridazin-3-yl]-N-{2-[(2S)-2-(methoxymethyl)pyrrolidin-1-yl]ethyl}-N'-(2-piperidin-1-ylethyl)biphenyl-3,5-dicarboxamide
N-Cyclopentyl-3'-[5-({[(3,5-dichloropyridin-4-yl)amino]carbonyl}amino)-1-ethyl-6-oxo-1,6-dihydropyridazin-3-yl]-N'-(2-piperidin-1-ylethyl)biphenyl-3,5-dicarboxamide
3'-[5-({[(3,5-Dichloropyridin-4-yl)amino]carbonyl}amino)-1-ethyl-6-oxo-1,6-dihydropyridazin-3-yl]-N-{4-[(2S)-2-(methoxymethyl)pyrrolidin-1-yl]butyl}-N'-(2-piperidin-1-ylethyl)biphenyl-3,5-dicarboxamide
2-{3'-[5-({[(3,5-Dichloropyridin-4-yl)amino]carbonyl}amino)-1-ethyl-6-oxo-1,6-dihydropyridazin-3-yl]biphenyl-3-yl}-N-(2-pyrrolidin-1-ylethyl)acetamide
2-{3'-[5-({[(3,5-Dichloropyridin-4-yl)amino]carbonyl}amino)-1-ethyl-6-oxo-1,6-dihydropyridazin-3-yl]biphenyl-4-yl}-N-(2-pyrrolidin-1-ylethyl)acetamide
3'-[5-({[(3,5-Dichloropyridin-4-yl)amino]carbonyl}amino)-1-ethyl-6-oxo-1,6-dihydropyridazin-3-yl]-N-(3-hydroxybenzyl)biphenyl-4-carboxamide
2-{3'-[5-({[(3,5-Dichloropyridin-4-yl)amino]carbonyl}amino)-1-ethyl-6-oxo-1,6-dihydropyridazin-3-yl]biphenyl-4-yl}-N-(3-hydroxybenzyl)acetamide
N-Cyclopropyl-N'-[3-(dimethylamino)propyl]-3'-[5-({[(3,5-dimethylpyridin-4-yl)amino]carbonyl}amino)-1-ethyl-6-oxo-1,6-dihydropyridazin-3-yl]biphenyl-3,5-dicarboxamide
3'-[5-({[(3,5-Dichloropyridin-4-yl)amino]carbonyl}amino)-1-ethyl-6-oxo-1,6-dihydropyridazin-3-yl]-N-{5-[(2-hydroxyethyl)(methyl)amino]pentyl}biphenyl-3-carboxamide
2-{3'-[5-({[(3,5-Dichloropyridin-4-yl)amino]carbonyl}amino)-1-ethyl-6-oxo-1,6-dihydropyridazin-3-yl]biphenyl-4-yl}-N-{5-[(2-hydroxyethyl)(methyl)amino]pentyl} acetamide
Ethyl 3'-[5-({[(3,5-dichloropyridin-4-yl)amino]carbonyl}amino)-1-ethyl-6-oxo-1,6-dihydropyridazin-3-yl]-5-(trifluoromethyl)biphenyl-3-carboxylate
3'-[5-({[(3,5-Dichloropyridin-4-yl)amino]carbonyl}amino)-1-ethyl-6-oxo-1,6-dihydropyridazin-3-yl]-5-(trifluoromethyl)biphenyl-3-carboxylic acid
N-Cyclopropyl-3'-[5-({[(3,5-dichloropyridin-4-yl)amino]carbonyl}amino)-1-ethyl-6-oxo-1,6-dihydropyridazin-3-yl]-5-(trifluoromethyl)biphenyl-3-carboxamide
3'-[5-({[(3,5-dichloropyridin-4-yl)amino]carbonyl}amino)-1-ethyl-6-oxo-1,6-dihydropyridazin-3-yl]-N-(2-piperidin-1-ylethyl)-5-(trifluoromethyl)biphenyl-3-carboxamide
3'-[5-({[(3,5-dichloropyridin-4-yl)amino]carbonyl}amino)-1-ethyl-6-oxo-1,6-dihydropyridazin-3-yl]-N-[3-(dimethylamino)propyl]-5-(trifluoromethyl)biphenyl-3-carboxamide
3'-[5-({[(3,5-dichloropyridin-4-yl)amino]carbonyl}amino)-1-ethyl-6-oxo-1,6-dihydropyridazin-3-yl]-N-{7-[(2-hydroxyethyl)(methyl)amino]heptyl}-5-(trifluoromethyl)biphenyl-3-carboxamide
ethyl 3'-[5-({[(3,5-dichloropyridin-4-yl)amino]carbonyl}amino)-1-ethyl-6-oxo-1,6-dihydropyridazin-3-yl]-5-(trifluoromethoxy)biphenyl-3-carboxylate
3'-[5-({[(3,5-dichloropyridin-4-yl)amino]carbonyl}amino)-1-ethyl-6-oxo-1,6-dihydropyridazin-3-yl]-5-(trifluoromethoxy)biphenyl-3-carboxylic acid
N-cyclopropyl-3'-[5-({[(3,5-dichloropyridin-4-yl)amino]carbonyl}amino)-1-ethyl-6-oxo-1,6-dihydropyridazin-3-yl]-5-(trifluoromethoxy)biphenyl-3-carboxamide
3'-[5-({[(3,5-dichloropyridin-4-yl)amino]carbonyl}amino)-1-ethyl-6-oxo-1,6-dihydropyridazin-3-yl]-N-(2-piperidin-1-ylethyl)-5-(trifluoromethoxy)biphenyl-3-carboxamide
3'-[5-({[(3,5-dichloropyridin-4-yl)amino]carbonyl}amino)-1-ethyl-6-oxo-1,6-dihydropyridazin-3-yl]-N-[3-(dimethylamino)propyl]-5-(trifluoromethoxy)biphenyl-3-carboxamide
3'-[5-({[(3,5-dichloropyridin-4-yl)amino]carbonyl}amino)-1-ethyl-6-oxo-1,6-dihydropyridazin-3-yl]-N-(2-pyrrolidin-1-ylethyl)-5-(trifluoromethoxy)biphenyl-3-carboxamide
ethyl 3-{3'-[5-({[(3,5-dichloropyridin-4-yl)amino]carbonyl}amino)-l-ethyl-6-oxo-1,6-dihydropyridazin-3-yl]biphenyl-3-yllpropanoate
3-{3'-[5-({[(3,5-dichloropyridin-4-yl)amino]carbonyl}amino)-1-ethyl-6-oxo-1,6-dihydropyridazin-3-yl]biphenyl-3-yl}propanoic acid
3-{3'-[5-({[(3,5-dichloropyridin-4-yl)amino]carbonyl}amino)-1-ethyl-6-oxo-1,6-dihydropyridazin-3-yl]biphenyl-3-yl}-N-{7-[(2-hydroxyethyl)(methyl)amino]heptyl}propanamide
3-{3'-[5-({[(3,5-dichloropyridin-4-yl)amino]carbonyl}amino)-1-ethyl-6-oxo-1,6-dihydropyridazin-3-yl]biphenyl-3-yl}-N-(4-hydroxybenzyl)propanamide
3-{3'-[5-({[(3,5-dichloropyridin-4-yl)amino]carbonyl}amino)-1-ethyl-6-oxo-1,6-dihydropyridazin-3-yl]biphenyl-3-yl}-N-(3-morpholin-4-ylpropyl)propanamide
3-{3'-[5-({[(3,5-dichloropyridin-4-yl)amino]carbonyl}amino)-1-ethyl-6-oxo-1,6-dihydropyridazin-3-yl]biphenyl-4-yl}-N-(3-morpholin-4-ylpropyl)propanamide
3-{3'-[5-({[(3,5-dichloropyridin-4-yl)amino]carbonyl}amino)-1-ethyl-6-oxo-1,6-dihydropyridazin-3-yl]biphenyl-4-yl}-N-{7-[(2-hydroxyethyl)(methyl)amino]heptyl}propanamide
3-{3'-[5-({[(3,5-dichloropyridin-4-yl)amino]carbonyl}amino)-1-ethyl-6-oxo-1,6-dihydropyridazin-3-yl]biphenyl-4-yl}-N-{5-[(2-hydroxyethyl)(methyl)amino]pentyl}propanamide
ethyl 5-(cyclopropylmethoxy)-3'-[5-({[(3,5-dichloropyridin-4-yl)amino]carbonyl}amino)-1-ethyl-6-oxo-1,6-dihydropyridazin-3-yl]biphenyl-3-carboxylate
5-(cyclopropylmethoxy)-3'-[5-({[(3,5-dichloropyridin-4-yl)amino]carbonyl}amino)-1-ethyl-6-oxo-1,6-dihydropyridazin-3-yl]biphenyl-3-carboxylic acid
5-(cyclopropylmethoxy)-3'-[5-({[(3,5-dichloropyridin-4-yl)amino]carbonyl}amino)-1-ethyl-6-oxo-1,6-dihydropyridazin-3-yl]-N-{7-[(2R)-2-(methoxymethyl)pyrrolidin-1-yl]heptyl}biphenyl-3-carboxamide
5-(cyclopropylmethoxy)-3'-[5-({[(3,5-dichloropyridin-4-yl)amino]carbonyl}amino)-1-ethyl-6-oxo-1,6-dihydropyridazin-3-yl]-N-(2-piperidin-1-ylethyl)biphenyl-3-carboxamide
5-(cyclopropylmethoxy)-3'-[5-({[(3,5-dichloropyridin-4-yl)amino]carbonyl}amino)-1-ethyl-6-oxo-1,6-dihydropyridazin-3-yl]-N-{7-[(2-hydroxyethyl)(methyl)amino]heptyl}biphenyl-3-carboxamide
5-(cyclopropylmethoxy)-3'-[5-({[(3,5-dichloropyridin-4-yl)amino]carbonyl}amino)-1-ethyl-6-oxo-1,6-dihydropyridazin-3-yl]-N-(1-methylpiperidin-4-yl)biphenyl-3-carboxamide
methyl 3'-[5-({[(3,5-dichloropyridin-4-yl)amino]carbonyl}amino)-1-ethyl-6-oxo-1,6-dihydropyridazin-3-yl]-5-{[(2-piperidin-1-ylethyl)amino]carbonyl}biphenyl-3-carboxylate
3'-[5-({[(3,5-dichloropyridin-4-yl)amino]carbonyl}amino)-1-ethyl-6-oxo-1,6-dihydropyridazin-3-yl]-5-{[(2-piperidin-1-ylethyl)amino]carbonyl}biphenyl-3-carboxylic acid
3'-[5-({[(3,5-dichloropyridin-4-yl)amino]carbonyl}amino)-1-ethyl-6-oxo-1,6-dihydropyridazin-3-yl]-N-ethyl-N'-(2-piperidin-1-ylethyl)biphenyl-3,5-dicarboxamide
3'-[5-({[(3,5-dichloropyridin-4-yl)amino]carbonyl}amino)-1-ethyl-6-oxo-1,6-dihydropyridazin-3-yl]-N-(2-piperidin-1-ylethyl)-N'-(2-pyrrolidin-1-ylethyl)biphenyl-3,5-dicarboxamide
3'-[5-({[(3,5-dichloropyridin-4-yl)amino]carbonyl}amino)-1-ethyl-6-oxo-1,6-dihydropyridazin-3-yl]-N-(2-piperidin-1-ylethyl)biphenyl-3,5-dicarboxamide
3'-[5-({[(3,5-dichloropyridin-4-yl)amino]carbonyl}amino)-1-ethyl-6-oxo-1,6-dihydropyridazin-3-yl]-N-(2-hydroxyethyl)-N'-(2-piperidin-1-ylethyl)biphenyl-3,5-dicarboxamide
3'-[5-({[(3,5-dichloropyridin-4-yl)amino]carbonyl}amino)-1-ethyl-6-oxo-1,6-dihydropyridazin-3-yl]-N,N-dimethyl-N'-(2-piperidin-1-ylethyl)biphenyl-3,5-dicarboxamide
ethyl 3'-[5-({[(3,5-dichloropyridin-4-yl)amino]carbonyl}amino)-1-ethyl-6-oxo-1,6-dihydropyridazin-3-yl]-5-ethoxybiphenyl-3-carboxylate
3'-[5-({[(3,5-dichloropyridin-4-yl)amino]carbonyl}amino)-1-ethyl-6-oxo-1,6-dihydropyridazin-3-yl]-5-ethoxybiphenyl-3-carboxylic acid
N-cyclopropyl-3'-[5-({[(3,5-dichloropyridin-4-yl)amin0]carbonyl}amino)-1-ethyl-6-oxo-1,6-dihydropyridazin-3-yl]-5-ethoxybiphenyl-3-carboxamide
3'-[5-({[(3,5-dichloropyridin-4-yl)amino]carbonyl}amino)-1-ethyl-6-oxo-1,6-dihydropyridazin-3-yl]-5-ethoxy-N-{7-[(2-hydroxyethyl)(methyl)amino]heptyl}biphenyl-3-carboxamide
3'-[5-({[(3,5-dichloropyridin-4-yl)amino]carbonyl}amino)-1-ethyl-6-oxo-1,6-dihydropyridazin-3-yl]-5-ethoxy-N-{7-[(2R)-2-(methoxymethyl)pyrrolidin-1-yl]heptyl}biphenyl-3-carboxamide
3'-[5-({[(3,5-dichloropyridin-4-yl)amino]carbonyl}amino)-1-ethyl-6-oxo-1,6-dihydropyridazin-3-yl]-5-(2-hydroxyethoxy)-N-{5-[(2-hydroxyethyl)(methyl)amino]pentyl}biphenyl-3-carboxamide
3'-[5-({[(3,5-dichloropyridin-4-yl)amino]carbonyl}amino)-1-ethyl-6-oxo-1,6-dihydropyridazin-3-yl]-5-(2-hydroxyethoxy)-N-(2-piperidin-1-ylethyl)biphenyl-3-carboxamide
3'-[5-({[(3,5-dichloropyridin-4-yl)amino]carbonyl}amino)-1-ethyl-6-oxo-1,6-dihydropyridazin-3-yl]-N-[3-(dimethylamino)propyl]-5-(2-hydroxyethoxy)biphenyl-3-carboxamide
3'-[5-({[(3,5-dichloropyridin-4-yl)amino]carbonyl}amino)-1-ethyl-6-oxo-1,6-dihydropyridazin-3-yl]-5-(2-hydroxyethoxy)-N-{7-[(2-hydroxyethyl)(methyl)amino]heptyl}biphenyl-3-carboxamide
3'-[5-({[(3,5-dichloropyridin-4-yl)amino]carbonyl}amino)-1-ethyl-6-oxo-1,6-dihydropyridazin-3-yl]-5-(2-hydroxyethoxy)-N-(1-methylpiperidin-4-yl)biphenyl-3-carboxamide
3'-[5-({[(3,5-dichloropyridin-4-yl)amino]carbonyl}amino)-1-ethyl-6-oxo-1,6-dihydropyridazin-3-yl]-N-{2-[(2-hydroxyethyl)(methyl)amino]ethyl}biphenyl-3-carboxamide
1-({3'-[5-({[(3,5-dichloropyridin-4-yl)amino]carbonyl}amino)-l-ethyl-6-oxo-1,6-dihydropyridazin-3-yl]biphenyl-3-yl}carbonyl)piperidine-4-carboxamide
N-({3'-[5-({[(3,5-dichloropyridin-4-yl)amino]carbonyl}amino)-l-ethyl-6-oxo-1,6-dihydropyridazin-3-yl]biphenyl-3-yl}carbonyl)glycine
(1 R,2S)-2-[({3'-[5-({[(3,5-dichloropyridin-4-yl)amino]carbonyl}amino)-1-ethyl-6-oxo-1,6-dihydropyridazin-3-yl]biphenyl-3-yl}carbonyl)amino]cyclopentanecarboxylicacid
3'-[5-({[(3,5-dichloropyridin-4-yl)amino]carbonyl}amino)-1-ethyl-6-oxo-1,6-dihydropyridazin-3-yl]-N-[3-(diethylamino)propyl]biphenyl-3-carboxamide
3'-[5-({[(3,5-dichloropyridin-4-yl)amino]carbonyl}amino)-1-ethyl-6-oxo-1,6-dihydropyridazin-3-yl]-N-{3-[(2-hydroxyethyl)(methyl)amino]propyl}-N'-(2-piperidin-1-ylethyl)biphenyl-3,5-dicarboxamide
3'-[5-({[(3,5-dichloropyridin-4-yl)amino]carbonyl}amino)-1-ethyl-6-oxo-1,6-dihydropyridazin-3-yl]-N-[3-(dimethylamino)propyl]-5-ethoxybiphenyl-3-carboxamide
3'-[5-({[(3,5-dichloropyridin-4-yl)amino]carbonyl}amino)-1-ethyl-6-oxo-1,6-dihydropyridazin-3-yl]-5-ethoxy-N-(2-piperidin-1-ylethyl)biphenyl-3-carboxamide
3'-[5-({[(3,5-dichloropyridin-4-yl)amino]carbonyl}amino)-1-ethyl-6-oxo-1,6-dihydropyridazin-3-yl]-5-ethoxy-N-(1-methylpiperidin-4-yl)biphenyl-3-carboxamide
ethyl 3'-[5-({[(3,5-dichloropyridin-4-yl)amino]carbonyl}amino)-1-ethyl-6-oxo-1,6-dihydropyridazin-3-yl]-5-{2-[(2-hydroxyethyl)(methyl)amino]ethoxy}biphenyl-3-carboxylate
ethyl 3'-[5-({[(3,5-dichloropyridin-4-yl)amino]carbonyl}amino)-1-ethyl-6-oxo-1,6-dihydropyridazin-3-yl]-5-[2-(dimethylamino)ethoxy]biphenyl-3-carboxylate
methyl 3'-[5-({[(3,5-dichloropyridin-4-yl)amino]carbonyl}amino)-1-ethyl-6-oxo-1,6-dihydropyridazin-3-yl]-5-(piperidin-1-ylcarbonyl)biphenyl-3-carboxylate
N-cyclopropyl-3'-[5-({[(3,5-dichloropyridin-4-yl)amino]carbonyl}amino)-1-ethyl-6-oxo-1,6-dihydropyridazin-3-yl]-6-methylbiphenyl-3-carboxamide
N-{6-[3'-(cyclopropyloxy)biphenyl-3-yl]-2-ethyl-3-oxo-2,3-dihydropyridazin-4-yl}-N'-(3,5-dichloropyridin-4-yl)urea
2-{3'-[5-({[(3,5-dichloropyridin-4-yl)amino]carbonyl}amino)-1-ethyl-6-oxo-1,6-dihydropyridazin-3-yl]biphenyl-4-yl}-N-[2-(4-methylpiperazin-1-yl)ethyl]acetamide
3'-[5-({[(3,5-dichloropyridin-4-yl)amino]carbonyl}amino)-1-ethyl-6-oxo-1,6-dihydropyridazin-3-yl]-5-hydroxy-N-{2-[(2R)-1-methylpyrrolidin-2-yl]ethyl}biphenyl-3-carboxamide
dimethyl 3'-[5-({[(3,5-dichloropyridin-4-yl)amino]carbonyl}amino)-1-ethyl-6-oxo-1,6-dihydropyridazin-3-yl]biphenyl-3,5-dicarboxylate
3'-[5-({[(3,5-dichloropyridin-4-yl)amino]carbonyl}amino)-1-ethyl-6-oxo-1,6-dihydropyridazin-3-yl]biphenyl-3,5-dicarboxylic acid
3'-[5-({[(3,5-dichloropyridin-4-yl)amino]carbonyl}amino)-1-ethyl-6-oxo-1,6-dihydropyridazin-3-yl]-N,N'-bis(2-piperidin-1-ylethyl)biphenyl-3,5-dicarboxamide
ethyl 3'-[5-({[(3,5-dichloropyridin-4-yl)amino]carbonyl}amino)-1-ethyl-6-oxo-1,6-dihydropyridazin-3-yl]-5-{[5-(dimethylamino)pentyl]oxy}biphenyl-3-carboxylate
3'-[5-({[(3,5-dichloropyridin-4-yl)amino]carbonyl}amino)-1-ethyl-6-oxo-1,6-dihydropyridazin-3-yl]-5-{[5-(dimethylamino)pentyl]oxy}biphenyl-3-carboxylicacid
N-cyclopropyl-3'-[5-({[(3,5-dichloropyridin-4-yl)amin0]carbonyl}amino)-1-ethyl-6-oxo-1,6-dihydropyridazin-3-yl]-5-{[5-(dimethylamino)pentyl]oxy}biphenyl-3-carboxamide
3'-[5-({[(3,5-dichloropyridin-4-yl)amino]carbonyl}amino)-1-ethyl-6-oxo-1,6-dihydropyridazin-3-yl]-N-[2-(dimethylamino)ethyl]-N'-(2-piperidin-1-ylethyl)biphenyl-3,5-dicarboxamide
N-(3,5-dichloropyridin-4-yl)-N'-[2-ethyl-6-(3'-formyl-5'-propoxybiphenyl-3-yl)-3-oxo-2,3-dihydropyridazin-4-yl]urea
N-(6-{3'-[(cyclopropylamino)methyl]-5'-propoxybiphenyl-3-yl}-2-ethyl-3-oxo-2,3-dihydropyridazin-4-yl)-N'-(3,5-dichloropyridin-4-yl)urea
N-(3,5-dichloropyridin-4-yl)-N'-(6-{3'-[(dimethylamino)methyl]-5'-propoxybiphenyl-3-yl}-2-ethyl-3-oxo-2,3-dihydropyridazin-4-yl)urea
N-{6-[3',5'-bis(piperidin-1-ylcarbonyl)biphenyl-3-yl]-2-ethyl-3-oxo-2,3-dihydropyridazin-4-yl}-N'-(3,5-dichloropyridin-4-yl)urea
N-(6-{3',5'-bis[(4-methylpiperazin-1-yl)carbonyl]biphenyl-3-yl}-2-ethyl-3-oxo-2,3-dihydropyridazin-4-yl)-N'-(3,5-dichloropyridin-4-yl)urea
3'-[5-({[(3,5-dichloropyridin-4-yl)amino]carbonyl}amino)-1ethyl-6-oxo-1,6-dihydropyridazin-3-yl]-N,N'-bis(2-pyrrolidin-1-ylethyl)biphenyl-3,5-dicarboxamide
3'-[5-({(3,5-dichloropyridin-4-yl)amino]carbonyl}amino)-1-ethyl-6-oxo-1,6-dihydropyridazin-3-yl]-N,N'-bis(2-morpholin-4-ylethyl)biphenyl-3,5-dicarboxamide
3'-[5-({[(3,5-dichloropyridin-4-yl)amino]carbonyl}amino)-1-ethyl-6-oxo-1,6-dihydropyridazin-3-yl]-N,N'-bis[2-(4-methylpiperazin-1-yl)ethyl]biphenyl-3,5-dicarboxamide
3'-[5-({[(3,5-dichloropyridin-4-yl)amino]carbonyl}amino)-1-ethyl-6-oxo-1,6-dihydropyridazin-3-yl]-N,N'-bis(1-methylpiperidin-4-yl)biphenyl-3,5-dicarboxamide
3'-[5-({[(3,5-dichoropyridin-4-yl)amino]carbonyl}amino)-1-ethyl-6-oxo-1,6-d ihyd ropyridazin-3-yl]-N, N, N', N'-tetramethylbiphenyl-3,5-dicarboxamide
3'-[5-({[(3,5-dichioropyridin-4-yl)amino]carbonyl}amino)-1-ethyl-6-oxo-1,6-dihydropyridazin-3-yl]-N,N'-bis[3-(dimethylamino)propyl]biphenyl-3,5-dicarboxamide
3'-[5-({[(3,5-dichloropyridin-4-yl)amino]carbonyl}amino)-1-ethyl-6-oxo-1,6-dihydropyridazin-3-yl]-N,N'-bis[3-(1H-imidazol-1-yl)propyl]biphenyl-3,5-dicarboxamide
3'-[5-({[(3,5-dichloropyridin-4-yl)amino]carbonyl}amino)-1-ethyl-6-oxo-1,6-dihydropyridazin-3-yl]-N,N'-bis(2-hydroxyethyl)biphenyl-3,5-dicarboxamide
3-{3'-[5-({[(3,5-dichioropyridin-4-yl)amino]carbonyl}amino)-1-ethyl-6-oxo-1,6-dihydropyridazin-3-yl]biphenyl-4-yl}-N-(2-pyrrolidin-1-ylethyl)propanamide
3-{3'-[5-({[(3,5-dichloropyridin-4-yl)amino]carbonyl}amino)-1-ethyl-6-oxo-1,6-dihydropyridazin-3-yl]biphenyl-4-yl}-N-(2-piperidin-1-ylethyl)propanamide
N-cyclopropyl-3-{3'-[5-({[(3,5-dichloropyridin-4-yl)amino]carbonyl}amino)-1-ethyl-6-oxo-1,6-dihydropyridazin-3-yl]biphenyl-3-yl}propanamide
3-{3'-[5-({[(3,5-dichloropyridin-4-yl)amino]carbonyl}amino)-1-ethyl-6-oxo-1,6-dihydropyridazin-3-yl]biphenyl-3-yl}-N-(2-piperidin-1-ylethyl)propanamide
N-(1-benzylpiperidin-4-yl)-N'-cyclopropyl-3'-[5-({[(3,5-dichloropyridin-4-yl)amino]carbonyl}amino)-1-ethyl-6-oxo-1,6-dihydropyridazin-3-yl]biphenyl-3,5-dicarboxamide
N-cyclopropyl-3'-[5-({[(3,5-dichloropyridin-4-yl)amino]carbonyl}amino)-1-ethyl-6-oxo-1,6-dihydropyridazin-3-yl]-N'-[1-(2-hydroxyethyl)piperidin-4-yl]biphenyl-3,5-dicarboxamide
N-cyclopropyl-3'-[5-({[(3,5-dichloropyridin-4-yl)amino]carbonyl}amino)-1-ethyl-6-oxo-1,6-dihydropyridazin-3-yl]-N'-[1-(5-hydroxypentyl)piperidin-4-yl]biphenyl-3,5-dicarboxamide
ethyl 5-cyano-3'-[5-({[(3,5-dichloropyridin-4-yl)amino]carbonyl}amino)-1-ethyl-6-oxo-1,6-dihydropyridazin-3-yl]biphenyl-3-carboxylate
5-cyano-3'-[5-({[(3,5-dichloropyridin-4-yl)amino]carbonyl}amino)-1-ethyl-6-oxo-1,6-dihydropyridazin-3-yl]biphenyl-3-carboxylic acid
5-cyano-N-cyclopropyl-3'-[5-({[(3,5-dichloropyridin-4-yl)amino]carbonyl}amino)-1-ethyl-6-oxo-1,6-dihydropyridazin-3-yl]biphenyl-3-carboxamide
5-cyano-3'-[5-({[(3,5-dichloropyridin-4-yl)amino]carbonyl}amino)-1-ethyl-6-oxo-1,6-dihydropyridazin-3-yl]-N-(2-piperidin-1-ylethyl)biphenyl-3-carboxamide
Methyl 3'-[5-({[(3,5-dichloropyridin-4-yl)amino]carbonyl}amino)-1-ethyl-6-oxo-1,6-dihydropyridazin-3-yl]-5-{[(2-pyrrolidin-1-ylethyl)amino]carbonyl}biphenyl-3-carboxylate
3'-[5-({[(3,5-dichloropyridin-4-yl)amino]carbonyl}amino)-1-ethyl-6-oxo-1,6-dihydropyridazin-3-yl]-5-({[3-(dimethylamino)propyl]amino}carbonyl)biphenyl-3-carboxylic acid
Methyl 5-[(cyclopropylamino)carbonyl]-3'-[5-({[(3,5-dichloropyridin-4-yl)amino]carbonyl}amino)-1-ethyl-6-oxo-1,6-dihydropyridazin-3-yl]biphenyl-3-carboxylate
3'-[5-({[(3,5-dichloropyridin-4-yl)amino]carbonyl}amino)-1-ethyl-6-oxo-1,6-dihydropyridazin-3-yl]-N-[3-(dimethylamino)propyl]-N'-ethylbiphenyl-3,5-dicarboxamide
3'-[5-({[(3,5-dichloropyridin-4-yl)amino]carbonyl}amino)-1-ethyl-6-oxo-1,6-dihydropyridazin-3-yl]-N'-[3-(dimethylamino)propyl]-N,N-dimethylbiphenyl-3,5-dicarboxamide
N-cyclopropyl-3'-[5-({[(3,5-dichloropyridin-4-yl)amino]carbonyl}amino)-1-ethyl-6-oxo-1,6-dihydropyridazin-3-yl]-N'-[3-(dimethylamino)propyl]biphenyl-3,5-dicarboxamide
N-cyclopropyl-3'-[5-({[(3,5-dichloropyridin-4-yl)amin0]carbonyl}amino)-1-ethyl-6-oxo-1,6-dihydropyridazin-3-yl]-N'-(2-morpholin-4-ylethyl)biphenyl-3,5-dicarboxamide
N-cyclopropyl-3'-[5-({[(3,5-dichloropyridin-4-yl)amin0]carbonyl}amino)-1-ethyl-6-oxo-1,6-dihydropyridazin-3-yl]-N'-(4-hydroxybenzyl)biphenyl-3,5-dicarboxamide
N-cyclopropyl-3'-[5-({[(3,5-dichloropyridin-4-yl)amino]carbonyl}amino)-1-ethyl-6-oxo-1,6-dihydropyridazin-3-yl]-N'-[3-(1H-imidazol-1-yl)propyl]biphenyl-3,5-dicarboxamide
N-cyclopropyl-3'-[5-({[(3,5-dichloropyridin-4-yl)amin0]carbonyl}amino)-1-ethyl-6-oxo-1,6-dihydropyridazin-3-yl]-N'-{5-[(2-hydroxyethyl)(methyl)amino]pentyl}biphenyl-3,5-dicarboxamide
N'-cyciopropyl-3'-[5-({[(3,5-dichioropyridin-4-yl)amino]carbonyl}amino)-1-ethyl-6-oxo-1,6-dihydropyridazin-3-yl]-N,N-dimethylbiphenyl-3,5-dicarboxamide
N-cyclopropyl-3'-[5-({[(3,5-dichloropyridin-4-yl)amino]carbonyl}amino)-1-ethyl-6-oxo-1,6-dihydropyridazin-3-yl]-N'-(2-hydroxyethyl)biphenyl-3,5-dicarboxamide
N-cyclopropyl-3'-[5-({[(3,5-dichloropyridin-4-yl)amino]carbonyl}amino)-1-ethyl-6-oxo-1,6-dihydropyridazin-3-yl]-N'-(2-pyrrolidin-1-ylethyl)biphenyl-3,5-dicarboxamide
N-cyclopropyl-3'-[5-({[(3,5-dichloropyridin-4-yl)amino]carbonyl}amino)-1-ethyl-6-oxo-1,6-dihydropyridazin-3-yl]biphenyl-3,5-dicarboxamide
1-({5-[(cyclopropylamino)carbonyl]-3'-[5-({[(3,5-dichloropyridin-4-yl)amino]carbonyl}amino)-1-ethyl-6-oxo-1,6-dihydropyridazin-3-yl]biphenyl-3-yl}carbonyl)piperidine-4-carboxamide
N'-cyclopropyl-3'-[5-({[(3,5-dichloropyridin-4-yl)amino]carbonyl}amino)-1-ethyl-6-oxo-1,6-dihydropyridazin-3-yl]-N-(2-hydroxyethyl)-N-methylbiphenyl-3,5-dicarboxamide
5-cyano-3'-[5-({[(3,5-dichioropyridin-4-yl)amino]carbonyl}amino)-1-ethyl-6-oxo-1,6-dihydropyridazin-3-yl]-N-(2-pyrrolidin-1-ylethyl)biphenyl-3-carboxamide
5-cyano-3'-[5-({[(3,5-dichioropyridin-4-yl)amino]carbonyl}amino)-1-ethyl-6-oxo-1,6-dihydropyridazin-3-yl]-N-(2-morpholin-4-ylethyl)biphenyl-3-carboxamide
Methyl 3'-[5-({[(3,5-dichloropyridin-4-yl)amino]carbonyl}amino)-1-ethyl-6-oxo-1,6-dihydropyridazin-3-yl]-5-[(ethylamino)carbonyl]biphenyl-3-carboxylate
Methyl 3'-[5-({[(3,5-dichloropyridin-4-yl)amino]carbonyl}amino)-1-ethyl-6-oxo-1,6-dihydropyridazin-3-yl]-5-{[(2-hydroxyethyl)amino]carbonyl}biphenyl-3-carboxylate
N-[6-(3'-cyanobiphenyl-3-yl)-2-ethyl-3-oxo-2,3-dihydropyridazin-4-yl]-N'-(3,5-dichloropyridin-4-yl)urea
N-[6-(4'-cyanobiphenyl-3-yl)-2-ethyl-3-oxo-2,3-dihydropyridazin-4-yl]-N'-(3,5-dichloropyridin-4-yl)urea
3'-[1-(cyclopropylmethyl)-5-({[(3,5-dichloropyridin-4-yl)amin0]carbonyl}amino)-6-oxo-1,6-dihydropyridazin-3-yl]-N-[3-(dimethylamino)propyl]biphenyl-3-carboxamide
N-cyclopropyl-3'-[1-(cyclopropylmethyl)-5-({[(3,5-dichloropyridin-4-yl)amino]carbonyl}amino)-6-oxo-1,6-dihydropyridazin-3-yl]biphenyl-3-carboxamide
N-cyclopropyl-3'-[5-({[(3,5-dimethylpyridin-4-yl)amino]carbonyl}amino)-1-ethyl-6-oxo-1,6-dihydropyridazin-3-yl]biphenyl-3-carboxamide
N-cyclopropyl-3'-[5-({[(3,5-dichloropyridin-4-yl)amino]carbonyl}amino)-6-oxo-1-(2,2,2-trifluoroethyl)-1,6-dihydropyridazin-3-yl]biphenyl-3-carboxamide
2-{3'-[1-(Cyclopropylmethyl)-5-({[(3,5-dichloropyridin-4-yl)amino]carbonyl}amino)-6-oxo-1,6-dihydropyridazin-3-yl]biphenyl-4-yl}-N-(2-morpholin-4-ylethyl)acetamide
2-{3'-[5-({[(3,5-Dichloropyridin-4-yl)amino]carbonyl}amino)-6-oxo-1-(2,2,2-trifluoroethyl)-1,6-dihydropyridazin-3-yl]biphenyl-4-yl}-N-(2-piperidin-1-ylethyl)acetamide
N-Cyclopropyl-3'-[5-({[(3,5-dichloropyridin-4-yl)amino]carbonyl}amino)-1-(2,2-difluoroethyl)-6-oxo-1,6-dihydropyridazin-3-yl]biphenyl-3-carboxamide
2-{3'-[5-({[(3,5-Dichloropyridin-4-yl)amino]carbonyl}amino)-1-(2,2-difluoroethyl)-6-oxo-1,6-dihydropyridazin-3-yl]biphenyl-4-yl}-N-(2-piperidin-1-ylethyl)acetamide
2-{3'-[1-(Cyclopropylmethyl)-5-({[(3,5-dichioropyridin-4-yl)amino]carbonyl}amino)-6-oxo-1,6-dihydropyridazin-3-yl]biphenyl-4-yl}-N-(2-piperidin-1-ylethyl)acetamide
2-{3'-[5-({[(3,5-Dimethylpyridin-4-yl)amino]carbonyl}amino)-1-ethyl-6-oxo-1,6-dihydropyridazin-3-yl]biphenyl-4-yl}-N-(2-piperidin-1-ylethyl)acetamide
N,N'-Dicyclopropyl-3'-[5-({[(3,5-dimethylpyridin-4-yl)amino]carbonyl}amino)-1-ethyl-6-oxo-1,6-dihydropyridazin-3-yl]biphenyl-3,5-dicarboxamide
N'-Cyclopropyl-3'-[5-({[(3,5-dimethylpyridin-4-yl)amino]carbonyl}amino)-1-ethyl-6-oxo-1,6-dihydropyridazin-3-yl]-N,N-diethylbiphenyl-3,5-dicarboxamide
N'-Cyclopropyl-3'-[5-({[(3,5-dimethylpyridin-4-yl)amino]carbonyl}amino)-1-ethyl-6-oxo-1,6-dihydropyridazin-3-yl]-N,N-dimethylbiphenyl-3,5-dicarboxamide
and pharmaceutically acceptable salts thereof.

Of outstanding interest are:
N-(3,5-Dichloropyridin-4-yl)-N'-[2-ethyl-6-(5'-fluoro-2'-hydroxybiphenyl-3-yl)-3-oxo-2,3-dihydropyridazin-4-yl]urea
3'-[5-({[(3,5-Dichloropyridin-4-yl)amino]carbonyl}amino)-1-ethyl-6-oxo-1,6-dihydropyridazin-3-yl]biphenyl-4-carboxylic acid
3'-[5-({[(3,5-Dichloropyridin-4-yl)amino]carbonyl}amino)-1-ethyl-6-oxo-1,6-dihydropyridazin-3-yl]-N-[2-(dimethylamino)ethyl]biphenyl-3-carboxamide
N-Cyclopropyl-3'-[5-({[(3,5-dichloropyridin-4-yl)amino]carbonyl}amino)-1-ethyl-6-oxo-1,6-dihydropyridazin-3-yl]biphenyl-3-carboxamide
3'-[5-({[(3,5-Dichloropyridin-4-yl)amino]carbonyl}amino)-1-ethyl-6-oxo-1,6-dihydropyridazin-3-yl]-N-[3-(dimethylamino)propyl]biphenyl-3-carboxamide
3'-[5-({[(3,5-Dichloropyridin-4-yl)amino]carbonyl}amino)-1-ethyl-6-oxo-1,6-dihydropyridazin-3-yl]-N-(2-piperidin-1-ylethyl)biphenyl-3-carboxamide
N-Cyclopropyl-3'-[5-({[(3,5-dichloropyridin-4-yl)amino]carbonyl}amino)-1-ethyl-6-oxo-1,6-dihydropyridazin-3-yl]-5-hydroxybiphenyl-3-carboxamide
N-Cyclopropyl-2-{3'-[5-({[(3,5-dichloropyridin-4-yl)amino]carbonyl}amino)-1-ethyl-6-oxo-1,6-dihydropyrudazin-3-yl]biphenyl-3-yl}acetamide
2-{3'-[5-({[(3,5-Dichloropyridin-4-yl)amino]carbonyl}amino)-1-ethyl-6-oxo-1,6-dihydropyridazin-3-yl]biphenyl-4-yl}-N-(2-piperidin-1-ylethyl)acetamide
2-{3'-[5-({[(3,5-Dichloropyridin-4-yl)amino]carbonyl}amino)-1-ethyl-6-oxo-1,6-dihydropyridazin-3-yl]biphenyl-3-yl}-N-(2-piperidin-1-ylethyl)acetamide
2-{3'-[5-({[(3,5-Dichloropyridin-4-yl)amino]carbonyl}amino)-1-ethyl-6-oxo-1,6-dihydropyridazin-3-yl]biphenyl-3-yl}-N-(2-morpholin-4-ylethyl)acetamide
3'-[5-({[(3,5-Dichloropyridin-4-yl)amino]carbonyl}amino)-1-ethyl-6-oxo-1,6-dihydropyridazin-3-yl]-N-{7-[(2-hydroxyethyl)(methyl)amino]heptyl}biphenyl-3-carboxamide
2-{3'-[5-({[(3,5-Dichloropyridin-4-yl)amino]carbonyl}amino)-1-ethyl-6-oxo-1,6-dihydropyridazin-3-yl]biphenyl-4-yl}-N-{7-[(2-hydroxyethyl)(methyl)amino]heptyl} acetamide
3'-[5-({[(3,5-Dichloropyridin-4-yl)amino]carbonyl}amino)-1-ethyl-6-oxo-1,6-dihydropyridazin-3-yl]-5-hydroxy-N-{7-[(2-hydroxyethyl)(methyl)amino]heptyl}biphenyl-3-carboxamide
3'-[5-({[(3,5-Dichloropyridin-4-yl)amino]carbonyl}amino)-1-ethyl-6-oxo-1,6-dihydropyridazin-3-yl]-N-(4-hydroxybenzyl)biphenyl-3-carboxamide
2-{3'-[5-({[(3,5-Dichloropyridin-4-yl)amino]carbonyl}amino)-1-ethyl-6-oxo-1,6-dihydropyridazin-3-yl]biphenyl-3-yl}-N-(3-morpholin-4-ylpropyl)acetamide
N,N'-Dicyclopropyl-3'-[5-({[(3,5-dichloropyridin-4-yl)amino]carbonyl}amino)-1-ethyl-6-oxo-1,6-dihydropyridazin-3-yl]biphenyl-3,5-dicarboxamide
N-Cyclopropyl-3'-[5-({[(3,5-dichloropyridin-4-yl)amino]carbonyl}amino)-1-ethyl-6-oxo-1,6-dihydropyridazin-3-yl]-N'-{2-[(2R)-1-methylpyrrolidin-2-yl]ethyl}biphenyl-3,5-dicarboxamide
N-Cyclopropyl-3'-[5-({[(3,5-dichloropyridin-4-yl)amino]carbonyl}amino)-1-ethyl-6-oxo-1,6-dihydropyridazin-3-yl]-N'-{7-[(2-hydroxyethyl)(methyl)amino]heptyl}biphenyl-3,5-dicarboxamide
Methyl 3'-[5-({[(3,5-dichloropyridin-4-yl)amino]carbonyl}amino)-1-ethyl-6-oxo-1,6-dihydropyridazin-3-yl]-5-({[3-(dimethylamino)propyl]amino}carbonyl)biphenyl-3-carboxylate
Methyl 5-[(cyclopropylamino)carbonyl]-3'-[5-({[(3,5-dichloropyridin-4-yl)amino]carbonyl}amino)-1-ethyl-6-oxo-1,6-dihydropyridazin-3-yl]biphenyl-3-carboxylate
N-{6-[3',5'-bis(Piperidin-1-ylcarbonyl)biphenyl-3-yl]-2-ethyl-3-oxo-2,3-dihydropyridazin-4-yl}-N'-(3,5-dichloropyridin-4-yl)urea
N-(6-{3',5'-bis[(4-Methylpiperazin-1-yl)carbonyl]biphenyl-3-yl}-2-ethyl-3-oxo-2,3-dihydropyridazin-4-yl)-N'-(3,5-dichloropyridin-4-yl)urea
3'-[5-({[(3,5-Dichloropyridin-4-yl)amino]carbonyl}amino)-1-ethyl-6-oxo-1,6-dihydropyridazin-3-yl]-N-[3-(dimethylamino)propyl]-N'-ethylbiphenyl-3,5-dicarboxamide
3'-[5-({[(3,5-Dichloropyridin-4-yl)amino]carbonyl}amino)-1-ethyl-6-oxo-1,6-dihydropyridazin-3-yl]-N,N'-bis(2-morpholin-4-ylethyl)biphenyl-3,5-dicarboxamide
N-Cyclopropyl-3'-[5-({[(3,5-dichloropyridin-4-yl)amino]carbonyl}amino)-1-ethyl-6-oxo-1,6-dihydropyridazin-3-yl]-N'-(2-morpholin-4-ylethyl)biphenyl-3,5-dicarboxamide
N-Cyclopropyl-3'-[5-({[(3,5-dichloropyridin-4-yl)amino]carbonyl}amino)-1-ethyl-6-oxo-1,6-dihydropyridazin-3-yl]-N'-(4-hydroxybenzyl)biphenyl-3,5-dicarboxamide
3'-[5-({[(3,5-Dichloropyridin-4-yl)amino]carbonyl}amino)-1-ethyl-6-oxo-1,6-dihydropyridazin-3-yl]-N,N'-bis[3-(1H-imidazol-1-yl)propyl]biphenyl-3,5-dicarboxamide
N-Cyclopropyl-3'-[5-({[(3,5-dichloropyridin-4-yl)amino]carbonyl}amino)-1-ethyl-6-oxo-1,6-dihydropyridazin-3-yl]-N'-[3-(1H-imidazol-1-yl)propyl]biphenyl-3,5-dicarboxamide
N-Cyclopropyl-3'-[5-({[(3,5-dichloropyridin-4-yl)amino]carbonyl}amino)-1-ethyl-6-oxo-1,6-dihydropyridazin-3-yl]-N'-{5-[(2-hydroxyethyl)(methyl)amino]pentyl}biphenyl-3,5-dicarboxamide
N-(1-Benzylpiperidin-4-yl)-N'-cyclopropyl-3'-[5-({[(3,5-dichloropyridin-4-yl)amino]carbonyl}amino)-1-ethyl-6-oxo-1,6-dihydropyridazin-3-yl]biphenyl-3,5-dicarboxamide
N-Cyclopropyl-3'-[5-({[(3,5-dichloropyridin-4-yl)amino]carbonyl}amino)-1-ethyl-6-oxo-1,6-dihydropyridazin-3-yl]-N'-[1-(2-hydroxyethyl)piperidin-4-yl]biphenyl-3,5-dicarboxamide
N-Cyclopropyl-3'-[5-({[(3,5-dichloropyridin-4-yl)amino]carbonyl}amino)-1-ethyl-6-oxo-1,6-dihydropyridazin-3-yl]-N'-[1-(5-hydroxypentyl)piperidin-4-yl]biphenyl-3,5-dicarboxamide
N-Cyclopropyl-3'-[5-({[(3,5-dichloropyridin-4-yl)amin0]carbonyl}amino)-1-ethyl-6-oxo-1,6-dihydropyridazin-3-yl]-N'-(2-hydroxyethyl)biphenyl-3,5-dicarboxamide
3'-[5-({[(3,5-Dichloropyridin-4-yl)amino]carbonyl}amino)-1-ethyl-6-oxo-1,6-dihydropyridazin-3-yl]-N-(2-piperidin-1-ylethyl)-N'-(2-pyrrolidin-1-ylethyl)biphenyl-3,5-dicarboxamide
Methyl 3'-[5-({[(3,5-dichloropyridin-4-yl)amino]carbonyl}amino)-1-ethyl-6-oxo-1,6-dihydropyridazin-3-yl]-5-(piperidin-1-ylcarbonyl)biphenyl-3-carboxylate
N-Cyclopropyl-3'-[5-({[(3,5-dichloropyridin-4-yl)amino]carbonyl}amino)-1-ethyl-6-oxo-1,6-dihydropyridazin-3-yl]biphenyl-3,5-dicarboxamide
1-({5-[(Cyclopropylamino)carbonyl]-3'-[5-({[(3,5-dichloropyridin-4-yl)amino]carbonyl}amino)-1-ethyl-6-oxo-1,6-dihydropyridazin-3-yl]biphenyl-3-yl}carbonyl)piperidine-4-carboxamide
3'-[5-({[(3,5-Dichioropyridin-4-yl)amino]carbonyl}amino)-1-ethyl-6-oxo-1,6-dihydropyridazin-3-yl]-N,N'-bis(2-hydroxyethyl)biphenyl-3,5-dicarboxamide
5-Cyano-N-cyclopropyl-3'-[5-({[(3,5-dichloropyridin-4-yl)amino]carbonyl}amino)-1-ethyl-6-oxo-1,6-dihydropyridazin-3-yl]biphenyl-3-carboxamide
3'-[5-({[(3,5-Dichioropyridin-4-yl)amino]carbonyl}amino)-1-ethyl-6-oxo-1,6-dihydropyridazin-3-yl]-N-{4-[(2S)-2-(methoxymethyl)pyrrolidin-1-yl]butyl}-N'-(2-piperidin-1-ylethyl)biphenyl-3,5-dicarboxamide
3'-[5-({[(3,5-Dichloropyridin-4-yl)amino]carbonyl}amino)-1-ethyl-6-oxo-1,6-dihydropyridazin-3-yl]-N-[2-(dimethylamino)ethyl]-N'-(2-piperidin-1-ylethyl)biphenyl-3,5-dicarboxamide
Methyl 3'-[5-({[(3,5-dichloropyridin-4-yl)amino]carbonyl}amino)-1-ethyl-6-oxo-1,6-dihydropyridazin-3-yl]-5-[(ethylamino)carbonyl]biphenyl-3-carboxylate
N-Cyclopropyl-N'-[3-(dimethylamino)propyl]-3'-[5-({[(3,5-dimethylpyridin-4-yl)amino]carbonyl}amino)-1-ethyl-6-oxo-1,6-dihydropyridazin-3-yl]biphenyl-3,5-dicarboxamide, and
Methyl 3'-[5-({[(3,5-dichloropyridin-4-yl)amino]carbonyl}amino)-1-ethyl-6-oxo-1,6-dihydropyridazin-3-yl]-5-{[(2-hydroxyethyl)amino]carbonyl}biphenyl-3-carboxylate

According to another embodiment the present invention covers pharmaceutical compositions comprising one or more of the compounds of formula (I), as hereinabove described, in admixture with pharmaceutically acceptable diluents or carriers.

In still another embodiment the present invention covers a combination product comprising (i) a compound of formula (I), as hereinabove described, and (ii) another compound selected from (a) β2-adrenergic agonists, (b) anti-cholinergics such as antagonists of M3 muscarinic receptors (c) antiinflammatory agents, (d) anti-allergic agents, (e) immunosuppressants and (f) anti-infectives; for simultaneous, separate or sequential use in the treatment of the human or animal body.

According to still another embodiment of the present invention is directed to compounds of formula (I), as hereinabove described, for use in the treatment of the pathological condition or disease susceptible to amelioration by inhibition of phosphodiesterase 4 and also for the use of compounds of the present invention for the manufacture of a medicament for the treatment or prevention of a pathological condition or disease susceptible to amelioration by inhibition of phosphodiesterase 4. It is a preferred embodiment to use the compound of formula (I) in the manufacture of a medicament for use in the treatment or prevention of a disorder which is asthma, chronic obstructive pulmonary disease, allergic rhinitis, rheumatoid arthritis, multiple sclerosis, atopic dermatitis, psoriasis, inflammatory bowel disease..

According to still another embodiment the present invention covers a method for treating a subject afflicted with a pathological condition or disease susceptible to amelioration by inhibition of phosphodiesterase 4, which method comprises administering to the said subject an effective amount of a compound of formula (I), as hereinabove described. In a preferred embodiment the method is used for treating a subject afflicted with a pathological condition or disease which is asthma, chronic obstructive pulmonary disease, allergic rhinitis, rheumatoid arthritis, multiple sclerosis, atopic dermatitis, psoriasis, inflammatory bowel disease..

The compounds of the present invention may be prepared by methods such as those illustrated in the following Schemes. Solvents, temperatures, pressures and other reaction conditions may readily be selected by one of ordinary skill in the art. Starting materials are commercially available or can be readily prepared by one of ordinary skill in the art using known methods. For all the schemes and compounds described below, R¹, R², R⁴, X, G¹, G² and n are as described for a compound of general formula (I).

Compounds of general formula (I) may be prepared following the synthetic scheme depicted in figure 1.

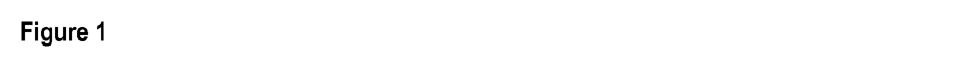

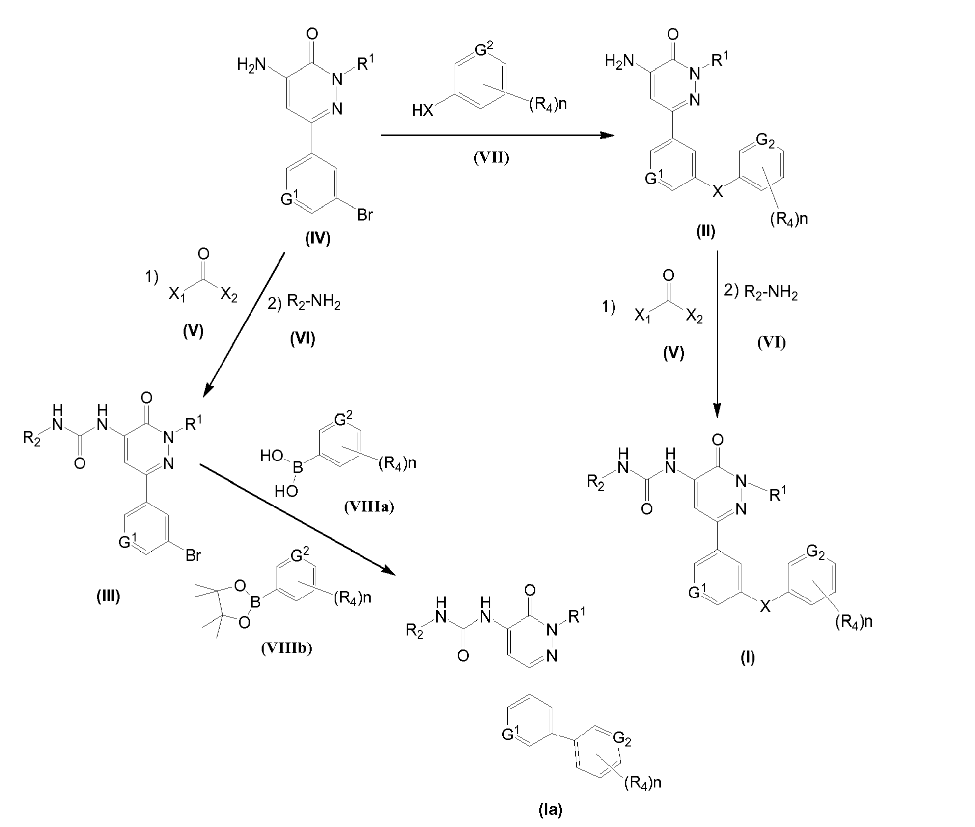

Compounds of general formula (I) may be prepared by condensation of an amine of formula (VI), wherein R₂ is as defined above, with the intermediate resulting from the reaction of 4-aminopyridazinone derivatives of formula (II) with an acylating agent of formula (V) wherein X¹ and X² represent good leaving groups such as chlorine, trichlorometoxy or imidazolyl groups. The reaction is carried out by mixing the aminopyridazinone derivative of formula (II) with an acylating agent (V) such as triphosgene or carbonyldiimidazole, in the presence of an organic base, preferably an amine base such as triethylamine, in an inert solvent such as dichloromethane, tetrahydrofurane or acetonitrile, at a temperature from -20°C to 80°C. The resulting intermediate is then mixed with a suspension of intermediate of formula (VI) in an inert solvent such as dichloromethane or tetrahydrofurane at a temperature from -20°C to 80°C, in the presence of a base such as sodium hydride, to yield the compound of formula (I). Intermediate of formula (II) may be prepared by coupling intermediates of formula (IV) with intermediates of formula (VII) by heating in a sealed tube a mixture of both intermediate in the presence with copper iodide and N,N-dimethylglycine in the presence of a base such as caesium carbonate in a solvent such as dioxane, wherein X represents -O-, or in the presence of tributyltin methoxide in a solvent such as xylene, in the case wherein X represents -S-

In the particular case wherein X is a direct bond, Compounds of formula (Ia) may be prepared by a coupling reaction between intermediate of formula (III) and a boronic acid (VIIIa) or a borolane derivative (Vlllb). The reaction is carried out using typical Suzuki-Miyaura reaction conditions (Miyaura, N.; Suzuki, A. Chem. Rev. 1995, 95, 2457) such as in the presence of tetrakis(triphenylphosphine)palladium(0) or [1,1'-bis(diphenylphosphino)ferrocene] palladium(II) dichloride dichloromethane complex (1:1) in solvents such as toluene or dioxane in an aqueous solution of a base such as sodium or cesium carbonate.

Intermediates of formula (III) may be prepared from intermediate of formula (I) following the same procedure described above for obtaining compounds of formula (I) from intermediates of formula (II). For example they can be prepared in a single procedure by condensation of an amine of formula (VI) with the intermediate resulting from the reaction of a 4-aminopyridazinone derivative of formula (IV) with an acylating agent of formula (V) wherein X1 and X2 represent good leaving groups, such as chlorine, trichlometoxy or imidazolyl groups.

In the particular case wherein R⁴ represents an amide derivative of formula -(CH₂)₍₀₋₄₎-C(O)-NR^{a}R^{b}, -(CH₂)₍₀₋₂₎-C(O)-NH-(CH₂)₍₁₋₃₎-R^{b} or -(CH₂)₍₀₋₂₎-C(O)-NH-(CH₂)₍₁₋₈₎-NR^{a}R^{b}, wherein R^{a} and R^{b} are as hereinbefore defined, compounds of formula (Id) may be prepared by reaction of the acid derivatives of formula (Ic), wherein R⁴ represents a - (CH₂)₍₀₋₄₎-CO₂H group, with an amine derivative of formula (IX), following the synthetic route depicted in Figure 2

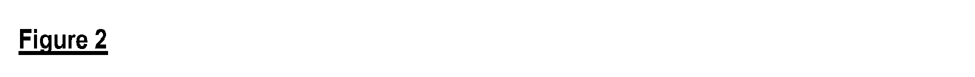

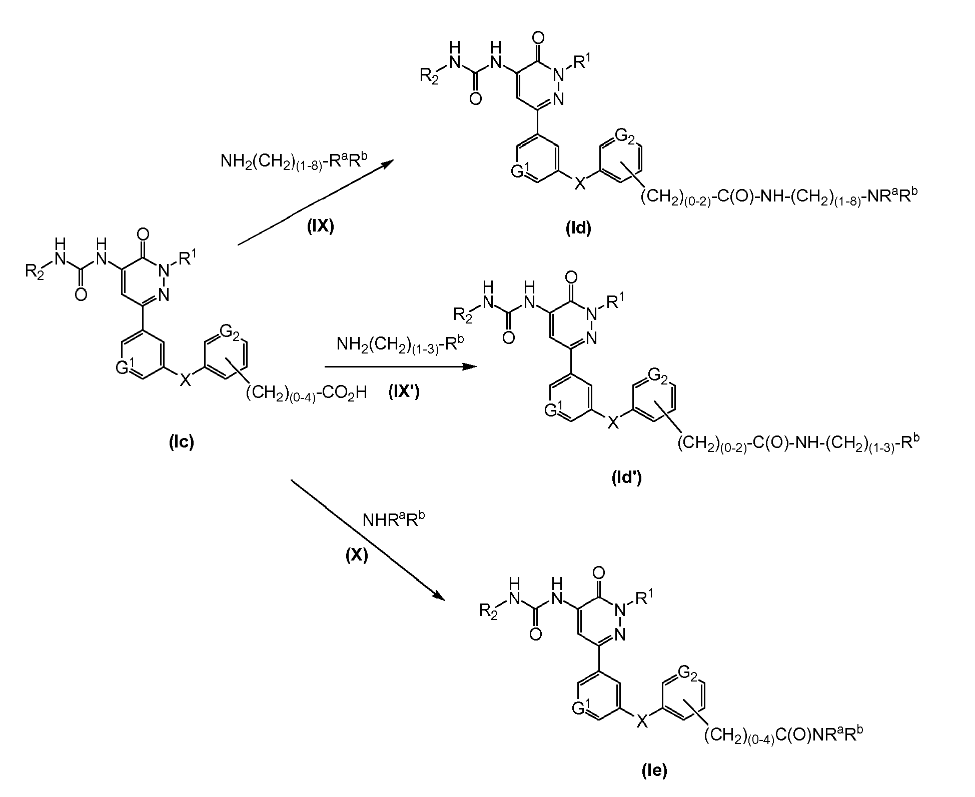

The reaction may be conducted using known amide bond formation procedures, for example by activation of the carboxylic acid of formula (Ic) with a coupling agent such as N-ethyl-N'-(3-dimethyl-aminopropyl)carbodiimide hydrochloride in the presence of 1-hydroxybenzotriazole or O-(benzotriazol-1-yl)-N,N,N',N'-tetramethyluronium hexafluorphosphate in the presence of a base, such as diisopropylethylamine in a solvent such as N,N-dimethylformamide followed by addition of intermediates of formula (IX), (IX') or (X), wherein R^{a} and R^{b} are as defined above, to yields compounds of formula (Id), (Id') or (Ie), respectively.

In the particular case wherein X is a -S(O)₂- group, compounds of general formula (Ig) may be prepared by oxidation of the corresponding compounds of formula (If), wherein X is a -S- group, following the synthetic route depicted in Figure 3.

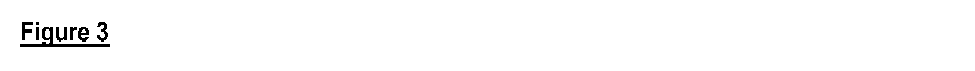

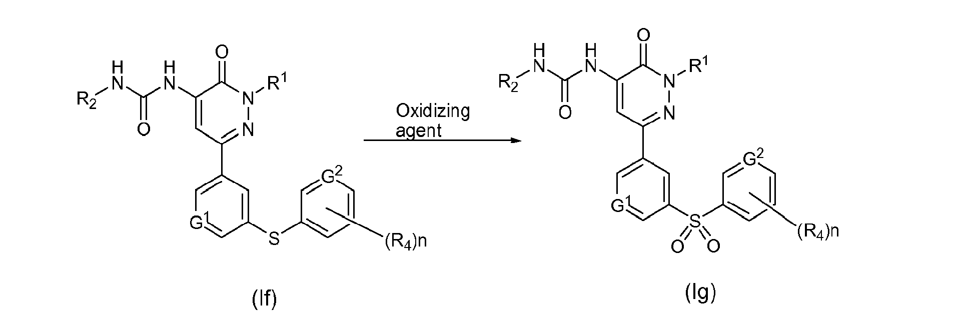

The reaction is carried out in the presence of an appropriate oxidizing agent such as a peroxide a peracid or oxone in an appropriate solvent such as dichloromethane, chloroform, tetrahydrofuran or dimethylformamide at a temperature from 0°C to the boiling point of the solvent.

Intermediates of formula (IV) may be prepared by a variety of methods. For example, as shown in figure 4, reaction of 1,3-dicarbonylic compounds of general formula (XI) with ethyl 2-chloro-2-(hydroxyimino)acetate following methods known per se, e. g. G. Renzi et al., Gazz. Chim. Ital. 1965, 95, 1478, yields to isoxazole derivatives of formula (XII). 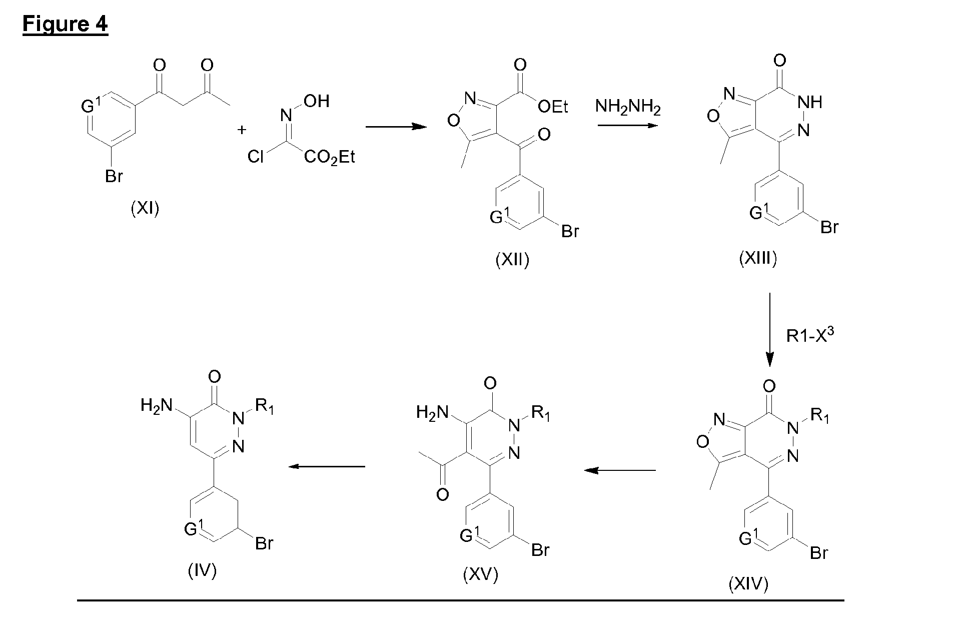 Isoxazole derivatives of formula (XII) are condensed with hydrazine, by methods known per se, e. g. G. Renzi et al., Gazz. Chim. Ital. 1965, 95, 1478 and V.Dal Piaz et al. Heterocycles 1991, 32, 1173, to give isoxazolo[3,4-d]pyridazin-7(6H)-ones of formula (XIII). Intermediates of formula (XIII) are reacted with an alkylating agent of formula R¹-X³, wherein R¹ is as hereinbefore defined and X³ is a leaving group such as a chlorine or a bromine atom or a methanesulfonate, p-toluenesulfonate or a benzenesulfonate group, by methods known per se, e. g. V. Dal Piaz et al. Drug Des. Discovery 1996, 14, to yield isoxazolo[3,4-d]pyridazin-7(6H)-ones derivatives of formula (XIV).

Intermediates of formula (XIV) are hydrogenated to yield 5-acetyl-4-amino(3-oxo)pyridazin-4-ylurea derivatives of formula (XV). The hydrogenation may be performed using for example hydrogen in the presence of a catalyst by methods known per se, e. g. V. Dal Piaz et al. Heterocycles, 1991, 32, 1173. Alternatively, the reaction may be accomplished by transfer hydrogenation using an organic hydrogen donor and a transfer agent, such as ammonium formate or hydrazine by methods known per se, e. g. V. Dal Piaz et al. Heterocycles, 1991, 32, 1173. Finally, treatment of 4-amino(3-oxo)pyridazin-4-ylurea derivatives (XV) with hydrobromic acid at reflux, gives Intermediate compounds of formula (IV).

Alternatively this intermediate of formula (IV) may be prepared through the reaction path shown in the Figure 5:

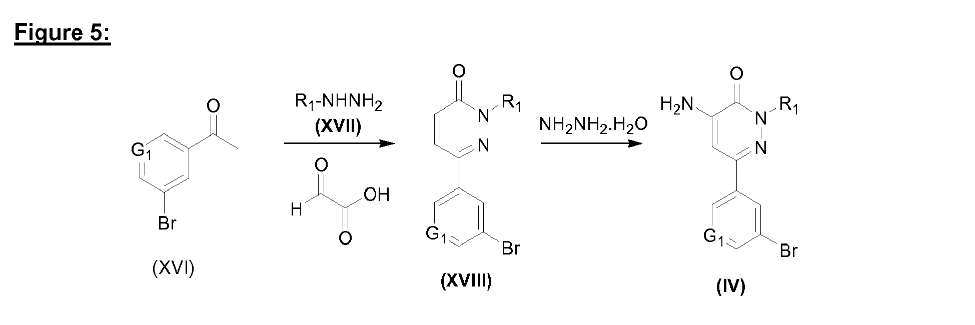

A methylketone derivative of formula (XVI) is condensed with a hydrazine of formula (XVII) wherein R¹ is as hereinbefore defined in the presence of oxoacetic acid by methods known per se, e.g. E. A. Steck et al., J. Heterocycl. Chem. 1974, 11, 755, to give a pyridazinone of formula (XXVII). Subsequent reaction of pyridazin-3(2H) one derivative of formula (XVIII) with hydrazine monohydrate by methods known per se, e.g. W. J. Coates et al., Heterocycles 1989, 29, 1077, yields to the intermediate 4-aminopyridazinones derivative of formula (IV).

Alternatively this intermediate of formula (IV) may be prepared through the reaction path shown in the Figure 6:

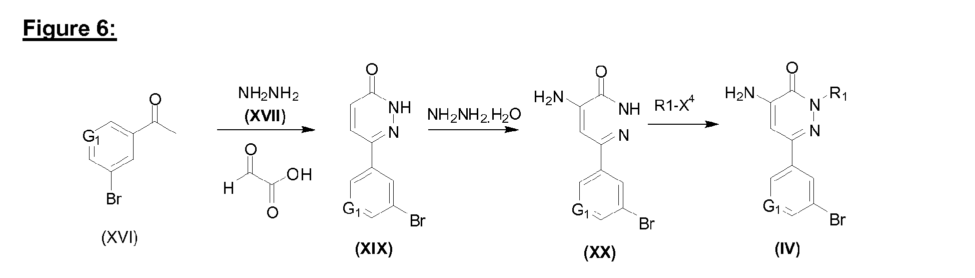

A methylketone derivative of formula (XVI) is condensed with hydrazine monohydrate of formula (XVII), in the presence of oxoacetic acid by methods known *per se*; for example, see e.g. E. A. Steck et al., J. Heterocycl. Chem. 1974, 11, 755. Subsequent reaction of pyridazin-3(2H) one derivative of formula (XIX) with hydrazine monohydrate yields the intermediate 4-aminopyridazinones derivative of formula (XX). This synthetic procedure is effected by methods known *per se*, e.g. W. J. Coates et al., Heterocycles 1989, 29, 1077.

Intermediates of formula (XX) are reacted with an alkylating agent of formula R¹-X⁴, wherein R¹ is as hereinbefore defined and X⁴ is a leaving group such as chlorine or a bromine atom or a methanesulfonate, p-toluenesulfonate or a benzenesulfonate group, by methods known per se, e. g. V. Dal Piaz et al. Drug Des. Discovery 1996, 14, to yield isoxazolo[3,4-d]pyridazin-7(6H)-ones derivatives of formula (IV).

When the defined R groups are susceptible to chemical reaction under the conditions of the hereinbefore described processes or are incompatible with said processes, conventional protecting groups may be used in accordance with standard practice, for example see T. W. Greene and P. G. M. Wuts in 'Protective Groups in Organic Chemistry', 3rd Edition, John Wiley & Sons (1999). It may be that deprotection will form the last step in the synthesis of compounds of formula (I).

The syntheses of the compounds of the invention and of the intermediates for use therein are illustrated by the following Examples (1-298) (including Preparation Examples (Preparations 1 to 120) which do not limit the scope of the invention in any way.

¹H Nuclear Magnetic Resonance Spectra were recorded on a Varian Gemini 300 spectrometer.

Low Resolution Mass Spectra (m/z) were recorded on a Micromass ZMD mass spectrometer using ESI ionization.

Melting points were recorded using a Perkin Elmer DSC-7 apparatus.

The chromatographic separations were obtained using a Waters 2690 system equipped with a Symmetry C18 (2.1 x 10 mm, 3.5 mM) column. The mobile phase was formic acid (0.4 mL), ammonia (0.1 mL), methanol (500 mL) and acetonitrile (500 mL) (B) and formic acid (0.46 mL), ammonia (0.115 mL) and water (1000 mL) (A): initially from 0% to 95% of B in 20 min, and then 4 min. with 95% of B. The reequilibration time between two injections was 5 min. The flow rate was 0.4 mL/min. The injection volume was 5 microliter. Diode array chromatograms were collected at 210 nM.

### Preparation Examples

### PREPARATION 1

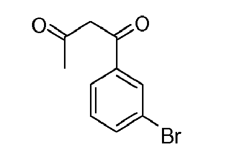

### 1-(3-Bromophenyl)butane-1,3-dione

50 g sodium hydride (60% dispersion in oil, previously washed with pentane, 1.25 mol) was suspended in 920 mL dry tetrahydrofuran. 122.5 mL ethyl acetate (1.25 mol) was added followed by 15 drops of absolute ethanol. The mixture was cooled in an ice-water bath. A solution of 125 g 1-(3-bromophenyl)ethanone (0.63 mol) dissolved in 330 mL tetrahydrofuran was added with stirring, maintaining the reaction temperature below 20 °C. Upon completion of addition 3.5 g 18-crown-6 (0.01 mol) was added. The mixture was stirred at room temperature for 15 min, at 40 °C for 15 min, then at reflux for 5.5 h and then overnight, cooling to room temperature.

The mixture was evaporated and the residue taken up in 1.5 L water. The aqueous mixture was extracted five times with 250 mL ether and was then cooled in an ice-bath and acidified to pH 1-2 with concentrated hydrochloric acid. The aqueous was extracted three times with 250 mL ether, the combined organics were washed twice with water, once with brine. The organics were dried over sodium sulphate, filtered and evaporated to give 124.9 g (82%) of the crude title compound as an oil, contaminated with several small impurities.
¹H NMR (300 MHz, CHLOROFORM-*d*) ppm 8.01 (1 H, s), 7.80 (1 H, d, *J*=7.7 Hz), 7.64 (1 H, d, *J*=7.7 Hz), 7.33 (1 H, t, *J*=8.0 Hz), 6.15 (1 H, s), 2.22 (3 H, s)

### PREPARATION 2

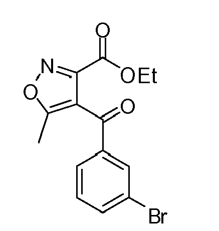

### Ethyl 4-(3-bromobenzoyl)-5-methylisoxazole-3-carboxylate

13 g Sodium metal (previously washed with pentane, 0.57 mol) was mechanically stirred in 50 mL absolute ethanol until completely dissolved. The solution was cooled to between 0 and -5 °C with a ice-salt bath and a solution of 124 g of the crude title compound from Preparation 1 (0.51 mol) dissolved in 325 ethanol was added drop-wise with stirring, maintaining the reaction temperature below 0 °C. The mixture was stirred for 15 min and then a solution of 85 g ethyl 2-chloro-2-(hydroxyimino)acetate (0.56 mol) dissolved in 180 mL ethanol was added drop-wise, maintaining the reaction temperature below 0 °C. The mixture was stirred for 30 min at 0 °C and then overnight, warming to room temperature. The solution (at pH 7) was acidified to pH 5 with 25 mL acetic acid and was evaporated to dryness. The residue was taken up in 750 mL ice-water and was extracted with 3 x 300 mL ether. The combined organics were washed successively with 4% sodium bicarbonate solution, water and brine. The organics were dried over sodium sulphate, decolourised at reflux with activated carbon and filtered. Evaporation under high vacuum gave 155.4 g (89%) of the crude title compound as an oil which solidified upon standing.
¹H NMR (300 MHz, CHLOROFORM-*d*) ppm 7.90 (1 H, s), 7.74 (1 H, d, *J*=7.7 Hz), 7.66 (1 H, d, *J*=7.4 Hz), 7.36 (1 H, t, *J*=7.8 Hz), 4.18 (2 H, q, *J*=7.0 Hz), 2.57 (3 H, s), 1.14 (3 H, t)

### PREPARATION 3

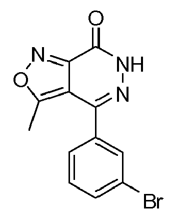

### 4-(3-Bromophenyl)-3-methylisoxazolo[3,4-d]pyridazin-7(6H)-one

155.4 g of the crude title compound from Preparation 2 (0.46 mol) was suspended in 1.15 L ethanol. 34.1 mL hydrazine hydrate (0.7 mol) was slowly added with stirring, dissolving the remaining solid. The mixture was stirred at room temperature overnight, precipitating a solid.

The mixture was cooled in an ice-water bath for 15 min and then filtered. The solid was washed with ethanol, then with ether and was dried in a stream of air and then at 50 °C under vacuum to give 101.5 g (72%) of the title compound.
¹H NMR (300 MHz, DMSO-*d*₆) ppm 7.83 (1 H, s), 7.77 (1 H, d, *J*=7.9 Hz), 7.66 (1 H, d, *J*=7.6 Hz), 7.53 (1 H, t, *J*=7.9 Hz), 3.35 (3 H, s)

### PREPARATION 4

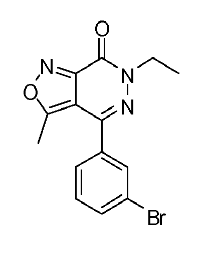

### 4-(3-Bromophenyl)-6-ethyl-3-methylisoxazolo[3,4-d]pyridazin-7(6H)-one

18.9 g of the title compound from Preparation 3 (61.8 mol) was dissolved in 200 mL anhydrous DMF. 25.7 g potassium carbonate (186 mmol) was added and the mixture stirred for 20 min. 13.9 mL bromoethane (186 mmol) was added drop-wise with stirring, maintaining the reaction temperature below 30 °C, and the mixture was stirred at room temperature overnight.

The mixture was evaporated to dryness, the residue taken up in 200 g ice-water and extracted several times with ethyl acetate. The combined organics were washed successively with water, 4% sodium bicarbonate solution, water, brine-water and brine. The organics were dried over sodium sulphate, decolourised at reflux with activated carbon and filtered. Evaporation gave a solid which was broken up in ether and collected by filtration. The solid was washed with ether and was dried in a stream of air and then at 50 °C under vacuum to give 17.6 g (85%) of the title compound.
¹H NMR (300 MHz, CHLOROFORM-*d*) ppm 7.72 (1 H, s), 7.67 (1 H, d, *J*=8.0 Hz), 7.50 (1 H, d, *J*=7.7 Hz), 7.43 (1 H, d, *J*=8.0 Hz), 4.28 (2 H, q, *J*=7.1 Hz), 2.58 (3 H, s), 1.42 (3 H, t, *J*=7.1 Hz)
HPLC/MS (15 min) retention time 8.83 min.
LRMS: *m*/*z* 334/336 (M+H⁺), 351/353 (M+NH₄⁺)

### PREPARATION 5

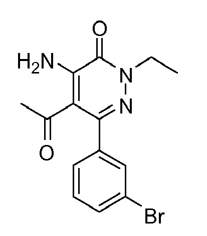

### 5-Acetyl-4-amino-6-(3-bromophenyl)-2-ethylpyridazin-3(2H)-one

34.4 g of the title compound from Preparation 4 (103 mmol) was dissolved in 500 mL tetrahydrofuran and 100 mL ethanol. A heaped spoonful of Raney nickel was added and the mixture agitated under 14 psi hydrogen overnight.

The mixture was filtered and the catalyst washed with tetrahydrofuran. The combined filtrate was evaporated and the solid residue was broken up in 100 mL hot ethanol. Upon cooling to room temperature, the solid was collected by filtration, was washed with ether and was dried in a stream of air and then at 50 °C under vacuum to give 31.2 g (90%) of the title compound.
¹H NMR (300 MHz, CHLOROFORM-*d*) ppm 7.66 (1 H, s), 7.60 (1 H, d, *J*=7.4 Hz), 7.29 - 7.40 (2 H, m), 4.25 (2 H, q, *J*=7.2 Hz), 1.83 (3 H, s), 1.42 (3 H, t, *J*=7.1 Hz)
HPLC/MS (15 min) retention time 7.77 min.
LRMS: *m*/*z* 336/338 (M+H⁺)

### PREPARATION 6

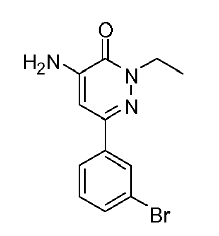

### 4-Amino-6-(3-bromophenyl)-2-ethylpyridazin-3(2H)-one

29.4 g of the title compound from Preparation 5 (87.5 mmol) was suspended in 215 mL 48% hydrobromic acid (3.96 mol) and was heated at 130 °C for 1.25 h. the mixture was allowed to cool and was carefully diluted with ice-water and basified with solid sodium carbonate. Once basic, the aqueous was extracted with 2 x 300 mL ethyl acetate. The combined organics were washed successively with water and brine. The organics were dried over sodium sulphate, decolourised at reflux with activated carbon and filtered. Evaporation gave a residue which was broken up in 100 mL hot diisopropyl ether. Upon cooling, the solid was collected by filtration, washed with diisopropyl ether and was dried in a stream of air and then at 50 °C under vacuum to give 21.1 g (82%) of the title compound.
¹H NMR (300 MHz, CHLOROFORM-*d*) ppm 7.93 (1 H, s), 7.67 (1 H, d, *J*=8.0 Hz), 7.52 (1 H, d, *J*=8.0 Hz), 7.30 (1 H, t, *J*=7.7 Hz), 6.67 (1 H, s), 5.02 (2 H, br. s.), 4.30 (2 H, q, *J*=7.1 Hz), 1.45 (3 H, t, *J*=7.3 Hz)
HPLC/MS (15 min) retention time 8.58 min.
LRMS: *m*/*z* 294/296 (M+H⁺)

### PREPARATION 7

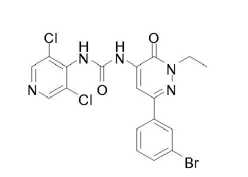

### N-(3,5-Dichloropyridin-4-yl)-N'-[2-ethyl-6-(3-bromophenyl)-3-oxo-2,3-dihydropyridazin-4-yl]urea

### Solution 1:

6.0 g (20.2 mmol) triphosgene was dissolved in 94 mL dichloromethane in a 500 mL flask. A filtered solution of 16.9 g of the title compound from Preparation 6 (57.5 mmol) and 8.5 mL triethylamine (60.9 mmol) in 165 mL dichloromethane was added and the mixture placed under argon atmosphere and stirred for 3 h. The mixture was evaporated to dryness and was re-evaporated twice more from tetrahydrofuran. The residue was taken up in 420 mL tetrahydrofuran and was filtered to remove triethylamine hydrochloride.

### Solution 2:

5.7 g sodium hydride (60% dispersion in oil, previously washed with pentane, 142 mmol) was suspended in 120 mL dry tetrahydrofuran in a 1 L flask. A solution of 4-amino-3,5-dichloropyridine dissolved in 140 mL tetrahydrofuran. The mixture was stirred for 2.5 h, forming a white suspension. The filtrate from solution 1 was added and the mixture stirred overnight at room temperature, precipitating a solid.

The mixture was filtered, keeping both the solid and the filtrate (*). The solid was dissolved in water and the solution acidified to pH 6-7 with 2M hydrochloric acid precipitating a solid. The solid was collected by filtration, washed with water, then with ether and was dried in a stream of air and then at 50 °C under vacuum to give 12.2 g (44%) of the title compound.

The filtrate (*) was evaporated to dryness. The residue was taken up in 700 mL water and acidified to pH 6-7 with 2M hydrochloric acid precipitating a solid. The solid was collected by filtration, washed with water, then with ether and was dried in a stream of air and then at 50 °C under vacuum. The solid was dissolved in 500 mL hot methanol-dichloromethane (30:70) and was decolourised with activated carbon. Filtration and concentration to approx 120 mL precipitated a solid. The solid was collected by filtration, washed with methanol and was dried in a stream of air and then at 50 °C under vacuum to give a further 7.2 g (26%) of the title compound.
m.p. 176-178 °C
¹H NMR (300 MHz, DMSO-*d*₆) ppm 9.94 (1 H, br. s.), 9.76 (1 H, s), 8.74 (2 H, s), 7.99 (1 H, s), 7.82 (1 H, d, *J*=8.0 Hz), 7.71 (1 H, d, *J*=8.0 Hz), 7.51 (1 H, t, *J*=7.8 Hz), 4.30 (2 H, q, *J*=7.1 Hz), 1.41 (3 H, t, *J*=7.1 Hz)
HPLC/MS (15 min) retention time 10.30 min.
LRMS: *m*/*z* 480/482/484 (M+1), 292/294

### PREPARATION 8

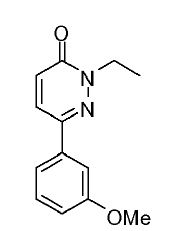

### 2-Ethyl-6-(3-methoxyphenyl)pyridazin-3(2H)-one

32 g 1-(3-methoxyphenyl)ethanone (0.23 mol) and 7 g 2-oxoacetic acid hydrate (76 mmol) were mixed neat and stirred at 105 °C for 5 h. The mixture was allowed to cool to around 40 °C and was diluted with 90 mL water and was basified to pH 8 with 5 mL ammonium hydroxide. The aqueous was extracted with ethyl acetate until no 1-(3-methoxyphenyl)ethanone remained (by TLC). 23 g ethylhydrazine oxalate (0.15 mol) was added and the aqueous mixture stirred at reflux overnight. 12 g ethylhydrazine oxalate (80 mmol) was added and the mixture stirred at reflux a further 5 h. The mixture was allowed to cool and was extracted thee times with ethyl acetate. The combined organics were washed with 4% w/v sodium bicarbonate solution, water and were dried over magnesium sulphate. Evaporation gave 16 g of the crude title compound.
¹H NMR (200 MHz, CHLOROFORM-*d*) ppm 7.62 (1 H, d, *J*=9.8 Hz), 7.22 - 7.42 (3 H, m), 6.87 - 7.05 (2 H, m), 4.27 (2 H, q, *J*=7.4 Hz), 3.84 (3 H, s), 1.42 (3 H, t)

### PREPARATION 9

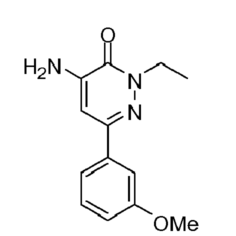

### 4-Amino-2-ethyl-6-(3-methoxyphenyl)pyridazin-3(2H)-one

10.5 g of the crude title compound from Preparation 8 (45 mmol) was suspended in 110 mL hydrazine hydrate and stirred at reflux. Additional portions of 55 mL hydrazine were added twice every day until no more starting material remained by TLC (approximately 700 mL hydrazine in total over 8 d). The mixture was allowed to cool, was diluted with 1 L water and was extracted with ethyl acetate. The organics were washed three times with 250 mL water, dried over magnesium sulphate and evaporated to give the title compound.
¹H NMR (200 MHz, DMSO-*d*₆) ppm 7.16 - 7.56 (3 H, m), 7.00 (1 H, d, *J*=7.4 Hz), 6.73 (1 H, s), 6.52 (2 H, br. s.), 4.14 (2 H, q, *J*=6.6 Hz), 3.81 (3 H, s), 1.31 (3 H, t, *J*=6.8 Hz)

### PREPARATION 10

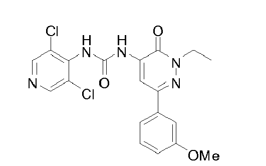

### N-(3,5-Dichloropyridin-4-yl)-N'-[2-ethyl-6-(3-methoxyphenyl)-3-oxo-2,3-dihydropyridazin-4-yl]urea

Reaction of 4.3 g of the title compound from Preparation 9 with 1.9 g triphosgene and 5.80 g 3,5-dichloropyridin-4-amine according to the method described in Preparation 7 and precipitation from ether gave 4.4 g (53%) of the title compound.
¹H NMR (200 MHz, CHLOROFORM-*d*) ppm 9.67 (1 H, s), 9.33 (1 H, s), 8.72 (1 H, s), 8.63 (2 H, s), 7.28 - 7.47 (3 H, m), 6.96 (1 H, d, *J*=8.2 Hz), 4.20 (2 H, q, *J*=7.3 Hz), 3.85 (3 H, s), 1.23 (3 H, t, *J*=7.0 Hz)
HPLC/MS (30 min) retention time 17.348 min.
LRMS: *m*/*z* 434 (M+1)

### PREPARATION 11

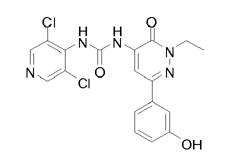

### N-(3,5-Dichloropyridin-4-yl)-N'-[2-ethyl-6-(3-hydroxyphenyl)-3-oxo-2,3-dihydropyridazin-4-yl]urea

Reaction of 1.90 g of the title compound from Preparation 10 with 20.0 mL boron tribromide solution (1M in dichloromethane) according to the method described in Preparation 17 gave 1.70 g (97%) of the title compound.
¹H NMR (400 MHz, DMSO-*d*₆) ppm 9.91 (1 H, s), 9.68 (1 H, br. s.), 9.65 (1 H, s), 8.70 (2 H, s), 8.32 (1 H, s), 7.28 (1 H, t, *J*=7.6 Hz), 7.15 - 7.25 (2 H, m), 6.84 (1 H, d, *J*=7.4 Hz), 4.24 (2 H, q, *J*=7.0 Hz), 1.37 (3 H, t, *J*=7.0 Hz)
HPLC/MS (30 min) retention time 14.602 min.
LRMS: *m*/*z* 420 (M+1)

### PREPARATION 12

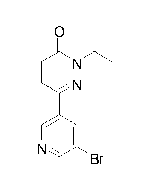

### 4-Amino-6-(5-bromopyridin-3-yl)-2-ethylpyridazin-3(2H)-one

A solution of 1-(5-bromopyridin-3-yl)ethanone (2.04 g, 27.5 mmol) in methanol (55 mL) was added slowly to a cold solution of 2-oxoacetic acid (2.04 g, 27.5 mmol) and potassium carbonate (7.22 g, 52.24 mmol) in water (50 mL). The mixture was stirred at room temperature for 2h.

The resultant solution was partially evaporated under reduced pressure at 30 °C to remove most of the methanol and then extracted three times with ethyl acetate. The cooled aqueous solution was carefully treated with acetic acid (11 mL, 192 mmol) and washed with dichloromethane. Ethylhydrazine oxalate (3.61 g, 26.1 mmol) was added to the aqueous phase, and the mixture heated under reflux for 2 h. A further 3.61 g ethylhydrazine oxalate (26.12mmols) was added and the mixture heated under reflux for 2 d.

The solution was neutralised with potassium carbonate to pH 7 and was extracted three times with ethyl acetate. The combined organic layers were washed with brine, and dried over anhydrous sodium sulphate. The solvent was removed under reduced pressure and the crude purified in a 40M column from Biotage® on a SP1® automatic purification system using hexane and ethyl acetate as solvents (0-100% ethyl acetate) in 20 column volumes. The appropriate fractions were collected and evaporated to give 3.71 g (47% yield) of the title compound.
HPLC/MS (9 min) retention time 5.32 min.
LRMS: *m*/*z* 282 (M+1)

### PREPARATION 13

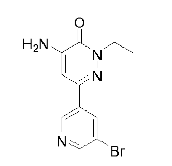

### N-[6-(5-Bromopyridin-3-yl)-2-ethyl-3-oxo-2,3-dihydropyridazin-4-yl]-N'-(3,5-dichloropyridin-4-yl)urea

A mixture of the title compound of Preparation 12 (3.71 g, 13.24 mmols) and hydrazine monohydrate (40 mL) was heated under reflux for 18h. An additional amount of hydrazine monohydrate (20 mL) was added and the reflux maintained for 8 h more. The double addition of hydrazine monohydrate was repeated during 2 further days.

The solution was cooled at room temperature. The suspension was filtered and the solid washed with water and dried under vacuum to give 2.31g (59%) of the title compound.
¹H NMR (200 MHz, CHLOROFORM-*d*) ppm 1.45 (t, *J*=7.25 Hz, 3 H), 4.30 (q, *J*=7.25 Hz, 2 H), 5.11 (s, 2 H), 6.67 (s, 1 H), 8.26 (s, 1 H), 8.68 (s, 1 H), 8.87 (s, 1 H)
HPLC/MS (9 min) retention time 5.45 min.
LRMS: *m*/*z* 297 (M+1)

### PREPARATION 14

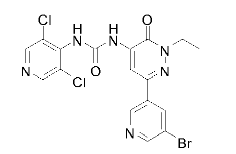

### N-[6-(5-Bromopyridin-3-yl)-2-ethyl-3-oxo-2,3-dihydropyridazin-4-yl]-N'-(3,5-dichloropyridin-4-yl)urea

Reaction of 0.6 g of the title compound of Preparation 13 according to the method described in Preparation 7 gave 0.684 g (63%) of the title compound.
¹H NMR (400 MHz, DMSO-*d*₆) ppm 1.38 (t, *J*=7.14 Hz, 3 H), 4.27 (q, *J*=7.14 Hz, 2 H), 8.32 (s, 1 H), 8.37 (t, *J*=1.96 Hz, 1 H), 8.70 (s, 2 H), 8.79 (d, *J*=2.35 Hz, 1 H), 8.94 (d, *J*=1.96 Hz, 1 H), 9.73 (s, 1 H), 9.90 (s, 1 H)
HPLC/MS (30 min) retention time 15.13 min.
LRMS: *m*/*z* 485 (M+)

### PREPARATION 15

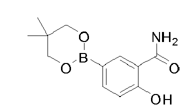

### 5-(5,5-Dimethyl-1,3,2-dioxaborinan-2-yl)-2-hydroxybenzamide

In a Schlenk tube, a mixture of 5-bromo-2-hydroxybenzamide (1.0 g, 4.63 mmol), bis(neopentylglycolato)diboron (2.09 g, 9.26 mmol) and potassium acetate (1.6 g, 13.9 mmol) was dissolved in dioxane (40 mL). The mixture was purged (vacuum-argon three times) and [1,1-Bis(diphenylphosphino)ferrocene]dichloro-palladium(II) dichloromethane complex (0.19 g, 0.23 mmol) and 1,1'-bis(diphenyl-phospheno)ferrocene (0.13 g, 0.23 mmol) were added. The mixture was purged again (vacuum-argon three times) and stirred at 90 °C for 18 h.

The suspension was removed by filtration and the filtrated diluted with water and extracted three times with ethyl acetate. The combined organic layers were washed with water, brine, and dried over anhydrous sodium sulphate. The solvent was removed in vacuum and the residue was purified by the SP1® automated purification system to give 0.4 g (1.54 mmol, 33%) of the title compound
HPLC/MS (9 min) retention time 3.49 min.
LRMS: *m*/*z* 180 (M-1)

### PREPARATION 16

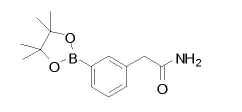

### 2-[3-(4,4,5,5-Tetramethyl-1,3,2-dioxaborolan-2-yl)phenyl]acetamide

In a Schlenk tube, a mixture of 2-(3-bromophenyl)acetamide (1.0 g, 4.67 mmol), bis(pinacolato)diboron (2.37 g, 9.34 mmol) and potassium acetate (1.38 g, 14 mmol) was dissolved in dioxane (40ml). The mixture was purged (vacuum-argon three times) and [1,1-Bis(diphenylphosphino)ferrocene]dichloropalladium(II)dichloromethane complex (0.19 g, 0.23 mmol) and 1,1'-bis(diphenylphospheno)ferrocene (0.13 g, 0.23 mmol) were added. The mixture was purged again (vacuum-argon three times) and stirred at 90 °C for 18 h.

The suspension was filtered off and the filtrated diluted with water and extracted three times with ethyl acetate. The combined organic layers were washed with water and brine, and dried over anhydrous sodium sulphate. Solvent was removed in vacuum and the residue was purified by the SP1® automated purification system to give 0.95 g (3.64 mmol) of the desired compound. Yield = 78%
HPLC/MS (15 min) retention time 6.28 min.
LRMS: *m*/*z* 262 (M+1)

### PREPARATION 17

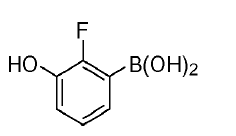

### 2-(Fluoro-3-hydroxyphenyl)boronic acid

2-Fluoro-3-methoxyphenylboronic acid (1.1g, 6.5 mmol) was dissolved in 30 mL dichloromethane and cooled to 5 °C under nitrogen. 32 mL boron tribromide solution (1M in dichloromethane, 32 mmol) was added drop-wise with stirring. Upon addition, the mixture was stirred for 1 h at room temperature. The mixture was added drop-wise to 50 mL cold ethanol. The mixture was then neutralised with portion-wise addition of excess solid sodium bicarbonate with cooling. The mixture was stirred for 15 min, filtered and the filtrate evaporated. The residue was taken up in tetrahydrofuran, re-filtered and re-evaporated to give 0.96 g (96%) of the title compound.
¹H NMR (200 MHz, DMSO-*d*₆) ppm 9.53 (1 H, s), 8.13 (2 H, m), 6.91 (3 H, m)

### PREPARATION 18

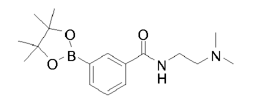

### N-[2-(dimethylamino)ethyl]-3-(4,4,5,5-tetramethyl-1,3,2-dioxaborolan-2-yl)benzamide

*N,N*-dimethylethane-1,2-diamine (0.71 g, 8.06 mmol) was added to a solution of 3-(4,4,5,5-tetramethyl-1,3,2-dioxaborolan-2-yl)benzoic acid (1.00 g, 4.03 mmol), 1-hydroxybenzotriazole hydrate (0.82 g, 6.05 mmol) and N-ethyl-N'-(3-dimethylaminopropyl)carbodiimide hydrochloride (1.16 g, 6.05 mmol) in 8ml dimethylformamide. The mixture was stirred at room temperature for 3 h.

The solvent was removed under reduced pressure and the residue dissolved in 4% sodium bicarbonate solution. The aqueous phase was extracted three times with ethyl acetate. The combined organic layers were washed with water and brine, and dried over anhydrous sodium sulphate. Solvent was removed in vacuum to give 0.81 g (2.55 mmol) of the title compound. Yield = 63%
HPLC/MS (9 min) retention time 4.23 min.
LRMS: *m*/*z* 319 (M+1)

### PREPARATION 19

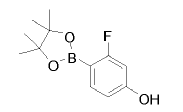

### 3-Fluoro-4-(4,4,5,5-tetramethyl-1,3,2-dioxaborolan-2-yl)phenol

Reaction of 1.00 g 4-bromo-3-fluorophenol with 2.66 g bis(pinacolato)diboron according to the method described in Preparation 16 gave 0.370 g (30%) of the title compound.
¹H NMR (200 MHz, CHLOROFORM-D) ppm 1.3 (s, 12 H) 6.5 (m, 1 H) 6.6 (m, 1 H) 7.6 (m, 1 H)
HPLC/MS (10 min) retention time 8.85 min.
LRMS: *m*/*z* 238 (M+)

### PREPARATION 20

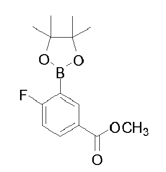

### Methyl 4-fluoro-3-(4,4,5,5-tetramethyl-1,3,2-dioxaborolan-2-yl)benzoate

Reaction of 1.05 g methyl 3-bromo-4-fluorobenzoate with 1.14 g bis(pinacolato)diboron according to the method described in Preparation 16 gave 1.52 g of the title compound, used in the next step without further purification.
HPLC/MS (10 min) retention time 4.47 min.

### PREPARATION 21

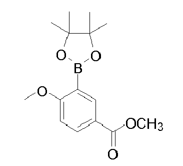

### Methyl 4-methoxy-(4,4,5,5-tetramethyl-1,3,2-dioxaborolan-2-yl)benzoate

Reaction of 2.63 g methyl 3-bromo-4-methoxybenzoate with 5.45 g bis(pinacolato)diboron according to the method described in Preparation 16 gave 4.58 g of an oil which was directly used in the next synthetic step without further purification.
HPLC/MS (10 min) retention time 8.85 min
LRMS: *m*/*z* 293 (M+1)

### PREPARATION 22

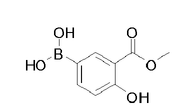

### [4-Hydroxy-3-(methoxycarbonyl)phenyl]boronic acid

Reaction of 1.00 g methyl 5-bromo-2-hydroxybenzoate with 1.96 g bis(neopentylglycolato)diboron according to the method described in Preparation 15 gave 0.07 g (6%) of the title compound.
HPLC/MS (9 min) retention time 4.76 min.
LRMS: *m*/*z* 195 (M-1)

### PREPARATION 23

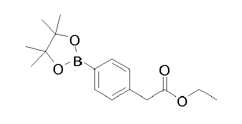

### Ethyl [4-(4,4,5,5-tetramethyl-1,3,2-dioxaborolan-2-yl)phenyl]acetate

Reaction of 1.00g ethyl (4-bromophenyl)acetate with 2.09g bis(pinacolato)diboron according to the method described in Preparation 16 gave 0.86 g (72%) of the title compound.
¹H NMR (200 MHz, CHLOROFORM-*d*) ppm 1.23 (t, *J*=6.83 Hz, 3 H), 1.34 (s, 12 H), 3.62 (s, 2 H), 4.13 (q, *J*=7.03 Hz, 2 H), 7.22 - 7.34 (m, 2 H), 7.77 (d, *J*=7.42 Hz, 2 H),
HPLC/MS (9 min) retention time 6.86 min.
LRMS: *m*/*z* 308 (M+1)

### PREPARATION 24

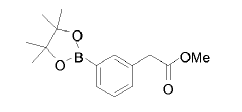

### Methyl [3-(4,4,5,5-tetramethyl-1,3,2-dioxaborolan-2-yl)phenyl]acetate

Reaction of 2.05 g methyl (3-bromophenyl)acetate with 4.55 g bis(pinacolato)diboron according to the method described in Preparation 16 gave 2.00 g (75%) of the title compound.
HPLC/MS (9 min) retention time 6.59 min.
LRMS: *m*/*z* 277 (M+1)

### PREPARATION 25

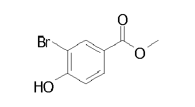

### Methyl 3-bromo-4-hydroxybenzoate

Reaction of 1.00g (4.65 mmol) methyl 3-bromo-4-hydroxybenzoate following W02006/051375 gave 1.07g (99% yield) of the title compound used in the next step without further purification
HPLC/MS (9 min) retention time 5.36 min.
LRMS: *m*/*z* 231 (M-1)

### PREPARATION 26

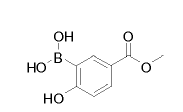

### [2-Hydroxy-5-(methoxycarbonyl)phenyl]boronic acid

Reaction of 1.07 g of the title compound of Preparation 25 (4.63 mmol) with 2.35 g (9.26 mmol) bis(pinacolato)diboron according to the method described in Preparation 16 gave 0.200 g (15%) of the title compound.
HPLC/MS (9 min) retention time 4.66 min.
LRMS: *m*/*z* 195 (M-1)

### PREPARATION 27

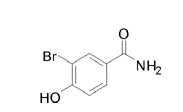

### 3-Bromo-4-hydroxybenzamide

Reaction of 1.5 g (6.91 mmols) 3-bromo-4-hydroxybenzoic acid with 0.554 mL (13.8 mmol) 32% ammonia solution, according to the method described in Preparation 18 gave 0.57 g (34%) of the title compound, after flash chromatography (hexane-ethyl acetate).
HPLC/MS (9 min) retention time 3.73 min.
LRMS: *m*/*z* 218 (M+1)

### PREPARATION 28

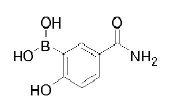

### [5-(Aminocarbonyl)-2-hydroxyphenyl]boronic acid

Reaction of 0.570 g (2.64 mmol) of the title compound of Preparation 27 with 1.34 g (5.28 mmol) bis(pinacolato)diboron according to the method described in Preparation 16 gave 0.245 g (35%) of the title compound.
HPLC/MS (9 min) retention time 3.35 min.
LRMS: *m*/*z* 182 (M+1)

### PREPARATION 29

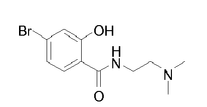

### 4-Bromo-N-[2-(dimethylamino)ethyl]-2-hydroxybenzamide

Reaction of 1.5 g (6.91 mmol) 4-bromo-2-hydroxybenzoic acid with 1.22 g (13.8 mmol) *N,N*-dimethylethane-1,2-diamine according to the method described in Preparation 18 gave 0.75 g (38%) of the title compound, after flash chromatography (chloroform-methanol).
HPLC/MS (9 min) retention time 3.79 min.
LRMS: *m*/*z* 289 (M+1)

### PREPARATION 30

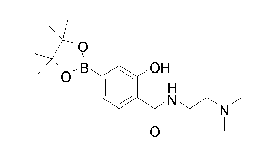

### N-[2-(Ddimethylamino)ethyl]-2-hydroxy-4-(4,4,5,5-tetramethyl-1,3,2-dioxaborolan-2-yl)benzamide

Reaction of 0.750 g (2.61 mmol) of the title compound of Preparation 29 with 1.32 g (5.22 mmol) bis(pinacolato)diboron according to the method described in Preparation 16 gave 0.82g (purity 55%, 52% yield) of the crude title compound, used without further purification.
HPLC/MS (9min) retention time 4.56min.
LRMS: *m*/*z* 335 (M+)

### PREPARATION 31

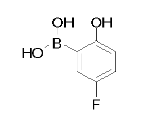

### 5-fluoro-2-hydroxyphenylboronic acid

Reaction of 3.0 g (15.24 mmol) 2-bromo-4-fluorophenol with 6.88 g (30.46 mmol) bis(neopentylglycolato)diboron according to the method described in Preparation 15 gave 3.84 g (62%) of an oil which was directly used in the next synthetic step without further purification.
HPLC/MS (9 min) retention time 4.60 min
LRMS: *m*/*z* 155 (M-1)

### PREPARATION 32

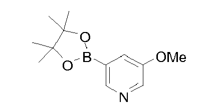

### 3-Methoxy-5-(4,4,5,5-tetramethyl-1,3,2-dioxaborolan-2-yl)pyridine

Reaction of 2.0 g (10.32 mmol) of 3-bromo-5-methoxypyridine with 2.88 g (11.35 mmol) of bis(pinacolato)diboron according to the method described in Preparation 16 gave 1.38 g of an oil which was directly used in the next synthetic step without further purification.
¹H NMR (200 MHz, CHLOROFORM-*d*) ppm 1.36 (br. s., 12 H), 3.87 (s, 3 H), 7.27 (s, 1 H), 8.37 (d, *J*=2.34 Hz, 1 H), 8.55 (s, 1 H)

### PREPARATION 33

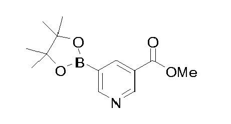

### Methyl 5-(4,4,5,5-tetramethyl-1,3,2-dioxaborolan-2-yl)nicotinate

Reaction of 1.0 g (4.63 mmol) of methyl 5-bromonicotinate with 1.29 g (5.08 mmol) of bis(pinacolato)diboron according to the method described in Preparation 16 gave 1.12 g of an oil which was directly used in the next synthetic step without further purification.
¹H NMR (200 MHz, CHLOROFORM-*d*) ppm 1.21 - 1.31 (m, 12 H), 3.95 (s, 3 H), 8.67 (br. s., 1 H), 9.09 (br. s., 1 H), 9.27 (br. s., 1 H)

### PREPARATION 34

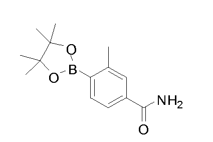

### 3-Methyl-4-(4,4,5,5-tetramethyl-1,3,2-dioxaborolan-2-yl)benzamide

Reaction of 0.6 g (2.80 mmol) of 4-bromo-3-methylbenzamide with 0.78 g (3.08 mmol) of bis(pinacolato)diboron according to the method described in Preparation 16 gave 1.38 g of an oil which was directly used in the next synthetic step without further purification.
¹H NMR (200 MHz, CHLOROFORM-*d*) ppm 1.26 (br. s., 12 H) 2.05 (br. s., 3 H) 4.04 - 4.21 (m, 2 H) 7.59 (d, *J*=9.37 Hz, 2 H) 7.82 (d, *J*=6.64 Hz, 1 H)

### PREPARATION 35

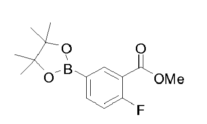

### Methyl 2-fluoro-5-(4,4,5,5-tetramethyl-1,3,2-dioxaborolan-2-yl)benzoate

Reaction of 0.5 g (2.15 mmol) of methyl 5-bromo-2-fluorobenzoate with 1.09 g (4.29 mmol) of bis(pinacolato)diboron according to the method described in Preparation 16 gave 1.04 g of an oil which was directly used in the next synthetic step without further purification.
¹H NMR (200 MHz, CHLOROFORM-*d*) ppm 1.26 (s, 12 H), 3.92 (s, 3 H), 7.04 - 7.18 (m, 1 H), 7.85 - 8.01 (m, 1 H), 8.36 (d, *J*=7.81 Hz, 1 H)

### PREPARATION 36

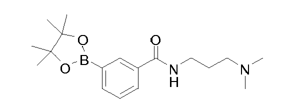

### N-[3-(Dimethylamino)propyl]-3-(4,4,5,5-tetramethyl-1,3,2-dioxaborolan-2-yl)benzamide

Reaction of 0.8 g (3.22 mmol) of 3-(4,4,5,5-tetramethyl-1,2,3-dioxaborolan-2-yl)benzoic acid with 0.66 g (6.45 mmol) of N,N-dimethylpropane-1,3-diamine according to the method described in Preparation 18 gave 0.16 g of an oil which was directly used in the next synthetic step without further purification.
HPLC/MS (9 min) retention time 4.43 min.
LRMS: *m*/*z* 333 (M+1)

### PREPARATION 37

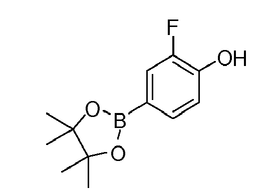

### 2-Fluoro-4-(4,4,5,5-tetramethyl-1,3,2-dioxaborolan-2-yl)phenol

Reaction of 0.50 g 4-bromo-2-fluorophenol (2.6 mmol) with 1.33 g bis(pinacolato)diboron (5.23 mmol) according to the method described in Preparation 16, chromatography (ethyl acetate-hexane, 20:80) and then reverse-phase chromatography by the SP1® automated purification system gave 0.25 g of the title compound (85% pure, 19% yield)
¹H NMR (200 MHz, METHANOL-*d*₄) ppm 7.26 - 7.41 (2 H, m), 6.87 (1 H, t, *J*=8.4 Hz), 1.31 (12 H, s)

### PREPARATION 38

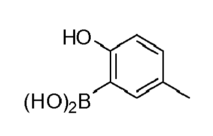

### (2-Hydroxy-5-methylphenyl)boronic acid

Reaction of 0.50 g 2-bromo-4-methylphenol (2.7 mmol) with 1.36 g bis(pinacolato)diboron (5.3 mmol) according to the method described in Preparation 16 and purification by the SP1® automated purification system gave 0.10 g of the title compound (24%)
¹H NMR (200 MHz, METHANOL-*d*₄) ppm 7.26 (1 H, br. s.), 6.83 - 7.16 (1 H, m), 6.41 - 6.73 (1 H, m), 2.16 (3 H, br. s.)
HPLC/MS (10 min) retention time 4.61 min
LRMS: *m*/*z* 151 (M-1)

### PREPARATION 39

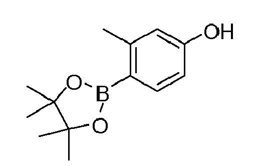

### 3-Methyl-4-(4,4,5,5-tetramethyl-1,3,2-dioxaborolan-2-yl)phenol

Reaction of 0.80 g 4-bromo-3-methylphenol (4.2 mmol) with 2.17 g bis(pinacolato)diboron (8.55 mmol) according to the method described in Preparation 16 and purification by the SP1® automated purification system gave 0.76 g of the title compound (90% pure, 68% yield)
¹H NMR (200 MHz, METHANOL-*d*₄) ppm 7.53 (1 H, d, *J*=7.8 Hz), 6.56 (1 H, s), 6.54 (1 H, dd, *J*=7.8 and 2.3 Hz), 4.90 (3 H, s), 1.31 (12 H, s)

### PREPARATION 40

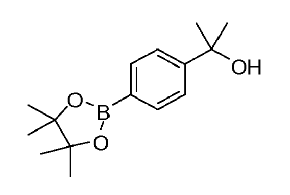

### 2-[4-(4,4,5,5-tetramethyl-1,3,2-dioxaborolan-2-yl)phenyl]propan-2-ol

1.5 g 1,4-dibromobenzene (6.4 mmol) was dissolved in 75 mL anhydrous tetrahydrofuran under nitrogen atmosphere and was cooled to -78 °C. 3.1 mL butyllithium (2.5 M in hexanes, 7.7 mmol) was added drop-wise and with stirring. The mixture was stirred at -78 °C for 1.5 h. 0.61 mL acetone (8.3 mmol) was added drop-wise and the mixture was stirred for a further 1 h at -78 °C. The mixture was allowed to warm to -10 °C and was quenched with 15 mL saturated ammonium chloride solution. The organic phase was separated and evaporated. The residue was re-suspended in ethyl acetate and was washed three times with water. The organics were dried over magnesium sulphate and evaporated. Chromatography (dichloromethane) gave 0.47 g (90% pure, 31% yield) of 2-(4-bromophenyl)propan-2-ol.
HPLC/MS (10 min) retention time 5.74 min
LRMS: *m*/*z* 197/199 (M+1-H₂O)

Reaction of 0.47 g 4-bromo-3-methylphenol (2.0 mmol) with 1.00 g bis(pinacolato)diboron (3.9 mmol) according to the method described in Preparation 16 and reverse-phase chromatography gave 0.10 g of the crude title compound (68% pure, 13% yield).
¹H NMR (200 MHz, METHANOL-*d*₄) ppm 7.68 (2 H, d, *J*=8.2 Hz), 7.47 (2 H, d, *J*=8.2 Hz), 1.33 (12 H, s)
HPLC/MS (10 min) retention time 6.22 min
LRMS: *m*/*z* 245 (M+1-H₂O)

### PREPARATION 41

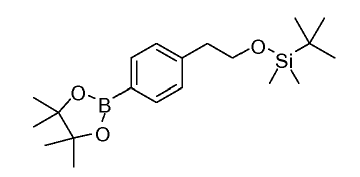

### tert-Butyl(dimethyl){2-[4-(4,4,5,5-tetramethyl-1,3,2-dioxaborolan-2-yl)phenyl]ethoxy}silane

3.0 g 2-(4-bromophenyl)ethanol (15 mmol) and 2.1 g imidazole (31 mmol) were dissolved in 20 mL dichloromethane. A solution of 2.3 g (tert-butyl)dimethylchlorosilane (15.5 mmol) dissolved in 20 mL dichloromethane was added and the mixture stirred overnight, forming a precipitate. The mixture was diluted with dichloromethane and washed three times with 4% w/v sodium carbonate solution, water and was dried over sodium sulphate. Evaporation and purification of the residue by chromatography (ethyl acetate-hexane gradient, 0:100 to 20:80) gave 3.6 g (76%) of [2-(4-bromophenyl)ethoxy](*tert-*butyl)dimethylsilane.
¹H NMR (200 MHz, CHLOROFORM-*d*) ppm 7.42 (2 H, d, *J*=8.2 Hz), 7.11 (2 H, d, *J*=8.2 Hz), 3.80 (2 H, t, *J*=6.6 Hz), 2.79 (2 H, t, *J*=6.8 Hz), 0.89 (9 H, s), 0.01 (6 H, s)

Reaction of 0.80 g [2-(4-bromophenyl)ethoxy](*tert*-butyl)dimethylsilane (2.54 mmol) with 1.29 g bis(pinacolato)diboron (5.07 mmol) according to the method described in Preparation 16 and chromatography (methanol-dichloromethane, 2:98) gave 0.45 g of the crude title compound (60% pure, 30% yield).
HPLC/MS (10 min) retention time 8.24 min
LRMS: *m*/*z* 363 (M+1)

### PREPARATION 42

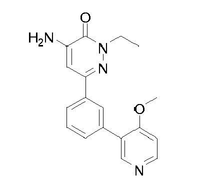

### 4-Amino-2-ethyl-6-[3-(4-methoxypyridin-3-yl)phenyl](3-oxo)pyridazin-4-ylurea

Reaction of 0.500 g of the title compound of Preparation 6 with 0.389 g (4-methoxypyridin-3-yl)boronic acid according to the method described in Example 1 gave 0.190 g (28%) of the title compound, after flash chromatography (hexane-ethyl acetate).
HPLC/MS (9 min) retention time 3.97 min
LRMS: *m*/*z* 323 (M+1)

### PREPARATION 43

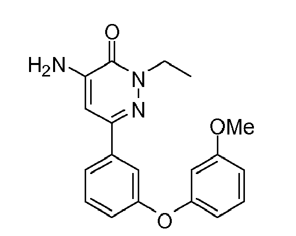

### 4-Amino-2-ethyl-6-[3-(3-methoxyphenoxy)phenyl]pyridazin-3(2H)-one

0.40 g of the title compound of Preparation 6 (1.36 mmol) and 1.33 g caesium carbonate (4.08 mmol) were loaded into a thick-walled Pyrex sealed tube and suspended in 8 mL degassed dioxane. 26 mg Copper(I) iodide (0.14 mmol), 57 mg N,N-dimethylglycine (0.41 mmol) and 0.30 g 3-methoxyphenol (2.72 mmol) were added sequentially, degassing the solvent with nitrogen between each subsequent addition. The tube was sealed and the mixture was stirred at 110 °C overnight. The solvent was evaporated under vacuum and the residue taken up in ethyl acetate. The organics were washed twice with water and twice with brine. The aqueous phases were re-extracted twice with ethyl acetate and the combined organics dried over magnesium sulphate and evaporated. Column chromatography (ethyl acetate-hexane gradient, 30:70 increasing to 50:50) gave 0.375 g (1.08 mmol, 80%) of the title compound
¹H NMR (200 MHz, CHLOROFORM-*d*) ppm 7.33 - 7.56 (3 H, m), 7.16 - 7.31 (1 H, m), 7.04 (1 H, d, *J*=7.8 Hz), 6.56 - 6.76 (4 H, m), 5.00 (2 H, br. s.), 4.28 (2 H, q, *J*=7.0 Hz), 3.79 (3 H, s), 1.43 (3 H, t, *J*=7.2 Hz)
HPLC/MS (10 min) retention time 6.69 min
LRMS: *m*/*z* 338 (M+1)

### PREPARATION 44

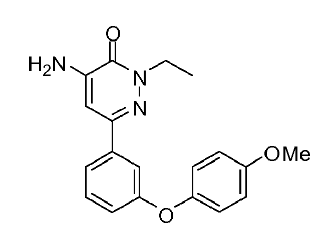

### 4-Amino-2-ethyl-6-[3-(4-methoxyphenoxy)phenyl]pyridazin-3(2H)-one

Reaction of 0.20 g of the title compound of Preparation 6 with 0.17 g 4-methoxyphenol according to the method described in Preparation 43 gave 0.17 g (72%) of the title compound.
HPLC/MS (10 min) retention time 6.62 min
LRMS: *m*/*z* 338 (M+1)

### PREPARATION 45

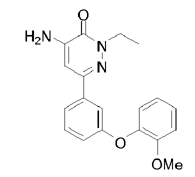

### 4-Amino-2-ethyl-6-[3-(2-methoxyphenoxy)phenyl]pyridazin-3(2H)-one

Reaction of 0.20 g of the title compound of Preparation 6 with 0.15 g 2-methoxyphenol according to the method described in Preparation 43 gave 0.14 g (61%) of the title compound.
HPLC/MS (10 min) retention time 6.40 min
LRMS: *m*/*z* 338 (M+1)

### PREPARATION 46

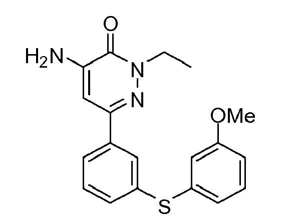

### 4-Amino-2-ethyl-6-{3-[(3-methoxyphenyl)thio]phenyl}pyridazin-3(2H)-one

0.40 g of the title compound of Preparation 6 (1.36 mmol) was dissolved in 8 mL xylene in a thick-walled Pyrex sealed tube. 0.34 mL 3-methoxybenzenethiol (2.72 mmol) and 0.78 mL tributyltin methoxide (2.72 mmol) were added and the mixture sealed and heated at 140 °C for 10 min. The mixture was allowed to cool, 0.31 g palladium tetrakis(triphenylphosphine) (0.27 mmol) was added and the mixture re-sealed and heated at 140 °C overnight.

The mixture was evaporated to dryness and partitioned between water and ethyl acetate. The organic phase was washed twice with water, twice with brine and dried over magnesium sulphate. The organics were filtered through a plug of celite and evaporated. Purification by chromatography (ethyl acetate-hexane, 50:50) gave 0.45 g (1.25 mmol, 92%) of the title compound.
HPLC/MS (10 min) retention time 7.00 min.
LRMS: *m*/*z* 354 (M+1)

### PREPARATION 47

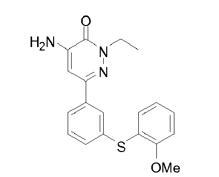

### 4-Amino-2-ethyl-6-{3-[(2-methoxyphenyl)thio]phenyl}pyridazin-3(2H)-one

Reaction of 0.40 g of the title compound of Preparation 6 with 0.25 g 2-methoxybenzenethiol according to the method described in Preparation 46 gave 0.39 g (79%) of the title compound.
HPLC/MS (10 min) retention time 6.77 min
LRMS: *m*/*z* 354 (M+1)

### PREPARATION 48

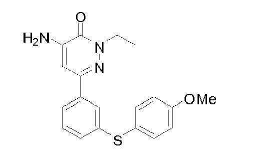

### 4-Amino-2-ethyl-6-{3-[(4-methoxyphenyl)thio]phenyl}pyridazin-3(2H)-one

Reaction of 0.40 g of the title compound of Preparation 6 with 0.34 g 4-methoxybenzenethiol according to the method described in Preparation 46 gave 0.44 g (90%) of the title compound.
HPLC/MS (10 min) retention time 6.96 min
LRMS: *m*/*z* 545 (M+1)

### PREPARATION 49

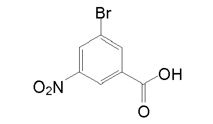

### 3-bromo-5-nitrobenzoic acid

A mixture of 3-nitrobenzoic acid (10 g, 0.06 mols), concentrated sulphuric acid (120ml), silver sulphate (9.33 g, 0.03 mols) and bromine (3.68 ml, 0.07 mols) was stirred at 100°C for 6h. The mixture was poured on ice, and the solid filtered off and extracted with sodium carbonate solution. The title compound was precipitated on acidification from dilute alcohol giving 7.11 g (47%).
HPLC/MS (10 min) retention time 5.69 min
LRMS: *m*/*z* 244 (M-1)

### PREPARATION 50

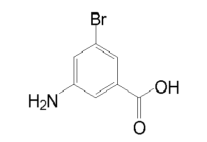

### 3-amino-5-bromobenzoic acid

Concentrated hydrochloric acid (30 ml) was gradually added to a solution of the title compound of Preparation 49 (7.02 g, 28.54 mmols) and ethanol (150 ml), the solution was stirred at room temperature during five minutes. Then Tin (II) chloride was added and the solution was stirred at 50°C for two hours.

The solution was cooled at room temperature. The solution basified to pH 9 with 8M sodium hydroxide precipitating a solid. The solid was collected by filtration and discarded. The filtrate was acidified with hydrochloric acid to pH 5 and precipitation from this solution gave 5.8 g (94%) of the title compound.
HPLC/MS (10 min) retention time 4.67 min
LRMS: *m*/*z* 216 (M-1)

### PREPARATION 51

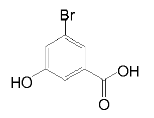

### 3-bromo-5-hydroxybenzoic acid

The title compound of Preparation 50 (5.80 g, 26.85 mmols), in 10% sulphuric acid (55 ml), was diazotised with sodium nitrite (1.85 g), and the solution boiled with dilute sulphuric acid (1:1) ( 30ml) for one hour and forty-five minutes.

The solution was cooled at room temperature. The suspension was filtered and the solid give 3.83 g (66%) of the title compound.
HPLC/MS (10 min) retention time 5.06 min
LRMS: *m*/*z* 215 (M-1)

### PREPARATION 52

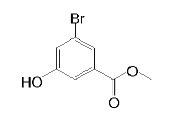

### Methyl 3-bromo-5-hydroxybenzoate

Reaction of 3.93 g (18.11 mmols) of the title compound of Preparation 51 according to the method described in Preparation 25 gave 4.00 g (96%) of the title compound.
HPLC/MS (10 min) retention time 5.87 min
LRMS: *m*/*z* 230 (M-1)

### PREPARATION 53

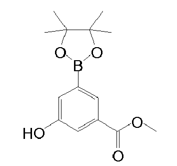

### Methyl 3-hydroxy-5-(4,4,5,5-tetramethyl-1,3,2-dioxaborolan-2-yl)benzoate

Reaction of 4.00 g (17.31 mmols) of the title compound of Preparation 52 with 8.79 g (34.63 mmol) bis(pinacolato)diboron according to the method described in Preparation 16 gave 5.19 g (98%) of the title compound.
¹H NMR (200 MHz, DMSO-*d*₆) ppm 0.94 - 1.44 (m, 12 H) 3.84 (s, 3 H) 7.30 (s, 1 H) 7.43 (s, 1 H) 7.71 (s, 1 H) 9.47 (s, 1 H)
HPLC/MS (10 min) retention time 5.53 min
LRMS: *m*/*z* 277 (M-1)

### PREPARATION 54

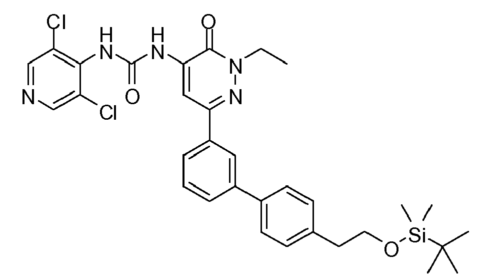

### N-{6-[4'-(2-{[tert-butyl(dimethyl)silyl]oxy}ethyl)biphenyl-3-yl]-2-ethyl-3-oxo-2,3-dihydropyridazin-4-yl}-N'-(3,5-dichloropyridin-4-yl)urea

Reaction of 0.25 g of the title compound of Preparation 7 with 0.45 g of the title compound of Preparation 41 according to the method described in Example 1 gave 0.32 g of the crude title compound. Used as such without further purification.
HPLC/MS (10 min) retention time 8.84 min
LRMS: *m*/*z* 636 (M+1)

### PREPARATION 55

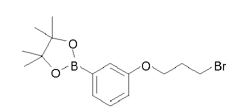

### 1-Bromo-3-[3-(4,4,5,5-tetramethyl-1,3,2-dioxaborolan-2-yl)phenyoxy]propane

3-(4,4,5,5-Tetramethyl-1,3,2-dioxaborolan-2-yl)phenol (0.41 g, 1.86 mmol) and caesium carbonate (0.69 g, 2.1 mmol) were suspended in 5 ml dry DMF. 1,3-Dibromopropane (1.0 ml, 1.0 mmol) was added and the mixture stirred overnight at room temperature.

The mixture was partitioned between water and ether. The aqueous phase was extracted twice with ether. The combined organics were washed with brine and dried over sodium sulphate. Flash chromatography (ethyl acetate-hexane gradient, 0:100, increasing polarity to 20:80) gave 0.36 g of an oil containing 85% of the desired product. Approximate yield 50%.
¹H NMR (200 MHz, CHLOROFORM-*d*) ppm 7.27 - 7.45 (3 H, m), 6.95 - 7.05 (1 H, m), 4.13 (2 H, t, *J*=5.9 Hz), 3.61 (2 H, t, *J*=6.4 Hz), 2.31 (2 H, quin, *J*=6.1 Hz), 1.34 (12 H, s)

### PREPARATION 56

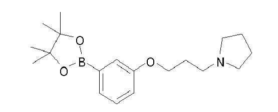

### N-{3-[3-(4,4,5,5-Tetramethyl-1,3,2-dioxaborolan-2-yl)phenyoxy]propyl}pyrrolidine

The crude title compound from preparation 55 (0.36 g) and pyrrolidine (0.41 ml, 5 mmol) were stirred together in 10 ml chloroform at 70 °C for 1 h. The mixture was allowed to cool and was evaporated. The residue was taken up in 2 N HCl and extracted three times with ether. The aqueous was then basified to pH 9 with 2N NaOH and was extracted three times with ethyl acetate. The ethyl acetate extracts were washed with brine and dried over sodium sulphate. Evaporation gave 170 mg of a 1:1 mixture of the title compound and 3-(3-(pyrrolidin-1-yl)propoxy)phenylboronic acid. Used without further purification.

### PREPARATION 57

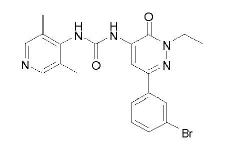

### 1-(6-(3-bromophenyl)-2-ethyl-3-oxo-2,3-dihydropyridazin-4-yl)-3-(3,5-dimethylpyridin-4-yl)urea

Reaction of 2.0 g of the title compound of Preparation 7 with 2.49 g of 3,5-dimethylpyridin-4-amine according to the method described in Preparation 8 and purification by chromatography (acetone-hexane, 10:50) gave 0.607 g (20%) of the title compound.

### PREPARATION 58

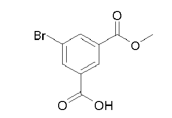

### 3-bromo-5-(methoxycarbonyl)benzoic acid

To a solution of dimethyl 5-bromoisophthalate (2.80g, 10.25mmols) in methanol (18.5ml) is added a solution of potassium hydroxide (0.29g, 5.13mmols) in 3.7ml of water. The mixture was stirred overnight under reflux.

The mixture was partitioned between water and ethyl ether. The aqueous phase was extracted twice with ether. The aqueous was then acidified to acid pH with 5N HCI and was extracted three times with ethyl ether. The combined organics were washed with brine and dried over sodium sulphate. Purification by chromatography (hexane 100% to ethyl acetate 100%) gave 0.700 g (2.70 mmol, 26%) of the title compound.
HPLC/MS (10 min) retention time 5.79 min
LRMS: *m*/*z* 259 (M-1)

### PREPARATION 59

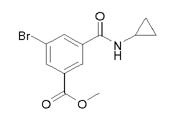

### Methyl 3-bromo-5-[(cyclopropylamino)carbonyl]benzoate

Reaction of 0.6 g (3.22 mmol) of title compound of preparation 58 with 322µl (4.63 mmol) of cyclopropanamine according to the method described in Preparation 18 gave 0.66 g (96%) of the title compound.
HPLC/MS (10 min) retention time 5.64 min
LRMS: *m*/*z* 296 (M-1)

### PREPARATION 60

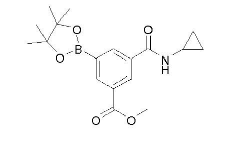

### Methyl 3-[(cyclopropylamino)carbonyl]-5-(4,4,5,5-tetramethyl-1,3,2-dioxaborolan-2-yl)benzoate

Reaction of 0.66 g (2.21 mmols) of the title compound of Preparation 59 with 1.12 g (4.43 mmol) bis(pinacolato)diboron according to the method described in Preparation 16 gave 0.572 g (75%) of the title compound.
HPLC/MS (10 min) retention time 6.31 min
LRMS: *m*/*z* 346 (M+1)

### PREPARATION 61

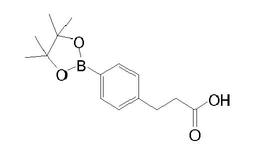

### 3-[4-(4,4,5,5-Tetramethyl-1,3,2-dioxaborolan-2-yl)phenyl]propanoic acid

Reaction of 1.5 g (6.42 mmols) of 3-(4-bromophenyl)propanoic acid with 2.44 g (9.61 mmol) bis(pinacolato)diboron according to the method described in Preparation 16 gave 1.28g (72%) of the title compound.
HPLC/MS (10 min) retention time 6.33 min
LRMS: *m*/*z* 294 (M+18)

### PREPARATION 62

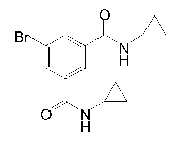

### 5-Bromo-N,N'-dicyclopropylisophthalamide

Cyclopropanamine (1.01ml, 14.49 mmol) was added to a solution of 5-bromoisophthalic acid (710 mg, 2.90 mmol), 1-hydroxybenzotriazole hydrate (1.17 g, 8.69 mmol) and N-ethyl-N'-(3-dimethylaminopropyl)carbodiimide hydrochloride (1.66 g, 8.69 mmol) in 20ml dimethylformamide. The mixture was stirred at room temperature for 2 h.

The solvent was removed under reduced pressure and the residue dissolved in 4% sodium bicarbonate solution precipitating a solid. The solid was collected by filtration and the solid give 0.65 g (70%) of the title compound.
HPLC/MS (10 min) retention time 5.03 min
LRMS: *m*/*z* 323 (M-1)

### PREPARATION 63

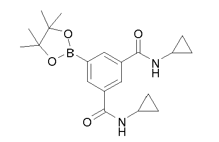

### N,N'-Dicyclopropyl-5-(4,4,5,5-tetramethyl-1,3,2-dioxaborolan-2-yl)isophthalamide

Reaction of 0.780 g (2.41 mmols) of the title compound of Preparation 62 with 1.22 g (4.83 mmol) bis(pinacolato)diboron according to the method described in Preparation 16 gave 0.640 g (72%) of the title compound.
HPLC/MS (10 min) retention time 5.77 min
LRMS: *m*/*z* 371 (M+1)

### PREPARATION 64

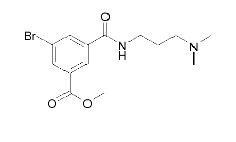

### Methyl 3-bromo-5-({[3-(dimethylamino)propyl]amino}carbonyl)benzoate

Reaction of 3.14 g (12.12 mmol) of title compound of preparation 58 with 2.48g (24.24mmol) of *N,N*-dimethylpropane-1,3-diamine according to the method described in Preparation 18 gave 3.98 g (94%) of the title compound.
HPLC/MS (10 min) retention time 3.82 min
LRMS: *m*/*z* 345 (M+1)

### PREPARATION 65

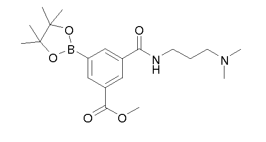

### Methyl 3-({[3-(dimethylamino)propyl]amino}carbonyl)-5-(4,4,5,5-tetramethyl-1,3,2-dioxaborolan-2-yl)benzoate

Reaction of 3.98g (11.34mmols) of the title compound of Preparation 64 with 5.76 g (22.68 mmol) bis(pinacolato)diboron according to the method described in Preparation 16 gave 3.00 g (68%) of the title compound.
HPLC/MS (10 min) retention time 3.82 min
LRMS: *m*/*z* 309 (M+1)

### PREPARATION 66

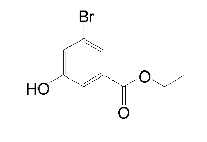

### Ethyl 3-bromo-5-hydroxybenzoate

2.32 g of the title compound of Preparation 51 (8.74 mmol) was dissolved in 25 mL ethanol. 2.10 mL sulphuric acid (39.4 mmols) was added and the mixture was heated at 90°C for 5 hours. The mixture was allowed to cool and was evaporated. The residue was taken up in water and basified to pH 9 with NaOH 32%. The aqueous was then extracted five times with dichloromethane. The organic phase was dried over magnesium sulphate. Purification by chromatography (hexane 100% to ethyl acetate 100%) gave 2.02 g (8.24 mmol, 94%) of the title compound.
HPLC/MS (10 min) retention time 6.08 min
LRMS: *m*/*z* 245 (M-1)

### PREPARATION 67

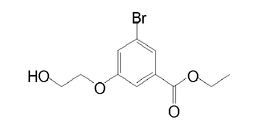

### Ethyl 3-bromo-5-(2-hydroxyethoxy)benzoate

1.25 g of the title compound of Preparation 66 (5.10 mmol) was dissolved in 20 mL dimethylformamide. 2.11 g of potassium carbonate (15.27 mmol) and 2.05 mL 2-chloroethanol (30.61 mmol) were added and the mixture heated at 100 °C for 3 hours. The mixture was allowed to cool and partitioned between water and ethyl ether. The organic phase was washed twice with water, twice with brine and dried over magnesium sulphate. Evaporation gave 1.54 g of the title compound. Used without further purification.
HPLC/MS (10 min) retention time 6.02 min
LRMS: *m*/*z* 290 (M+1)

### PREPARATION 68

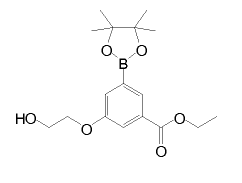

### Ethyl 3-(2-hydroxyethoxy)-5-(4,4,5,5-tetramethyl-1,3,2-dioxaborolan-2-yl)benzoate

Reaction of 1.54 g (5.33 mmols) of the title compound of Preparation 67 with 2.03 g (7.99 mmol) bis(pinacolato)diboron according to the method described in Preparation 16 gave 1.17g (65%) of the title compound.
HPLC/MS (10 min) retention time 6.5 min
LRMS: *m*/*z* 337 (M+1)

### PREPARATION 69

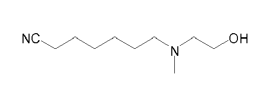

### 7-[(2-hydroxyethyl)(methyl)amino]heptanenitrile

1.90 ml of 2-(methylamino)ethanol (23.67 mmol) was dissolved in 90 ml of methylisobutylketone and 3.27 g of potassium carbonate (23.67 mmol) were added. To this mixture was added a solution of 3.00 g of 7-bromoheptanenitrile (15.78 mmol) in 10 ml of methylisobutylketone. The reaction was kept overnight at 70 °C.

The solvent was evaporated under vacuum and the residue taken up in ethyl acetate. The organics were washed with water and brine, dried over magnesium sulphate, filtered and evaporated. 2.81 g (97%) of the title compound were obtained as a yellow oil.
HPLC/MS (10 min) retention time 0.67 min.
LRMS: *m*/*z* 185 (M+1)

### PREPARATION 70

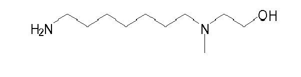

### 2-[(7-aminoheptyl)(methyl)amino]ethanol

1.74 g of lithium aluminum hydride (45.75 mmol) were suspended in 40 mL of dry tetrahydrofuran under nitrogen atmosphere. Then a solution of 2.81 g of the title compound of Preparation 69 (15.25 mmol) in 10 mL of dry tetrahydrofuran was added, followed by additional 50 ml of solvent. The reaction was kept for 3h at room temperature. The reaction was quenched with successive additions of 1.75 mL of water, 1.75 mL of aqueous solution of sodium hydroxide 4M and 5.25 mL of water. This mixture was filtered though celite and the filtrates were evaporated under vacuum. The residue was dissolved in methylene chloride and the organics were dried over magnesium sulphate, filtered and evaporated. 2.32 g (81%) of the title compound were obtained with no need of futher purification.
HPLC/MS (10 min) retention time 0.64 min.
LRMS: *m*/*z* 189 (M+1)

### PREPARATION 71

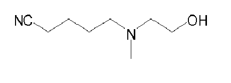

### 5-[(2-hydroxyethyl)(methyl)amino]pentanenitrile

Reaction of 2.22 ml of 2-(methylamino)ethanol (27.8 mmol) and 3.00 g of 5-bromopentanenitrile (18.5 mmol) following the method described in Preparation 69 yielded 2.70 g (93%) of the title compound as a yellow oil.
HPLC/MS (10 min) retention time 0.52 min.
LRMS: *m*/*z* 157 (M+1)

### PREPARATION 72

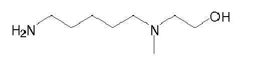

### 2-[(5-aminopentyl)(methyl)amino]ethanol

Reaction of 2.81 g (15.25 mmol) of the title compound of Preparation 71 following the method described in preparation 70 gave 2.32 g (81%) of the title compound.
HPLC/MS (10 min) retention time 0.50 min.
LRMS: *m*/*z* 161 (M+1)

### PREPARATION 73

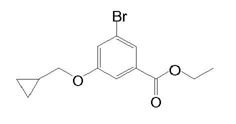

### Ethyl 3-bromo-5-(cyclopropylmethoxy)benzoate

1 g of the title compound of Preparation 66 (4.08 mmol) was dissolved in 15 mL dimethylformamide. 1.13 g of potassium carbonate (8.18 mmol) and 0.66 g of bromomethylcyclopropane (4.89 mmol) were added and the mixture heated at 100 °C overnight. The mixture was allowed to cool and partitioned between water and ethyl ether. The organic phase was washed twice with water, twice with brine and dried over magnesium sulphate. Evaporation gave 0.982 g (75%) of the title compound. Used without further purification.
HPLC/MS (10 min) retention time 7.46 min

### PREPARATION 74

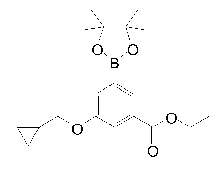

### Ethyl 3-(cyclopropylmethoxy)-5-(4,4,5,5-tetramethyl-1,3,2-dioxaborolan-2-yl)benzoate

Reaction of 0.982 g (3.04 mmols) of the title compound of Preparation 73 with 1.157 g (4.56 mmol) bis(pinacolato)diboron according to the method described in Preparation 16 gave after purification by chromatography (hexane 100% to ethyl acetate 100%) 0.92 g (49%) of the title compound.
HPLC/MS (10 min) retention time 7.57 min
LRMS: *m*/*z* 347 (M+1)

### PREPARATION 75

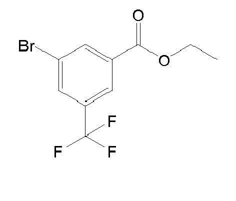

### Ethyl 3-bromo-5-(trifluoromethyl)benzoate

5.20g of 3-bromo-5-(trifluoromethyl)benzoic acid (19.3 mmol) were dissolved in 80 mL of ethanol and 5.27 ml of concentrated sulphuric acid were added and the reaction mixture was refluxed overnight. The solvent vas evaporated under vacuum and the residue was partitioned between water and ethyl acetate. The organic phase was separated, washed twice with water and with brine, dried over magnesium sulphate, filtered and evaporated. 5.43g (95%) of the title compound were obtained.
¹H NMR (200 MHz, CHLOROFORM-D) ppm 1.4 (t, *J*=7.2 Hz, 3 H) 4.4 (q, *J*=7.3 Hz, 2 H) 7.9 (s, 1 H) 8.2 (s, 1 H) 8.4 (s, 1 H)
HPLC/MS (10 min) retention time 7.32 min.
LRMS: *m*/*z* 298 (M+1)

### PREPARATION 76

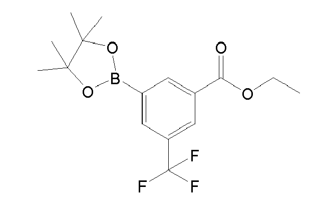

### Ethyl 3-(4,4,5,5-tetramethyl-1,3,2-dioxaborolan-2-yl)-5-(trifluoromethyl)benzoate

Reaction of 5.43 g of the compound of Preparation 75 (18.28 mmol) with 9.28 g of bis(pinacolato)diboron (36.54 mmol) according to the method described in Preparation 16 gave 4.82 g (77%) of the title compound.
HPLC/MS (10 min) retention time 6.28 min.
LRMS: *m*/*z* 345 (M+1)

### PREPARATION 77

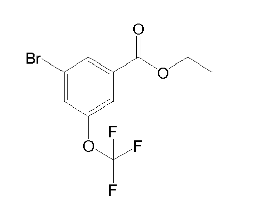

### Ethyl 3-bromo-5-(trifluoromethoxy)benzoate

Reaction of 1.00 g of 3-bromo-5-(trifluoromethoxy)benzoic acid (3.51 mmol) according to the method described in Preparation 75 (reaction time 4 hours) gave 1.01 g (92%) of the title compound.
¹H NMR (200 MHz, CHLOROFORM-D) ppm 1.4 (t, *J*=7.0 Hz, 3 H) 4.4 (q, *J*=7.2 Hz, 2 H) 7.6 (s, 1 H) 7.8 (s, 1 H) 8.1 (m, 1 H)
HPLC/MS (10 min) retention time 7.45 min.
LRMS: *m*/*z* 314 (M+1)

### PREPARATION 78

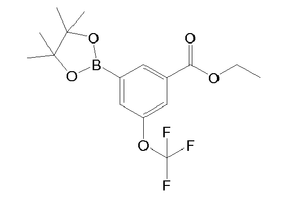

### Ethyl 3-(4,4,5,5-tetramethyl-1,3,2-dioxaborolan-2-yl)-5-(trifluoromethoxy)benzoate

Reaction of 1.01 g of compound of Preparation 77 (3.23 mmol) with 1.64 g of bis(pinacolato)diboron (6.46 mmol) according to the method described in Preparation 16 gave 1.07 g (92%) of the title compound.
HPLC/MS (10 min) retention time 6.40 min.
LRMS: *m*/*z* 360 (M+1)

### PREPARATION 79

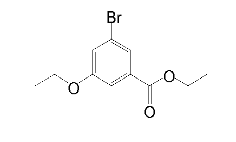

### Ethyl 3-bromo-5-ethoxybenzoate

The title compound of Preparation 51 (1.12 g, 5.16 mmol) and potassium carbonate (3.57 g, 25.83 mmol) were suspended in 100 ml of acetonitrile. Iodoethane (0.91 mL, 11.35 mmol) was added and the resulting reaction mixture was heated under reflux overnight. The solvent was evaporated under vacuum and the residue was partitioned between chloroform and water. The organic phase was separated and the aqueous phase was extracted twice with chloroform. The combined organics were washed with brine, dried over magnesium sulphate and filtered. The solvent was evaporated to give 1.15 g (82%) of the title compound.
¹H NMR (200 MHz, CHLOROFORM-D) ppm 1.4 (m, 6 H) 4.1 (q, *J*=7.0 Hz, 2 H) 4.4 (q, *J*=7.0 Hz, 2 H) 7.2 (m, 1 H) 7.5 (dd, *J*=2.3, 1.2 Hz, 1 H) 7.7 (m, 1 H) HPLC/MS (10 min) retention time 7.27 min.

### PREPARATION 80

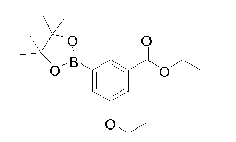

### Ethyl 3-ethoxy-5-(4,4,5,5-tetramethyl-1,3,2-dioxaborolan-2-yl)benzoate

Reaction of 0.95 g (3.27 mmols) of the title compound of Preparation 79 with 1.25 g (4.92 mmol) bis(pinacolato)diboron according to the method described in Preparation 16 gave after purification by chromatography (hexane 100% to ethyl acetate 300%) 0.97 g (52%) of the title compound.
HPLC/MS (10 min) retention time 7.46 min
LRMS: *m*/*z* 321 (M+)

### PREPARATION 81

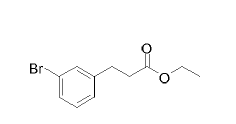

### Ethyl 3-(3-bromophenyl)propanoate

Reaction of 2 g (8.38 mmols) of 3-(3-bromophenyl)propanoic acid according to the method described in Preparation 66 gave 2.04g (92%) of the title compound. Used without further purification.
HPLC/MS (10 min) retention time 6.81 min

### PREPARATION 82

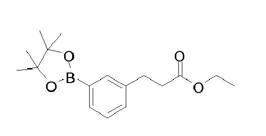

### Ethyl 3-[3-(4,4,5,5-tetramethyl-1,3,2-dioxaborolan-2-yl)phenyl]propanoate

Reaction of 2.04 g (5.33 mmols) of the title compound of Preparation 81 with 2.98 g (11.5 mmol) bis(pinacolato)diboron according to the method described in Preparation 16 gave after purification by chromatography (hexane 100% to ethyl acetate 100%) 2.26g (97%) of the title compound.
HPLC/MS (10 min) retention time 6.5 min
LRMS: *m*/*z* 305 (M+1)

### PREPARATION 83

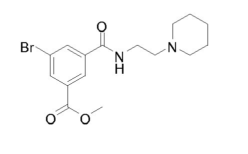

### Methyl 3-bromo-5-(2-(piperidin-1-yl)ethylcarbamoyl)benzoate

Reaction of 2.0 g (7.72 mmol) of title compound of preparation 58 with 2.2 ml (15.49 mmol) of 2-(piperidin-1-yl)ethanamine according to the method described in Preparation 18 gave 2.80 g (96%) of the title compound.
HPLC/MS (10 min) retention time 4.08 min
LRMS: *m*/*z* 370 (M+)
¹H NMR (200 MHz, CHLOROFORM-*d*) δ ppm 1.40 - 1.54 (m, 2 H) 1.54 - 1.70 (m, 3 H) 2.39 - 2.51 (m, 4 H) 2.52 - 2.64 (m, 2 H) 2.68 - 2.79 (m, 1 H) 3.45 - 3.64 (m, 2 H) 3.95 (s, 3 H) 7.13 - 7.25 (m, 1 H) 8.18 (t, *J*=1.76 Hz, 1 H) 8.24 - 8.30 (m, 1 H) 8.33 (t, *J*=1.56 Hz, 1 H)

### PREPARATION 84

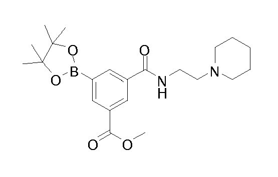

### Methyl 3-(2-(piperidin-1-yl)ethylcarbamoyl)-5-(4,4,5,5-tetramethyl-1,3,2-dioxaborolan-2-yl)benzoate

Reaction of 2.80 g (7.58 mmols) of the title compound of Preparation 83 with 2.90 g (11.42 mmol) bis(pinacolato)diboron according to the method described in Preparation 16 gave 2.0 g (59%) of the title compound.
HPLC/MS (10 min) retention time 4.78 min
LRMS: *m*/*z* 417 (M+)

### PREPARATION 85

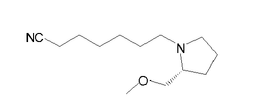

### 7-[(2R)-2-(methoxymethyl)pyrrolidin-1-yl]heptanenitrile

1.90 ml of (2R)-2-(methoxymethyl)pyrrolidine (1.1 ml, 8.9 mmol) were dissolved in 25 ml of methylisobutylketone and 1.2 g of potassium carbonate (8.68 mmol) were added. To this mixture was added 0.85 ml g of 7-bromoheptanenitrile (15.78 mmol). The reaction was kept overnight at 70 °C.

The solvent was evaporated under vacuum and the residue taken up in ethyl acetate. The organics were washed with water and brine, dried over magnesium sulphate, filtered and evaporated. 0.99 g (68%) of the title compound were obtained as an oil.
HPLC/MS (10 min) retention time 0.7 min.
LRMS: *m*/*z* 225 (M+1)

### PREPARATION 86

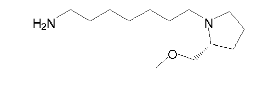

### {7-[(2R)-2-(methoxymethyl)pyrrolidin-1-yl]heptyl}amine

0.5 g of Lithium Aluminum hydride (13.18 mmol) were suspended in 30 mL of dry tetrahydrofuran under nitrogen atmosphere. Then a solution of 0.99 g of the title compound of Preparation 85 (3.88 mmol) in 30 mL of dry tetrahydrofuran was added. The reaction was kept for 3h at room temperature.

The reaction was quenched with successive additions of 0.5 mL of water, 0.5 mL of aqueous solution of sodium hydroxide 4M and 1.5 mL of water. This mixture was filtered though Celite and the filtrates were evaporated under vacuum. 0.89 g (72%) of the title compound were obtained with no need of further purification.
HPLC/MS (10 min) retention time 0.6 min.
LRMS: *m*/*z* 229 (M+1)

### PREPARATION 87

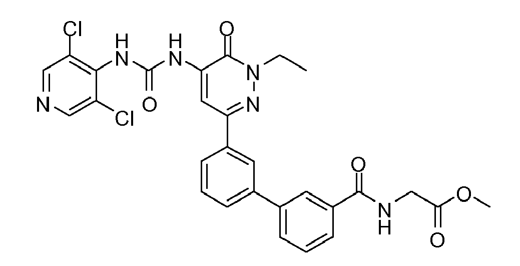

### Methyl N-({3'-[5-({[(3,5-dichloropyridin-4-yl)amino]carbonyl}amino)-1-ethyl-6-oxo-1,6-dihydropyridazin-3-yl]biphenyl-3-yl}carbonyl)glycinate

Reaction of the title compound of Example 48 (150 mg, 0.29 mmol) with methyl 2-aminoacetate (51 mg, 0.57 mmol) according to the method described in Example 49 gave 70 mg (33%) of the title compound.
HPLC/MS (15 min) retention time 8.43 min.
LRMS: *m*/*z* 595 (M+)

### PREPARATION 88

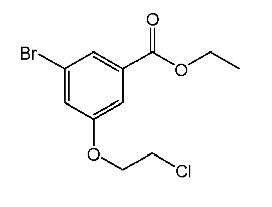

### Ethyl 3-bromo-5-(2-chloroethoxy)benzoate

1.92 g of the title compound of Preparation 66 (7.83 mmol) was dissolved in 20 mL dimethylformamide. 4.33 g of potassium carbonate (31.33 mmol) and 2.61 mL of 1-bromo-2-chloroethane (31.30 mmol) were added and the mixture heated at 90 °C for 1.5 hours. The mixture was allowed to cool and partitioned between water and ethyl acetate. The organic phase was washed with brine and dried over magnesium sulphate. Evaporation gave 2.40 g of the title compound. Used without further purification.
¹H NMR (200 MHz, CHLOROFORM-*d*) ppm 1.4 (t, *J*=7.2 Hz, 3 H) 3.8 (t, *J*=5.9 Hz, 2 H) 4.3 (t, *J*=5.7 Hz, 2 H) 4.4 (q, *J*=7.0 Hz, 2 H) 7.3 (m, 1 H) 7.5 (m, 1 H) 7.8 (m, 1 H)
HPLC/MS (10 min) retention time 7.05 min.

### PREPARATION 89

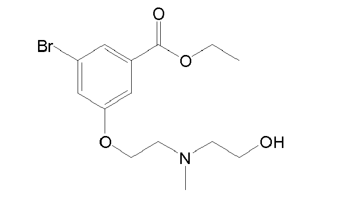

### Ethyl 3-bromo-5-{2-[(2-hydroxyethyl)(methyl)amino]ethoxy}benzoate

1.20 g of the title compound of Preparation 88 (3.90 mmol) was dissolved in 100 mL of isobutylmethylketone. 1.56 ml of 2-(methylamino)ethanol (19.52 mmol) and 1.62 g of potassium carbonate (11.72 mmol) were added and the mixture heated under reflux overnight.

1 mL of dimethylformamide was added and the reaction refluxed for 4h more. The solvent was evaporated under vacuum and the residue was partitioned between water and ethyl acetate. The organic phase was extracted three times with 2N solution of clorhidric acid and the combined aqueous phase was basified with 8N sodium hydroxide solution and the product was reextracted with ethyl acetate. The organics were washed with brine and dried over magnesium sulphate. Evaporation gave 460 mg (34%) of the title compound. Used without further purification.
¹H NMR (200 MHz, CHLOROFORM-*d*) ppm 1.4 (t, *J*=7.2 Hz, 3 H) 2.4 (m, 3 H) 2.7 (m, 2 H) 2.9 (t, *J*=5.5 Hz, 2 H) 3.6 (m, 2 H) 4.1 (t, *J*=5.5 Hz, 2 H) 4.4 (q, *J*=7.3 Hz, 2 H) 7.3 (dd, *J*=4.3, 2.0 Hz, 1 H) 7.5 (m, 1 H) 7.8 (m, 1 H)
HPLC/MS (10 min) retention time 4.17 min.
LRMS: *m*/*z* 347 (M+1)

### PREPARATION 90

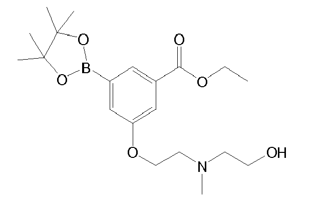

### Ethyl 3-{2-[(2-hydroxyethyl)(methyl)amino]ethoxy}-5-(4,4,5,5-tetramethyl-1,3,2-dioxaborolan-2-yl)benzoate

Reaction of 460 mg (1.33 mmols) of the title compound of Preparation 89 with 759 mg (2.99 mmol) of bis(pinacolato)diboron according to the method described in Preparation 16 gave, after purification by SP1® automated purification system, 300 mg (57%) of the title compound.
HPLC/MS (10 min) retention time 4.90 min
LRMS: *m*/*z* 394 (M+1)

### PREPARATION 91

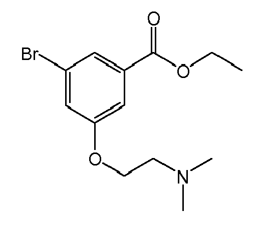

### Ethyl 3-bromo-5-[2-(dimethylamino)ethoxy]benzoate

1.30 g of the title compound of Preparation 88 (4.23 mmol) was dissolved in 10 mL of ethanol. 5 ml of dimethylamine (solution 5.6M in ethanol, 28 mmol) were added and the mixture was heated to 130 °C for 5h under microwaves.

The solvent was evaporated under vacuum and the residue was partitioned between water and ethyl acetate. The organic phase was extracted twice with 2N solution of clorhidric acid and the combined aqueous phase was basified with 8N sodium hydroxide solution and the product was reextracted with ethyl acetate. The organics were washed with brine and dried over magnesium sulphate. Evaporation gave 1.03 g (77%) of the title compound. Used without further purification.
¹H NMR (200 MHz, CHLOROFORM-*d*) ppm 1.4 (t, *J*=7.0 Hz, 3 H) 2.3 (s, 6 H) 2.7 (s, 2 H) 4.1 (t, *J*=5.7 Hz, 2 H) 4.4 (q, *J*=7.3 Hz, 2 H) 7.3 (s, 1 H) 7.5 (s, 1 H) 7.8 (s, 1 H)
HPLC/MS (10 min) retention time 4.10 min
LRMS: *m*/*z* 317 (M+1)

### PREPARATION 92

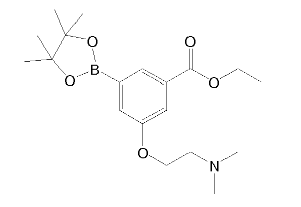

### Ethyl 3-[2-(dimethylamino)ethoxy]-5-(4,4,5,5-tetramethyl-1,3,2-dioxaborolan-2-yl)benzoate

Reaction of 1.03 g (3.26 mmols) of the title compound of Preparation 91 with 1.65 g (6.50 mmol) of bis(pinacolato)diboron according to the method described in Preparation 16 gave, after purification by SP1® automated purification system, 270 mg (23%) of the title compound.
HPLC/MS (10 min) retention time 3.92 min
LRMS: *m*/*z* 364 (M+1)

### PREPARATION 92

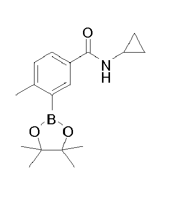

### N-cyclopropyl-4-methyl-3-(4,4,5,5-tetramethyl-1,3,2-dioxaborolan-2-yl)benzamide

A mixture of 4-methyl-3-(4,4,5,5-tetramethyl-1,3,2-dioxaborolan-2-yl)benzoic acid (300mg, 1.14mmol), cyclopropanamine (78.41mg, 1.37mmol), HATU (417mg, 1.10mmol) and DIEA (438 I, 2.2mmol) in DMF (3ml) was stirred overnight at r.t.

The solvent was removed under reduced pressure and the residue dissolved in 4% sodium bicarbonate solution. The aqueous phase was extracted three times with ethyl acetate. The combined organic layers were washed with water and brine, and dried over anhydrous sodium sulphate. Solvent was removed in vacuum to give 176mg of the title compound after triturating with isopropyl eter. Yield = 51 %
HPLC/MS (9 min) retention time 6.43 min.
LRMS: *m*/*z* 302 (M+1)

### PREPARATION 93

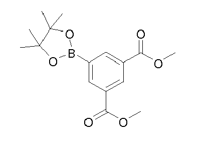

### Dimethyl 5-(4,4,5,5-tetramethyl-1,3,2-dioxaborolan-2-yl)isophthalate

Reaction of 2.00 g (7.32 mmols) of dimethyl 5-bromoisophthalate with 3.72 g (14.65 mmol) bis(pinacolato)diboron according to the method described in Preparation 16 gave 2.10 g (Purity: 94%; Yeld:84%) of the title compound.
HPLC/MS (10 min) retention time 7.08 min
LRMS: *m*/*z* 320 (M+1)

### PREPARATION 94

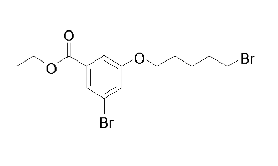

### Ethyl 3-bromo-5-[(5-bromopentyl)oxy]benzoate

1.0 g of the title compound of Preparation 66 (4.08 mmol) was dissolved in 15 mL dimethylformamide. 2.82 g of potassium carbonate (20.40 mmol) and 1.11 mL 1,5-dibromopentane (8.16 mmol) were added and the mixture stirred 1 hour at room temperature. The mixture was then partitioned between water and ethyl ether. The organic phase was washed twice with water, twice with brine and dried over magnesium sulphate. Evaporation gave 2.41 g (100%) of the title compound. Used without further purification.
HPLC/MS (10 min) retention time 7.83 min
LRMS: *m*/*z* 412 (M+18)

### PREPARATION 95

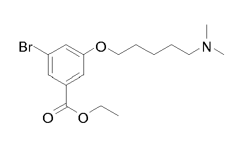

### Ethyl 3-bromo-5-{[5-(dimethylamino)pentyl]oxy}benzoate

2.41 g of the title compound of Preparation 94 (4.28 mmol) was dissolved in 15 mL dimethylformamide. 0.89 g of potassium carbonate (6.44 mmol) and 3.21 mL N-methylmethanamine (6.42 mmol) were added and the mixture heated at 70 °C for 48h hours. The mixture was allowed to cool and partitioned between water and ethyl ether. The organic phase was washed twice with water, twice with brine and dried over magnesium sulphate. Evaporation gave 0.51 g of the title compound after purification by SP1® automated purification system.
HPLC/MS (10 min) retention time 5.03 min
LRMS: *m*/*z* 359 (M+1)
1H NMR (200 MHz, CHLOROFORM-*d*) d ppm 1.39 (t, *J*=7.03 Hz, 3 H) 1.48 - 1.64 (m, 2 H) 1.71 - 1.93 (m, 4 H) 2.70 (s, 6 H) 2.85 - 2.97 (m, 2 H) 3.99 (t, *J*=6.05 Hz, 2 H) 4.37 (q, *J*=7.03 Hz, 2 H) 7.19 - 7.24 (m, 1 H) 7.45 - 7.50 (m, 1 H) 7.73 - 7.78 (m, 1 H)

### PREPARATION 96

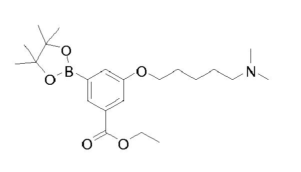

### Ethyl 3-{[5-(dimethylamino)pentyl]oxy}-5-(4,4,5,5-tetramethyl-1,3,2-dioxaborolan-2-yl)benzoate

Reaction of 508.7 mg (1.42 mmols) of the title compound of Preparation 95 with 540.85 mg (2.13 mmol) bis(pinacolato)diboron according to the method described in Preparation 16 gave 251.7 mg (44%) of the title compound after purification by SP1® automated purification system.
HPLC/MS (10 min) retention time 7.33 min
LRMS: *m*/*z* 406 (M+1)

### PREPARATION 97

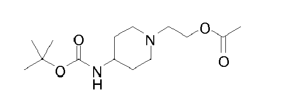

### 2-{4-[(tert-Butoxycarbonyl)amino]piperidin-1-yl}ethyl acetate

A mixture of *tert*-butyl piperidin-4-ylcarbamate (1.95 g, 10 mmol), potassium carbonate (11 g, 80 mmol) and 2-bromoethyl acetate (1.4 ml, 12.5 mmol) in 40 ml acetonitrile was stirred at reflux for 5 h and was then allowed to cool to room temperature and was left stirring for 3 d. The mixture was filtered and the solid washed with ethyl acetate. The combined filtrate was evaporated and the residue purified by column chromatography (MeOH-DCM (5:95), increasing to (10:90) to give 1.76 g (6.1 mmol, 63%) of the title compound as a white solid.
1H NMR (200 MHz, CHLOROFORM-*d*) ppm 4.45 (1 H, br. d, *J*=5.9 Hz), 4.14 (2 H, t, *J*=6.1 Hz), 3.27 - 3.58 (1 H, m), 2.82 (2 H, br. d, *J*=12.1 Hz), 2.58 (2 H, t, *J*=6.1 Hz), 2.09 - 2.23 (2 H, m), 2.03 (3 H, s), 1.89 (2 H, br. d, *J*=11.7 Hz), 1.50-1.30 (2 H, m), 1.41 (9 H, s).
LRMS: *m*/*z* 287 (M+1)

### PREPARATION 98

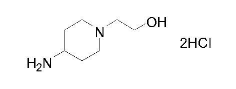

### 2-(4-Aminopiperidin-1-yl)ethanol dihydrochloride

The title compound from preparation 97 (1.7 g, 6.0 mmol) was dissolved in 10 ml dioxane. Hydrochloric acid (7 ml, 4M in dioxane, 28 mmol) was added and the mixture stirred at 95 °C for 1 h, precipitating a white solid. 5 ml 2N hydrochloric acid was added, dissolving the solid and the mixture was stirred at 95 °C for a further 3 h. The mixture was evaporated at 60° C for 2 h to give 1.31 g of the title compound.
1H NMR (200 MHz, DMSO-*d*₆) ppm 10.74 (1 H, br. s., minor isomer), 10.58 (1 H, br. s. major isomer), 8.75 (3 H, br. s., minor isomer), 8.62 (3 H, br. s., major isomer), 3.70 - 3.88 (2 H, m), 3.58 (2 H, d, *J*=11.7 Hz), 2.98 - 3.37 (5 H, m), 1.83 - 2.28 (4 H, m).
LRMS: *m*/*z* 145 (M+1)

### PREPARATION 99

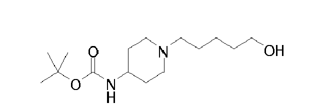

### 5-{4-[(tert-Butoxycarbonyl)amino]piperidin-1-yl}pentan-1-ol

tert-Butyl piperidin-4-ylcarbamate (1.98 g, 9.9 mmol) was dissolved in 40 ml acetonitrile. Potassium carbonate (11 g, 80 mmol) was added and the suspension stirred. 5-Bromopentyl acetate (2.0 ml, 12 mmol) was added and the mixture stirred at reflux for 5 h. The mixture was removed from the oil bath, 20 ml water was added and the mixture was left to stir overnight, cooling to room temperature. The mixture was evaporated to near-dryness, and the residue was diluted with brine. The aqueous was extracted three times with ethyl acetate, the combined organics were dried over sodium sulfate and evaporated. The residue was purified by column chromatography (MeOH-DCM gradient, 5:95, increasing 10:90, 15:85 and finally 20:80) to give 0.90 g (3.0 mmol, 30%) of the title compound.
1H NMR (200 MHz, CHLOROFORM-*d*) ppm 4.47 (1 H, d, *J*=6.2 Hz), 3.64 (2 H, t, *J*=6.2 Hz), 3.49 (1 H, br. s.), 2.89 (2 H, br. d, *J*=11.7 Hz), 2.38 (2 H, t, *J*=7.4 Hz), 1.86 - 2.19 (4 H, m), 1.48 - 1.68 (8 H, m), 1.44 (9 H, s).
LRMS: *m*/*z* 287 (M+1)

### PREPARATION 100

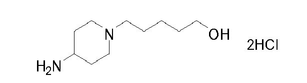

### 5-(4-aminopiperidin-1-yl)pentan-1-ol dihydrochloride

The title compound of preparation 99 (1.4 g, 4.7 mmol) was dissolved in 10 ml dioxane. Hydrochloric acid (8 ml, 4M in dioxane, 32 mmol) was added and the mixture stirred at 95 °C for 4 h, precipitating a white solid. The mixture was evaporated at 60° C for 2 h to give 1.23 g of the title compound.
1H NMR (200 MHz, DMSO-*d*₆) ppm 11.02* (1 H, br. s. minor isomer), 10.81* (1 H, br. s. major isomer), 8.61 - 8.75* (3 H, br. s. minor isomer), 8.53* (3 H, br. s. major isomer), 3.12 - 3.74 (6 H, m), 2.81 - 3.13 (4 H, m), 1.84 - 2.29 (2 H, m), 1.71 (2 H, br. s.), 1.16 - 1.56 (4 H, m).
* interchange with D2O

### PREPARATION 101

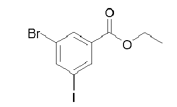

### Ethyl 3-bromo-5-iodobenzoate

Reaction of 2.00 g (6.12 mmols) of 3-bromo-5-iodobenzoic acid according to the method described in Preparation 66 gave 2.18 g (Purity: 87%; Yield: 85%) of the title compound. Used without further purification.
¹H NMR (200 MHz, CHLOROFORM-*d*) ppm 1.40 (t, *J*=7.03 Hz, 3 H) 4.38 (q, *J*=7.03 Hz, 2 H) 8.03 (t, *J*=1.76 Hz, 1 H) 8.13 (t, *J*=1.56 Hz, 1 H) 8.29 (t, *J*=1.37 Hz, 1 H)
HPLC/MS (10 min) retention time 3.54 min
LRMS: *m*/*z* 354 (M+1)

### PREPARATION 102

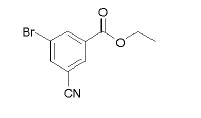

### Ethyl 3-bromo-5-cyanobenzoate

Reaction of 1.59g (4.48 mmol) the title compound of Preparation 101 following W0 02/068417 (page 79) gave 0.66 g (58% yield) of the title compound.
¹H NMR (200 MHz, DMSO-*d*₆) ppm 1.34 (t, *J*=7.03 Hz, 3 H) 4.36 (q, *J*=7.16 Hz, 2 H) 8.34 (s, 2 H) 8.48 (d, *J*=1.95 Hz, 1 H)
HPLC/MS (10 min) retention time 6.43 min
LRMS: *m*/*z* 254 (M+1)

### PREPARATION 103

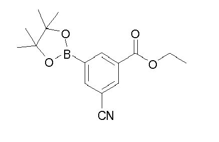

### Ethyl 3-cyano-5-(4,4,5,5-tetramethyl-1,3,2-dioxaborolan-2-yl)benzoate

Reaction of 660 mg (2.60 mmols) of the title compound of Preparation 102 with 1.32 g (5.20 mmol) bis(pinacolato)diboron according to the method described in Preparation 16 gave 650 mg (Yield:84%) of the title compound.
¹H NMR (200 MHz, DMSO-*d*₆) ppm 1.15 - 1.57 (m, 15 H) 4.37 (q, *J*=7.16 Hz, 2 H) 8.21 (s, 1 H) 8.44 (d, *J*=1.56 Hz, 2 H)
HPLC/MS (10 min) retention time 5.32 min
LRMS: *m*/*z* 301 (M+1)

### PREPARATION 104

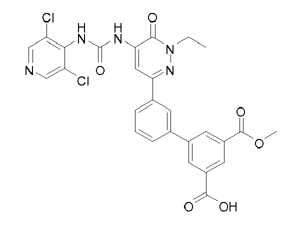

### 3'-[5-({[(3,5-dichloropyridin-4-yl)amino]carbonyl}amino)-1-ethyl-6-oxo-1,6-dihydropyridazin-3-yl]-5-(methoxycarbonyl)biphenyl-3-carboxylic acid

2.5 g of the title compound of Example 233 (4.19 mmols) and 0.09 g of lithium hydroxide (2.10 mmols) were dissolved in a mixture 1:1:1 water/THF/MeOH (30ml/30ml/30ml). The solution was stirred at room temperature during 2h, and then two additional portions of 0.09 g of lithium hydroxide were added during the next 4h.

The THF and the MeOH were evaporated under vacuum and the residue was diluted with water and acidified with 2N hydrochloric acid precipitating a solid, which was collected by filtration and dried in a stream of air. Obtaining 1.45 g (Purity: 64%; Yield: 38%) of the title compound after triturating with methanol.
HPLC/MS (15 min) retention time 9.02 min
LRMS: *m*/*z* 580 (M-)

### PREPARATION 105

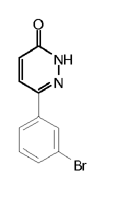

### 6-(3-bromophenyl)pyridazin-3(2H)-one

6.48 g of 1-(3-bromophenyl)ethanone (32.59 mmol) and 1.0 g of glyoxilic acid (10.86 mmol) were mixed neat and stirred at 105 °C for 3 h. The mixture was allowed to cool to around 40 °C and was diluted with 14 mL water and was basified to pH 8 with ammonium hydroxide. The aqueous phase was extracted with dichloromethane and the solid formed filtered off. 0.55 g of hydrazine monohydrate (11.07 mol) were added to the aqueous phase and the resulting mixture stirred at reflux overnight. The resulting precipitate was filtered and dried to yield 1.69 g (62%) of the title compound as a light brown solid.

### PREPARATION 106

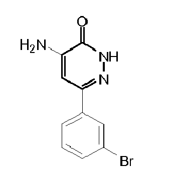

### 4-Amino-6-(3-bromophenyl)pyridazin-3(2H)-one

A mixture of 11.1 g (44.20 mmol) of the title compound of Preparation 105 and 110 mL of hydrazine monohydrate was heated at reflux for four days. After allowing the mixture to cool at room temperature, the resulting precipitate was collected by filtration and dried in a stream of air to yield 10.57 g (90 %) of the title compound as a pale yellow solid.
¹H NMR (200 MHz, DMSO-*d*₆) ppm 6.51 (bs, 2H), 6.73 (s, 1 H), 7.40 (t, 1 H), 7.58 (d, 1 H), 7.71 (d, 1 H), 7.88 (s, 1 H)

### PREPARATION 107

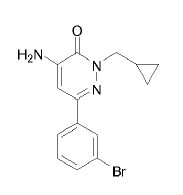

### 4-Amino-6-(3-bromophenyl)-2-(cyclopropylmethyl)pyridazin-3(2H)-one

To a suspension of 10.5 g (39.46 mmol) of the title compound of Preparation 106 in 100 mL of DMF were added 8.0 g (59.19 mmol) of (bromomethyl)cyclopropane and 1.9 g of sodium hydride and the resulting mixture was heated at 60° C under inert atmosphere for 2.5 h., then it was allowed to cool and the solvent removed under vacuo. The resulting residue was dissolved in ethyl acetate and washed with water. The organic phase was dried over sodium sulphate, filtered and evaporated to give 9.5 g (75%) of title compound as light brown solid.
¹H NMR (200 MHz, DMSO-*d*₆) ppm 0.47 (m, 4H), 1.31 (m, 1H), 3.97 (d, 2H), 6.58 (s, 2H), 6.74 (s, 1H), 7.42 (t, 1H), 7.59 (d, 1H), 7.74 (d, 1H), 7.91 (1H)

### PREPARATION 108

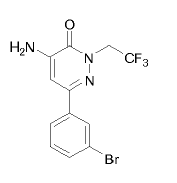

### 4-Amino-6-(3-bromophenyl)-2-(2,2,2-trifluoroethyl)pyridazin-3(2H)-one

Reaction of the title compound of Preparation 106 (6.63 g, 24.91 mmol) with 2,2,2-trifluoroethyl 4-methylbenzenesulfonate (10.77 g, 42.35 mmol) according to the method described in Preparation 107 gave 4.58 g (53%) of the title compound.
¹H NMR (200 MHz, DMSO-*d*₆) ppm 4.99 (m, 2H), 6.75 (s, 1H), 6.82 (bs, 2H), 7.44 (t, 1H), 7.63 (d, 1H), 7.72 (d, 1H), 7.91 (bs, 1H).

### PREPARATION 109

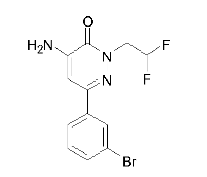

### 4-Amino-6-(3-bromophenyl)-2-(2,2-difluoroethyl)pyridazin-3(2H)-one

Reaction of the title compound of Preparation 106 (3.70 g, 13.90 mmol) with 1,1-difluoro-2-iodoethane (4.0 g, 20.8 mmol) according to the method described in Preparation 107 gave 4.4 g (98%) of the title compound.
¹H NMR (200 MHz, DMSO-*d*₆) ppm 4.55 (td, 2H), 6.46 (tt, 1H), 6.75 (s, 3H), 7.43 (t, 1H), 7.62 (d, 1H), 7.74 (d, 1H), 7.94 (bs, 1H).

### PREPARATION 110

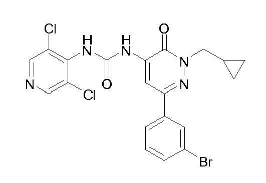

### N-[6-(3-Bromophenyl)-2-(cyclopropylmethyl)-3-oxo-2,3-dihydropyridazin-4-yl]-N'-(3,5-dichloropyridin-4-yl)urea

Reaction of the title compound of Preparation 107 (5 g, 15.62 mmol) with triphosgene (1.85g, 6.25 mmol), 4-amino-3,5-dichloropyridine (5.0 g, 31.24 mmol) and sodium hydride 1.90 g (47.35 mmol) according to the method described in Preparation 7 gave 3.1 g (36%) of the title compound.
¹H NMR (200 MHz, DMSO-*d*₆) ppm 9.89 (sa, 1H, NH), 9.72 (sa, 1H, NH), 8.69 (s, 2H, Ar-H), 8.32 (s, 1H, Ar-H), 7.93 (m, 1H, Ar-H), 7.77 (d, 7.4 Hz, 1H, Ar-H), 7.65 (d, 6.8 Hz, 1H, Ar-H), 7.45 (t, 8.0 Hz, 1H, Ar-H), 4.10 (d, 7.1 Hz, 2H, CH2), 1.35 (m, 1H, CH), 0.49 (m, 4H, CH2)

### PREPARATION 111

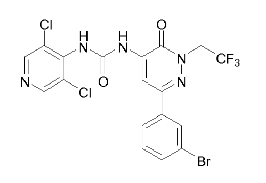

### N-[6-(3-Bromophenyl)-3-oxo-2-(2,2,2-trifluoroethyl)-2,3-dihydropyridazin-4-yl]-N'-(3,5-dichloropyridin-4-yl)urea

Reaction of the title compound of Preparation 108 (4.58 g, 13.15 mmol) with triphosgene (1.56g, 5.26 mmol), 4-amino-3,5-dichloropyridine (4.29 g, 26.31 mmol) and sodium hydride (1.31, 32.89 mmol) according to the method described in Preparation 7 gave 0.64 g (9%) of the title compound.

### PREPARATION 112

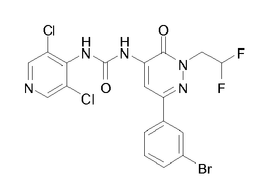

### 1-(6-(3-Bromophenyl)-2-(2,2-difluoroethyl)-3-oxo-2,3-dihydropyridazin-4-yl)-3-(3,5-dichloropyridin-4-yl)urea

Reaction of the title compound of Preparation 109 (4.34 g, 13.90 mmol) with triphosgene (1.56g, 6.25 mmol), 4-amino-3,5-dichloropyridine (4.28 g, 26.29 mmol) and sodium hydride (1.31 g, 32.86) according to the method described in Preparation 7 gave 475 mg (7%) of the title compound.

### PREPARATION 113

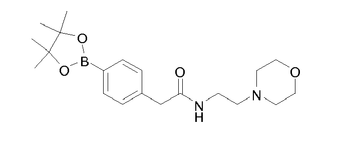

### N-(2-morpholinoethyl)-2-(4-(4,4,5,5-tetramethyl-1,3,2-dioxaborolan-2-yl)phenyl)acetamide

1. To a solution of 2-(4-bromophenyl)acetic acid (1.5 g, 6.98 mmol) in DMF (15 mL) under inert atmosphere were added 2-morpholinoethanamine (1.82 g, 13.95 mmol), 1-ethyl-3-(3-dimethylaminopropyl)carbodiimide hydrochloride (2.04 g, 10.43 mmol) and N-hydroxybenzotriazole (1.41 g, 10.47 mmol). The resulting mixture was stirred for 4 h. After removing the solvent under vacuo, water (15 mL) was added and the mixture extracted with ethyl acetate (3 x 15 mL). The combined organic fractions were dried and concentrated in vacuo to yield a residue which was purified by flash column chromatography (CH2CI2-MeOH 95:5) to give 1.85 g (81%) of 2-(4-bromophenyl)-N-(2-morpholinoethyl)acetamide.
   ¹H NMR (200 MHz, DMSO-*d*₆) ppm 1.74 (s, 2H), 2.36 (m, 6H), 3.27 (m, 2H), 3.53 (m, 4H), 5.98 (s, 1H), 7.14 (d, 2H), 7.47 (d, 2H).
2. Reaction of 2-(4-bromophenyl)-N-(2-morpholinoethyl)acetamide (1.0 g, 3.05 mmol) with bis(pinacolato)diboron (1.55 g, 6.11 mmol) according to the method described in Preparation 16 gave 451 mg (40%) of the title compound.

### PREPARATION 114

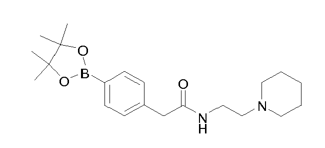

### N-(2-(piperidin-1-yl)ethyl)-2-(4-(4,4,5,5-tetramethyl-1,3,2-dioxaborolan-2-yl)phenyl)acetamide

Reaction of 2-(4-bromophenyl)acetic acid (1.0 g, 4.65 mmol) with 2-(piperidin-1-yl)ethanamine (1.19 g, 9.30 mmol) according to the method described in Preparation 113-1 gave 1.44 g mg (95%) of 2-(4-bromophenyl)-N-(2-(piperidin-1-yl)ethyl)acetamide.
¹H NMR (200 MHz, DMSO-*d*₆) ppm 1.46 (m, 6H), 2.38 (m, 6H), 3.29 (m, 2H), 3.51 (m, 2H), 6.46 (s, 1H), 7.15 (d, 2H), 7.46 (d, 2H).

Reaction of 2-(4-bromophenyl)-N-(2-(piperidin-1-yl)ethyl)acetamide (2.94 g, 9.04 mmol) with bis(pinacolato)diboron (4.59 g, 18.08 mmol) according to the method described in Preparation 16 gave 1.0 g mg (30%) of the title compound.
¹H NMR (200 MHz, DMSO-*d*₆) ppm 1.46 (m, 6H), 2.38 (m, 6H), 3.29 (m, 2H), 3.51 (m, 2H), 6.46 (s, 1H), 7.15 (d, 2H), 7.46 (d, 2H).

### PREPARATION 114

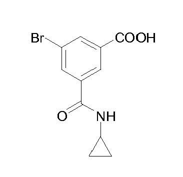

### 3-bromo-5-[(cyclopropylamino)carbonyl]benzoic acid

To a solution of the title compound of Preparation 59 (5.46 g) in THF (100 mL) was added an aqueous solution of lithium hydroxyde 2M (15 mL, 29.30 mmol). The resulting mixture was stirred at room temperature for 3 h. After neutralization with aqueous 10% HCI solution, it was extracted with ethyl acetate (50 mL). The aqueous phase is then acidified to pH = 1-2 with aqueous 10 % HCI and the resulting precipitate is collected by filtration under vacuo, washed with water and then dried (40 °C, 40 mbar, 8h) to yield 4.9 g (94%) of the title compound as a white solid.

### PREPARATION 115

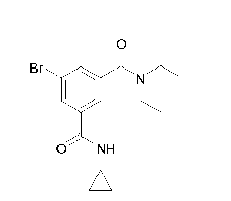

### 5-bromo-N¹-cyclopropyl-N³,N³-diethylisophthalamide

Reaction of the title compound of Preparation 114 (1.0 g, 3.52 mmol) with diethylamine (0.73 mL, 7.04 mmol) according to the method described in Preparation 113.1 gave 0.80 g (67%) of of the title product as a white solid.

### PREPARATION 116

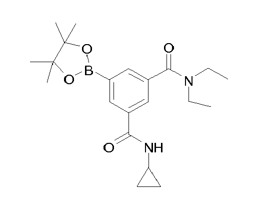

### N¹-cyclopropyl-N³,N³-diethyl-5-(4,4,5,5-tetramethyl-1,3,2-dioxaborolan-2-yl)isophthalamide

Reaction of the title compound of Preparation 115 (0.80 g, 2.36 mmol) with bis(pinacolato)diboron (1.20 g, 4.72 mmol) according to the method described in Preparation 16 gave 0.79 g mg (86%) of the title compound as a brown solid.

### PREPARATION 117

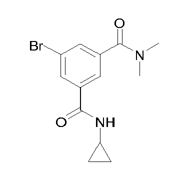

### 5-bromo-N¹-cyclopropyl-N³,N³-dimethylisophthalamide

Reaction of the title compound of Preparation 114 (1.0 g, 3.52 mmol) with a solution of dimethylamine 2.0 M in tetrahydrofuran (3.52 mL, 7.04 mmol) according to the method described in Preparation 113.1 gave 0.70 g (63%) of of the title product as a white solid.

### PREPARATION 118

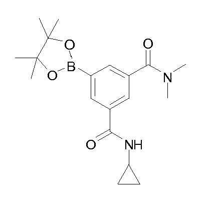

Reaction of the title compound of Preparation 117 (0.69 g, 2.23 mmol) with bis(pinacolato)diboron (1.13 g, 4.47 mmol) according to the method described in Preparation 16 gave 0.44 g mg (55%) of the title compound as a brown solid.

### PREPARATION 119

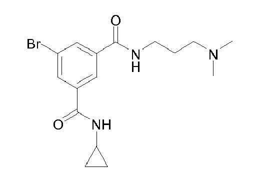

### 5-bromo-N1-cyclopropyl-N3-(3-(dimethylamino)propyl)isophthalamide

Reaction of the title compound of Preparation 114 (1.0 g, 3.52 mmol) with N¹,N¹-dimethylpropane-1,3-diamine (3.52 mL, 7.04 mmol) according to the method described in Preparation 113.1 gave 0.77 g (60%) of of the title product as a yellow solid.

### PREPARATION 120

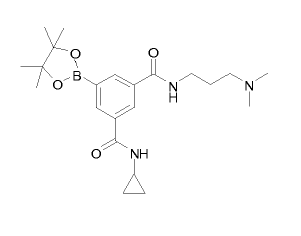

### 5-bromo-N1-cyclopropyl-N3-(3-(dimethylamino)propyl)isophthalamide

Reaction of the title compound of Preparation 119 (0.77 g, 2.29 mmol) with bis(pinacolato)diboron (1.06 g, 4.18 mmol) according to the method described in Preparation 16 gave 0.9 g of of the title product as a brown solid.

### EXAMPLE 1

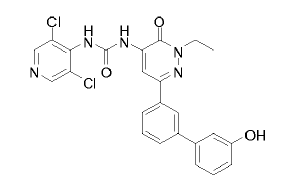

### N-(3,5-Dichloropyridin-4-yl)-N'-[2-ethyl-6-(3'-hydroxybiphenyl-3-yl)-3-oxo-2,3-dihydropyridazin-4-yl]urea

In a Schlenk tube, the title compound of Preparation 7 (3.00 g, 6.21 mmol) and 3-(4,4,5,5-tetramethyl-1,3,2-dioxaborolan-2-yl)phenol (2.05 g, 6.21 mmol) were dissolved in dioxane (100 mL). Caesium carbonate solution (2M, 9.31 mL, 18.6 mmol) was added and the mixture was purged (vacuum-argon three times). [1,1-Bis(diphenylphosphino)-ferrocene]dichloropalladium(II)dichloromethane complex (0.30 g, 0.37mmol) was added and the mixture purged again (vacuum-argon three times). The mixture was stirred at 90 °C for 3 h.

The reaction mixture was cooled to room temperature, diluted with water and extracted three times with ethyl acetate. The combined organics were washed with water and brine, and dried over anhydrous sodium sulphate. The solvent was removed in vacuum and the residue was purified by the SP1® automated purification system to give 1.26 g (2.54 mmol, 41 %) of the title compound.
¹H NMR (400 MHz, DMSO-*d*₆) ppm 9.48 - 9.80 (m, 3 H), 8.69 (s, 2 H), 8.40 (s, 1 H), 7.93 (s, 1 H), 7.76 (d, *J*=7.83 Hz, 1 H), 7.68 (d, *J*=8.22 Hz, 1 H), 7.57 (t, *J*=7.63 Hz, 1 H), 7.29 (t, *J*=7.83 Hz, 1 H), 7.10 (d, *J*=8.22 Hz, 1 H), 7.01 - 7.07 (m, 1 H), 6.80 (dd, *J*=8.22, 1.57 Hz, 1 H), 4.28 (q, *J*=7.43 Hz, 2 H), 1.39 (t, *J*=7.24 Hz, 3 H)
HPLC/MS (30 min) retention time 15.94 min
LRMS: *m*/*z* 496 (M+)

### EXAMPLE 2

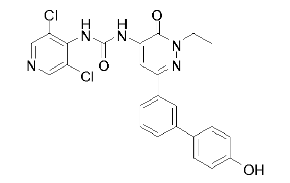

### N-(3,5-Dichloropyridin-4-yl)-N'-[2-ethyl-6-(4'-hydroxybiphenyl-3-yl)-3-oxo-2,3-dihydropyridazin-4-yl]urea

Reaction of the title compound of Preparation 7 (200 mg, 0.41 mmol) with 4-hydroxyphenylboronic acid (85 mg, 0.62 mmol) according to the method described in Example 1 gave 30 mg (15%) of the title compound after crystallization with ethanol.
¹H NMR (200 MHz, DMSO-*d*₆) ppm 1.47 (t, *J*=6.83 Hz, 3 H), 4.35 (q, *J*=6.77 Hz, 2 H), 6.93 (d, *J*=8.20 Hz, 2 H), 7.23 - 7.63 (m, 5 H), 7.73 (d, *J*=7.42 Hz, 1 H), 7.97 (br. s., 1 H), 8.52 (s, 2 H), 8.74 (s, 1 H), 9.58 (d, *J*=19.13 Hz, 2 H)
HPLC/MS (30 min) retention time 17.33 min
LRMS: *m*/*z* 496 (M+)

### EXAMPLE 3

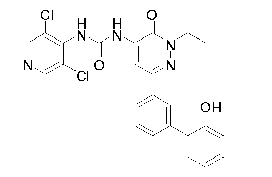

### N-(3,5-Dichloropyridin-4-yl)-N'-[2-ethyl-6-(2'-hydroxybiphenyl-3-yl)-3-oxo-2,3-dihydropyridazin-4-yl]urea

Reaction of the title compound of Preparation 7 (200 mg, 0.41 mmol) with 2-(4,4,5,5-tetramethyl-1,3,2-dioxaborolan-2-yl)phenol (61 mg, 0.28 mmol) according to the method described in Example 1 gave 50 mg (34.5%) of the title compound after crystallization with ethanol.
¹H NMR (200 MHz, DMSO-*d*₆) ppm 1.38 (t, *J*=6.83 Hz, 3 H), 4.26 (q, *J*=6.90 Hz, 2 H), 6.80 - 7.06 (m, 2 H), 7.10 - 7.36 (m, 2 H), 7.44 - 7.67 (m, 2 H), 7.73 (d, *J*=7.03 Hz, 1 H), 7.95 (s, 1 H), 8.39 (s, 1 H), 8.69 (s, 2 H), 9.61 (s, 1 H), 9.69 (s, 1 H), 9.90 (s, 1 H)
HPLC/MS (30 min) retention time 18.12 min
LRMS: *m*/*z* 496 (M+)

### EXAMPLE 4

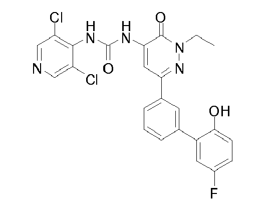

### N-(3,5-Dichloropyridin-4-yl)-N'-[2-ethyl-6-(5'-fluoro-2'-hydroxybiphenyl-3-yl)-3-oxo-2,3-dihydropyridazin-4-yl]urea

Reaction of the title compound of Preparation 7 (2.69 g, 5.40 mmol) with 2-(5,5-dimethyl-1,3,2-dioxaborinan-2-yl)-4-fluorophenol (3.3 g, 8.10 mmol) according to the method described in Example 1 gave 1.65 g (59%) of the title compound after crystallization with dichloromethane-methanol (6:4).
¹H NMR (400 MHz, DMSO-*d*₆) ppm 1.37 (t, *J*=7.04 Hz, 3 H), 4.26 (q, *J*=7.04 Hz, 2 H), 6.87 - 7.22 (m, 4 H), 7.50 - 7.66 (m, 2 H), 7.75 (d, *J*=7.83 Hz, 1 H), 7.97 (s, 1 H), 8.38 (s, 1 H), 8.68 (s, 2 H), 9.67 (br. s., 2 H)
HPLC/MS (30 min) retention time 18.31 min
LRMS: *m*/*z* 514 (M+)

### EXAMPLE 5

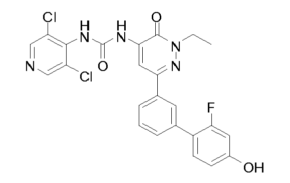

### N-(3,5-Dichloropyridin-4-yl)-N'-[2-ethyl-6-(2'-fluoro-4'-hydroxybiphenyl-3-yl)-3-oxo-2,3-dihydropyridazin-4-yl]urea

Reaction of 0.30 g of the title compound of Preparation 7 with 0.37 g of the title compound of Preparation 19 according to the method described in Example 1 gave 0.075 g (37%) of the title compound.
¹H NMR (200 MHz, DMSO-*d*₆) ppm 1.4 (m, 3 H), 4.3 (m, 2 H), 6.7 (m, 2 H), 7.4 (t, J=9.0 Hz, 1 H), 7.6 (d, *J*=5.1 Hz, 2 H), 7.7 (m, 1 H), 7.8 (s, 1 H), 8.4 (s, 1 H), 8.7 (s, 2 H), 9.7 (s, 1 H), 9.9 (s, 1 H), 10.1 (s, 1 H)
HPLC/MS (30 min) retention time 17.81 min
LRMS: m/z 514 (M+)

### EXAMPLE 6

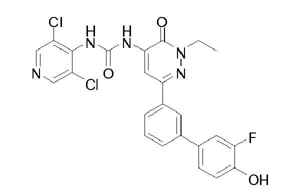

### N-(3,5-Dichloropyridin-4-yl)-N'-[2-ethyl-6-(3'-fluoro-4'-hydroxybiphenyl-3-yl)-3-oxo-2,3-dihydropyridazin-4-yl]urea

Reaction of 0.25 g of the title compound of Preparation 7 with 0.22 g of the title compound of Preparation 37 according to the method described in Example 1 and purification by chromatography (methanol-dichloromethane, 5:95) gave 0.04 g (16%) of the title compound.
¹H NMR (200 MHz, DMSO-*d*₆) ppm 10.07 (1 H, s), 9.91 (1 H, br. s.), 9.69 (1 H, s), 8.70 (2 H, s), 8.38 (1 H, s), 7.93 (1 H, s), 7.70 (2 H, d), 7.44 - 7.62 (2 H, m), 7.36 (1 H, d, *J*=8.2 Hz), 7.06 (1 H, t, *J*=8.8 Hz), 4.28 (2 H, q, *J*=7.0 Hz), 1.39 (3 H, t, *J*=7.2 Hz)
HPLC/MS (30 min) retention time 17.73 min.
LRMS: *m*/*z* 514 (M+)

### EXAMPLE 7

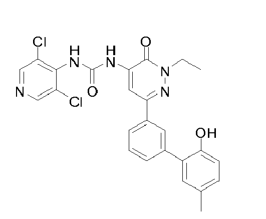

### N-(3,5-dichloropyridin-4-yl)-N'-[2-ethyl-6-(2'-hydroxy-5'-methylbiphenyl-3-yl)-3-oxo-2,3-dihydropyridazin-4-yl]urea

Reaction of 0.20 g of the title compound of Preparation 7 with 0.095 g of the title compound of Preparation 38 according to the method described in Example 1 and purification first by chromatography (methanol-dichloromethane, 2:98) and then by the SP1® automated purification system gave 0.11 g (50%) of the title compound.
1H NMR (200 MHz, DMSO-*d*₆) ppm 9.92 (1 H, br. s.), 9.65 (1 H, br. s.), 9.35 (1 H, s), 8.68 (2 H, s), 8.38 (1 H, s), 7.93 (1 H, s), 7.72 (1 H, d, *J*=7.0 Hz), 7.41 - 7.65 (2 H, m), 7.09 (1 H, s), 6.99 (1 H, dd, *J*=8.2, 1.5 Hz), 6.84 (1 H, d, *J*=8.2 Hz), 4.26 (2 H, q, *J*=6.9 Hz), 2.24 (3 H, s), 1.37 (3 H, t, *J*=7.0 Hz)
HPLC/MS (30 min) retention time 18.67 min
LRMS: *m*/*z* 510 (M+)

### EXAMPLE 8

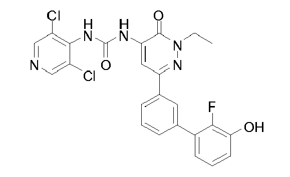

### N-(3,5-Dichloropyridin-4-yl)-N'-[2-ethyl-6-(2'-fluoro-3'-hydroxybiphenyl-3-yl)-3-oxo-2,3-dihydropyridazin-4-yl]urea

Reaction of 0.25 g of the title compound of Preparation 7 with 0.125 g of the title compound of Preparation 17 according to the method described in Example 1 and purification by the SP1® automated purification system gave 0.025 g (9%) of the title compound.
¹H NMR (200 MHz, DMSO-*d*₆) ppm 9.99 (2 H, br. s.), 9.67 (1 H, br. s.), 8.69 (2 H, s), 8.39 (1 H, s), 7.88 (1 H, s), 7.82 (1 H, t, *J*=4.5 Hz), 7.52 - 7.66 (2 H, m), 6.85 - 7.17 (3 H, m), 4.26 (2 H, q, *J*=7.0 Hz), 1.37 (3 H, t, *J*=7.0 Hz)
HPLC/MS (30 min) retention time 17.77 min
LRMS: *m*/*z* 514 (M+)

### EXAMPLE 9

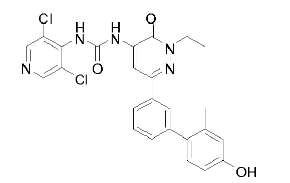

### N-(3,5-Dichloropyridin-4-yl)-N'-[2-ethyl-6-(4'-hydroxy-2'-methylbiphenyl-3-yl)-3-oxo-2,3-dihydropyridazin-4-yl]urea

Reaction of 0.25 g of the title compound of Preparation 7 with 0.20 g of the title compound of Preparation 39 according to the method described in Example 1 and purification by the SP1® automated purification system gave 0.06 g (24%) of the title compound.
¹H NMR (200 MHz, DMSO-*d*₆) ppm 9.89 (1 H, br. s.), 9.68 (1 H, s), 9.42 (1 H, s), 8.69 (2 H, s), 8.37 (1 H, s), 7.72 (1 H, d, *J*=7.8 Hz), 7.63 (1 H, s), 7.52 (1 H, t, *J*=7.6 Hz), 7.37 (1 H, d, *J*=7.4 Hz), 7.05 (1 H, d, *J*=7.8 Hz), 6.60 - 6.78 (2 H, m), 4.25 (2 H, q, *J*=7.0 Hz), 2.17 (3 H, s), 1.37 (3 H, t, *J*=7.0 Hz)
HPLC/MS (30 min) retention time 17.88 min
LRMS: *m*/*z* 510 (M+)

### EXAMPLE 10

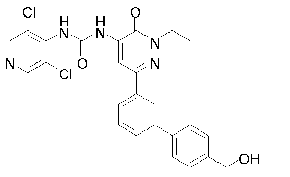

### N-(3,5-dichloropyridin-4-yl)-N'-{2-ethyl-6-[4'-(hydroxymethyl)biphenyl-3-yl]-3-oxo-2,3-dihydropyridazin-4-yl}urea

Reaction of 0.25 g of the title compound of Preparation 7 with 0.12 g 4-(hydroxymethyl)phenylboronic acid according to the method described in Example 1 and purification by the SP1® automated purification system gave 0.05 g (20%) of the title compound.
¹H NMR (200 MHz, DMSO-*d*₆) ppm 9.69 (2 H, br. s.), 8.69 (2 H, s), 8.40 (1 H, s), 7.99 (1 H, s), 7.52 - 7.83 (5 H, m), 7.44 (2 H, d, *J*=8.2 Hz), 5.25 (1 H, br. s.), 4.55 (2 H, d, *J*=3.9 Hz), 4.28 (2 H, q, *J*=7.0 Hz), 1.39 (3 H, t, *J*=7.0 Hz)
HPLC/MS (30 min) retention time 17.23 min
LRMS: *m*/*z* 510 (M+)

### EXAMPLE 11

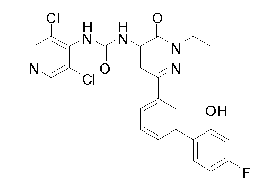

### N-(3,5-dichloropyridin-4-yl)-N'-[2-ethyl-6-(4'-fluoro-2'-hydroxybiphenyl-3-yl)-3-oxo-2,3-dihydropyridazin-4-yl]urea

Reaction of 0.20 g of the title compound of Preparation 7 with 0.10 g 4-fluoro-2-hydroxyphenylboronic acid according to the method described in Example 1 and purification by the SP1® automated purification system gave 0.06 g (28%) of the title compound.
¹H NMR (200 MHz, DMSO-*d*₆) ppm 10.19 (1 H, br. s.), 9.93 (1 H, br. s.), 9.68 (1 H, br. s.), 8.69 (2 H, s), 8.38 (1 H, s), 7.91 (1 H, s), 7.74 (1 H, d, *J*=6.2 Hz), 7.46 - 7.66 (2 H, m), 7.33 (1 H, t, *J*=7.8 Hz), 6.75 (2 H, d, *J*=9.8 Hz), 4.26 (2 H, q, *J*=6.9 Hz), 1.37 (3 H, t, *J*=7.0 Hz)
HPLC/MS (30 min) retention time 18.45 min
LRMS: *m*/*z* 514 (M+)

### EXAMPLE 12

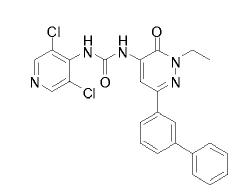

### N-(6-biphenyl-3-yl-2-ethyl-3-oxo-2,3-dihydropyridazin-4-yl)-N'-(3,5-dichloropyridin-4-yl)urea

Reaction of 0.25 g of the title compound of Preparation 7 with 0.095 g phenylboronic acid according to the method described in Example 1 and purification by the SP1® automated purification system gave 0.047 g (18%) of the title compound.
¹H NMR (200 MHz, CHLOROFORM-d) ppm 9.42 (1 H, s), 8.80 (1 H, br. s.), 8.79 (1 H, s), 8.71 (1 H, s), 8.62 (2 H, s), 8.04 (1 H, s), 7.82 (1 H, d, *J*=7.8 Hz), 7.34 - 7.70 (7 H, m), 4.26 (2 H, q, *J*=7.3 Hz), 1.29 (3 H, t, *J*=7.2 Hz)
HPLC/MS (30 min) retention time 20.09 min
LRMS: *m*/*z* 480 (M+)

### EXAMPLE 13

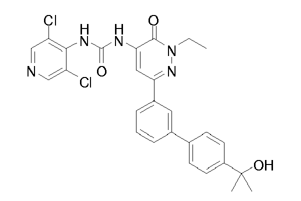

### N-(3,5-dichloropyridin-4-yl)-N'-{2-ethyl-6-[4'-(1-hydroxy-1-methylethyl)biphenyl-3-yl]-3-oxo-2,3-dihydropyridazin-4-yl}urea

Reaction of 0.10 g of the title compound of Preparation 7 with 0.10 of the title compound of Preparation 40 according to the method described in Example 1 and purification by the SP1® automated purification system gave 0.032 g (30%) of the title compound.
¹H NMR (200 MHz, DMSO-*d*₆) ppm 9.92 (1 H, br. s.), 9.67 (1 H, br. s.), 8.69 (2 H,s), 8.41 (1 H,s), 7.98 (1 H, s), 7.74 (2 H, t, *J*=6.6 Hz), 7.51 - 7.68 (5 H, m), 5.07 (1 H, s), 4.28 (2 H, q, *J*=6.6 Hz), 1.46 (6 H, s), 1.39 (3 H, t, *J*=7.2 Hz)
HPLC/MS (30 min) retention time 18.55 min
LRMS: *m*/*z* 538 (M+)

### EXAMPLE 14

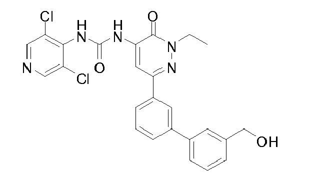

### N-(3,5-Dichloropyridin-4-yl)-N'-{2-ethyl-6-[3'-(hydroxymethyl)biphenyl-3-yl]-3-oxo-2,3-dihydropyridazin-4-yl}urea

Reaction of 0.25 g of the title compound of Preparation 7 with 0.11 g 4-(hydroxymethyl)phenylboronic acid according to the method described in Example 1 and purification by the SP1® automated purification system gave 0.03 g (12%) of the title compound.
¹H NMR (200 MHz, DMSO-*d*₆) ppm 9.92 (1 H, br. s.), 9.67 (1 H, br. s.), 8.69 (2 H, s), 8.40 (1 H, s), 7.99 (1 H, s), 7.75 (2 H, t, *J*=7.8 Hz), 7.51 - 7.67 (3 H, m), 7.45 (1 H, t, *J*=7.4 Hz), 7.30 - 7.40 (1 H, m), 5.27 (1 H, t, *J*=5.7 Hz), 4.58 (2 H, d, *J*=5.5 Hz), 4.28 (2 H, q, *J*=7.3 Hz), 1.39 (3 H, t, *J*=7.2 Hz)
HPLC/MS (30 min) retention time 17.43 min
LRMS: *m*/*z* 510 (M+)

### EXAMPLE 15

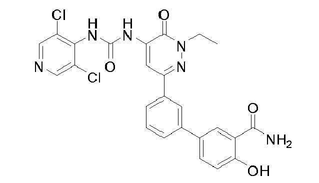

### 3'-[5-({[(3,5-Dichloropyridin-4-yl)amino]carbonyl}amino)-1-ethyl-6-oxo-1,6-dihydropyridazin-3-yl]-4-hydroxybiphenyl-3-carboxamide

Reaction of 0.500 g of the title compound of Preparation 7 with 0.256 g of the title compound of Preparation 15 according to the method described in Example 1 gave 0.018 g (4%) of the title compound.
¹H NMR (400 MHz, DMSO-*d*₆) ppm 1.39 (t, *J*=7.14 Hz, 3 H), 4.28 (q, *J*=7.14 Hz, 2 H), 7.01 (d, *J*=8.61 Hz, 1 H), 7.58 (t, *J*=7.63 Hz, 1 H), 7.69 - 7.82 (m, 3 H), 7.99 (s, 2 H), 8.22 (d, *J*=1.96 Hz, 1 H), 8.39 (s, 1 H), 8.62 (s, 1 H), 8.70 (s, 2 H), 9.70 (s, 1 H), 9.91 (s, 1 H), 13.16 (s, 1 H)
HPLC/MS (30 min) retention time 17.38min.
LRMS: *m*/*z* 539 (M+)

### EXAMPLE 16

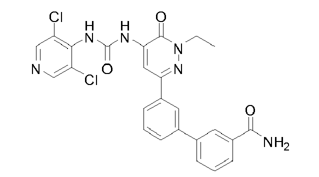

### 3'-[5-({[(3,5-Dichloropyridin-4-yl)amino]carbonyl}amino)-1-ethyl-6-oxo-1,6-dihydropyridazin-3-yl]biphenyl-3-carboxamide

Reaction of 0.400 g of the title compound of Preparation 7 with 0.204 g [3-(aminocarbonyl)phenyl]boronic acid according to the method described in Example 1 gave 0.055 g (13%) of the title compound after crystallisation with dichloromethane-methanol.
¹H NMR (400 MHz, DMSO-*d*₆) ppm 1.39 (t, *J*=7.24 Hz, 3 H), 4.29 (q, *J*=7.24 Hz, 2 H), 7.45 (s, 1 H), 7.53 - 7.67 (m, 2 H), 7.75 - 7.93 (m, 4 H), 8.05 (s, 1 H), 8.13 (s, 1 H), 8.19 (s, 1 H), 8.40 (s, 1 H), 8.70 (s, 2 H), 9.71 (s, 1 H), 9.90 (s, 1 H)
HPLC/MS (30 min) retention time 15.98 min.
LRMS: *m*/*z* 523 (M+)

### EXAMPLE 17

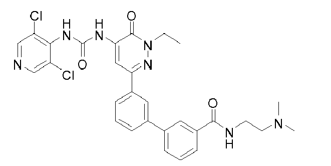

### 3'-[5-({[(3,5-Dichloropyridin-4-yl)amino]carbonyl}amino)-1-ethyl-6-oxo-1,6-dihydropyridazin-3-yl]-N-[2-(dimethylamino)ethyl]biphenyl-3-carboxamide

Reaction of 0.500 g of the title compound of Preparation 7 with 0.493 g of the title compound of Preparation 18 according to the method described in Example 1 gave 0.320 g (51 %) of the title compound.
¹H NMR (400 MHz, DMSO-*d*₆) ppm 1.39 (t, *J*=7.16 Hz, 3 H), 2.25 (s, 6 H), 3.32 - 3.48 (m, 2 H), 4.28 (q, *J*=7.16 Hz, 2 H), 7.53 - 7.68 (m, 2 H), 7.76 - 7.83 (m, 2 H), 7.86 (t, *J*=7.05 Hz, 2 H), 8.04 (s, 1 H), 8.14 (s, 1 H), 8.19 (s, 1 H), 8.40 (s, 1 H), 8.59 (t, *J*=5.60 Hz, 1 H), 8.69 (s, 2 H), 9.71 (s, 1 H)
HPLC/MS (30 min) retention time 12.32 min.
LRMS: *m*/*z* 594 (M+)

### EXAMPLE 18

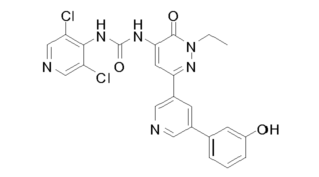

### N-(3,5-Dichloropyridin-4-yl)-N'-{2-ethyl-6-[5-(3-hydroxyphenyl)pyridin-3-yl]-3-oxo-2,3-dihydropyridazin-4-yl}urea

Reaction of 0.250 g of the title compound of the Preparation 14 with 0.170 g (3-hydroxyphenyl)boronic acid according to the method described in Example 1 gave 0.039 g (15.3%) of the title compound, after crystallisation with dichloromethane-methanol (7:3).
¹H NMR (400 MHz, DMSO-*d*₆) ppm 1.40 (t, *J*=7.04 Hz, 3 H), 4.29 (q, *J*=7.04 Hz, 2 H), 6.79 - 6.90 (m, 1 H), 7.11 (s, 1 H), 7.18 (d, *J*=8.12 Hz, 1 H), 7.32 (t, *J*=8.12 Hz, 1 H), 8.25 (t, *J*=2.25 Hz, 1 H), 8.40 (s, 1 H), 8.70 (s, 2 H), 8.88 (d, *J*=2.25 Hz, 1 H), 8.95 (d, *J*=1.96 Hz, 1 H), 9.68 (s, 1 H), 9.74 (s, 1 H), 9.91 (s, 1 H)
HPLC/MS (30 min) retention time 14.89 min.
LRMS: *m*/*z* 497 (M+)

### EXAMPLE 19

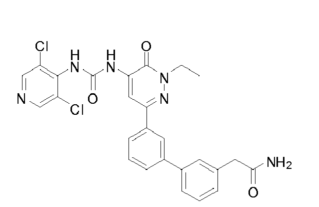

### 2-{3'-[5-({[(3,5-Dichloropyridin-4-yl)amino]carbonyl}amino)-1-ethyl-6-oxo-1,6-dihydropyridazin-3-yl]biphenyl-3-yl}acetamide

Reaction of 0.500 g of the title compound of Preparation 7 with 0.405 g of the title compound of Preparation 16 according to the method described in Example 1 gave 0.156 g (28%) of the title compound, after crystallisation with dichloromethane-methanol (7:3).
¹H NMR (400 MHz, DMSO-*d*₆) ppm 1.39 (t, *J*=7.14 Hz, 3 H), 3.46 (s, 2 H), 4.28 (q, *J*=7.14 Hz, 2 H), 6.90 (s, 1 H), 7.29 (d, *J*=7.43 Hz, 1 H), 7.42 (t, *J*=7.63 Hz, 1 H), 7.47 - 7.65 (m, 4 H), 7.71 (d, *J*=7.43 Hz, 1 H), 7.76 (d, *J*=7.43 Hz, 1 H), 7.98 (s, 1 H), 8.39 (s, 1 H), 8.70 (s, 2 H), 9.70 (s, 1 H), 9.91 (s, 1 H)
HPLC/MS (30 min) retention time 16.15 min.
LRMS: *m*/*z* 537 (M+)

### EXAMPLE 20

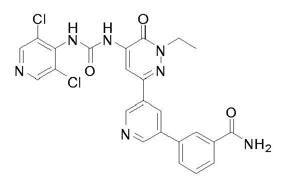

### 3-{5-[5-({[(3,5-Dichloropyridin-4-yl)amino]carbonyl}amino)-1-ethyl-6-oxo-1,6-dihydropyridazin-3-yl]pyridin-3-yl}benzamide

Reaction of 0.250 g of the title compound of Preparation 14 with 0.127 g [3-(aminocarbonyl)phenyl]boronic acid according to the method described in Example 1 gave 0.051 g (10%) of the title compound, after crystallisation with dichloromethane-methanol (7:3).
¹H NMR (400 MHz, DMSO-*d*₆) ppm 1.40 (t, *J*=7.44 Hz, 3 H), 4.30 (d, *J*=7.44 Hz, 2 H), 7.48 (s, 1 H), 7.57 - 7.68 (m, 1 H), 7.95 (d, *J*=7.83 Hz, 2 H), 8.14 (s, 1 H), 8.26 (s, 1 H), 8.41 (s, 2 H), 8.70 (s, 1 H), 8.98 (s, 1 H), 9.02 (s, 1 H), 9.74 (s, 1 H), 9.91 (s, 1 H)
HPLC/MS (30 min) retention time 13.73 min.
LRMS: *m*/*z* 524 (M+)

### EXAMPLE 21

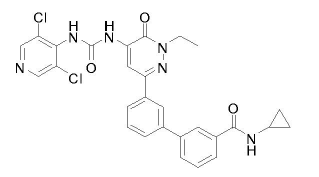

### N-Cyclopropyl-3'-[5-({[(3,5-dichloropyridin-4-yl)amino]carbonyl}amino)-1-ethyl-6-oxo-1,6-dihydropyridazin-3-yl]biphenyl-3-carboxamide

Reaction of 0.200 g of the title compound of Preparation 7 with 0.142 g N-cyclopropyl-3-(4,4,5,5-tetramethyl-1,3,2-dioxaborolan-2-yl)benzamide according to the method described in Example 1 gave 0.072 g (31%) of the title compound.
¹H NMR (400 MHz, DMSO-*d*₆) ppm 0.55 - 0.62 (m, 2 H), 0.66 - 0.75 (m, 2 H), 1.39 (t, *J*=7.04 Hz, 3 H), 2.79 - 2.99 (m, 1 H), 4.28 (q, *J*=7.04 Hz, 2 H), 7.53 - 7.66 (m, 2 H), 7.74 - 7.88 (m, 4 H), 8.03 (s, 1 H), 8.10 (s, 1 H), 8.40 (s, 1 H), 8.57 (d, *J*=4.30 Hz, 1 H), 8.70 (s, 2 H), 9.71 (s, 1 H), 9.90 (s, 1 H)
HPLC/MS (30 min) retention time 17.51 min.
LRMS: *m*/*z* 563 (M+)

### EXAMPLE 22

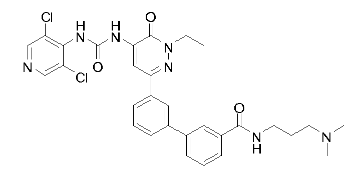

### 3'-[5-({[(3,5-Dichloropyridin-4-yl)amino]carbonyl}amino)-1-ethyl-6-oxo-1,6-dihydropyridazin-3-yl]-N-[3-(dimethylamino)propyl]biphenyl-3-carboxamide

Reaction of the title compound of Preparation 7 (252 mg, 0.51 mmol) with the title compound of Preparation 36 (252 mg, 0.76 mmol) according to the method described in Example 1 gave 130 mg (42%) of the title compound.
¹H NMR (200 MHz, DMSO-*d*₆) ppm 1.39 (t, *J*=7.03 Hz, 3 H), 1.69 (t, *J*=6.83 Hz, 2 H), 2.18 (s, 6 H), 2.34 (t, *J*=6.83 Hz, 2 H), 3.31 (q, *J*=6.51 Hz, 2 H), 4.28 (q, *J*=6.64 Hz, 2 H), 7.49 - 8.01 (m, 6 H), 8.04 (s, 1 H), 8.14 (s, 1 H), 8.29 (br. s., 1 H), 8.41 (s, 1 H), 8.68 (bs, 3 H)
HPLC/MS (30min) retention time 12.81 min
LRMS: *m*/*z* 608 (M+)

### EXAMPLE 23

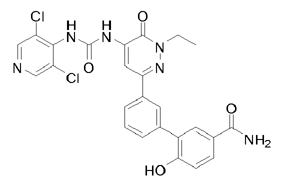

### 3'-[5-({[(3,5-Dichloropyridin-4-yl)amino]carbonyl}amino)-1-ethyl-6-oxo-1,6-dihydropyridazin-3-yl]-6-hydroxybiphenyl-3-carboxamide

Reaction of 0.300 g of the title compound of Preparation 7 with 0.245 g of the title compound of Preparation 28 according to the method described in Example 1 gave 0.057 g (17%) of the title compound.
¹H NMR (400 MHz, DMSO-*d*₆) ppm 1.37 (t, *J*=7.04 Hz, 3 H), 4.26 (q, *J*=7.04 Hz, 2 H), 7.00 (d, *J*=8.61 Hz, 1 H), 7.13 (s, 1 H), 7.55 (t, *J*=7.63 Hz, 1 H), 7.63 (d, *J*=7.83 Hz, 1 H), 7.72 - 7.78 (m, 2 H), 7.81 - 7.90 (m, 2 H), 7.98 (s, 1 H), 8.38 (s, 1 H), 8.69 (s, 2 H), 9.70 (s, 1 H), 9.94 (s, 1 H), 10.26 (s, 1 H)
HPLC/MS (30 min) retention time 13.74 min.
LRMS: *m*/*z* 539 (M+)

### EXAMPLE 24

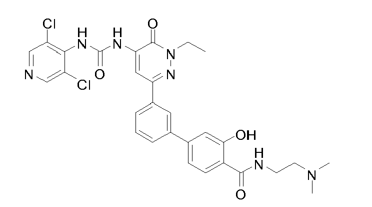

### 3'-[5-({[(3,5-Dichloropyridin-4-yl)amino]carbonyl}amino)-1-ethyl-6-oxo-1,6-dihydropyridazin-3-yl]-N-[2-(dimethylamino)ethyl]-3-hydroxybiphenyl-4-carboxamide

Reaction of 0.200 g of the title compound of Preparation 7 with 0.30 g of the title compound of Preparation 30 according to the method described in Example 1 gave 0.052 g (21 %) of the title compound.
¹H NMR (200 MHz, DMSO-*d*₆) ppm 1.39 (t, 3 H), 2.21 (s, 6 H), 3.32 - 3.52 (m, 2 H), 4.28 (q, 2 H), 7.18 - 7.29 (m, 2 H), 7.60 (t, *J*=7.81 Hz, 1 H), 7.72 - 7.85 (m, 2 H), 7.95 (d, *J*=8.20 Hz, 1 H), 8.01 (t, *J*=1.56 Hz, 1 H), 8.17 (s, 1 H), 8.40 (s, 1 H), 8.69 (s, 2 H), 8.82 (t, *J*=5.86 Hz, 1 H), 9.69 (s, 1 H)
HPLC/MS (30 min) retention time 12.8 min.
LRMS: *m*/*z* 610 (M+)

### EXAMPLE 25

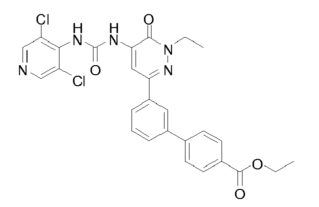

### Ethyl 3'-[5-({[(3,5-dichloropyridin-4-yl)amino]carbonyl}amino)-1-ethyl-6-oxo-1,6-dihydropyridazin-3-yl]biphenyl-4-carboxylate

Reaction of the title compound of Preparation 7 (350 mg, 0.72 mmol) with 4-(ethoxycarbonyl)phenyl boronic acid (211 mg, 1.09 mmol) according to the method described in Example 1 gave 190 mg (46%) of the title compound after triturating with diethyl ether.
HPLC/MS (9 min) retention time 7.71 min
LRMS: *m*/*z* 552 (M+1)

### EXAMPLE 26

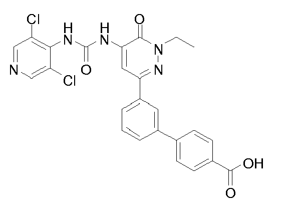

### 3'-[5-({[(3,5-Dichloropyridin-4-yl)amino]carbonyl}amino)-1-ethyl-6-oxo-1,6-dihydropyridazin-3-yl]biphenyl-4-carboxylic acid

A solution of lithium hydroxide (234 mg, 5.58 mmol) in 7 mL water was added drop-wise to a stirred solution of the title compound of Example 25 (880 mg, 1.12 mmol) in 19 mL methanol-tetrahydrofuran (2:1). The mixture was stirred at room temperature for 24 h, then diluted with water and acidified with 2N hydrochloric acid precipitating a solid, which was collected by filtration and dried in a stream of air. Crystallisation with dichloromethane-methanol (7:3) gave 440 mg (74.5%) of the title compound.
¹H NMR (200 MHz, DMSO-*d*₆) ppm 1.39 (t, 3 H), 4.28 (q, 2 H), 7.54 - 8.27 (m, 8 H), 8.41 (br. s., 1 H), 8.70 (br. s., 2 H), 9.71 (br. s., 1 H), 9.91 (br. s., 1 H), 12.99 (br. s., 1 H)
HPLC/MS (30 min) retention time 17.61 min
LRMS: m/z 524 (M+)

### EXAMPLE 27

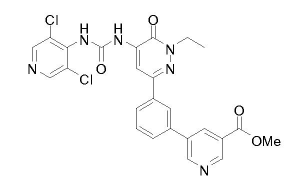

### Methyl 5-{3-[5-({[(3,5-dichloropyridin-4-yl)amino]carbonyl}amino)-1-ethyl-6-oxo-1,6-dihydropyridazin-3-yl]phenyl}nicotinate

Reaction of the title compound of Preparation 7 (500 mg, 1 mmol) with the title compound of Preparation 33 (396 mg, 1.51 mmol) according to the method described in Example 1 gave 160 mg (27%) of the title compound after crystallization with dichloromethane-methanol (8:2).
HPLC/MS (9 min) retention time 6.83 min
LRMS: *m*/*z* 537 (M-1)

### EXAMPLE 28

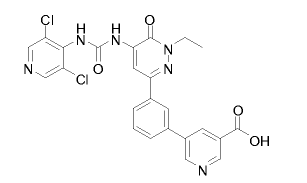

### 5-{3-[5-({[(3,5-Dichloropyridin-4-yl)amino]carbonyl}amino)-1-ethyl-6-oxo-1,6-dihydropyridazin-3-yl]phenyl}nicotinic acid

Reaction of the title compound of Example 27 (160 mg, 0.27 mmol) according to the method described in Example 26 gave 52 mg (36%) of the title compound.
¹H NMR (200 MHz, DMSO-*d*₆) ppm 1.39 (t, *J*=6.64 Hz, 3 H), 4.29 (q, 2 H), 7.56 - 7.78 (m, 1 H), 7.87 (d, *J*=7.03 Hz, 2 H), 8.10 (br. s., 1 H), 8.41 (br. s., 1 H), 8.49 (br. s., 1 H), 8.69 (s, 2 H), 9.09 (br. s., 1 H), 9.14 (br. s., 1 H), 9.71 (br. s., 1 H), 9.91 (br. s., 1 H)
HPLC/MS (30 min) retention time 15.39 min
LRMS: *m*/*z* 525 (M+)

### EXAMPLE 29

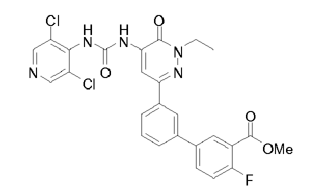

### Methyl 3'-[5-({[(3,5-dichloropyridin-4-yl)amino]carbonyl}amino)-1-ethyl-6-oxo-1,6-dihydropyridazin-3-yl]-4-fluorobiphenyl-3-carboxylate

Reaction of the title compound of Preparation 7 (535 mg, 1.07 mmol) with the title compound of Preparation 35 (1.0 g, 3.57 mmol) according to the method described in Example 1 gave 420 mg (47%) of the title compound after crystallization with dichloromethane-methanol (8:2).
HPLC/MS (9 min) retention time 7.54 min.
LRMS: *m*/*z* 556 (M+)

### EXAMPLE 30

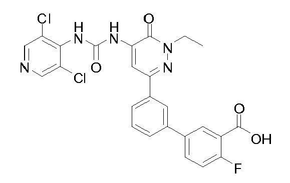

### 3'-[5-({[(3,5-Dichloropyridin-4-yl)amino]carbonyl}amino)-1-ethyl-6-oxo-1,6-dihydropyridazin-3-yl]-4-fluorobiphenyl-3-carboxylic acid

Reaction of the title compound of Example 29 (420 mg, 0.51 mmol) according to the method described in Example 26 gave 38mg (13.5%) of the title compound.
¹H NMR (200 MHz, DMSO-*d*₆) ppm 1.38 (t, *J*=7.03 Hz, 3 H), 4.28 (q, *J*=6.90 Hz, 2 H), 7.30 - 7.68 (m, 2 H), 7.68 - 8.18 (m, 6 H), 8.40 (s, 1 H) 8.70 (s, 2 H), 9.71 (br. s., 1 H)
HPLC/MS (30 min) retention time 15.39 min
LRMS: *m*/*z* 525 (M+)

### EXAMPLE 31

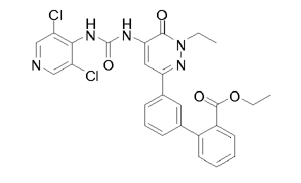

### Ethyl 3'-[5-({[(3,5-dichloropyridin-4-yl)amino]carbonyl}amino)-1-ethyl-6-oxo-1,6-dihydropyridazin-3-yl]biphenyl-2-carboxylate

Reaction of the title compound of Preparation 7 (700 mg, 1.41 mmol) with ethyl 2-(4,4,5,5-tetramethyl-1,3,2-dioxaborolan-2-yl)benzoate (600 mg, 2.11 mmol) according to the method described in Example 1 gave 440 mg (50%) of the title compound after triturating with diethyl ether.
HPLC/MS (9min) retention time 7.57 min.
LRMS: *m*/*z* 552 (M+1)

### EXAMPLE 32

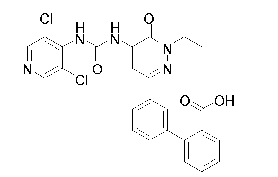

### 3'-[5-({[(3,5-Dichloropyridin-4-yl)amino]carbonyl}amino)-1-ethyl-6-oxo-1,6-dihydropyridazin-3-yl]biphenyl-2-carboxylic acid

Reaction of the title compound of Example 31 (440 mg, 0.71 mmol) according to the method described in Example 26 gave 130 mg (34%) of the title compound.
¹H NMR (200 MHz, DMSO-*d*₆) ppm 1.37 (t, 3 H), 4.25 (q, 2 H), 7.25 - 7.99 (m, 8 H), 8.38 (br. s., 1 H), 8.69 (br. s., 2 H), 9.71 (br. s., 1 H), 9.90 (br. s., 1 H), 12.81 (br. s., 1 H)
HPLC/MS (30 min) retention time 17.28 min
LRMS: *m*/*z* 524 (M+)

### EXAMPLE 33

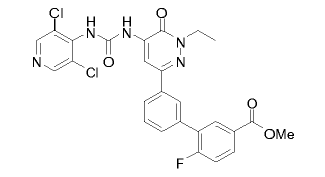

### Methyl 3'-(5-(3-(3,5-dichloropyridin-4-yl)ureido)-1-ethyl-6-oxo-1,6-dihydropyridazin-3-yl)-6-fluorobiphenyl-3-carboxylate

Reaction of 1.00 g of the title compound of Preparation 7 with 1.40 g of title compound of Preparation 20 according to the method described in Example 1 gave 0.55 g (48%) of the title compound.
HPLC/MS (9 min) retention time 7.53 min
LRMS: m/z 556 (M+)

### EXAMPLE 34

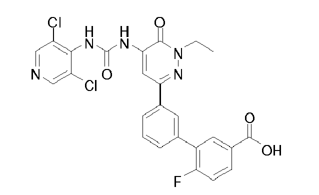

### 3'-[5-({[(3,5-Dichloropyridin-4-yl)amino]carbonyl}amino)-1-ethyl-6-oxo-1,6-dihydropyridazin-3-yl]-6-fluorobiphenyl-3-carboxylic acid

Reaction of the title compound of Example 33 (550 mg, 0.99 mmol) according to the method described in Example 26 and crystallisation from ethanol-chloroform (1:1) gave 274 mg (51%) of the title compound.
¹H NMR (200 MHz, DMSO-*d*₆) δ ppm 1.4 (t, *J*=7.0 Hz, 3 H), 4.3 (q, *J*=7.0 Hz, 2 H), 7.5 (m, 1 H), 7.6 (m, 2 H), 8.0 (m, 4 H), 8.4 (s, 1 H), 8.7 (s, 2 H), 9.7 (s, 1 H), 9.9 (s, 1 H), 13.2 (s, 1 H)
HPLC/MS (30 min) retention time 17.87 min.
LRMS: *m*/*z* 542 (M+)

### EXAMPLE 35

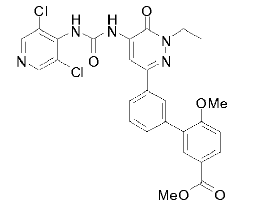

### Methyl 3'-(5-(3-(3,5-dichloropyridin-4-yl)ureido)-1-ethyl-6-oxo-1,6-dihydropyridazin-3-yl)-6-methoxybiphenyl-3-carboxylate

Reaction of 0.50 g of the title compound of Preparation 7 with 0.33 g of the title compound of Preparation 21 according to the method described in Example 1 gave 0.60 g (80%) of the crude title compound.
HPLC/MS (10 min) retention time 7.50 min.
LRMS: m/z 568 (M+)

### EXAMPLE 36

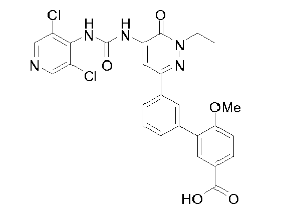

### 3'-[5-({[(3,5-Dichloropyridin-4-yl)amino]carbonyl}amino)-1-ethyl-6-oxo-1,6-dihydropyridazin-3-yl]-6-methoxybiphenyl-3-carboxylic acid

Reaction of the crude title compound of Example 35 (600 mg, 1.06 mmol) according to the method described in Example 26 and purification by the SP1® automated purification system gave 270 mg (487 mmol, 46%) of the title compound.
¹H NMR (200 MHz, DMSO-*d*₆) ppm 1.37 (t, J=7.03 Hz, 3 H), 3.85 (s, 3 H), 4.26 (q, J=7.16 Hz, 2 H), 7.24 (d, J=8.59 Hz, 1 H), 7.56 (d, J=4.69 Hz, 2 H), 7.71 - 7.82 (m, 1 H), 7.86 (d, J=1.95 Hz, 2 H), 7.98 (dd, J=8.59, 2.34 Hz, 2 H), 8.37 (s, 1 H), 8.69 (s, 2 H), 9.70 (s, 1 H)
HPLC/MS (30 min) retention time 17.36 min.
LRMS: m/z 554 (M+)

### EXAMPLE 37

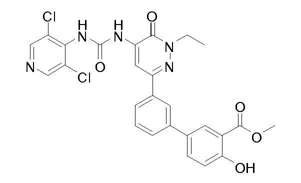

### Methyl 3'-[5-({[(3,5-dichloropyridin-4-yl)amino]carbonyl}amino)-1-ethyl-6-oxo-1,6-dihydropyridazin-3-yl]-4-hydroxybiphenyl-3-carboxylate

Reaction of 0.100 g of the title compound of Preparation 7 with 0.061 g of the title compound of Preparation 22 according to the method described in Example 1 gave 0.093 g (58%) of the crude title compound, used in the next step without further purification.
HPLC/MS (15 min) retention time 10.00 min.
LRMS: *m*/*z* 552 (M-1)

### EXAMPLE 38

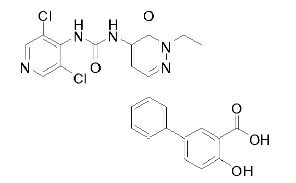

### 3'-[5-({[(3,5-Dichloropyridin-4-yl)amino]carbonyl}amino)-1-ethyl-6-oxo-1,6-dihydropyridazin-3-yl]-4-hydroxybiphenyl-3-carboxylic acid

Reaction of 0.093 g (0.17mmols) of the title compound of Example 37 according to the method described in Example 26 gave 0.003 g (3%) of the title compound.
HPLC/MS (30 min) retention time 18.46 min.
LRMS: *m*/*z* 540 (M+)

### EXAMPLE 39

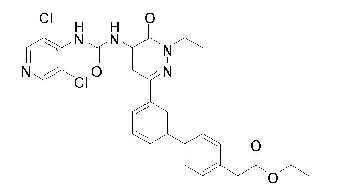

### Ehyl {3'-[5-({[(3,5-dichloropyridin-4-yl)amino]carbonyl}amino)-1-ethyl-6-oxo-1,6-dihydropyridazin-3-yl]biphenyl-4-yl}acetate

Reaction of 1.00g of the title compound of Preparation 7 with 0.90 g of the title compound of Preparation 23 according to the method described in Example 1 gave 0.65 g (55%) of the title compound.
¹H NMR (200 MHz, DMSO-*d*₆) ppm 1.20 (t, *J*=7.12 Hz, 3 H), 1.39 (t, *J*=7.12 Hz, 3 H), 3.72 (s, 2 H), 4.10 (q, *J*=7.29 Hz, 2 H), 4.28 (q, *J*=7.42 Hz, 2 H), 7.37 (s, 1 H), 7.41 (s, 1 H), 7.56 - 7.82 (m, 5 H), 7.99 (s, 1 H), 8.40 (s, 1 H), 8.70 (s, 2 H), 9.70 (s, 1 H), 9.90 (s, 1 H)
HPLC/MS (9 min) retention time 7.56 min.
LRMS: *m*/*z* 565 (M+)

### EXAMPLE 40

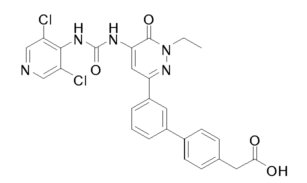

### {3'-[5-({[(3,5-Dichloropyridin-4-yl)amino]carbonyl}amino)-1-ethyl-6-oxo-1,6-dihydropyridazin-3-yl]biphenyl-4-yl}acetic acid

Reaction of 0.650 g of the title compound of Example 39 according to the method described in example 26 gave 0.303 g (49%) of the title compound.
¹H NMR (400 MHz, DMSO-*d*₆) ppm 1.39 (t, *J*=7.28 Hz, 3 H), 3.62 (s, 2 H), 4.28 (q, *J*=7.28 Hz, 2 H), 7.38 (d, *J*=8.61 Hz, 2 H), 7.51 - 7.68 (m, 3 H), 7.69 - 7.81 (m, 2 H), 7.99 (s, 1 H), 8.40 (s, 1 H), 8.70 (s, 2 H), 9.70 (s, 1 H), 9.90 (s, 1 H), 12.37 (s, 1 H)
HPLC/MS (15 min) retention time 9.10 min.
LRMS: *m*/*z* 538 (M+)

### EXAMPLE 41

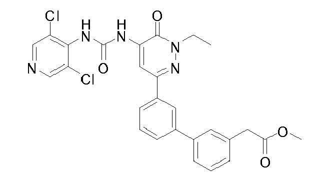

### Methyl {3'-[5-({[(3,5-dichloropyridin-4-yl)amino]carbonyl}amino)-1-ethyl-6-oxo-1,6-dihydropyridazin-3-yl]biphenyl-3-yl}acetate

Reaction of 1.00 g of the title compound of Preparation 7 with 0.85 g of the title compound of Preparation 24 according to the method described in Example 1 gave 0.76 g (66%) of the title compound used in the next step without further purification.
¹H NMR (400 MHz, DMSO-*d*₆) ppm 1.36 (t, *J*=7.20 Hz, 3 H), 3.59 (s, 3 H), 3.75 (s, 2 H), 4.25 (q, *J*=7.20 Hz, 2 H), 7.27 (d, *J*=7.83 Hz, 1 H), 7.42 (t, *J*=8.02 Hz, 1 H), 7.51 - 7.61 (m, 3 H), 7.69 (d, *J*=8.22 Hz, 1 H), 7.74 (d, *J*=7.83 Hz, 1 H), 7.95 (s, 1 H), 8.37 (s, 1 H), 8.67 (s, 2 H), 9.67 (s, 1 H), 9.87 (s, 1 H)
HPLC/MS (9 min) retention time 7.33 min.
LRMS: *m*/*z* 550 (M-1)

### EXAMPLE 42

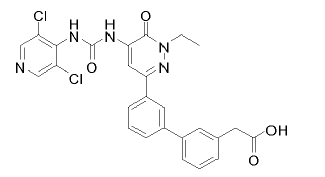

### {3'-[5-({[(3,5-Dichloropyridin-4-yl)amino]carbonyl}amino)-1-ethyl-6-oxo-1,6-dihydropyridazin-3-yl]biphenyl-3-yl}acetic acid

Reaction of 0.760 g of the title compound of Example 41 according to the method described in example 26 gave 0.330 g (44%) of the title compound, after crystallisation with dichloromethane-methanol (7:3).
¹H NMR (400 MHz, DMSO-*d*₆) ppm 1.36 (t, *J*=7.14 Hz, 3 H), 3.64 (s, 2 H), 4.25 (q, *J*=7.14 Hz, 2 H), 7.26 (d, *J*=7.83 Hz, 1 H), 7.41 (t, *J*=7.63 Hz, 1 H), 7.50 - 7.60 (m, 3 H),
7.69 (d, *J*=8.22 Hz, 1 H), 7.73 (d, *J*=7.8.22 Hz, 1 H), 7.95 (s, 1 H), 8.36 (s, 1 H), 8.66 (s, 2 H), 9.67 (s, 1 H), 9.88 (s, 1 H), 12.33 (s, 1 H),
HPLC/MS (30 min) retention time 17.47 min.
LRMS: *m*/*z* 538 (M+)

### EXAMPLE 43

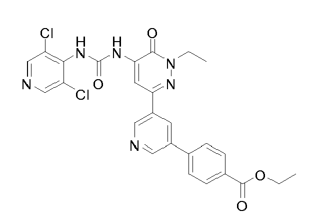

### Ethyl 4-{5-[5-({[(3,5-dichloropyridin-4-yl)amino]carbonyl}amino)-1-ethyl-6-oxo-1,6-dihydropyridazin-3-yl]pyridin-3-yl}benzoate

Reaction of 0.240 g of the title compound of Preparation 14 with 0.144 g [3-(ethoxycarbonyl)phenyl]boronic acid according to the method described in Example 1 gave 0.293 g (70%) of the title compound, after crystallisation with dichloromethane-methanol (7:3).
HPLC/MS (9 min) retention time 7.17 min.
LRMS: *m*/*z* 553 (M+)

### EXAMPLE 44

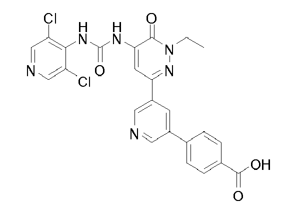

### 4-{5-[5-({[(3,5-Dichloropyridin-4-yl)amino]carbonyl}amino)-1-ethyl-6-oxo-1,6-dihydropyridazin-3-yl]pyridin-3-yl}benzoic acid

Reaction of 0.296 g of the title compound of Example 43 according to the method described in example 26 gave 0.046 g (25%) of the title compound, after crystallisation with dichloromethane-methanol (7:3).
¹H NMR (200 MHz, DMSO-*d*₆) ppm 1.40 (t, *J*=7.03 Hz, 3 H), 4.30 (q, *J*=7.03 Hz, 2 H), 7.89 - 8.00 (m, 2 H), 8.02 - 8.12 (m, 2 H), 8.41 (s, 2 H), 8.70 (s, 2 H), 9.01 (s, 2 H), 9.74 (s, 1 H), 9.90 (s, 1 H), 13.06 (s, 1 H)
HPLC/MS (30 min) retention time 14.48 min.
LRMS: *m*/*z* 525 (M+)

### EXAMPLE 45

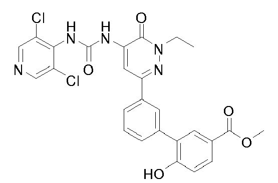

### Methyl 3'-[5-({[(3,5-dichloropyridin-4-yl)amino]carbonyl}amino)-1-ethyl-6-oxo-1,6-dihydropyridazin-3-yl]-6-hydroxybiphenyl-3-carboxylate

Reaction of 0.425 g of the title compound of Preparation 7 with 0.206 g of the title compound of Preparation 26 according to the method described in Example 1 gave 0.325 g (57%) of the title compound after triturating with methanol.
HPLC/MS (15 min) retention time 6.86 min.
LRMS: *m*/*z* 552 (M-1)

### EXAMPLE 46

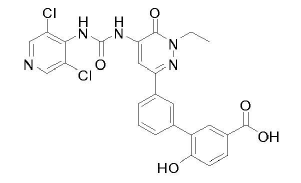

### 3'-[5-({[(3,5-Dichloropyridin-4-yl)amino]carbonyl}amino)-1-ethyl-6-oxo-1,6-dihydropyridazin-3-yl]-6-hydroxybiphenyl-3-carboxylic acid

Reaction of 0.325 g of the title compound of Example 45 according to the method described in example 26 gave 0.008 g (3%) of the title compound.
¹H NMR (400 MHz, DMSO-*d*₆) ppm 1.37 (t, *J*=7.09 Hz, 3 H), 4.26 (q, *J*=7.09 Hz, 2 H), 7.03 (d, *J*=8.22 Hz, 1 H), 7.50 - 7.63 (m, 2 H), 7.71 - 7.82 (m, 2 H), 7.86 (d, *J*=1.96 Hz, 1 H), 7.95 (s, 1 H), 8.39 (s, 1 H), 8.68 (s, 2 H), 9.64 (s, 1 H).
HPLC/MS (30 min) retention time 15.40 min.
LRMS: *m*/*z* 540 (M+)

### EXAMPLE 47

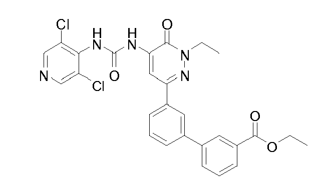

### Ethyl 3'-[5-({[(3,5-dichloropyridin-4-yl)aminolcarbonyl}amino)-1-ethyl-6-oxo-1,6-dihydropyridazin-3-yl]biphenyl-3-carboxylate

Reaction of 0.250 g from the title compound of Preparation 7 with 0.150 g [3-(ethoxycarbonyl)phenyl]boronic acid according to the method described in Example 1 gave 0.159 g (56%) of the title compound after trituration with methanol.
HPLC/MS (9 min) retention time 7.69 min.
LRMS: *m*/*z* 552 (M+)

### EXAMPLE 48

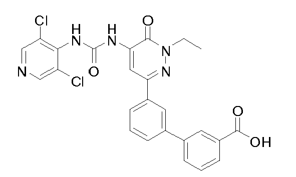

### 3'-[5-({[(3,5-dichloropyridin-4-yl)amino]carbonyl}amino)-1-ethyl-6-oxo-1,6-dihydropyridazin-3-yl]biphenyl-3-carboxylic acid

Reaction of 0.159 g from the title compound of the Example 47 according to the method described in example 26 gave 0.043 g (28%) of the title compound.
¹H NMR (400 MHz, DMSO-*d*₆) ppm 1.38 (t, *J*=7.20 Hz, 3 H), 4.28 (q, *J*=7.20 Hz, 2 H), 7.56 - 7.65 (m, 2 H), 7.79 (t, *J*=8.41 Hz, 2 H), 7.90 (d, *J*=7.63 Hz, 1 H), 7.96 (d, *J*=7.63 Hz, 1 H), 8.02 (s, 1 H), 8.20 (s, 1 H), 8.41 (s, 1 H), 8.69 (s, 2 H), 9.70 (s, 1 H)
HPLC/MS (30 min) retention time 18.01 min.
LRMS: *m*/*z* 524 (M+)

### EXAMPLE 49

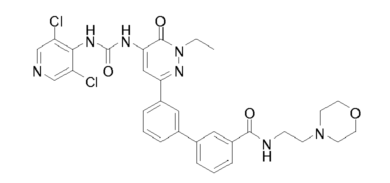

### 3'-[5-({[(3,5-Dichloropyridin-4-yl)amino]carbonyl}amino)-1-ethyl-6-oxo-1,6-dihydropyridazin-3-yl]-N-(2-morpholin-4-ylethyl)biphenyl-3-carboxamide

The title compound of Example 48 (170 mg, 0.29 mmol) and 2-morpholinoethanamine (77 mg, 0.59 mmol) were added to a solution of 1-hydroxybenzotriazole hydrate (60 mg, 0.44 mmol) and N-Ethyl-N'-(3-dimethyl-aminopropyl)carbodiimide hydrochloride (85 mg, 0.44mmol) in dimethylformamide (3.5 mL). The mixture was stirred at room temperature for 3 h.

The solvent was removed under reduced pressure and the residue dissolved in 4% sodium bicarbonate solution. The aqueous phase was extracted three times with ethyl acetate. The combined organic layers were washed with water and brine, and dried over anhydrous sodium sulphate.

The solvent was removed in vacuum and the residue purified by crystallization from dichloromethane-methanol (8:2) to yield the title compound (70 mg, 37%).
¹H NMR (200 MHz, DMSO-*d*₆) ppm 1.39 (t, *J*=6.64 Hz, 3 H), 2.35 - 2.45 (m, 8 H), 3.56 (br. s., 4 H), 4.28 (q, 2 H), 7.53 - 7.96 (m, 6 H), 8.04 (br. s., 1 H), 8.13 (br. s., 1 H), 8.41 (s, 1 H), 8.58 (br. s., 1 H), 8.70 (s, 2 H), 9.72 (br. s., 1 H), 9.92 (br. s., 1 H)
HPLC/MS (30 min) retention time 12.54 min
LRMS: *m*/*z* 636 (M+)

### EXAMPLE 50

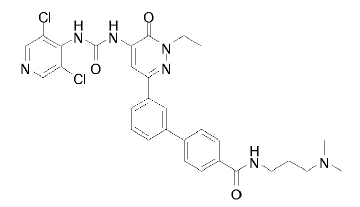

### 3'-[5-({[(3,5-Dichloropyridin-4-yl)amino]carbonyl}amino)-1-ethyl-6-oxo-1,6-dihydropyridazin-3-yl]-N-[3-(dimethylamino)propyl]biphenyl-4-carboxamide

Reaction of the title compound of Example 26 (100 mg, 0.18 mmol) with N,N-dimethylethane-1,2-diamine (32 mg, 0.36 mmol) according to the method described in Example 49 gave 27 mg (24%) of the title compound.
¹H NMR (200 MHz, DMSO-*d*₆) ppm 1.39 (t, *J*=7.03 Hz, 3 H), 1.60 - 1.85 (m, 2 H), 2.21 (s, 6 H), 2.36 (t, *J*=6.64 Hz, 2 H), 3.46 - 3.63 (m, 2 H), 4.28 (q, 2 H), 7.55 - 8.14 (m, 8 H), 8.41 (s, 1 H), 8.60 (br. s., 1 H), 8.70 (s, 2 H), 9.70 (br. s., 1 H)
HPLC/MS (30 min) retention time 12.38 min
LRMS: *m*/*z* 608 (M+)

### EXAMPLE 51

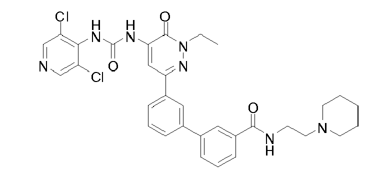

### 3'-[5-({[(3,5-Dichloropyridin-4-yl)amino]carbonyl}amino)-1-ethyl-6-oxo-1,6-dihydropyridazin-3-yl]-N-(2-piperidin-1-ylethyl)biphenyl-3-carboxamide

Reaction of the title compound of Example 48 (170 mg, 0.29 mmol) with 2-(piperidin-1-yl)ethanamine (75 mg, 0.59 mmol) according to the method described in Example 49 gave 70mg (37%) of the title compound.
¹H NMR (200 MHz, DMSO-*d*₆) ppm 1.39 (t, *J*=6.64 Hz, 3 H), 1.44 - 1.60 (m, 6 H), 2.32 - 2.52 (m, 6 H), 3.39 - 3.48 (m, 2 H), 4.28 (q, 2 H), 7.83 (t, *J*=8.39 Hz, 6 H), 8.04 (br. s., 1 H), 8.13 (br. s., 1 H), 8.41 (s, 1 H), 8.53 (br. s., 1 H), 8.68 (s, 2 H), 9.68 (br. s., 1 H), 9.94 (br. s., 1 H)
HPLC/MS (30 min) retention time 11.85 min
LRMS: *m*/*z* 634 (M+)

### EXAMPLE 52

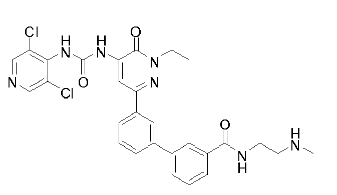

### 3'-[5-({[(3,5-Dichloropyridin-4-yl)amino]carbonyl}amino)-1-ethyl-6-oxo-1,6-dihydropyridazin-3-yl]-N-[2-(methylamino)ethyl]biphenyl-3-carboxamide

Reaction of the title compound of Example 48 (170 mg, 0.29 mmol) with N-methylethane-1,2-diamine (44 mg, 0.59 mmol) according to the method described in Example 49 gave 15mg (9%) of the title compound after purification by SP1® automated purification system.
¹H NMR (400 MHz, DMSO-*d*₆) ppm 1.39 (t, *J*=6.85 Hz, 3 H), 2.42 (s, 3 H), 2.92 (br. s., 2 H), 4.28 (q, *J*=6.65 Hz, 2 H), 7.50 - 7.70 (m, 2 H), 7.72 - 7.93 (m, 3 H), 8.04 (br. s., 1 H), 8.18 (br. s., 1 H), 8.29 (br. s., 1 H), 8.41 (s, 1 H), 8.67 (s, 2 H), 8.85 (br. s., 1 H), 9.61 (br. s., 2 H).
HPLC/MS (30 min) retention time 11.25 min
LRMS: *m*/*z* 580 (M+)

### EXAMPLE 53

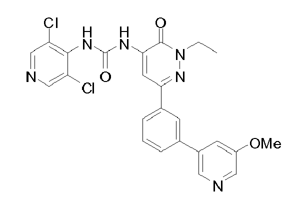

### N-(3,5-Dichloropyridin-4-yl)-N'-{2-ethyl-6-[3-(5-methoxypyridin-3-yl)phenyl]-3-oxo-2,3-dihydropyridazin-4-yl}urea

Reaction of the title compound of Preparation 7 (250 mg, 0.50 mmol) with the title compound of Preparation 32 (177 mg, 0.75 mmol) according to the method described in Example 1 gave 50 mg (19%) of the title compound after purification by flash chromatography (hexane-ethyl acetate, 2:1).
¹H NMR (200 MHz, DMSO-*d*₆) ppm 1.39 (t, *J*=7.22 Hz, 3 H), 3.91 (s, 3 H), 4.28 (q, *J*=7.03 Hz, 2 H), 7.59 - 7.71 (m, 2 H), 7.82 (d, *J*=7.81 Hz, 2 H), 8.06 (s, 1 H), 8.33 (d, *J*=2.73 Hz, 1 H), 8.40 (s, 1 H), 8.50 (s, 1 H), 8.69 (s, 2 H), 9.69 (s, 1 H), 9.90 (s, 1 H)
HPLC/MS (30 min) retention time 16.92 min
LRMS: *m*/*z* 511 (M+)

### EXAMPLE 54

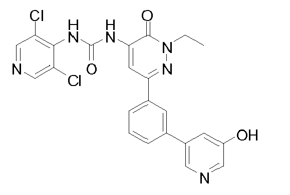

### N-(3,5-Dichloropyridin-4-yl)-N'-{2-ethyl-6-[3-(5-hydroxypyridin-3-yl)phenyl]-3-oxo-2,3-dihydropyridazin-4-yl}urea

The title compound of Example 53 (150 mg, 0.28 mmol) was added to a solution of boron tribromide-methyl sulphide complex (711 mg, 2.27 mmol) in 11 mL of 1,2-dichloroethane. The mixture was stirred at reflux for 10 d. Additional boron tribromide-methyl sulphide complex (2492 mg, 7.97 mmol) was added during reaction until the starting material had disappeared.

The reaction mixture was diluted with water and neutralised with ammonium hydroxide solution to pH 7-8. Dichloromethane was added precipitating a solid, which was collected by filtration and dried in a stream of air. Purification by preparative HPLC gave 12 mg (9%) of the title compound.
HPLC/MS (30 min) retention time 14.23 min
LRMS: *m*/*z* 497 (M+)

### EXAMPLE 55

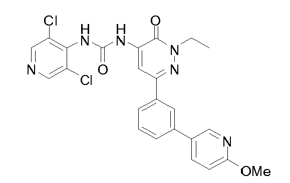

### N-(3,5-Dichloropyridin-4-yl)-N'-{2-ethyl-6-[3-(6-methoxypyridin-3-yl)phenyl]-3-oxo-2,3-dihydropyridazin-4-yl}urea

Reaction of 0.30 g of the title compound of Preparation 7 with 0.19 g 6-methoxypyridin-3-ylboronic acid according to the method described in Example 1 gave 0.45 g (74%) of the title compound.
¹H NMR (200 MHz, DMSO-*d*₆) ppm 1.4 (t, *J*=7.2 Hz, 3 H), 3.9 (s, 3 H), 4.3 (m, 2 H), 6.9 (d, *J*=8.6 Hz, 1 H), 7.6 (t, *J*=7.6 Hz, 1 H), 7.8 (m, 2 H), 8.0 (m, 2 H), 8.4 (s, 1 H), 8.5 (d, *J*=2.3 Hz, 1 H), 8.7 (s, 2 H), 9.7 (s, 1 H), 9.9 (s, 1 H)
HPLC/MS (30 min) retention time 18.98 min
LRMS: m/z 511 (M+)

### EXAMPLE 56

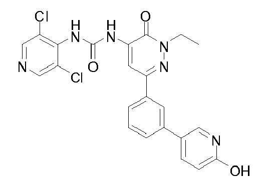

### N-(3,5-Dichloropyridin-4-yl)-N'-{2-ethyl-6-[3-(6-hydroxypyridin-3-yl)phenyl]-3-oxo-2,3-dihydropyridazin-4-yl}urea

The title compound of Example 55 (153 mg, 0,3 mmol) was suspended in acetonitrile (10 mL) in a microwave vial. Sodium iodide (225 mg, 1.50 mmol) was added. The vial was sealed, purged with argon and then trimethylsilyl chloride (190 µl, 1.50 mmol) was added. The mixture was microwaved at 100 °C for 5h. The solvent was evaporated under reduced pressure and the residue directly purified by the SP1® automated purification system to give 60 mg (0.121 mmol) of the desired compound. Yield = 40%
¹H NMR (200 MHz, DMSO-*d*₆) ppm 1.4 (t, *J*=7.2 Hz, 3 H), 4.3 (q, *J*=7.4 Hz, 2 H), 6.5 (d, *J*=9.8 Hz, 1 H), 7.5 (t, *J*=7.8 Hz, 1 H), 7.7 (m, *J*=7.0, 1.0 Hz, 2 H), 7.8 (d, *J*=2.0 Hz, 1 H), 7.9 (m, *J*=2.7 Hz, 2 H), 8.4 (s, 1 H), 8.7 (s, 2 H), 9.7 (s, 1 H), 9.9 (s, 1 H), 11.9 (s, 1 H)
HPLC/MS (30 min) retention time 14.66 min.
LRMS: *m*/*z* 497 (M+)

### EXAMPLE 57

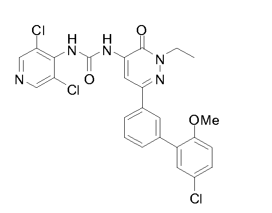

### N-[6-(5'-chloro-2'-methoxybiphenyl-3-yl)-2-ethyl-3-oxo-2,3-dihydropyridazin-4-yl]-N'-(3,5-dichloropyridin-4-yl)urea

Reaction of 0.40 g of the title compound of Preparation 7 with 0.23 g 5-chloro-2-methoxyphenylboronic acid according to the method described in Example 1 and purification by the SP1® automated purification system gave 0.115 g (26%) of the title compound.
HPLC/MS (10 min) retention time 7.74 min
LRMS: *m*/*z* 542 (M-1)

### EXAMPLE 58

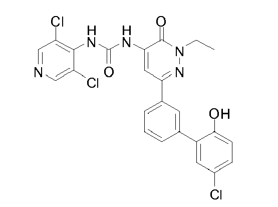

### N-[6-(5'-chloro-2'-hydroxybiphenyl-3-yl)-2-ethyl-3-oxo-2,3-dihydropyridazin-4-yl]-N'-(3,5-dichloropyridin-4-yl)urea

Reaction of 0.115 g of the title compound of Example 57 with 0.95 mL boron tribromide solution (1M in dichloromethane) according to the method described in Preparation 17 and purification by the SP1® automated purification system gave 0.052 g (46%) of the title compound.
¹H NMR (200 MHz, DMSO-*d*₆) ppm 9.98 (2 H, br. s.), 9.66 (1 H, br. s.), 8.69 (2 H, s), 8.38 (1 H, s), 7.95 (1 H, s), 7.76 (1 H, d, *J*=7.0 Hz), 7.48 - 7.69 (2 H, m), 7.32 (1 H, d, *J*=2.3 Hz), 7.24 (1 H, dd, *J*=8.6, 2.7 Hz), 6.97 (1 H, d, *J*=8.6 Hz), 4.26 (2 H, q, *J*=7.0 Hz), 1.37 (3 H, t, *J*=7.0 Hz)
HPLC/MS (30 min) retention time 19.34 min
LRMS: *m*/*z* 530 (M+)

### EXAMPLE 59

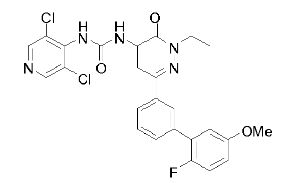

### N-(3,5-dichloropyridin-4-yl)-N'-[2-ethyl-6-(2'-fluoro-5'-methoxybiphenyl-3-yl)-3-oxo-2,3-dihydropyridazin-4-yl]urea

Reaction of 0.41 g of the title compound of Preparation 7 with 0.21 g 2-fluoro-5-methoxyphenylboronic acid according to the method described in Example 1 and purification by the SP1® automated purification system gave 0.11 g (25%) of the title compound.
¹H NMR (200 MHz, CHLOROFORM-*d*) ppm 9.56 (1 H, s), 9.08 (1 H, s), 8.73 (1 H, s), 8.62 (2 H, s), 7.99 (1 H, s), 7.85 (1 H, d, *J*=7.4 Hz), 7.40 - 7.71 (2 H, m), 7.08 (1 H, t, *J*=9.4 Hz), 6.77 - 7.01 (2 H, m), 4.23 (2 H, q, *J*=7.0 Hz), 3.83 (3 H, s), 1.26 (3 H, t, *J*=7.0 Hz)
HPLC/MS (15 min) retention time 9.73 min
LRMS: *m*/*z* 526/528/530 (M-H⁺)

### EXAMPLE 60

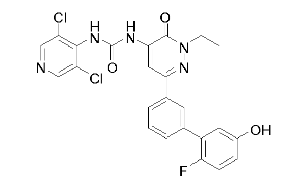

### N-(3,5-dichloropyridin-4-yl)-N'-[2-ethyl-6-(2'-fluoro-5'-hydroxybiphenyl-3-yl)-3-oxo-2,3-dihydropyridazin-4-yl]urea

Reaction of 10 mg of the title compound of Example 59 with 0.095 mL boron tribromide solution (1M in dichloromethane) according to the method described in Preaparation 17 gave 8 mg (83%) of the title compound
HPLC/MS (30 min) retention time 17.78 min
LRMS: *m*/*z* 514 (M+)

### EXAMPLE 61

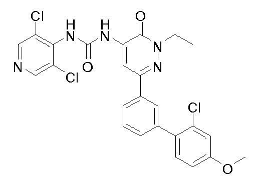

### N-[6-(2'-Chloro-4'-methoxybiphenyl-3-yl)-2-ethyl-3-oxo-2,3-dihydropyridazin-4-yl]-N'-(3,5-dichloropyridin-4-yl)urea

Reaction of 0.425 g of the title compound of Preparation 7 with 0.335 g (2-chloro-4-methoxyphenyl)boronic acid according to the method described in Example 1 gave 0.146 g (30%) of the title compound.
¹H NMR (200 MHz, DMSO-*d*₆) ppm 1.37 (t, 3 H), 3.82 (s, 3 H), 4.26 (q, 2 H), 7.03 (dd, *J*=8.59, 2.34 Hz, 1 H), 7.17 (d, *J*=2.34 Hz, 1 H), 7.35 - 7.63 (m, 3 H), 7.72 - 7.85 (m, 2 H), 8.38 (s, 1 H), 8.69 (s, 2 H), 9.70 (s, 1 H), 9.90 (s, 1 H)
HPLC/MS (9 min) retention time 7.68min.
LRMS: *m*/*z* 544 (M+)

### EXAMPLE 62

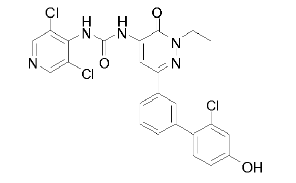

### N-[6-(2'-Chloro-4'-hydroxybiphenyl-3-yl)-2-ethyl-3-oxo-2,3-dihydropyridazin-4-yl]-N'-(3,5-dichloropyridin-4-yl)urea

Reaction of 0.146 g from the title compound of Example 61 according to the method described in preparation 17 gave 0.038 g (27%) of the title compound.
¹H NMR (200 MHz, DMSO-*d*₆) ppm 1.37 (t, *J*=7.42 Hz, 3 H), 4.25 (q, *J*=7.42 Hz, 2 H), 6.85 (dd, *J*=8.40, 2.34 Hz, 1 H), 6.95 (d, *J*=2.34 Hz, 1 H), 7.27 (d, *J*=8.40 Hz, 1 H), 7.40 - 7.62 (m, 2 H), 7.70 - 7.83 (m, 2 H), 8.38 (s, 1 H), 8.67 (s, 2 H)
HPLC/MS (30 min) retention time 18.25 min.
LRMS: *m*/*z* 530 (M+)

### EXAMPLE 63

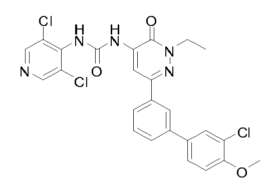

### N-[6-(3'-Chloro-4'-methoxybiphenyl-3-yl)-2-ethyl-3-oxo-2,3-dihydropyridazin-4-yl]-N'-(3,5-dichloropyridin-4-yl)urea

Reaction of 0.250 g from the title compound of Preparation 7 with 0.144 g (3-chloro-4-methoxyphenyl)boronic acid according to the method described in Example 1 gave 0.225 g (82%) of the title compound.
HPLC/MS (9 min) retention time 7.65 min.
LRMS: *m*/*z* 546 (M-1)

### EXAMPLE 64

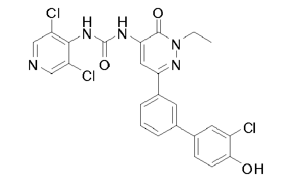

### N-[6-(3'-Chloro-4'-hydroxybiphenyl-3-yl)-2-ethyl-3-oxo-2,3-dihydropyridazin-4-yl]-N'-(3,5-dichloropyridin-4-yl)urea

Reaction of 0.225 g of the title compound of Example 63 according to the method described in preparation 17 gave 0.068 g (31%) of the title compound.
¹H NMR (200 MHz, DMSO-*d*₆) ppm 1.31 (t, *J*=6.83 Hz, 3 H), 4.20 (q, *J*=6.83 Hz, 2 H), 7.02 (d, *J*=8.20 Hz, 1 H), 7.33 - 7.54 (m, 2 H), 7.54 - 7.71 (m, 3 H), 7.85 (s, 1 H), 8.32 (s, 1 H), 8.61 (s, 2 H)
HPLC/MS (30 min) retention time 18.64 min.
LRMS: *m*/*z* 530 (M+)

### EXAMPLE 65

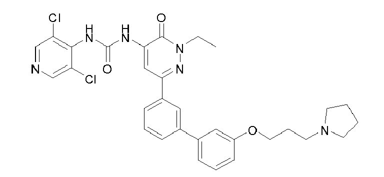

### N-(3,5-Dichloropyridin-4-yl)-N'-{2-ethyl-6-[3'-(3-pyrrolidin-1-ylpropoxy)biphenyl-3-yl]-3-oxo-2,3-dihydropyridazin-4-yl}urea

Reaction of the title compound of Preparation 7 (185 mg, 0.38 mmol) with the crude title compound of Preparation 56 (170 mg) according to the method described in Example 1 and purification by the SP1® automated purification system gave 25 mg (11%) of the title compound.
¹H NMR (200 MHz, CHLOROFORM-*d*) ppm 9.55 (1 H, s), 8.64 (1 H, s), 8.59 (2 H, s), 8.51 (1 H, s), 7.96 (1 H, s), 7.79 (1 H, d, *J*=7.8 Hz), 7.58 (1 H, d, *J*=7.4 Hz), 7.47 (1 H, t, *J*=7.6 Hz), 7.34 (1 H, t, *J*=7.8 Hz), 7.17 (1 H, d, *J*=7.8 Hz), 7.06 (1 H, s), 6.86 (1 H, d, *J*=7.8 Hz), 4.25 (2 H, q, *J*=7.2 Hz), 4.11 (2 H, t, *J*=5.1 Hz), 3.27 (6 H, br. s.), 2.21 - 2.42 (2 H, m), 2.00 - 2.16 (4 H, m), 1.31 (3 H, t, *J*=7.0 Hz).
HPLC/MS (30 min) retention time 12.58 min.
LRMS: *m*/*z* 607 (M+).

### EXAMPLE 66

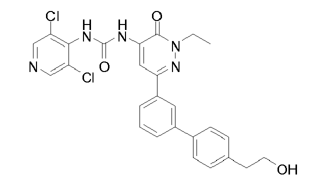

### N-(3,5-dichloropyridin-4-yl)-N'-{2-ethyl-6-[4'-(2-hydroxyethyl)biphenyl-3-yl]-3-oxo-2,3-dihydropyridazin-4-yl}urea

0.32 g of the crude title compound of Preparation 54 (0.50 mmol) was dissolved in 6 mL anhydrous tetrahydrofuran under nitrogen atmosphere. 1 mL tetrabutylammonium fluoride (1 M in tetrahydrofuran, 1 mmol) was added and the mixture stirred overnight at room temperature. The mixture was evaporated and the residue purified by the SP1® automated purification system to give 64 mg (24%) of the title compound.
¹H NMR (200 MHz, DMSO-*d*₆) ppm 9.91 (1 H, br. s.), 9.68 (1 H, br. s.), 8.69 (2 H, s), 8.40 (1 H, s), 7.96 (1 H, br. s.), 7.66 - 7.86 (2 H, m), 7.50 - 7.65 (3 H, m), 7.34 (2 H, d, *J*=7.4 Hz), 4.67 (1 H, br. s.), 4.17 - 4.38 (2 H, m), 3.50 - 3.75 (2 H, m), 2.66 - 2.90 (2 H, m), 1.27 - 1.51 (3 H, m)
HPLC/MS (30 min) retention time 17.85 min
LRMS: *m*/*z* 524 (M+)

### EXAMPLE 67

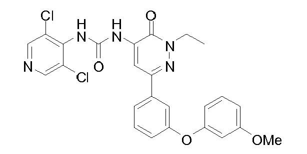

### N-(3,5-Dichloropyridin-4-yl)-N'-{2-ethyl-6-[3-(3-methoxyphenoxy)phenyl]-3-oxo-2,3-dihydropyridazin-4-yl}urea

Reaction of 0.37 g of the title compound of Preparation 43 with 0.11 g triphosgene and 0.36 g 3,5-dichloropyridin-4-amine according to the method described in Preparation 7 and purification by the SP1® automated purification system gave 0.20 g (34%) of the title compound.
¹H NMR (200 MHz, DMSO-*d*₆) ppm 9.89 (1 H, br. s.), 9.68 (1 H, br. s.), 8.69 (2 H, s), 8.31 (1 H, s), 7.39 - 7.59 (3 H, m), 7.29 (1 H, t, *J*=8.0 Hz), 7.03 - 7.14 (1 H, m), 6.74 (1 H, dd, *J*=8.2, 2.0 Hz), 6.62 - 6.67 (1 H, m), 6.58 (1 H, d, *J*=7.8 Hz), 4.23 (2 H, q, *J*=7.0 Hz), 3.74 (3 H, s), 1.34 (3 H, t, *J*=7.0 Hz)
HPLC/MS (30 min) retention time 19.80 min
LRMS: *m*/*z* 526 (M+)

### EXAMPLE 68

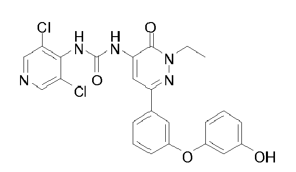

### N-(3,5-Dichloropyridin-4-yl)-N'-{2-ethyl-6-[3-(3-hydroxyphenoxy)phenyl]-3-oxo-2,3-dihydropyridazin-4-yl}urea

Reaction of 0.10 g of the title compound of Example 67 with 0.85 mL boron tribromide solution (1M in dichloromethane) according to the method described in Preparation 17 and purification by the SP1® automated purification system gave 0.036 g (36%) of the title compound.
¹H NMR (200 MHz, DMSO-*d*₆) ppm 9.64 (3 H, br. s.), 8.67 (2 H, s), 8.32 (1 H, s), 7.37 - 7.64 (3 H, m), 7.02 - 7.28 (2 H, m), 6.34 - 6.61 (3 H, m), 4.23 (2 H, q, *J*=6.9 Hz), 1.34 (3 H, t, *J*=7.0 Hz)
HPLC/MS (30 min) retention time 17.82 min
LRMS: *m*/*z* 512 (M+)

### EXAMPLE 69

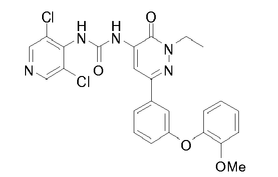

### N-(3,5-Dichloropyridin-4-yl)-N'-{2-ethyl-6-[3-(2-methoxyphenoxy)phenyl]-3-oxo-2,3-dihydropyridazin-4-yl}urea

Reaction of 0.14 g of the title compound of Preparation 45 with 0.043 g triphosgene and 0.135 g 3,5-dichloropyridin-4-amine according to the method described in Preparation 7 and precipitation from ethyl acetate gave 0.109 g (48%) of the title compound.
HPLC/MS (10 min) retention time 7.28 min
LRMS: *m*/*z* 527 (M+1)

### EXAMPLE 70

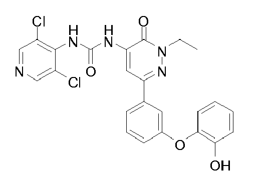

### N-(3,5-Dichloropyridin-4-yl)-N'-{2-ethyl-6-[3-(2-hydroxyphenoxy)phenyl]-3-oxo-2,3-dihydropyridazin-4-yl}urea

Reaction of 0.108 g of the title compound of Example 69 with 0.89 mL boron tribromide solution (1M in dichloromethane) according to the method described in Preparation 17, purification by chromatography (ethyl acetate-hexane, 50:50) and re-crystallisation from ethanol gave 0.037 g (34%) of the title compound.
¹H NMR (200 MHz, DMSO-*d*₆) ppm 9.91 (1 H, br. s.), 9.67 (1 H, br. s.), 9.58 (1 H, s), 8.70 (2 H, s), 8.30 (1 H, s), 7.24 - 7.56 (3 H, m), 6.70 - 7.18 (5 H, m), 4.22 (2 H, q), 1.34 (3 H, t, *J*=6.6 Hz)
HPLC/MS (30 min) retention time 17.80
LRMS: *m*/*z* 512 (M+)

### EXAMPLE 71

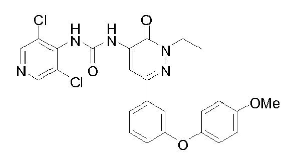

### N-(3,5-Dichloropyridin-4-yl)-N'-{2-ethyl-6-[3-(4-methoxyphenoxy)phenyl]-3-oxo-2,3-dihydropyridazin-4-yl}urea

Reaction of 0.167 g of the title compound of Preparation 44 with 0.051 g triphosgene and 0.162 g 3,5-dichloropyridin-4-amine according to the method described in Preparation 7 and precipitation from ethyl acetate gave 0.153 g (57%) of the title compound.
HPLC/MS (10 min) retention time 7.47 min
LRMS: *m*/*z* 527 (M+1)

### EXAMPLE 72

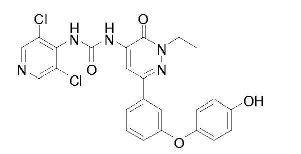

### N-(3,5-Dichloropyridin-4-yl)-N'-{2-ethyl-6-[3-(4-hydroxyphenoxy)phenyl]-3-oxo-2,3-dihydropyridazin-4-yl}urea

Reaction of 0.148 g of the title compound of Example 71 with 1.16 mL boron tribromide solution (1M in dichloromethane) according to the method described in Preparation 17 and purification by the SP1® automated purification system gave 0.016 g (11%) of the title compound.
¹H NMR (200 MHz, DMSO-*d*₆) ppm 9.58 (m), 8.66 (2 H, s), 8.29 (1 H, s), 7.26 - 7.56 (3 H, m), 6.64 - 7.03 (5 H, m), 4.21 (2 H, q, *J*=7.0 Hz), 1.33 (3 H, t, *J*=7.0 Hz)
HPLC/MS (30 min) retention time 17.44 min
LRMS: *m*/*z* 512 (M+)

### EXAMPLE 73

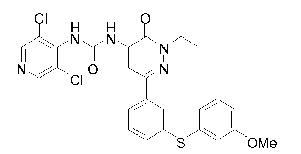

### N-(3,5-Dichloropyridin-4-yl)-N'-(2-ethyl-6-{3-[(3-methoxyphenyl)thio]phenyl}-3-oxo-2,3-dihydropyridazin-4-yl)urea

Reaction of 0.45 g of the title compound of Preparation 46 with 0.13 g triphosgene and 0.42 g 3,5-dichloropyridin-4-amine according to the method described in Preparation 7 and precipitation from ethyl acetate gave 0.39 g (54%) of the title compound.
HPLC/MS (10 min) retention time 7.72 min
LRMS: *m*/*z* 540/542/544 (M-1)

### EXAMPLE 74

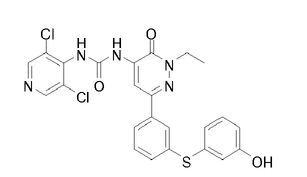

### N-(3,5-Dichloropyridin-4-yl)-N'-(2-ethyl-6-{3-[(3-hydroxyphenyl)thio]phenyl}-3-oxo-2,3-dihydropyridazin-4-yl)urea

Reaction of 0.15 g of the title compound of Example 73 with 1.18 mL boron tribromide solution (1M in dichloromethane) according to the method described in Preparation 17 and purification by the SP1® automated purification system gave 0.034 g (24%) of the title compound.
¹H NMR (200 MHz, DMSO-*d*₆) ppm 9.89 (1 H, s), 9.69 (1 H, br. s.), 9.64 (1 H, s), 8.69 (2 H, s), 8.31 (1 H, s), 7.75 (1 H, s), 7.70 (1 H, d, *J*=7.8 Hz), 7.51 (1 H, t, *J*=7.6 Hz), 7.33 - 7.43 (1 H, m), 7.19 (1 H, t, *J*=8.2 Hz), 6.79 (1 H, d, *J*=7.8 Hz), 6.72 (2 H, br. s.), 4.23 (2 H, q, *J*=6.9 Hz), 1.35 (3 H, t, *J*=7.0 Hz)
HPLC/MS (30 min) retention time 18.69 min
LRMS: *m*/*z* 528 (M+)

### EXAMPLE 75

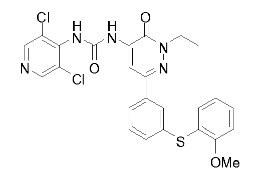

### N-(3,5-dichloropyridin-4-yl)-N'-(2-ethyl-6-{3-[(2-methoxyphenyl)thio]phenyl}-3-oxo-2,3-dihydropyridazin-4-yl)urea

Reaction of 0.38 g of the title compound of Preparation 47 with 0.11 g triphosgene and 0.35 g 3,5-dichloropyridin-4-amine according to the method described in Preparation 7 and precipitation from ethyl acetate gave 0.45 g (74%) of the title compound.
HPLC/MS (10 min) retention time 7.57 min
LRMS: *m*/*z* 540/542/544 (M-1)

### EXAMPLE 76

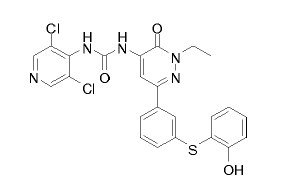

### N-(3,5-Dichloropyridin-4-yl)-N'-(2-ethyl-6-{3-[(2-hydroxyphenyl)thio]phenyl}-3-oxo-2,3-dihydropyridazin-4-yl)urea

Reaction of 0.20 g of the title compound of Example 75 with 1.6 mL boron tribromide solution (1M in dichloromethane) according to the method described in Preparation 17 and purification by the SP1® automated purification system gave 0.057 g (30%) of the title compound.
¹H NMR (200 MHz, DMSO-*d*₆) ppm 10.00 (1 H, s), 9.90 (1 H, br. s.), 9.66 (1 H, br. s.), 8.69 (2 H, s), 8.28 (1 H, s), 7.52 - 7.66 (2 H, m), 7.42 (1 H, t, *J*=7.6 Hz), 7.12 - 7.30 (3 H, m), 6.95 (1 H, d, *J*=7.8 Hz), 6.82 (1 H, t, *J*=7.4 Hz), 4.21 (2 H, q, *J*=7.0 Hz), 1.33 (3 H, t)
HPLC/MS (30 min) retention time 18.76 min
LRMS: *m*/*z* 528 (M+)

### EXAMPLE 77

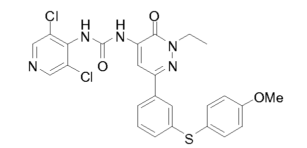

### N-(3,5-Dichloropyridin-4-yl)-N'-(2-ethyl-6-{3-[(4-methoxyphenyl)thio]phenyl}-3-oxo-2,3-dihydropyridazin-4-yl)urea

Reaction of 0.44 g of the title compound of Preparation 48 with 0.13 g triphosgene and 0.41 g 3,5-dichloropyridin-4-amine according to the method described in Preparation 7 and precipitation from ethyl acetate gave 0.48 g (69%) of the title compound.
HPLC/MS (10 min) retention time 7.75 min
LRMS: *m*/*z* 540/542/544 (M-1)

### EXAMPLE 78

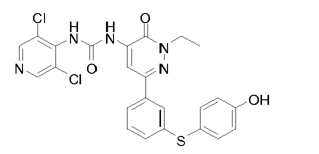

### N-(3,5-Dichloropyridin-4-yl)-N'-(2-ethyl-6-{3-[(4-hydroxyphenyl)thio]phenyl}-3-oxo-2,3-dihydropyridazin-4-yl)urea

Reaction of 0.20 g of the title compound of Example 77 with 1.6 mL boron tribromide solution (1M in dichloromethane) according to the method described in Preparation 17 and purification by the SP1® automated purification system gave 0.053 g (28%) of the title compound.
¹H NMR (200 MHz, DMSO-*d*₆) ppm 9.91 (2 H, br. s.), 9.65 (1 H, br. s.), 8.69 (2 H, s), 8.27 (1 H, s), 7.46 - 7.61 (2 H, m), 7.23 - 7.46 (3 H, m), 7.10 (1 H, d, *J*=7.8 Hz), 6.85 (2 H, d, *J*=8.6 Hz), 4.21 (2 H, q, *J*=7.0 Hz), 1.32 (3 H, t, *J*=7.2 Hz)
HPLC/MS (30 min) retention time 18.77 min
LRMS: *m*/*z* 528 (M+)

### EXAMPLE 79

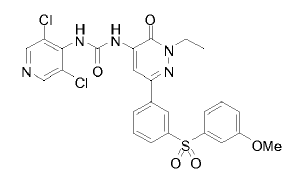

### N-(3,5-Dichloropyridin-4-yl)-N'-(2-ethyl-6-{3-[(3-methoxyphenyl)sulfonyl]phenyl}-3-oxo-2,3-dihydropyridazin-4-yl)urea

0.15 g of the title compound of Example 73 (0.26 mmol) was dissolved in 5 mL DMF and cooled to 0 °C. 0.39 g Oxone (0.63 mmol) was added in portions over 10 min. The mixture was stirred for 2 h at room temperature. A further 0.32 g Oxone (0.52 mmol) was added and the mixture stirred for a further 2 h. The mixture was then poured into a 40% w/v solution of sodium bisulfite. The mixture was partitioned between water and chloroform. The organic phase was washed with water, brine and then dried over magnesium sulphate. Evaporation gave 0.14 g (0.24 mmol, 91%) of the title compound.
HPLC/MS (5 min) retention time 3.51 min
LRMS: *m*/*z* 574/576 (M+1)

### EXAMPLE 80

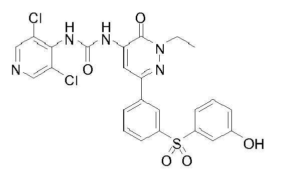

### N-(3,5-Dichloropyridin-4-yl)-N'-(2-ethyl-6-{3-[(3-hydroxyphenyl)sulfonyl]phenyl}-3-oxo-2,3-dihydropyridazin-4-yl)urea

Reaction of 0.14 g of the title compound of Example 79 with portion-wise addition of 2.1 mL boron tribromide solution (1 M in dichloromethane) according to the method described in Preparation 17 and trituration with dichloromethane gave 0.10 g (70%) of the title compound.
¹H NMR (200 MHz, DMSO-*d*₆) ppm 10.29 (1 H, br. s.), 9.92 (1 H, br. s.), 9.74 (1 H, br. s.), 8.70 (2 H, s), 8.37 (1 H, s), 8.28 (1 H, s), 8.08 (1 H, d, *J*=7.8 Hz), 7.99 (1 H, d, *J*=7.8 Hz), 7.75 (1 H, t, *J*=7.8 Hz), 7.33 - 7.50 (2 H, m), 7.30 (1 H, s), 7.06 (1 H, d, *J*=7.0 Hz), 4.28 (2 H, q, *J*=6.9 Hz), 1.38 (3 H, t, *J*=7.0 Hz)
HPLC/MS (30 min) retention time 15.99 min
LRMS: *m*/*z* 560 (M+)

### EXAMPLE 81

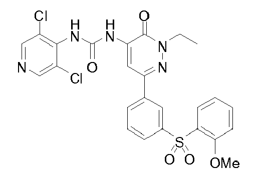

### N-(3,5-Dichloropyridin-4-yl)-N'-(2-ethyl-6-{3-[(2-methoxyphenyl)sulfonyl]phenyl}-3-oxo-2,3-dihydropyridazin-4-yl)urea

Reaction of 0.25 g of the title compound of Example 75 with a total of 0.66 g Oxone according to the method described in Example 79 gave 0.14 g (55%) of the title compound.
HPLC/MS (10 min) retention time 6.75 min
LRMS: *m*/*z* 574/576 (M+1)

### EXAMPLE 82

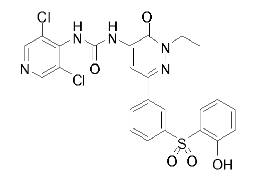

### N-(3,5-Dichloropyridin-4-yl)-N'-(2-ethyl-6-{3-[(2-hydroxyphenyl)sulfonyl]phenyl}-3-oxo-2,3-dihydropyridazin-4-yl)urea

Reaction of 0.14 g of the title compound of Example 81 with 1.1 mL boron tribromide solution (1M in dichloromethane) according to the method described in Preparation 17 and purification by the SP1® automated purification system gave 0.006 g (4%) of the title compound.
HPLC/MS (30 min) retention time 16.10 min
LRMS: *m*/*z* 560 (M+)

### EXAMPLE 83

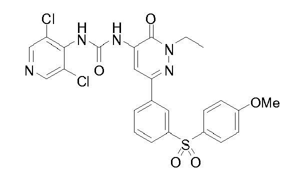

### N-(3,5-Dichloropyridin-4-yl)-N'-(2-ethyl-6-{3-[(4-methoxyphenyl)sulfonyl]phenyl}-3-oxo-2,3-dihydropyridazin-4-yl)urea

Reaction of 0.28 g of the title compound of Example 77 with a total of 0.74 g Oxone according to the method described in Example 79 gave 0.19 g (68%) of the title compound.
HPLC/MS (10 min) retention time 6.75 min
LRMS: *m*/*z* 574/576 (M+1)

### EXAMPLE 84

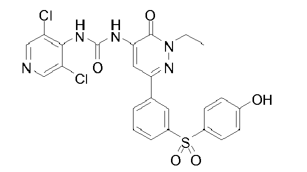

### N-(3,5-Dichloropyridin-4-yl)-N'-(2-ethyl-6-{3-[(4-hydroxyphenyl)sulfonyl]phenyl}-3-oxo-2,3-dihydropyridazin-4-yl)urea

Reaction of 0.19 g of the title compound of Example 83 with portion-wise addition of 5.5 mL boron tribromide solution (1 M in dichloromethane) according to the method described in Preparation 17 and precipitation with water gave 0.12 g (60%) of the title compound.
¹H NMR (200 MHz, DMSO-*d*₆) ppm 9.70 (1 H, br. s.), 8.69 (2 H, s), 8.35 (1 H, s), 8.25 (1 H, s), 8.03 (1 H, d, *J*=7.8 Hz), 7.95 (1 H, d, *J*=7.8 Hz), 7.80 (2 H, d, *J*=8.6 Hz), 7.71 (1 H, t, *J*=7.8 Hz), 6.93 (2 H, d, *J*=9.0 Hz), 4.27 (2 H, q, *J*=7.0 Hz), 1.38 (3 H, t, *J*=7.2 Hz)
HPLC/MS (30 min) retention time 15.57 min
LRMS: *m*/*z* 560 (M+)

### EXAMPLE 85

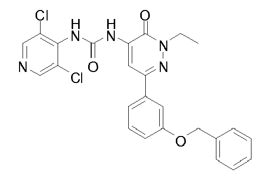

### N-{6-[3-(Benzyloxy)phenyl]-2-ethyl-3-oxo-2,3-dihydropyridazin-4-yl}-N'-(3,5-dichloropyridin-4-yl)urea

200 mg of the title compound of Preparation 11 (0.48 mmol) was dissolved in 4 mL anhydrous dimethylformamide. 21 mg sodium hydride (60% dispersion in oil, 0.52 mmol) was added and the mixture stirred at room temperature for 30 min. The mixture was cooled in an ice-water bath and 0.11 mL benzyl chloride (0.95 mmol) was added. The mixture was stirred for 1 h at room temperature. 10 mg sodium hydride was added and the mixture stirred overnight. A further 20 mg sodium hydride was added and the mixture stirred overnight. The mixture was poured onto ice-water and was extracted five times with ether. The organics were washed twice with water, once with brine, dried over magnesium sulphate and evaporated. Purification by chromatography (ethyl acetate-hexane gradient) gave 63 mg (25%) of the title compound.
¹H NMR (200 MHz, CHLOROFORM-*d*) ppm 9.69 (1 H, br. s.), 9.37 (1 H, br s), 8.72 (1 H, m), 8.62 (2 H, 2), 7.15 - 7.75 (8 H, m), 7.02 (1 H, d, *J*=6.6 Hz), 5.08 (2 H, s), 4.19 (2 H, d, *J*=7.0 Hz), 1.22 (3 H, t, *J*=6.6 Hz)
HPLC/MS (30 min) retention time 19.53 min
LRMS: *m*/*z* 510 (M+)

### EXAMPLE 86

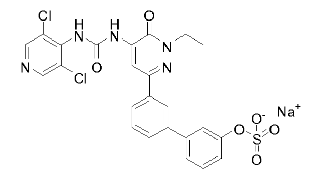

### 3'-[5-({[(3,5-Dichloropyridin-4-yl)amino]carbonyl}amino)-1-ethyl-6-oxo-1,6-dihydropyridazin-3-yl]biphenyl-3-yl hydrogen sulphate (sodium salt)

The title product of Example 1 (0.100 g, 0.20 mmol) was added in portions under nitrogen to a stirred suspension of pyridine-sulphur trioxide complex (0.160 g, 1.01 mmol) in dry pyridine (2 mL). The mixture was stirred for 18 h at room temperature. After removal of the pyridine under reduced pressure, the residue was dissolved in dry methanol and sodium methoxide (0.020 g) was added. After stirring for 1 h the solvent was evaporated. The residue was purified by the SP1® automated purification system to give 0.080g (0.142 mmol, 71 %) of the title compound.
¹H NMR (400 MHz, DMSO-*d*₆) ppm 1.39 (t, *J*=7.14 Hz, 3 H), 4.28 (q, *J*=7.14 Hz, 2 H), 7.18 - 7.24 (m, 1 H), 7.36 - 7.43 (m, 1 H), 7.46 (s, 1 H), 7.59 (t, *J*=7.79 Hz, 1 H), 7.70 (d, *J*=7.79 Hz, 1 H), 7.75 (d, *J*=7.79 Hz, 1 H), 7.99 (s, 1 H), 8.15 (s, 1 H), 8.40 (s, 1 H), 8.70 (s, 2 H), 9.70 (s, 1 H)
HPLC/MS (30min) retention time 17.66 min.
LRMS: *m*/*z* 576 (M+)

### EXAMPLE 87

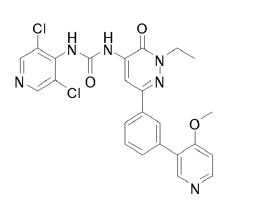

### N-(3,5-Dichloropyridin-4-yl)-N'-{2-ethyl-6-[3-(4-methoxypyridin-3-yl)phenyl]-3-oxo-2,3-dihydropyridazin-4-yl}urea

Reaction of 0.190 g of the title compound of Preparation 42 according to the method described in Preparation 7 gave 0.020 g (8%) of the title compound, after crystallisation with dichloromethane-methanol (7:3).
¹H NMR (400 MHz, DMSO-*d*₆) ppm 1.37 (t, *J*=6.85 Hz, 3 H), 3.86 (s, 3 H), 4.26 (q, 2 H), 7.19 (d, *J*=4.30 Hz, 1 H), 7.58 (s, 2 H), 7.73 - 7.83 (m, 1 H), 7.87 (s, 1 H), 8.37 (s, 1 H), 8.41 (s, 1 H), 8.48 (d, *J*=3.91 Hz, 1 H), 8.69 (s, 2 H), 9.71 (s, 1 H), 9.93 (s, 1 H)
HPLC/MS (30 min) retention time 11.32 min
LRMS: *m*/*z* 511 (M+)

### EXAMPLE 88

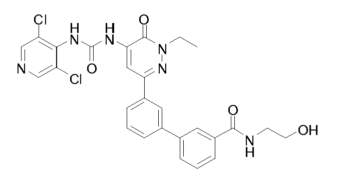

### 3'-[5-({[(3,5-dichloropyridin-4-yl)amino]carbonyl}amino)-1-ethyl-6-oxo-1,6-dihydropyridazin-3-yl]-N-(2-hydroxyethyl)biphenyl-3-carboxamide

Reaction of the title compound of Example 48 (400 mg, 0.69 mmol) with 2-aminoethanol (83.3 mg, 1.39 mmol) according to the method described in Example 49 gave 30 mg (8%) of the title compound after purification by SP1® automated purification system.
¹H NMR (400 MHz, DMSO-*d*₆) ppm 1.41 (t, *J*=7.04 Hz, 3 H) 3.50 - 3.65 (m, 4 H) 4.30 (q, *J*=6.39 Hz, 2 H) 7.55 - 7.70 (m, 2 H) 7.77 - 7.95 (m, 4 H) 8.06 (s, 1 H) 8.19 (s, 1 H) 8.43 (s, 1 H) 8.65 (d, 1 H) 8.68 (s, 2 H) 9.61 (br. s., 1 H)
HPLC/MS (30 min) retention time 16.02 min
LRMS: *m*/*z* 567 (M+)

### EXAMPLE 89

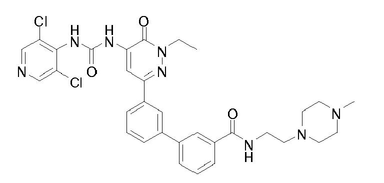

### 3'-[5-({[(3,5-dichloropyridin-4-yl)amino]carbonyl}amino)-1-ethyl-6-oxo-1,6-dihydropyridazin-3-yl]-N-[2-(4-methylpiperazin-1-yl)ethyl]biphenyl-3-carboxamide

Reaction of the title compound of Example 48 (250 mg, 0.42 mmol) with 2-(4-methylpiperazin-1-yl)ethaneamine (121.00 mg, 0.84 mmol) according to the method described in Example 49 gave 60 mg (22%) of the title compound.
¹H NMR (200 MHz, DMSO-*d*₆) ppm 1.39 (t, *J*=6.83 Hz, 3 H) 2.13 (s, 3 H) 2.31 (br. s., 6 H) 2.43 (br. s., 4 H) 3.40 (br. s., 2 H) 4.28 (q, 2 H) 7.61 (q, *J*=7.94 Hz, 2 H) 7.83 (t, *J*=8.39 Hz, 4 H) 8.03 (s, 1 H) 8.12 (s, 1 H) 8.40 (s, 1 H) 8.54 (br. s., 1 H) 8.69 (s, 2 H) 9.68 (br. s., 1 H) 9.90 (br. s., 1 H)
HPLC/MS (30 min) retention time 12.77 min
LRMS: *m*/*z* 649 (M+)

### EXAMPLE 90

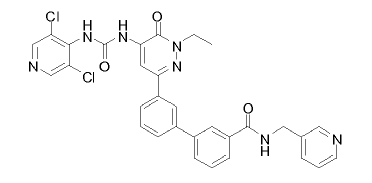

### 3'-[5-({[(3,5-dichloropyridin-4-yl)amino]carbonyl}amino)-1-ethyl-6-oxo-1,6-dihydropyridazin-3-yl]-N-(pyridin-3-ylmethyl)biphenyl-3-carboxamide

Reaction of the title compound of Example 48 (200 mg, 0.34 mmol) with pyridine-3-ylmethanamine (73.00 mg, 0.68 mmol) according to the method described in Example 49 gave 130 mg (62%) of the title compound.
¹H NMR (200 MHz, DMSO-*d*₆) ppm 1.39 (t, *J*=7.03 Hz, 3 H) 4.28 (q, 2 H) 4.53 (d, *J*=5.47 Hz, 2 H) 7.35 (m, 1 H) 7.54 - 7.71 (m, 2 H) 7.70 - 8.02 (m, 5 H) 8.04 (s, 1 H) 8.20 (s, 1 H) 8.40 (s, 1 H) 8.46 (d, *J*=3.90 Hz, 1 H) 8.58 (s, 1 H) 8.70 (s, 2 H) 9.17 - 9.35 (m, 1 H) 9.70 (br. s., 1 H) 9.91 (br. s., 1 H)
HPLC/MS (30 min) retention time 15.49 min
LRMS: *m*/*z* 614 (M+)

### EXAMPLE 91

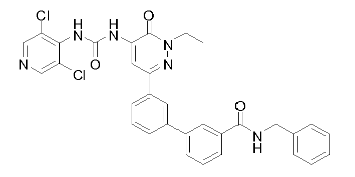

### N-benzyl-3'-[5-({[(3,5-dichloropyridin-4-yl)amino]carbonyl}amino)-1-ethyl-6-oxo-1,6-dihydropyridazin-3-yl]biphenyl-3-carboxamide

Reaction of the title compound of Example 48 (200 mg, 0.34 mmol) with pyridine-3-ylmethanamine (73.00 mg, 0.68 mmol) according to the method described in Example 49 gave 100 mg (48%) of the title compound after purification by SP1® automated purification system.
¹H NMR (400 MHz, DMSO-*d*₆) ppm 1.39 (t, *J*=7.04 Hz, 3 H) 4.28 (q, 2 H) 4.52 (d, *J*=5.48 Hz, 2 H) 7.24 (d, *J*=5.87 Hz, 1 H) 7.29 - 7.40 (m, 4 H) 7.55 - 7.71 (m, 2 H) 7.77 - 7.84 (m, 2 H) 7.87 (d, *J*=7.83 Hz, 1 H) 7.93 (d, *J*=7.43 Hz, 1 H) 8.05 (s, 1 H) 8.22 (s, 1 H) 8.41 (s, 1 H) 8.68 (s, 2 H) 9.20 (t, *J*=5.48 Hz, 1 H) 9.66 (br. s., 1 H) 9.92 (br. s., 1 H)
HPLC/MS (30 min) retention time 18.61 min
LRMS: *m*/*z* 613 (M+)

### EXAMPLE 92

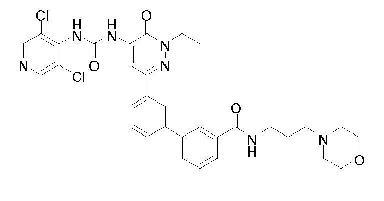

### 3'-[5-({[(3,5-dichloropyridin-4-yl)amino]carbonyl}amino)-1-ethyl-6-oxo-1,6-dihydropyridazin-3-yl]-N-(3-morpholin-4-ylpropyl)biphenyl-3-carboxamide

Reaction of the title compound of Example 48 (200 mg, 0.34 mmol) with 3-morpholinopropan-1-amine (98.00 mg, 0.68 mmol) according to the method described in Example 49 gave 70 mg (32%) of the title compound after purification by SP1® automated purification system.
¹H NMR (400 MHz, DMSO-*d*₆) ppm 1.39 (t, *J*=7.04 Hz, 3 H) 1.70 (quin, *J*=6.95 Hz, 2 H) 2.26 - 2.43 (m, 6 H) 3.32 (q, *J*=6.52 Hz, 2 H) 3.55 (t, *J*=4.11 Hz, 4 H) 4.28 (q, *J*=6.91 Hz, 2 H) 7.54 - 7.67 (m, 2 H) 7.76 - 7.92 (m, 4 H) 8.03 (s, 1 H) 8.13 (s, 1 H) 8.31 (br. s., 2 H) 8.40 (s, 1 H) 8.62 (t, *J*=5.09 Hz, 1 H) 8.67 (s, 2 H)
HPLC/MS (30 min) retention time 12.25 min
LRMS: *m*/*z* 650 (M+)

### EXAMPLE 93

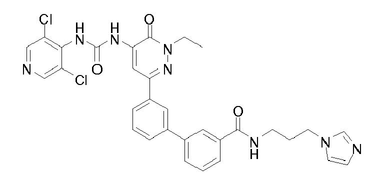

### 3'-[5-({[(3,5-dichloropyridin-4-yl)amino]carbonyl}amino)-1-ethyl-6-oxo-1,6-dihydropyridazin-3-yl]-N-[3-(1H-imidazol-1-yl)propyl]biphenyl-3-carboxamide

Reaction of the title compound of Example 48 (200 mg, 0.34 mmol) with 3-(1H-imidazol-1-yl)propan-1-amine (85.00 mg, 0.68 mmol) according to the method described in Example 49 gave 90 mg (42%) of the title compound after purification by SP1® automated purification system.
¹H NMR (400 MHz, DMSO-*d*₆) ppm 1.39 (t, *J*=7.04 Hz, 3 H) 1.98 (dq, *J*=7.04, 6.78 Hz, 2 H) 3.27 (q, *J*=6.52 Hz, 2 H) 4.03 (t, *J*=6.85 Hz, 2 H) 4.28 (q, *J*=7.30 Hz, 2 H) 6.89 (s, 1 H) 7.21 (s, 1 H) 7.56 - 7.71 (m, 3 H) 7.75 - 7.93 (m, 4 H) 8.04 (s, 1 H) 8.14 (s, 1 H) 8.28 (br. s., 2 H) 8.41 (s, 1 H) 8.65 (t, *J*=5.48 Hz, 1 H) 8.68 (s, 2 H)
HPLC/MS (30 min) retention time 12.46 min
LRMS: *m*/*z* 631 (M+)

### EXAMPLE 94

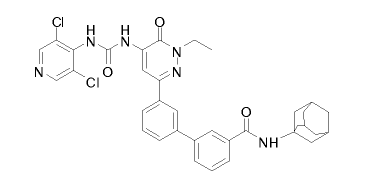

### N-1-adamantyl-3'-[5-({[(3,5-dichloropyridin-4-yl)amino]carbonyl}amino)-1-ethyl-6-oxo-1,6-dihydropyridazin-3-yl]biphenyl-3-carboxamide

The title compound of Example 48 (200 mg, 0.34 mmol) and 1-adamantylamine (103.00 mg, 0.68 mmol) were added to a solution of O-(Benzotriazol-1-yl)-N,N,N',N'-tetramethyluronium hexafluorphosphate (129 mg, 0.34 mmol) and N,N-diisopropylethylamine (59 mg, 0.34mmol) in dimethylformamide (5 mL). The mixture was stirred at room temperature for 15 h.

The solvent was removed under reduced pressure and 4% sodium bicarbonate solution was added. The precipitate was filtrated, washed with water and dried in a vacuum oven. The product was purified by crystallization from dichloromethane-methanol (8:2) and by SP1® automated purification system to yields the title compound (110 mg, 49%).
¹H NMR (400 MHz, DMSO-*d*₆) ppm 1.39 (t, *J*=7.04 Hz, 3 H) 1.66 (br. s., 6 H) 1.98 - 2.17 (m, 9 H) 4.28 (q, *J*=7.04 Hz, 2 H) 7.55 (t, *J*=7.63 Hz, 1 H) 7.62 (t, *J*=7.83 Hz, 1 H) 7.71 - 7.85 (m, 5 H) 8.03 (s, 2 H) 8.41 (s, 1 H) 8.68 (s, 2 H) 9.68 (br. s., 1 H) 9.90 (br. s., 1 H)
HPLC/MS (30 min) retention time 21.13 min
LRMS: *m*/*z* 657 (M+)

### EXAMPLE 95

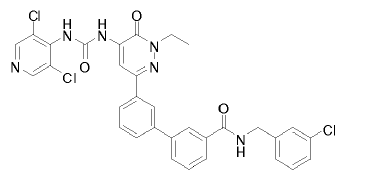

### N-(3-chlorobenzyl)-3'-[5-({[(3,5-dichloropyridin-4-yl)amino]carbonyl}amino)-1-ethyl-6-oxo-1,6-dihydropyridazin-3-yl]biphenyl-3-carboxamide

Reaction of the title compound of Example 48 (200 mg, 0.34 mmol) with (3-chloroohenyl)methanamine (96.00 mg, 0.68 mmol) according to the method described in Example 49 gave 75 mg (34%) of the title compound after purification by SP1® automated purification system.
¹H NMR (400 MHz, DMSO-*d*₆) ppm 1.39 (t, *J*=7.24 Hz, 3 H) 4.28 (q, *J*=7.04 Hz, 2 H) 4.51 (d, *J*=5.87 Hz, 2 H) 7.26 - 7.43 (m, 4 H) 7.57 - 7.68 (m, 2 H) 7.81 (t, *J*=6.46 Hz, 2 H) 7.88 (d, *J*=7.83 Hz, 1 H) 7.93 (d, *J*=7.83 Hz, 1 H) 8.05 (s, 1 H) 8.21 (s, 1 H) 8.40 (s, 1 H) 8.68 (s, 2 H) 9.22 (t, *J*=5.87 Hz, 1 H) 9.68 (br. s., 1 H) 9.90 (br. s., 1 H)
HPLC/MS (30 min) retention time 19.53 min
LRMS: *m*/*z* 647 (M+)

### EXAMPLE 96

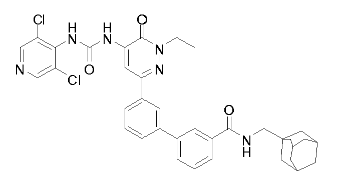

### N-(1-adamantylmethyl)-3'-[5-({[(3,5-dichloropyridin-4-yl)amino]carbonyl}amino)-1-ethyl-6-oxo-1,6-dihydropyridazin-3-yl]biphenyl-3-carboxamide

Reaction of the title compound of Example 48 (200 mg, 0.34 mmol) with 1-adamantanemethylamine (112.00 mg, 0.68 mmol) according to the method described in Example 94 gave 110 mg (48%) of the title compound.
¹H NMR (400 MHz, DMSO-*d*₆) ppm 1.39 (t, *J*=7.24 Hz, 3 H) 1.51 (br. s., 6 H) 1.53 - 1.71 (m, 6 H) 1.91 (br. s., 3 H) 3.02 (d, *J*=6.26 Hz, 2 H) 4.28 (q, *J*=7.04 Hz, 2 H) 7.55 - 7.66 (m, 2 H) 7.78 - 7.92 (m, 4 H) 8.05 (s, 1 H) 8.15 (s, 1 H) 8.41 (s, 1 H) 8.45 (t, *J*=6.06 Hz, 1 H) 8.69 (s, 2 H) 9.69 (br. s., 1 H) 9.91 (br. s., 1 H)
HPLC/MS (30 min) retention time 21.41 min
LRMS: *m*/*z* 671 (M+)

### EXAMPLE 97

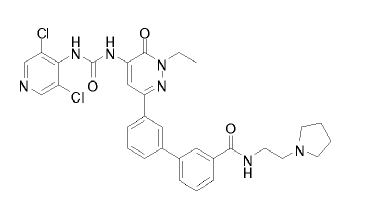

### 3'-[5-({[(3,5-dichloropyridin-4-yl)amino]carbonyl}amino)-1-ethyl-6-oxo-1,6-dihydropyridazin-3-yl]-N-(2-pyrrolidin-1-ylethyl)biphenyl-3-carboxamide

Reaction of the title compound of Example 48 (200 mg, 0.34 mmol) with 2-(pyrrolidinyl)ethanamine (77.00 mg, 0.67 mmol) according to the method described in Example 49 gave 87 mg (41%) of the title compound after purification by SP1® automated purification system.
¹H NMR (400 MHz, DMSO-*d*₆) ppm 1.39 (t, *J*=7.24 Hz, 3 H) 1.60 - 1.76 (m, 4 H) 2.50 - 2.56 (m, 4 H) 2.64 (t, *J*=6.85 Hz, 2 H) 3.42 (q, *J*=6.65 Hz, 2 H) 4.28 (q, *J*=7.04 Hz, 2 H) 7.52 - 7.71 (m, 2 H) 7.76 - 7.92 (m, 4 H) 8.04 (s, 1 H) 8.15 (s, 1 H) 8.33 (br. s., 2 H) 8.41 (s, 1 H) 8.66 (t, 1 H) 8.68 (s, 2 H)
HPLC/MS (30 min) retention time 12.24 min
LRMS: *m*/*z* 620 (M+)

### EXAMPLE 98

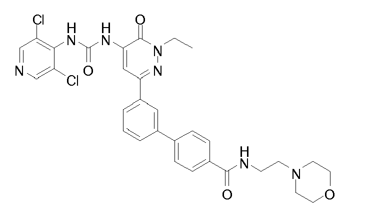

### 3'-[5-({[(3,5-dichloropyridin-4-yl)amino]carbonyl}amino)-1-ethyl-6-oxo-1,6-dihydropyridazin-3-yl]-N-(2-morpholin-4-ylethyl)biphenyl-4-carboxamide

Reaction of the title compound of Example 26 (200 mg, 0.37 mmol) with 2-morpholinoethanamine (97 mg, 0.75 mmol) according to the method described in Example 49 gave 110 mg (46%) of the title compound.
¹H NMR (200 MHz, DMSO-*d*₆) ppm 1.39 (t, *J*=6.83 Hz, 3 H) 2.35 - 2.46 (m, 6 H) 3.36 - 3.49 (m, 2 H) 3.58 (br. s., 4 H) 4.28 (q, 2 H) 7.62 (t, 1 H) 7.80 (d, *J*=7.03 Hz, 4 H) 7.95 (d, *J*=8.20 Hz, 2 H) 8.04 (br. s., 1 H) 8.41 (s, 1 H) 8.47 (t, *J*=5.66 Hz, 1 H) 8.70 (s, 2 H) 9.71 (br. s., 1 H) 9.90 (s, 1 H)
HPLC/MS (30 min) retention time 12.14 min
LRMS: *m*/*z* 636 (M+)

### EXAMPLE 99

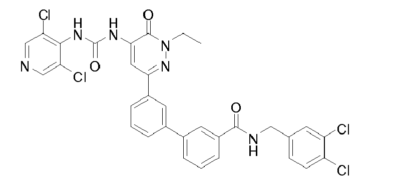

### N-(3,4-dichlorobenzyl)-3'-[5-({[(3,5-dichloropyridin-4-yl)amino]carbonyl}amino)-1-ethyl-6-oxo-1,6-dihydropyridazin-3-yl]biphenyl-3-carboxamide

Reaction of the title compound of Example 48 (200 mg, 0.34 mmol) with (3,4-dichlorophenyl)methanamine (119 mg, 0.68 mmol) according to the method described in Example 49 gave 130 mg (56%) of the title compound after purification by SP1® automated purification system.
¹H NMR (400 MHz, DMSO-*d*₆) ppm 1.38 (t, *J*=7.04 Hz, 3 H) 4.28 (q, *J*=7.04 Hz, 2 H) 4.50 (d, *J*=5.87 Hz, 2 H) 7.35 (d, *J*=1.57 Hz, 1 H) 7.55 - 7.69 (m, 4 H) 7.81 (t, *J*=6.26 Hz, 2 H) 7.90 (dd, *J*=15.06, 7.63 Hz, 2 H) 8.04 (s, 1 H) 8.20 (s, 1 H) 8.40 (s, 1 H) 8.69 (s, 2 H) 9.26 (t, *J*=5.87 Hz, 1 H) 9.70 (br. s., 1 H) 9.92 (br. s., 1 H)
HPLC/MS (30 min) retention time 20.24 min
LRMS: *m*/*z* 682 (M+)

### EXAMPLE 100

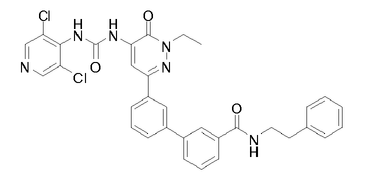

### 3'-[5-({[(3,5-dichloropyridin-4-yl)amino]carbonyl}amino)-1-ethyl-6-oxo-1,6-dihydropyridazin-3-yl]-N-(2-phenylethyl)biphenyl-3-carboxamide

Reaction of the title compound of Example 48 (175 mg, 0.30 mmol) with 2-phenylethaneamine (73 mg, 0.60 mmol) according to the method described in Example 49 gave 108 mg (57%) of the title compound after purification by SP1® automated purification system.
¹H NMR (400 MHz, DMSO-*d*₆) ppm 1.39 (t, *J*=7.04 Hz, 3 H) 2.87 (t, *J*=7.43 Hz, 2 H) 3.45 - 3.57 (m, 2 H) 4.29 (q, *J*=7.04 Hz, 2 H) 7.20 (d, *J*=7.04 Hz, 1 H) 7.23 - 7.36 (m, 4 H) 7.53 - 7.69 (m, 2 H) 7.83 (dd, *J*=18.00, 7.83 Hz, 4 H) 8.04 (s, 1 H) 8.11 (s, 1 H) 8.41 (s, 1 H) 8.68 (s, 2 H) 8.73 (t, *J*=5.28 Hz, 1 H) 9.70 (br. s., 1 H) 9.92 (br. s., 1 H)
HPLC/MS (30 min) retention time 19.08 min
LRMS: *m*/*z* 627 (M+)

### EXAMPLE 101

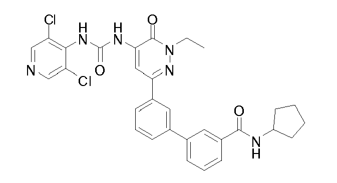

### N-cyclopentyl-3'-[5-({[(3,5-dichloropyridin-4-yl)amino]carbonyl}amino)-1-ethyl-6-oxo-1,6-dihydropyridazin-3-yl]biphenyl-3-carboxamide

Reaction of the title compound of Example 48 (200 mg, 0.34 mmol) with cyclopentanamine (58 mg, 0.68 mmol) according to the method described in Example 49 gave 111 mg (55%) of the title compound after purification by SP1® automated purification system.
¹H NMR (400 MHz, DMSO-*d*₆) ppm 1.39 (t, *J*=7.24 Hz, 3 H) 1.46 - 1.63 (m, 4 H) 1.63 - 1.78 (m, 2 H) 1.82 - 1.96 (m, 2 H) 4.18 - 4.34 (m, 3 H) 7.51 - 7.68 (m, 3 H) 7.76 - 7.93 (m, 3 H) 8.04 (s, 1 H) 8.12 (s, 1 H) 8.41 (s, 1 H) 8.44 (br. s., 1 H) 8.70 (s, 2 H) 9.69 (br. s., 1 H) 9.91 (br. s., 1 H)
HPLC/MS (30 min) retention time 18.91 min
LRMS: *m*/*z* 591 (M+)

### EXAMPLE 102

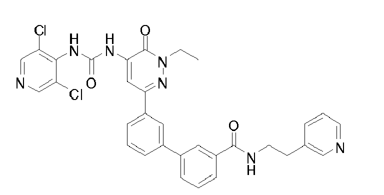

### 3'-[5-({[(3,5-dichloropyridin-4-yl)amino]carbonyl}amino)-1-ethyl-6-oxo-1,6-dihydropyridazin-3-yl]-N-(2-pyridin-3-ylethyl)biphenyl-3-carboxamide

Reaction of the title compound of Example 48 (175 mg, 0.30 mmol) with 2-(pyridin-3-yl)ethanamine (73 mg, 0.60 mmol) according to the method described in Example 49 gave 110 mg (57%) of the title compound after purification by SP1® automated purification system.
¹H NMR (400 MHz, DMSO-*d*₆) ppm 1.37 (t, *J*=7.24 Hz, 3 H) 2.87 (t, *J*=7.04 Hz, 2 H) 3.52 (q, *J*=6.65 Hz, 2 H) 4.27 (q, *J*=7.04 Hz, 2 H) 7.29 (dd, *J*=7.63, 4.89 Hz, 2 H) 7.50 - 7.71 (m, 3 H) 7.73 - 7.87 (m, 3 H) 8.01 (s, 1 H) 8.08 (s, 1 H) 8.36 - 8.42 (m, 2 H) 8.45 (s, 1 H) 8.66 (s, 2 H) 8.71 (t, *J*=5.48 Hz, 1 H) 9.67 (br. s., 1 H) 9.88 (br. s., 1 H)
HPLC/MS (30 min) retention time 14.69 min
LRMS: *m*/*z* 628 (M+)

### EXAMPLE 103

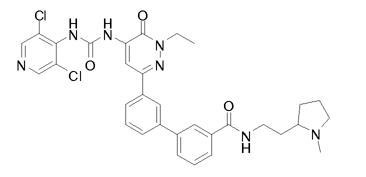

### 3'-[5-({[(3,5-dichloropyridin-4-yl)amino]carbonyl}amino)-1-ethyl-6-oxo-1,6-dihydropyridazin-3-yl]-N-[2-(1-methylpyrrolidin-2-yl)ethyl]biphenyl-3-carboxamide

Reaction of the title compound of Example 48 (175 mg, 0.30 mmol) with 2-(1-methylpyrrolidin-2-yl)ethanamine (77 mg, 0.60 mmol) according to the method described in Example 49 gave 70 mg (37%) of the title compound after purification by SP1® automated purification system.
¹H NMR (400 MHz, DMSO-*d*₆) ppm 1.39 (t, *J*=7.04 Hz, 3 H) 1.42 - 1.56 (m, 2 H) 1.58 - 1.73 (m, 2 H) 1.84 - 2.05 (m, 2 H) 2.07 - 2.22 (m, 2 H) 2.25 (s, 3 H) 2.94 - 3.03 (m, 1 H) 3.32 (q, *J*=6.65 Hz, 2 H) 4.28 (q, *J*=7.04 Hz, 2 H) 7.54 - 7.67 (m, 2 H) 7.73 - 7.92 (m, 4 H) 8.03 (s, 1 H) 8.13 (s, 1 H) 8.31 (br. s., 1 H) 8.41 (s, 1 H) 8.68 (s, 3 H)
HPLC/MS (30 min) retention time 12.39 min
LRMS: *m*/*z* 634 (M+)

### EXAMPLE 104

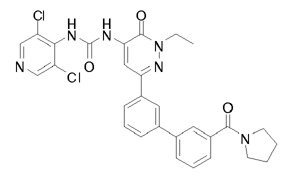

### N-(3,5-dichloropyridin-4-yl)-N'-{2-ethyl-3-oxo-6-[3'-(pyrrolidin-1-ylcarbonyl)biphenyl-3-yl]-2,3-dihydropyridazin-4-yl}urea

Reaction of the title compound of Example 48 (175 mg, 0.30 mmol) with pyrrolidine (43 mg, 0.60 mmol) according to the method described in Example 49 gave 120 mg (69%) of the title compound.
¹H NMR (400 MHz, DMSO-*d*₆) ppm 1.39 (t, *J*=7.04 Hz, 3 H) 1.70 - 1.91 (m, 4 H) 3.38 - 3.53 (m, 4 H) 4.28 (q, *J*=7.04 Hz, 2 H) 7.47 - 7.65 (m, 3 H) 7.72 - 7.87 (m, 4 H) 8.00 (s, 1 H) 8.42 (s, 1 H) 8.70 (s, 2 H) 9.70 (s, 1 H) 9.90 (s, 1 H)
HPLC/MS (30 min) retention time 18.31 min
LRMS: *m*/*z* 577 (M+)

### EXAMPLE 105

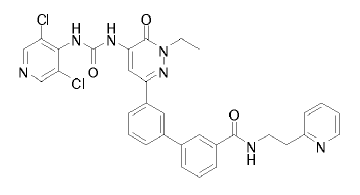

### 3'-[5-({[(3,5-dichloropyridin-4-yl)amino]carbonyl}amino)-1-ethyl-6-oxo-1,6-dihydropyridazin-3-yl]-N-(2-pyridin-2-ylethyl)biphenyl-3-carboxamide

Reaction of the title compound of Example 48 (175 mg, 0.30 mmol) with 2-(pyridin-2-yl)ethanamine (73 mg, 0.60 mmol) according to the method described in Example 49 gave 115 mg (61%) of the title compound after purification by SP1® automated purification system.
¹H NMR (400 MHz, DMSO-*d*₆) ppm 1.39 (t, *J*=7.04 Hz, 3 H) 3.02 (t, *J*=7.43 Hz, 2 H) 3.64 (q, *J*=6.91 Hz, 2 H) 4.29 (q, *J*=7.04 Hz, 2 H) 7.21 (dd, *J=*7.43, 5.09 Hz, 1 H) 7.29 (d, *J*=7.83 Hz, 1 H) 7.51 - 7.75 (m, 3 H) 7.76 - 7.90 (m, 4 H) 8.03 (s, 1 H) 8.11 (s, 1 H) 8.41 (s, 1 H) 8.51 (d, *J*=4.70 Hz, 1 H) 8.68 (s, 2 H) 8.74 (t, *J*=5.48 Hz, 1 H) 9.65 (br. s., 2 H)
HPLC/MS (30 min) retention time 15.13 min
LRMS: *m*/*z* 628 (M+)

### EXAMPLE 106

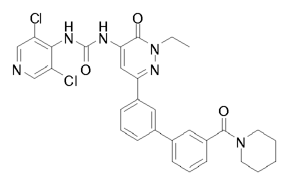

### N-(3,5-dichloropyridin-4-yl)-N'-{2-ethyl-3-oxo-6-[3'-(piperidin-1-ylcarbonyl)biphenyl-3-yl]-2,3-dihydropyridazin-4-yl}urea

Reaction of the title compound of Example 48 (175 mg, 0.30 mmol) with piperidine (51 mg, 0.60 mmol) according to the method described in Example 49 gave 100 mg (55%) of the title compound after purification by SP1® automated purification system.
¹H NMR (400 MHz, DMSO-*d*₆) ppm 1.38 (t, *J*=7.04 Hz, 3 H) 1.57 (br. s., 6 H) 3.34 (s, 2 H) 3.59 (br. s., 2 H) 4.28 (q, *J*=7.04 Hz, 2 H) 7.39 (d, *J*=7.43 Hz, 1 H) 7.59 (dt, *J*=18.49, 7.78 Hz, 3 H) 7.72 - 7.85 (m, 3 H) 8.01 (s, 1 H) 8.42 (s, 1 H) 8.69 (s, 2 H) 9.69 (br. s., 1 H) 9.90 (br. s., 1 H)
HPLC/MS (30 min) retention time 18.91 min
LRMS: *m*/*z* 591 (M+)

### EXAMPLE 107

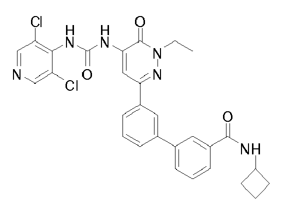

### N-cyclobutyl-3'-[5-({[(3,5-dichloropyridin-4-yl)amino]carbonyl}amino)-1-ethyl-6-oxo-1,6-dihydropyridazin-3-yl]biphenyl-3-carboxamide

Reaction of the title compound of Example 48 (175 mg, 0.30 mmol) with cyclobutanamine (43 mg, 0.60 mmol) according to the method described in Example 49 gave 59 mg (34%) of the title compound after purification by SP1® automated purification system.
¹H NMR (400 MHz, DMSO-*d*₆) ppm 1.39 (t, *J*=7.04 Hz, 3 H) 1.61 - 1.73 (m, 2 H) 2.08 (quin, *J*=9.39 Hz, 2 H) 2.16 - 2.31 (m, 2 H) 4.28 (q, *J*=7.04 Hz, 2 H) 4.45 (sxt, *J*=8.14 Hz, 1 H) 7.50 - 7.68 (m, 2 H) 7.74 - 7.94 (m, 4 H) 8.04 (s, 1 H) 8.14 (s, 1 H) 8.42 (s, 1 H) 8.68 (s, 2 H) 8.74 (d, *J*=7.83 Hz, 1 H) 9.66 (br. s., 1 H) 9.92 (br. s., 1 H)
HPLC/MS (30 min) retention time 18.33 min
LRMS: *m*/*z* 577 (M+)

### EXAMPLE 108

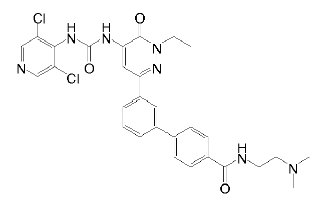

### 3'-[5-({[(3,5-dichloropyridin-4-yl)amino]carbonyl}amino)-1-ethyl-6-oxo-1,6-dihydropyridazin-3-yl]-N-[2-(dimethylamino)ethyl]biphenyl-4-carboxamide

Reaction of the title compound of Example 26 (200 mg, 0.38 mmol) with *N*,*N-*dimethylethane-1,2-diamine (67.2 mg, 0.76 mmol) according to the method described in Example 49 gave 206 mg (91%) of the title compound after purification by SP1® automated purification system.
¹H NMR (400 MHz, DMSO-*d*₆) ppm 1.38 (t, *J*=7.04 Hz, 3 H) 2.19 (s, 6 H) 2.42 (t, *J*=6.85 Hz, 2 H) 3.32 - 3.40 (m, 2 H) 4.27 (q, *J*=7.04 Hz, 2 H) 7.61 (t, *J*=7.63 Hz, 1 H) 7.78 (d, *J*=1.96 Hz, 2 H) 7.79 - 7.81 (m, 2 H) 7.93 (s, 1 H) 7.95 (s, 1 H) 8.03 (s, 1 H) 8.24 (s, 1 H) 8.40 (s, 1 H) 8.46 (t, *J*=5.67 Hz, 1 H) 8.68 (s, 2 H)
HPLC/MS (15 min) retention time 6.29 min
LRMS: *m*/*z* 594 (M+)

### EXAMPLE 109

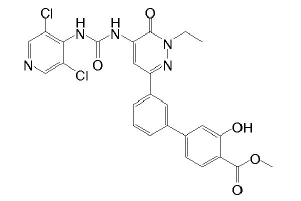

### Methyl 3'-[5-({[(3,5-dichloropyridin-4-yl)amino]carbonyl}amino)-1-ethyl-6-oxo-1,6-dihydropyridazin-3-yl]-3-hydroxybiphenyl-4-carboxylate

Reaction of the title compound of Preparation 7 (350 mg, 0.72 mmol) with methyl 2-hydroxy-4-(4,4,5,5-tetramethyl-1,3,2-dioxaborolan-2-yl)benzoate (241 mg, 0.87 mmol) according to the method described in Example 1 gave 267 mg (44%) of the title compound after triturating with diethyl ether.
HPLC/MS (9 min) retention time 7.73 min
LRMS: *m*/*z* 552 (M-)

### EXAMPLE 110

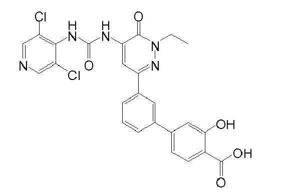

### 3'-[5-({[(3,5-dichloropyridin-4-yl)amino]carbonyl}amino)-1-ethyl-6-oxo-1,6-dihydropyridazin-3-yl]-3-hydroxybiphenyl-4-carboxylic acid

Reaction of the title compound of Example 109 (267 mg, 0.48 mmol) according to the method described in Example 26 gave 12.4 mg (5%) of the title compound after purification by SP1® automated purification system.
¹H NMR (400 MHz, DMSO-*d*₆) ppm 1.38 (t, *J*=7.04 Hz, 3 H) 4.27 (q, *J*=7.04 Hz, 2 H) 6.87 - 6.92 (m, 2 H) 7.55 (t, *J*=7.83 Hz, 1 H) 7.71 (t, *J*=9.00 Hz, 2 H) 7.98 (s, 1 H) 8.42 (d, *J*=2.35 Hz, 2 H) 8.61 (s, 2 H) 10.11 (s, 1 H)
HPLC/MS (30 min) retention time 16.8 min
LRMS: *m*/*z* 538 (M-)

### EXAMPLE 111

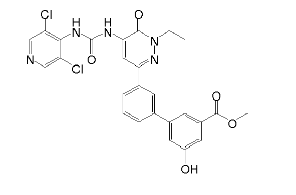

### Methyl 3'-[5-({[(3,5-dichloropyridin-4-yl)amino]carbonyl}amino)-1-ethyl-6-oxo-1,6-dihydropyridazin-3-yl]-5-hydroxybiphenyl-3-carboxylate

Reaction of the title compound of Preparation 7 (2 g, 4.14 mmol) with (1.73 g, 6.21 mmol) of the title compound of Preparation 53 according to the method described in Example 1 gave 1.12 g (40 %) of the title compound after triturating with methanol.
HPLC/MS (9 min) retention time 6.97min
LRMS: *m*/*z* 554 (M+)

### EXAMPLE 112

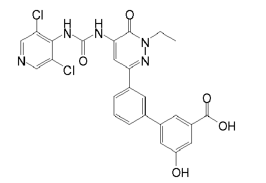

### 3'-[5-({[(3,5-dichloropyridin-4-yl)amino]carbonyl}amino)-1-ethyl-6-oxo-1,6-dihydropyridazin-3-yl]-5-hydroxybiphenyl-3-carboxylic acid

Reaction of the title compound of Example 111 (1.12 g, 2.02 mmol) according to the method described in Example 26 gave 0.90 g (74.2%) of the title compound.
¹H NMR (400 MHz, DMSO-*d*₆) ppm 1.39 (t, *J*=7.04 Hz, 3 H) 4.28 (q, *J*=7.17 Hz, 2 H) 7.27 (s, 1 H) 7.37 (s, 1 H) 7.60 (t, *J*=7.83 Hz, 1 H) 7.65 (s, 1 H) 7.72 (d, *J*=7.83 Hz, 1 H) 7.79 (d, *J*=7.83 Hz, 1 H) 7.95 (s, 1 H) 8.40 (s, 1 H) 8.70 (s, 2 H) 9.70 (s, 1 H) 10.00 (s, 1 H)
HPLC/MS (30 min) retention time 16.2 min
LRMS: *m*/*z* 540 (M+)

### EXAMPLE 113

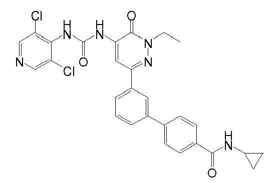

### N-cyclopropyl-3'-[5-({[(3,5-dichloropyridin-4-yl)amino]carbonyl}amino)-1-ethyl-6-oxo-1,6-dihydropyridazin-3-yl]biphenyl-4-carboxamide

Reaction of the title compound of Example 26 (200 mg, 0.38 mmol) with *N* cyclopropanamine (47.5 µl, 0.69 mmol) according to the method described in Example 49 gave 122 mg (56%) of the title compound after purification by SP1® automated purification system.
¹H NMR (200 MHz, DMSO-*d*₆) ppm 0.47 - 0.65 (m, 2 H) 0.65 - 0.79 (m, 2 H) 1.39 (t, 3 H) 2.70 - 3.04 (m, 1 H) 4.28 (q, *J*=7.16 Hz, 2 H) 7.52 - 7.67 (m, 1 H) 7.72 - 7.85 (m, 4 H) 7.91 (s, 1 H) 7.95 (s, 1 H) 8.03 (s, 1 H) 8.41 (s, 1 H) 8.49 (d, *J*=4.29 Hz, 1 H) 8.68 (s, 2 H) 9.65 (s, 1 H)
HPLC/MS (30 min) retention time 17.22 min
LRMS: *m*/*z* 563 (M+)

### EXAMPLE 114

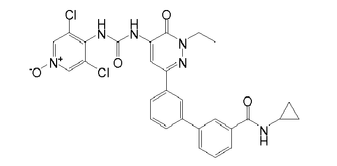

### N-cyclopropyl-3'-[5-({[(3,5-dichloro-1-oxidopyridin-4-yl)amino]carbonyl}amino)-1-ethyl-6-oxo-1,6-dihydropyridazin-3-yl]biphenyl-3-carboxamide

To a solution of the title compound of example 21 (300 mg, 1.06 mmols) in 60 ml of dichloromethane is added 3-chloroperoxybenzoic acid (239 mg, 1.06 mmols), the reaction mixture was stirred under nitrogen at room temperature during eleven days.

The reaction mixture was diluted with dichloromethane, washed with saturated aqueous NaHCO3 (x3), dried over sodium sulphate and evaporated under reduced pressure to afford a solid. The solid was purified by the SP1® automated purification system to give 25 mg (8%) of the title compound.
¹H NMR (400 MHz, DMSO-*d*₆) ppm 0.54 - 0.65 (m, 2 H) 0.66 - 0.76 (m, 2 H) 1.38 (t, *J*=7.04 Hz, 3 H) 2.80 - 2.95 (m, 1 H) 4.28 (q, *J*=7.04 Hz, 2 H) 7.54 - 7.66 (m, 2 H) 7.76 - 7.87 (m, 3 H) 8.03 (s, 1 H) 8.10 (s, 1 H) 8.40 (s, 1 H) 8.58 (d, *J*=4.30 Hz, 1 H) 8.68 (s, 2 H) 9.65 (s, 2 H)
HPLC/MS (30 min) retention time 15.63 min
LRMS: *m*/*z* 579 (M+)

### EXAMPLE 115

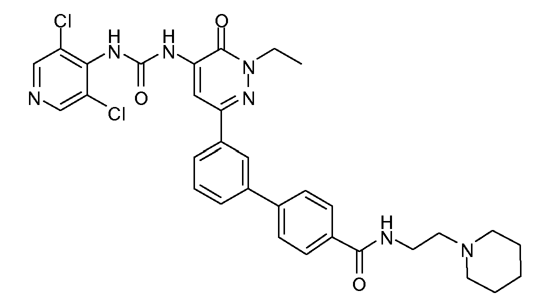

### 3'-(5-(3-(3,5-dichloropyridin-4-yl)ureido)-1-ethyl-6-oxo-1,6-dihydropyridazin-3-yl)-N-(2-(piperidin-1-yl)ethyl)biphenyl-4-carboxamide

Reaction of the title compound of Example 26 (200 mg, 0.38 mmol) with 2-(piperidin-1-yl)ethanamine (109 µl, 0.76 mmol) according to the method described in Example 49 gave 47 mg (19%) of the title compound.
¹H NMR (200 MHz, DMSO-*d*₆) ppm 1.28 (t, *J*=7.22 Hz, 3 H) 1.42 - 1.81 (m, 6 H) 2.35 - 2.50 (m, 4 H) 2.58 (t, *J*=5.86 Hz, 2 H) 3.36 - 3.68 (m, 2 H) 4.25 (q, *J*=7.42 Hz, 2 H) 6.90 - 7.15 (m, 1 H) 7.42 - 7.61 (m, 2 H) 7.59 - 7.76 (m, 3 H) 7.77 - 7.97 (m, 3 H) 8.06 (s, 1 H) 8.62 (s, 2 H) 8.73 (s, 1 H) 9.52 (s, 1 H)
HPLC/MS (30 min) retention time 12.23 min
LRMS: *m*/*z* 634 (M+)

### EXAMPLE 116

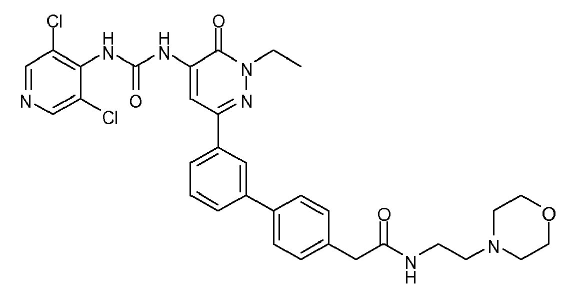

### 2-(3'-(5-(3-(3,5-dichloropyridin-4-yl)ureido)-1-ethyl-6-oxo-1,6-dihydropyridazin-3-yl)biphenyl-4-yl)-N-(2-morpholinoethyl)acetamide

Reaction of the title compound of Example 40 (300 mg, 0.56 mmol) with 2-morpholinoethanamine (146 µl, 1.11 mmol) according to the method described in Example 49 gave 141 mg (38.9%) of the title compound.
¹H NMR (200 MHz, DMSO-*d*₆) ppm 1.29 (t, 3 H) 2.40 (s, 4 H) 2.54 - 2.78 (m, 4 H) 3.47 (q, *J*=5.21 Hz, 2 H) 3.55 - 3.78 (m, 4 H) 4.19 (q, *J*=7.16 Hz, 2 H) 6.59 (s, 1 H) 7.28 - 7.47 (m, 3 H) 7.47 - 7.71 (m, 4 H) 7.94 (s, 1 H) 8.49 - 8.68 (m, 3 H) 9.39 (s, 1 H) 9.63 (s, 1 H)
HPLC/MS (30 min) retention time 12.34 min
LRMS: *m*/*z* 650 (M+)

### EXAMPLE 117

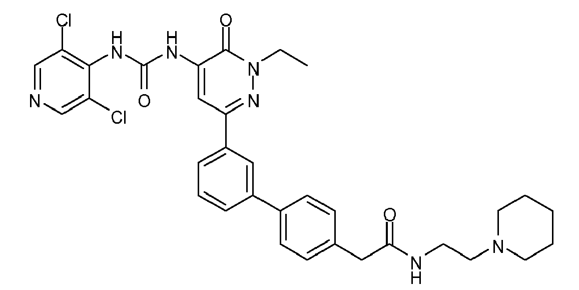

### 2-(3'-(5-(3-(3,5-dichloropyridin-4-yl)ureido)-1-ethyl-6-oxo-1,6-dihydropyridazin-3-yl)biphenyl-4-yl)-N-(2-(piperidin-1-yl)ethyl)acetamide

Reaction of the title compound of Example 40 (300 mg, 0.56 mmol) with 2-(piperidin-1-yl)ethanamine (160 µl, 1.12 mmol) according to the method described in Example 49 gave 185 mg (51.2%) of the title compound.
¹H NMR (200 MHz, DMSO-*d*₆) ppm 1.27 (t, 3 H) 1.48 - 2.06 (m, 8 H) 3.09 - 3.35 (m, 4 H) 3.66 (s, 2 H) 3.73 (bs, 2 H) 4.05 (q, 2 H) 7.12 - 7.24 (m, 3 H) 7.28 - 7.47 (m, 4 H) 7.82 (bs, 2 H) 8.32 (s, 1 H) 8.58 (s, 2 H) 8.80 (bs, 1 H) 9.08 (bs, 1 H)
HPLC/MS (30 min) retention time 12.64 min
LRMS: *m*/*z* 648 (M+)

### EXAMPLE 118

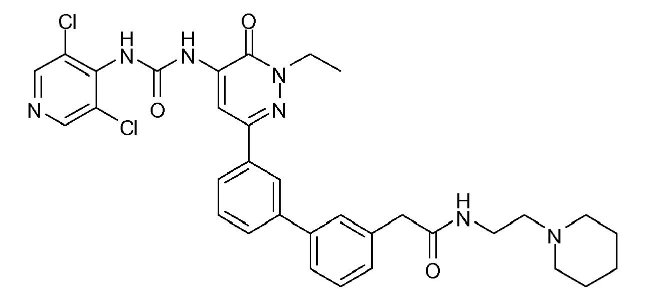

### 2-(3'-(5-(3-(3,5-dichloropyridin-4-yl)ureido)-1-ethyl-6-oxo-1,6-dihydropyridazin-3-yl)biphenyl-3-yl)-N-(2-(piperidin-1-yl)ethyl)acetamide

Reaction of the title compound of Example 42 (300 mg, 0.56 mmol) with 2-(piperidin-1-yl)ethanamine (160 µl, 1.12 mmol) according to the method described in Example 49 gave 146 mg (40.4%) of the title compound.
¹H NMR (200 MHz, DMSO-*d*₆) ppm 1.21 (t, *J*=6.64 Hz, 3 H) 1.41 - 1.66 (m, 2 H) 1.64 - 1.95 (m, 4 H) 3.03 (d, *J*=21.47 Hz, 6 H) 3.55 - 3.79 (m, 4 H) 3.95 (q, *J*=6.64 Hz, 2 H) 7.12 - 7.53 (m, 4 H) 7.53 - 7.76 (m, 3 H) 7.88 (s, 1 H) 8.24 (bs, 1 H) 8.36 (s, 1 H) 8.56 (s, 2 H) 8.78 (bs, 1 H) 9.36 (bs, 1 H)
HPLC/MS (30 min) retention time 12.88 min
LRMS: *m*/*z* 648 (M+)

### EXAMPLE 119

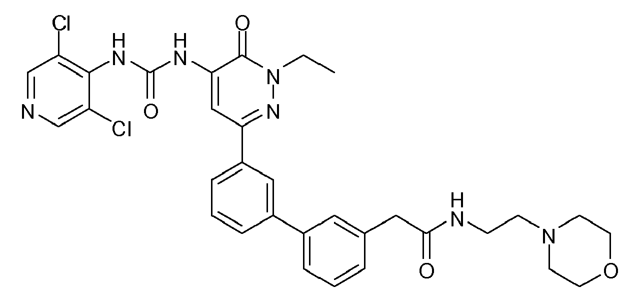

### 2-(3'-(5-(3-(3,5-dichloropyridin-4-yl)ureido)-1-ethyl-6-oxo-1,6-dihydropyridazin-3-yl)biphenyl-3-yl)-N-(2-morpholinoethyl)acetamide

Reaction of the title compound of Example 42 (300 mg, 0.56 mmol) with 2-morpholinoethanamine (146 µl, 1.11 mmol) according to the method described in Example 49 gave 244 mg (67.3%) of the title compound.
¹H NMR (200 MHz, DMSO-*d*₆) ppm 1.24 (t, *J*=7.29 Hz, 3 H) 2.55 - 2.97 (m, 5 H) 3.31 - 3.59 (m, 2 H) 3.60 - 3.80 (m, 4 H) 4.08 (q, *J*=7.29 Hz, 2 H) 5.08 (s, 3 H) 7.19 (t, *J*=5.27 Hz, 1 H) 7.23 - 7.31 (m, 1 H) 7.32 - 7.67 (m, 4 H) 7.75 (d, *J*=7.81 Hz, 1 H) 7.93 (bs, 1 H) 8.47 (bs, 1 H) 8.52 (s, 1 H) 8.58 (s, 2 H) 9.42 (bs, 1 H) 9.73 (bs, 1 H)
HPLC/MS (30 min) retention time 12.57 min
LRMS: *m*/*z* 650 (M+)

### EXAMPLE 120

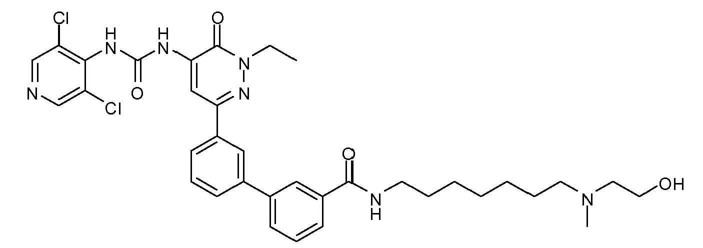

### 3'-(5-(3-(3,5-dichloropyridin-4-yl)ureido)-1-ethyl-6-oxo-1,6-dihydropyridazin-3-yl)-N-(7-((2-hydroxyethyl)(methyl)amino)heptyl)biphenyl-3-carboxamide

Reaction of the title compound of Example 48 (300 mg, 0.57 mmol) with 2-[(7-aminoheptyl)(methyl)amino]ethanol (215 mg, 1.14 mmol) according to the method described in Example 49 gave 169 mg (42.5%) of the title compound.
¹H NMR (200 MHz, DMSO-*d*₆) ppm 1.12 - 1.48 (m, 8 H) 1.49 - 1.88 (m, 3 H) 2.76 (s, 3 H) 2.86 - 3.20 (m, 3 H) 3.44 (q, *J*=6.38 Hz, 2 H) 3.77 - 4.07 (m, 2 H) 4.25 (q, *J*=7.29 Hz, 2 H) 4.91 (s, 4 H) 6.67 (t, *J*=5.66 Hz, 1 H) 7.48 (t, *J*=7.81 Hz, 3 H) 7.55 - 7.89 (m, 3 H) 8.02 (d, *J*=1.95 Hz, 2 H) 8.49 (bs, 1 H) 8.57 (s, 2 H) 8.66 (s, 1 H) 9.59 (bs, 1 H)
HPLC/MS (30 min) retention time 13.44 min
LRMS: *m*/*z* 694 (M+)

### EXAMPLE 121

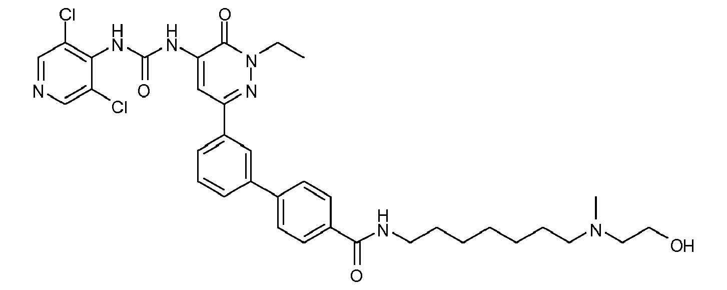

### 3'-(5-(3-(3,5-dichloropyridin-4-yl)ureido)-1-ethyl-6-oxo-1,6-dihydropyridazin-3-yl)-N-(7-((2-hydroxyethyl)(methyl)amino)heptyl)biphenyl-4-carboxamide

Reaction of the title compound of Example 26 (300 mg, 0.57 mmol) with 2-[(7-aminoheptyl)(methyl)amino]ethanol (215 mg, 1.14 mmol) according to the method described in Example 49 gave 130 mg (32.7%) of the title compound.
¹H NMR (200 MHz, DMSO-*d*₆) ppm 1.29 (t, *J*=7.22 Hz, 3 H) 1.35 - 1.47 (m, 6 H) 1.51 - 1.74 (m, 4 H) 2.46 (s, 3 H) 2.54 - 2.70 (m, 2 H) 2.70 - 2.83 (m, 2 H) 3.48 (q, *J*=6.90 Hz, 2 H) 3.61 - 3.89 (m, 2 H) 4.25 (q, *J*=7.16 Hz, 2 H) 6.30 (t, *J*=5.86 Hz, 1 H) 7.52 (s, 1 H) 7.56 (s, 1 H) 7.59 - 7.73 (m, 4 H) 7.83 (bs, 1 H) 7.87 (bs, 1 H) 8.04 (m, 1 H) 8.57 (bs, 1 H) 8.61 (s, 2 H) 8.71 (s, 1 H) 9.52 (bs, 1 H)
HPLC/MS (30 min) retention time 13.26 min
LRMS: *m*/*z* 694 (M+)

### EXAMPLE 122

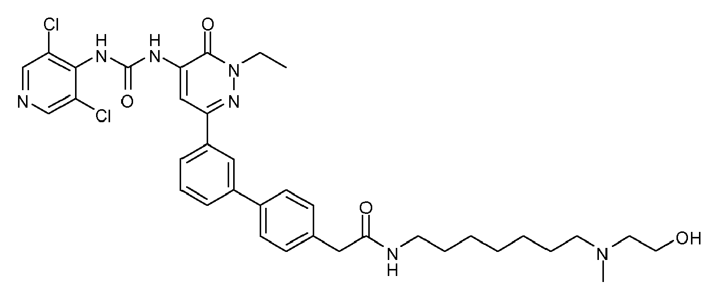

### 2-(3'-(5-(3-(3,5-dichloropyridin-4-yl)ureido)-1-ethyl-6-oxo-1,6-dihydropyridazin-3-yl)biphenyl-4-yl)-N-(7-((2-hydroxyethyl)(methyl)amino)heptyl)acetamide

Reaction of the title compound of Example 40 (300 mg, 0.56 mmol) with 2-[(7-aminoheptyl)(methyl)amino]ethanol (209 mg, 1.11 mmol) according to the method described in Example 49 gave 97 mg (24.6%) of the title compound.
¹H NMR (200 MHz, DMSO-*d*₆) ppm 1.08 - 1.79 (m, 11 H) 2.38 - 2.54 (m, 3 H) 2.56 - 2.70 (m, 2 H) 2.71 - 2.85 (m, 2 H) 3.02 - 3.41 (m, 4 H) 3.62 (s, 2 H) 3.67 - 3.83 (m, 2 H) 4.25 (q, *J*=7.29 Hz, 2 H) 5.60 (t, *J*=5.66 Hz, 1 H) 7.33 (d, *J*=8.59 Hz, 2 H) 7.41 - 7.68 (m, 4 H) 7.83 (d, *J*=7.81 Hz, 2 H) 7.93 - 8.04 (m, 2 H) 8.57 (s, 2 H) 8.70 (s, 1 H) 9.61 (bs, 1 H)
HPLC/MS (30 min) retention time 13.22 min
LRMS: *m*/*z* 708 (M+)

### EXAMPLE 123

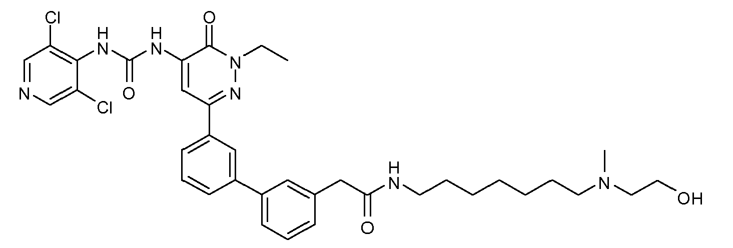

### 2-(3'-(5-(3-(3,5-dichloropyridin-4-yl)ureido)-1-ethyl-6-oxo-1,6-dihydropyridazin-3-yl)biphenyl-3-yl)-N-(7-((2-hydroxyethyl)(methyl)amino)heptyl)acetamide

Reaction of the title compound of Example 42 (300 mg, 0.56 mmol) with 2-[(7-aminoheptyl)(methyl)amino]ethanol (209 mg, 1.11 mmol) according to the method described in Example 49 gave 89 mg (22.5%) of the title compound.
¹H NMR (200 MHz, DMSO-*d*₆) ppm 1.13 - 1.53 (m, 10 H) 1.56 - 1.85 (m, 2 H) 2.76 (s, 3 H) 2.85 - 3.00 (m, 2 H) 3.02 - 3.12 (m, 2 H) 3.20 (q, *J*=6.64 Hz, 2 H) 3.64 (s, 2 H) 3.86 - 4.04 (m, 2 H) 4.26 (q, *J*=7.29 Hz, 2 H) 5.65 (s, 2 H) 5.81 (t, *J*=5.86 Hz, 1 H) 7.19 - 7.31 (m, 2 H) 7.32 - 7.66 (m, 4 H) 7.81 (d, *J*=7.81 Hz, 1 H) 7.93 (bs, 1 H) 8.52 (bs, 1 H) 8.55 (s, 2 H) 8.63 (s, 1 H) 9.60 (bs, 1 H)
HPLC/MS (30 min) retention time 13.21 min
LRMS: *m*/*z* 708 (M+)

### EXAMPLE 124

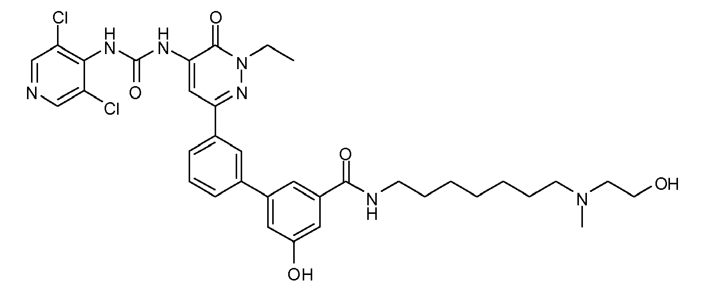

### 3'-(5-(3-(3,5-dichloropyridin-4-yl)ureido)-1-ethyl-6-oxo-1,6-dihydropyridazin-3-yl)-5-hydroxy-N-(7-((2-hydroxyethyl)(methyl)amino)heptyl)biphenyl-3-carboxamide

Reaction of the title compound of Example 112 (300 mg, 0.56 mmol) with 2-[(7-aminoheptyl)(methyl)amino]ethanol (209 mg, 1.11 mmol) according to the method described in Example 49 gave 87 mg (22.1 %) of the title compound.
¹H NMR (200 MHz, DMSO-*d*₆) ppm 0.94 - 1.63 (m, 8 H) 2.16 (s, 3 H) 2.23 - 2.62 (m, 8 H) 3.08 - 3.33 (m, 6 H) 3.45 (t, 2 H) 4.28 (q, 2 H) 7.18 (bs, 1 H) 7.26 (bs, 1 H) 7.50 - 7.66 (m, 2 H) 7.76 (t, *J*=7.61 Hz, 2 H) 7.97 (bs, 1 H) 8.26 (bs, 1 H) 8.40 (s, 1 H) 8.42 - 8.55 (m, 1 H) 8.68 (s, 2 H)
HPLC/MS (30 min) retention time 12.45 min
LRMS: *m*/*z* 710 (M+)

### EXAMPLE 125

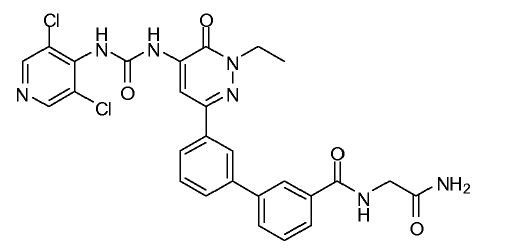

### N-(2-amino-2-oxoethyl)-3'-(5-(3-(3,5-dichloropyridin-4-yl)ureido)-1-ethyl-6-oxo-1,6-dihydropyridazin-3-yl)biphenyl-3-carboxamide

Reaction of the title compound of Example 48 (100 mg, 0.19 mmol) with 2-aminoacetamide (19 mg, 0.25 mmol) according to the method described in Example 49 gave 67 mg (59.2%) of the title compound.
¹H NMR (200 MHz, DMSO-*d*₆) ppm 1.39 (t, *J*=7.03 Hz, 3 H) 3.71 - 3.95 (m, 2 H) 4.29 (q, *J*=7.03 Hz, 2 H) 7.05 (s, 1 H) 7.41 (s, 1 H) 7.64 (s, 2 H) 7.83 (d, *J*=21.86 Hz, 4 H) 8.05 (s, 1 H) 8.21 (s, 1 H) 8.40 (s, 1 H) 8.70 (s, 2 H) 8.86 (bs, 1 H) 9.71 (bs, 1 H) 9.91 (bs, 1 H)
HPLC/MS (30 min) retention time 15.4 min
LRMS: *m*/*z* 580 (M+)

### EXAMPLE 126

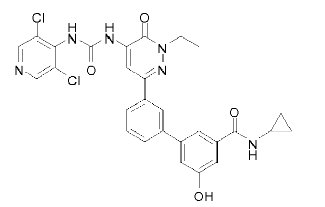

### N-cyclopropyl-3'-[5-({[(3,5-dichloropyridin-4-yl)amino]carbonyl}amino)-1-ethyl-6-oxo-1,6-dihydropyridazin-3-yl]-5-hydroxybiphenyl-3-carboxamide

Reaction of the title compound of Example 112 (150 mg, 0.28 mmol) with *N* cyclopropanamine (34.8 µl, 0.5 mmol) according to the method described in Example 49 gave 76 mg (47%) of the title compound.
¹H NMR (400 MHz, DMSO-*d*₆) ppm 0.49 - 0.63 (m, 2 H) 0.61 - 0.73 (m, 2 H) 1.39 (t, *J*=7.04 Hz, 3 H) 2.72 - 2.98 (m, 1 H) 4.28 (q, *J*=7.04 Hz, 2 H) 7.16 - 7.20 (m, 1 H) 7.20 - 7.27 (m, 1 H) 7.54 (s, 1 H) 7.60 (t, *J*=7.83 Hz, 1 H) 7.74 (d, *J*=8.22 Hz, 1 H) 7.78 (d, *J*=7.83 Hz, 1 H) 7.96 (s, 1 H) 8.40 (s, 1 H) 8.48 (d, *J*=4.30 Hz, 1 H) 8.68 (s, 2 H) 9.58 - 9.72 (m, 1 H) 9.79 - 9.94 (m, 1 H)
HPLC/MS (30 min) retention time 16.2 min
LRMS: *m*/*z* 579 (M+)

### EXAMPLE 127

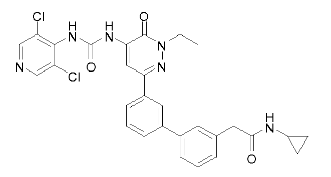

### N-cyclopropyl-2-{3'-[5-({[(3,5-dichloropyridin-4-yl)amino]carbonyl}amino)-1-ethyl-6-oxo-1,6-dihydropyridazin-3-yl]biphenyl-3-yl}acetamide

Reaction of the title compound of Example 42 (200 mg, 0.37 mmol) with *N* cyclopropanamine (46.33 µl, 0.67 mmol) according to the method described in Example 49 gave 59 mg (27%) of the title compound.
¹H NMR (400 MHz, DMSO-*d*₆) ppm 0.26 - 0.45 (m, 2 H) 0.53 - 0.62 (m, 2 H) 1.39 (t, *J*=7.24 Hz, 3 H) 2.57 - 2.64 (m, 1 H) 3.43 (s, 2 H) 4.27 (q, 2 H) 7.26 (d, *J*=7.83 Hz, 1 H) 7.42 (t, *J*=7.83 Hz, 1 H) 7.52 - 7.56 (m, 2 H) 7.60 (t, *J*=7.63 Hz, 1 H) 7.70 (d, *J*=7.83 Hz, 1 H) 7.77 (d, *J*=8.22 Hz, 1 H) 7.96 (s, 1 H) 8.18 (d, *J*=3.91 Hz, 1 H) 8.41 (s, 1 H) 8.68 (s, 1 H) 9.72 (s, 1 H) 9.91 (s, 1 H)
HPLC/MS (30 min) retention time 17.2 min
LRMS: *m*/*z* 577 (M+)

### EXAMPLE 128

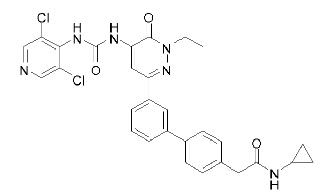

### N-cyclopropyl-2-{3'-[5-({[(3,5-dichloropyridin-4-yl)amino]carbonyl}amino)-1-ethyl-6-oxo-1,6-dihydropyridazin-3-yl]biphenyl-4-yl}acetamide

Reaction of the title compound of Example 40 (200 mg, 0.37 mmol) with *N* cyclopropanamine (46.33 µl, 0.67 mmol) according to the method described in Example 49 gave 35 mg (16%) of the title compound.
¹H NMR (400 MHz, DMSO-*d*₆) ppm 0.34 - 0.44 (m, 2 H) 0.55 - 0.66 (m, 2 H) 1.38 (t, *J*=7.24 Hz, 3 H) 2.53 - 2.67 (m, 1 H) 4.27 (q, *J*=6.91 Hz, 2 H) 7.34 (s, 1 H) 7.36 (s, 1 H) 7.55 - 7.65 (m, 2 H) 7.68 - 7.78 (m, 2 H) 7.98 (s, 1 H) 8.17 (d, *J*=4.30 Hz, 1 H) 8.40 (s, 1 H) 8.69 (s, 2 H) 9.70 (s, 1 H) 9.90 (s, 1 H)
HPLC/MS (30 min) retention time 17.02 min
LRMS: *m*/*z* 577 (M+)

### EXAMPLE 129

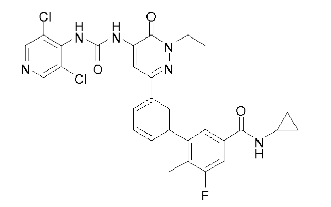

### N-cyclopropyl-3'-[5-({[(3,5-dichloropyridin-4-yl)amino]carbonyl}amino)-1-ethyl-6-oxo-1,6-dihydropyridazin-3-yl]-5-fluoro-6-methylbiphenyl-3-carboxamide

Reaction of the title compound of Preparation 7 (200 mg, 0.41 mmol) with N-cyclopropyl-3-fluoro-4-methyl-5-(4,4,5,5-tetramethyl-1,3,2-dioxaborolan-2-yl)benzamide (171.7 mg, 0.54 mmol) according to the method described in Example 1 gave 64 mg (26%) of the title compound after triturating with diethyl ether.
¹H NMR (400 MHz, DMSO-*d*₆) ppm 0.47 - 0.61 (m, 2 H) 0.61 - 0.73 (m, 2 H) 1.36 (t, *J*=7.24 Hz, 3 H) 2.16 (s, 3 H) 2.76 - 2.91 (m, 1 H) 4.26 (q, *J*=6.91 Hz, 2 H) 7.48 (d, *J*=7.43 Hz, 1 H) 7.56 - 7.67 (m, 3 H) 7.72 (s, 1 H) 7.83 (d, *J*=8.22 Hz, 1 H) 8.37 (s, 1 H) 8.51 (d, *J*=4.30 Hz, 1 H) 8.68 (s, 2 H) 9.70 (s, 1 H) 9.89 (s, 1 H)
HPLC/MS (30 min) retention time 18.6 min
LRMS: *m*/*z* 595 (M+)

### EXAMPLE 130

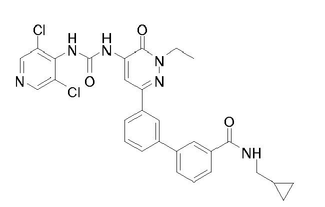

### N-(cyclopropylmethyl)-3'-[5-({[(3,5-dichloropyridin-4-yl)amino]carbonyl}amino)-1-ethyl-6-oxo-1,6-dihydropyridazin-3-yl]biphenyl-3-carboxamide

Reaction of the title compound of Example 48 (175 mg, 0.30 mmol) with cyclopropylmethanamine (43 mg, 0.60 mmol) according to the method described in Example 49 gave 110 mg (62%) of the title compound after purification by SP1® automated purification system.
¹H NMR (400 MHz, DMSO-*d*₆) ppm 0.21 - 0.27 (m, 2 H) 0.40 - 0.47 (m, 2 H) 0.96 - 1.11 (m, 1 H) 1.39 (t, *J*=7.24 Hz, 3 H) 3.17 (t, *J*=6.06 Hz, 2 H) 4.29 (q, *J*=7.04 Hz, 2 H) 7.55 - 7.70 (m, 2 H) 7.75 - 7.95 (m, 4 H) 8.05 (s, 1 H) 8.16 (s, 1 H) 8.41 (s, 1 H) 8.69 (s, 2 H) 8.72 (t, *J*=5.67 Hz, 1 H) 9.68 (br. s., 1 H) 9.92 (br. s., 1 H)
HPLC/MS (30 min) retention time 18.21 min
LRMS: *m*/*z* 577 (M+)

### EXAMPLE 131

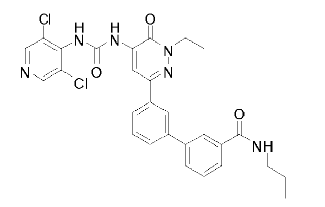

### 3'-[5-({[(3,5-dichloropyridin-4-yl)amino]carbonyl}amino)-1-ethyl-6-oxo-1,6-dihydropyridazin-3-yl]-N-propylbiphenyl-3-carboxamide

Reaction of the title compound of Example 48 (125 mg, 0.21 mmol) with propan-1-amine (25 mg, 0.42 mmol) according to the method described in Example 49 gave 70 mg (57%) of the title compound after purification by SP1® automated purification system.
¹H NMR (400 MHz, DMSO-*d*₆) ppm 0.90 (t, *J*=7.43 Hz, 3 H) 1.39 (t, *J*=7.04 Hz, 3 H) 1.55 (sxt, *J*=7.20 Hz, 2 H) 3.25 (q, *J*=6.65 Hz, 2 H) 4.28 (q, *J*=7.04 Hz, 2 H) 7.53 - 7.68 (m, 2 H) 7.75 - 7.95 (m, 4 H) 8.04 (s, 1 H) 8.14 (s, 1 H) 8.41 (s, 1 H) 8.60 (t, *J*=5.48 Hz, 1 H) 8.68 (s, 2 H) 9.67 (br. s., 1 H) 9.92 (br. s., 1 H)
HPLC/MS (30 min) retention time 17.98 min
LRMS: *m*/*z* 565 (M+)

### EXAMPLE 132

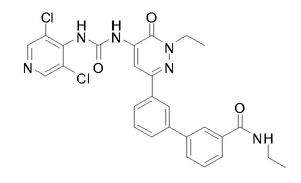

### 3'-[5-({[(3,5-dichloropyridin-4-yl)amino]carbonyl}amino)-1-ethyl-6-oxo-1,6-dihydropyridazin-3-yl]-N-ethylbiphenyl-3-carboxamide

Reaction of the title compound of Example 48 (175 mg, 0.30 mmol) with ethanamine (313 µl, 0.63 mmol) according to the method described in Example 49 gave 30 mg (17%) of the title compound after purification by SP1® automated purification system.
¹H NMR (400 MHz, DMSO-*d*₆) ppm 1.14 (t, *J*=7.24 Hz, 3 H) 1.39 (t, *J*=7.24 Hz, 3 H) 3.27 - 3.33 (m, 2 H) 4.28 (q, *J*=7.30 Hz, 2 H) 7.53 - 7.68 (m, 2 H) 7.75 - 7.90 (m, 4 H) 8.04 (s, 1 H) 8.14 (s, 1 H) 8.41 (s, 1 H) 8.62 (t, *J*=5.28 Hz, 1 H) 8.68 (s, 2 H) 9.66 (br. s., 1 H) 9.93 (br. s., 1 H)
HPLC/MS (30 min) retention time 17.49 min
LRMS: *m*/*z* 551 (M+)

### EXAMPLE 133

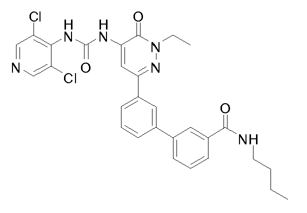

### N-butyl-3'-[5-({[(3,5-dichloropyridin-4-yl)amino]carbonyl}amino)-1-ethyl-6-oxo-1,6-dihydropyridazin-3-yl]biphenyl-3-carboxamide

Reaction of the title compound of Example 48 (175 mg, 0.30 mmol) with butan-1-amine (62 µl, 0.62 mmol) according to the method described in Example 49 gave 70 mg (39%) of the title compound after purification by SP1® automated purification system.
¹H NMR (400 MHz, DMSO-*d*₆) ppm 0.90 (t, *J*=7.43 Hz, 3 H) 1.29 - 1.37 (m, 2 H) 1.39 (t, *J*=7.24 Hz, 3 H) 1.52 (quin, *J*=7.14 Hz, 2 H) 3.24-3.33 (m, 2 H) 4.28 (q, *J*=7.04 Hz, 2 H) 7.54 - 7.69 (m, 2 H) 7.74 - 7.92 (m, 4 H) 8.04 (s, 1 H) 8.14 (s, 1 H) 8.41 (s, 1 H) 8.59 (t, *J*=5.48 Hz, 1 H) 8.69 (s, 2 H) 9.70 (br. s., 1 H) 9.91 (br. s., 1 H)
HPLC/MS (30 min) retention time 18.88 min
LRMS: *m*/*z* 579 (M+)

### EXAMPLE 134

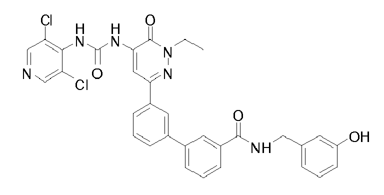

### 3'-[5-({[(3,5-dichloropyridin-4-yl)amino]carbonyl}amino)-1-ethyl-6-oxo-1,6-dihydropyridazin-3-yl]-N-(3-hydroxybenzyl)biphenyl-3-carboxamide

Reaction of the title compound of Example 48 (150 mg, 0.27 mmol) with 3-(aminomethyl)phenol (66 mg, 0.54 mmol) according to the method described in Example 49 gave 80 mg (62.5%) of the title compound after purification by SP1® automated purification system.
¹H NMR (400 MHz, DMSO-*d*₆) ppm 1.38 (t, *J*=7.24 Hz, 3 H) 4.28 (q, *J*=7.04 Hz, 2 H) 4.44 (d, *J*=5.87 Hz, 2 H) 6.59 - 6.65 (m, 1 H) 6.71 - 6.79 (m, 2 H) 7.10 (t, *J*=8.02 Hz, 1 H) 7.62 (q, *J*=7.83 Hz, 2 H) 7.81 (t, *J*=8.80 Hz, 2 H) 7.87 (d, *J*=7.83 Hz, 1 H) 7.93 (d, *J*=7.83 Hz, 1 H) 8.05 (s, 1 H) 8.22 (s, 1 H) 8.40 (s, 1 H) 8.68 (s, 2 H) 9.16 (t, *J*=5.87 Hz, 1 H) 9.33 (s, 1 H) 9.65 (br. s., 1 H) 9.87 (br. s., 1 H)
HPLC/MS (30 min) retention time 17.46 min
LRMS: *m*/*z* 629 (M+)

### EXAMPLE 135

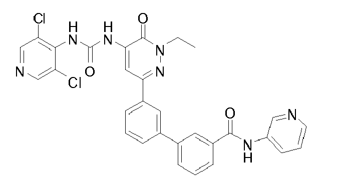

### 3'-[5-({[(3,5-dichloropyridin-4-yl)amino]carbonyl}amino)-1-ethyl-6-oxo-1,6-dihydropyridazin-3-yl]-N-pyridin-3-ylbiphenyl-3-carboxamide

Reaction of the title compound of Example 48 (150 mg, 0.27 mmol) with pyridin-3-amine (50 mg, 0.53 mmol) according to the method described in Example 49 gave 84 mg (52%) of the title compound after purification by SP1® automated purification system.
¹H NMR (400 MHz, DMSO-*d*₆) ppm 1.39 (t, *J*=7.24 Hz, 3 H) 4.29 (q, *J*=7.04 Hz, 2 H) 7.38 - 7.45 (m, 1 H) 7.62 - 7.72 (m, 2 H) 7.76 - 7.91 (m, 2 H) 7.91 - 8.03 (m, 2 H) 8.09 (s, 1 H) 8.21 (d, *J*=8.22 Hz, 1 H) 8.27 (s, 1 H) 8.33 (d, *J*=4.70 Hz, 1 H) 8.42 (s, 1 H) 8.68 (s, 2 H) 8.94 (d, *J*=2.35 Hz, 1 H) 9.67 (br. s., 1 H) 9.92 (br. s., 1 H) 10.57 (s, 1 H)
HPLC/MS (30 min) retention time 17.37 min
LRMS: *m*/*z* 600 (M+)

### EXAMPLE 136

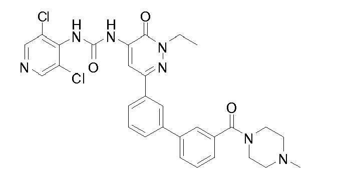

### N-(3,5-dichloropyridin-4-yl)-N'-(2-ethyl-6-{3'-[(4-methylpiperazin-1-yl)carbonyl]biphenyl-3-yl}-3-oxo-2,3-dihydropyridazin-4-yl)urea

Reaction of the title compound of Example 48 (175 mg, 0.30 mmol) with 1-methylpiperazine (63 mg, 0.63 mmol) according to the method described in Example 49 gave 150 mg (79%) of the title compound after purification by SP1® automated purification system.
¹H NMR (400 MHz, DMSO-*d*₆) ppm 1.39 (t, *J*=7.04 Hz, 3 H) 2.21 - 2.43 (m, 4 H) 3.36 (br. s., 2 H) 3.63 (br. s., 2 H) 4.28 (q, *J*=7.04 Hz, 2 H) 7.40 (d, *J*=7.83 Hz, 1 H) 7.54 - 7.65 (m, 2 H) 7.66 (s, 1 H) 7.75 - 7.84 (m, 3 H) 8.01 (s, 1 H) 8.26 (br. s., 2 H) 8.41 (s, 1 H) 8.69 (s, 2 H)
HPLC/MS (30 min) retention time 11.81 min
LRMS: *m*/*z* 606 (M+)

### EXAMPLE 137

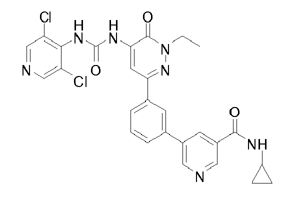

### N-cyclopropyl-5-{3-[5-({[(3,5-dichloropyridin-4-yl)amino]carbonyl}amino)-1-ethyl-6-oxo-1,6-dihydropyridazin-3-yl]phenyl}nicotinamide

Reaction of the title compound of Example 28 (200 mg, 0.34 mmol) with cyclopropanamine (39 mg, 0.68 mmol) according to the method described in Example 49 gave 130 mg (67%) of the title compound after purification by SP1® automated purification system.
¹H NMR (400 MHz, DMSO-*d*₆) ppm 0.56 - 0.65 (m, 2 H) 0.70 - 0.76 (m, 2 H) 1.39 (t, *J*=7.24 Hz, 3 H) 2.84 - 2.93 (m, 1 H) 4.29 (q, *J*=7.30 Hz, 2 H) 7.66 (t, *J*=7.83 Hz, 1 H) 7.86 (t, *J*=6.85 Hz, 2 H) 8.10 (s, 1 H) 8.41 (s, 1 H) 8.44 (s, 1 H) 8.69 (s, 2 H) 8.75 (d, *J*=3.91 Hz, 1 H) 8.98 (d, *J*=1.57 Hz, 1 H) 9.05 (d, *J*=2.35 Hz, 1 H) 9.70 (br. s., 1 H) 9.91 (br. s., 1 H)
HPLC/MS (30 min) retention time 15.95 min
LRMS: *m*/*z* 564 (M+)

### EXAMPLE 138

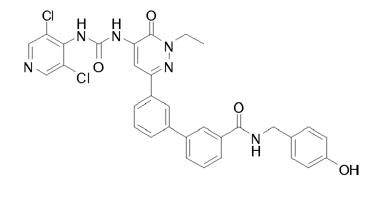

### 3'-[5-({[(3,5-dichloropyridin-4-yl)amino]carbonyl}amino)-1-ethyl-6-oxo-1,6-dihydropyridazin-3-yl]-N-(4-hydroxybenzyl)biphenyl-3-carboxamide

Reaction of the title compound of Example 48 (175 mg, 0.30 mmol) with 4-(aminomethyl)phenol (77 mg, 0.63 mmol) according to the method described in Example 49 gave 85 mg (43%) of the title compound after purification by SP1® automated purification system.
¹H NMR (400 MHz, DMSO-*d*₆) ppm 1.32 - 1.41 (m, 3 H) 4.19 - 4.31 (m, 2 H) 4.35 (d, *J*=4.70 Hz, 2 H) 6.67 (dd, 2 H) 7.11 (dd, 2 H) 7.52 - 7.63 (m, 2 H) 7.73 - 7.90 (m, 4 H) 8.01 (br. s., 1 H) 8.16 (br. s., 1 H) 8.36 (d, *J*=3.52 Hz, 1 H) 8.66 (d, *J*=3.91 Hz, 2 H) 9.01 - 9.10 (m, 1 H) 9.27 (br. s., 1 H) 9.65 (br. s., 1 H)
HPLC/MS (30 min) retention time 17.30 min
LRMS: *m*/*z* 629 (M+)

### EXAMPLE 139

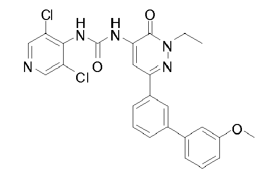

### N-(3,5-dichloropyridin-4-yl)-N'-[2-ethyl-6-(3'-methoxybiphenyl-3-yl)-3-oxo-2,3-dihydropyridazin-4-yl]urea

Reaction of the title compound of Preparation 7 (400 mg, 0.80 mmol) with 3-methoxyphenyl boronic acid (183 mg, 1.20 mmol) according to the method described in Example 1 gave 140 mg (34%) of the title compound after purification by flash chromatography (hexane/ethyl acetate, 4-3:1).
¹H NMR (200 MHz, DMSO-*d*₆) ppm 1.38 (t, *J*=6.83 Hz, 3 H) 3.82 (s, 3 H) 4.28 (q, *J*=6.77 Hz, 2 H) 6.98 (d, *J*=7.42 Hz, 1 H) 7.11 - 7.67 (m, 4 H) 7.76 (t, *J*=6.25 Hz, 2 H) 7.98 (s, 1 H) 8.39 (s, 1 H) 8.69 (s, 2 H) 9.70 (s, 1 H) 9.90 (br. s., 1 H)
HPLC/MS (30 min) retention time 19.66 min.
LRMS: *m*/*z* 510 (M+)

### EXAMPLE 140

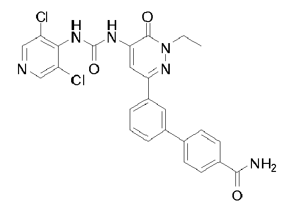

### 3'-[5-({[(3,5-dichloropyridin-4-yl)amino]carbonyl}amino)-1-ethyl-6-oxo-1,6-dihydropyridazin-3-yl]biphenyl-4-carboxamide

Reaction of the title compound of Preparation 7 (300 mg, 0.60 mmol) with 4-carbamoylphenyl boronic acid (149 mg, 0.90 mmol) according to the method described in Example 1 gave 130 mg (14%) of the title compound after purification by chromatography (alumina neutral, ethyl acetate/methanol, 10:0.10-0.35).
¹H NMR (200 MHz, DMSO-*d*₆) ppm 1.39 (t, *J*=6.83 Hz, 3 H) 4.28 (q, 2 H) 7.34 - 7.89 (m, 5 H) 7.88 - 8.15 (m, 3 H) 8.41 (s, 1 H) 8.69 (s, 2 H) 9.69 (br. s., 1 H) 9.91 (br. s., 1 H)
HPLC/MS (30 min) retention time 16.11 min.
LRMS: *m*/*z* 516 (M+)

### EXAMPLE 141

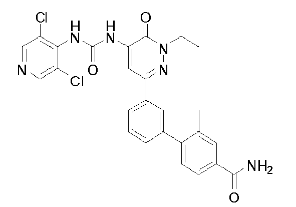

### 3'-[5-({[(3,5-dichloropyridin-4-yl)amino]carbonyl}amino)-1-ethyl-6-oxo-1,6-dihydropyridazin-3-yl]-2-methylbiphenyl-4-carboxamide

Reaction of the title compound of Preparation 7 (400 mg, 0.80 mmol) with methyl 3-methyl-4-(4,4,5,5-tetramethyl-1,3,2-dioxaborolan-2-yl)benzamide (550 mg, 2.11 mmol) (obtained following Preparation 34) according to the method described in Example 1 gave 16 mg (4%) of the title compound after purification by preparative HPLC-EM.
HPLC/MS (30 min) retention time 16.25 min.
LRMS: *m*/*z* 537 (M+)

### EXAMPLE 142

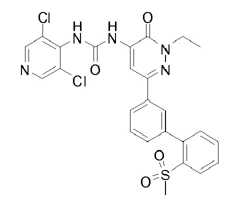

### N-(3,5-dichloropyridin-4-yl)-N'-{2-ethyl-6-[2'-(methylsulfonyl)biphenyl-3-yl]-3-oxo-2,3-dihydropyridazin-4-yl}urea

Reaction of the title compound of Preparation 7 (300 mg, 0.62 mmol) with 2-(methylsulfonyl)phenylboronic acid (190 mg, 0.93 mmol) according to the method described in Example 1 gave 108 mg (31%) of the title compound after purification by chromatography (hexane/ethyl acetate 60:40).
¹H NMR (400 MHz, DMSO-*d*₆) ppm 1.37 (t, *J*=7 Hz, 3 H), 2.84 (s, 3 H), 4.25 (q, 2 H), 7.45 - 7.86 (m, 7 H), 8.10 (d, 1 H), 8.34 (s, 1 H), 8.61 (s, 2 H)
LRMS: *m*/*z* 558 (M+)

### EXAMPLE 143

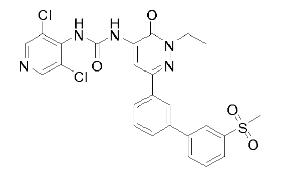

### N-(3,5-dichloropyridin-4-yl)-N'-{2-ethyl-6-[3'-(methylsulfonyl)biphenyl-3-yl]-3-oxo-2,3-dihydropyridazin-4-yl}urea

Reaction of the title compound of Preparation 7 (300 mg, 0.62 mmol) with 3-(methylsulfonyl)phenylboronic acid (190 mg, 0.93 mmol) according to the method described in Example 1 gave 59 mg (17%) of the title compound after purification by chromatography (hexane/ethyl acetate 60:40).
¹H NMR (400 MHz, DMSO-*d*₆) ppm 1.42 (t, *J*=7 Hz, 3 H), 3.25 (s, 3 H), 4.30 (q, 2 H), 7.64 - 8.04 (m, 7 H), 8.17 (s, 1 H), 8.39 (s, 1 H), 8.61 (s, 2 H), 9.45 (bs), 9.79 (bs)
LRMS: *m*/*z* 558 (M+)

### EXAMPLE 144

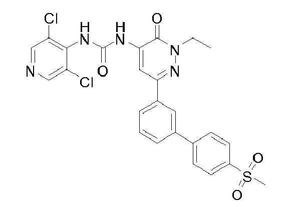

### N-(3,5-dichloropyridin-4-yl)-N'-{2-ethyl-6-[4'-(methylsulfonyl)biphenyl-3-yl]-3-oxo-2,3-dihydropyridazin-4-yl}urea

Reaction of the title compound of Preparation 7 (250 mg, 0.52 mmol) with 4-(methylsulfonyl)phenylboronic acid (160 mg, 0.78 mmol) according to the method described in Example 1 gave 49 mg (17%) of the title compound after purification by chromatography (hexane/ethyl acetate 60:40).
¹H NMR (400 MHz, DMSO-*d*₆) ppm 1.41 (t, *J*=7 Hz, 3 H), 3.20 (s, 3 H), 4.29 (q, 2 H), 7.63 - 8.03 (m, 8 H), 8.40 (s, 1 H), 8.62 (s, 2 H), 9.49 (bs), 9.77 (bs) LRMS: *m*/*z* 558 (M+)

### EXAMPLE 145

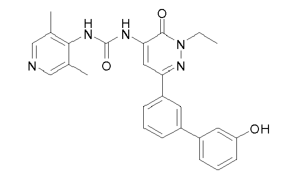

### N-(3,5-dimethylpyridin-4-yl)-N'-[2-ethyl-6-(3'-hydroxybiphenyl-3-yl)-3-oxo-2,3-dihydropyridazin-4-yl]urea

Reaction of the title compound of Preparation 57 (150 mg, 0.34 mmol) with 3-hydroxyphenylboronic acid (60 mg, 0.41 mmol) according to the method described in Example 1 gave 64 mg (40%) of the title compound after purification by chromatography (acetone/hexane 40:60).
¹H NMR (300 MHz, DMSO-*d*₆) ppm 1.39 (t, *J*=7 Hz, 3 H), 2.22 (s, 6H), 4.27 (q, 2 H), 6.79 (d, *J*=7 Hz, 1 H), 7.06 - 7.92 (m, 6 H), 8.29 (bs, 2H), 8.42 (s, 1 H), 9.29 (bs, 1 H), 9.49 (bs), 9.77 (bs)
LRMS: *m*/*z* 455 (M+)

### EXAMPLE 146

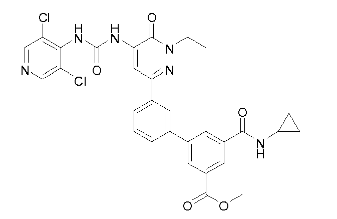

### Methyl 5-[(cyclopropylamino)carbonyl]-3'-[5-({[(3,5-dichloropyridin-4-yl)amino]carbonyl}amino)-1-ethyl-6-oxo-1,6-dihydropyridazin-3-yl]biphenyl-3-carboxylate

Reaction of the title compound of Preparation 7 (0.62 g, 1.28 mmol) with (0.576 g, 1.67 mmol) of the title compound of Preparation 60 according to the method described in Example 1 gave 0.54 g (68 %) of the title compound.
HPLC/MS (9 min) retention time 7.08 min
LRMS: *m*/*z* 621 (M+)

### EXAMPLE 147

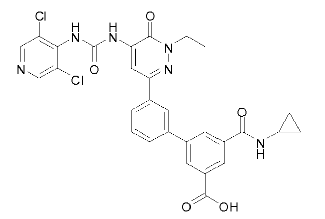

### 5-[(cyclopropylamino)carbonyl]-3'-[5-({[(3,5-dichloropyridin-4-yl)amino]carbonyl} amino)-1-ethyl-6-oxo-1,6-dihydropyridazin-3-yl]biphenyl-3-carboxylic acid

Reaction of the title compound of Example 146 (540 mg, 0.87 mmol) according to the method described in Example 26 gave 520 mg (98%) of the title compound without further purification.
¹H NMR (400 MHz, DMSO-*d*₆) ppm 0.50 - 0.66 (m, 2 H) 0.67 - 0.83 (m, 2 H) 1.39 (t, *J*=7.04 Hz, 3 H) 2.81 - 3.02 (m, 1 H) 4.29 (q, *J*=7.04 Hz, 2 H) 7.65 (t, *J*=7.83 Hz, 1 H) 7.79 - 7.90 (m, 2 H) 8.06 (s, 1 H) 8.31 (s, 1 H) 8.35 (s, 1 H) 8.37 - 8.46 (m, 2 H) 8.70 (s, 2 H) 8.78 (d, *J*=4.30 Hz, 1 H) 9.73 (s, 1 H) 9.94 (s, 1 H)
HPLC/MS (9 min) retention time 6.60 min
LRMS: *m*/*z* 607 (M+)

### EXAMPLE 148

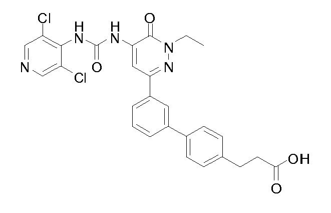

### 3-{3'-[5-({[(3,5-dichloropyridin-4-yl)amino]carbonyl}amino)-1-ethyl-6-oxo-1,6-dihydropyridazin-3-yl]biphenyl-4-yl}propanoic acid

In a Schlenk tube, the title compound of Preparation 7 (0.5 g, 1.03 mmol) and 0.414 g of the title compound of the Preparation 61 were dissolved in dioxane (10 mL). Caesium carbonate solution (2M, 1.55 mL, 3.1 mmol) was added and the mixture was purged (vacuum-argon three times). [1,1-Bis(diphenylphosphino)ferrocene]dichloropalladium (II)dichloromethane complex (0.042 g, 0.05mmol) was added and the mixture purged again (vacuum-argon three times). The mixture was stirred at 90 °C for 18 h.

The reaction mixture was cooled to room temperature, diluted with water and extracted three times with ethyl acetate. The combined organics were washed with water and brine, and dried over anhydrous sodium sulphate. The solvent was removed in vacuum and the residue was purified by the SP1® automated purification system to give 0.178 g (0.098 mmol, 31 %) of the title compound.
¹H NMR (200 MHz, DMSO-*d*₆) d ppm 1.39 (t, *J*=7.03 Hz, 3 H) 2.55 - 2.65 (m, 2 H) 2.80 - 2.96 (m, 2 H) 4.20 - 4.37 (m, 2 H) 7.35 (d, *J*=8.20 Hz, 2 H) 7.55 - 7.65 (m, 3 H) 7.74 (t, *J*=6.83 Hz, 2 H) 7.97 (s, 1 H) 8.40 (s, 1 H) 8.69 (s, 2 H) 9.67 (s, 1 H)
HPLC/MS (30 min) retention time 16.67 min
LRMS: *m*/*z* 553 (M+1)

### EXAMPLE 149

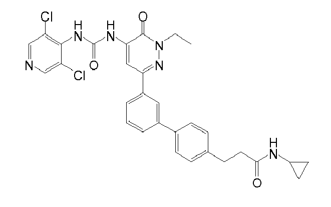

### N-cyclopropyl-3-{3'-[5-({[(3,5-dichloropyridin-4-yl)amino]carbonyl}amino)-1-ethyl-6-oxo-1,6-dihydropyridazin-3-yl]biphenyl-4-yl}propanamide

The title compound of Example 148 (122 mg, 0.22 mmol) , 1-hydroxybenzotriazole hydrate (43.4 mg, 0.32 mmol) and N-Ethyl-N'-(3-dimethyl-aminopropyl)carbodiimide hydrochloride (61.6 mg, 0.32mmol) were suspended in 5ml of DMF. The mixture was stirred at room temperature for 30 min. Then *N* cyclopropanamine (30.8 µl, 0.44 mmol) was added and the mixture was stirred at 50°C for 4 h.

The solvent was removed under reduced pressure. Purification by SP1® automated purification system gave 117.4mg (0.069 mmols, 89%).
¹H NMR (200 MHz, CHLOROFORM-*d*) d ppm 0.35 - 0.47 (m, 2 H) 0.67 - 0.79 (m, 2 H) 1.27 (t, *J*=7.22 Hz, 3 H) 2.45 (t, *J*=7.61 Hz, 2 H) 2.61 - 2.74 (m, 1 H) 3.00 (t, *J*=7.22 Hz, 2 H) 4.24 (q, *J*=7.03 Hz, 2 H) 5.47 (s, 1 H) 7.27 - 7.32 (m, 2 H) 7.46 - 7.65 (m, 4 H) 7.80 (d, *J*=8.20 Hz, 1 H) 8.00 (s, 1 H) 8.61 (s, 2 H) 8.73 (s, 1 H) 9.20 (s, 1 H) 9.63 (s, 1 H)
HPLC/MS (30 min) retention time 16.13 min
LRMS: *m*/*z* 592 (M+1)

### EXAMPLE 150

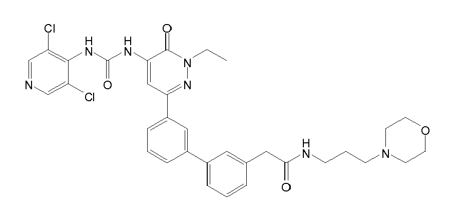

### 2-(3'-(5-(3-(3,5-dichloropyridin-4-yl)ureido)-1-ethyl-6-oxo-1,6-dihydropyridazin-3-yl)biphenyl-3-yl)-N-(2-morpholinopropyl)acetamide

The title compound of Example 42 (200 mg, 0.37 mmol), 1-hydroxybenzotriazole hydrate (75 mg, 0.56 mmol) and N-Ethyl-N'-(3-dimethyl-aminopropyl)carbodiimide hydrochloride (106 mg, 0.55 mmol) were dissolved in 3 ml of DMF. The mixture was stirred at room temperature for 1 hour. Then 3-morpholinopropan-1-amine (108 µl, 0.74 mmol) was added and the mixture was stirred at room temperature for 16 h.

The solvent was removed under reduced pressure. Purification by SP1® automated purification system gave 125 mg (51%) of the title compound.
¹H NMR (200 MHz, CHLOROFORM-D) ppm 1.3 (t, *J*=7.2 Hz, 3 H) 1.8 (m, 2 H) 2.6 (m, 6 H) 3.3 (q, *J*=5.9 Hz, 2 H) 3.7 (m, 6 H) 4.2 (q, *J*=7.0 Hz, 2 H) 6.8 (t, *J*=5.5 Hz, 1 H) 7.3-7.6 (m, 5 H) 7.8 (d, *J*=7.4 Hz, 1 H) 8.0 (s, 1 H) 8.3 (s, 1 H) 8.6 (s, 2 H) 8.6 (s, 1 H) 9.4 (s, 1 H) 9.5 (s, 1 H)
HPLC/MS (30 min) retention time 11.54 min
LRMS: *m*/*z* 665 (M+)

### EXAMPLE 151

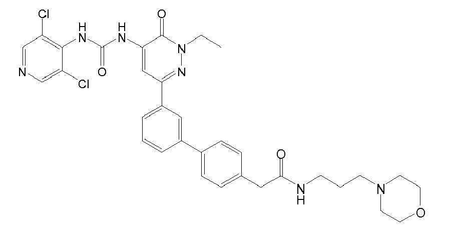

### 2-(3'-(5-(3-(3,5-dichloropyridin-4-yl)ureido)-1-ethyl-6-oxo-1,6-dihydropyridazin-3-yl)biphenyl-4-yl)-N-(2-morpholinopropyl)acetamide

The title compound of Example 40 (200 mg, 0.37 mmol), 1-hydroxybenzotriazole hydrate (75 mg, 0.56 mmol) and N-Ethyl-N'-(3-dimethyl-aminopropyl)carbodiimide hydrochloride (106 mg, 0.55 mmol) were dissolved in 3 ml of DMF. The mixture was stirred at room temperature for 1 hour. Then 3-morpholinopropan-1-amine (108 µl, 0.74 mmol) was added and the mixture was stirred at room temperature for 16 h.

The solvent was removed under reduced pressure. Purification by SP1® automated purification system gave 151 mg (61 %) of the title compound.
¹H NMR (200 MHz, CHLOROFORM-D) ppm 1.3 (t, *J*=7.0 Hz, 3 H) 1.8 (m, 2 H) 2.7 (m, 6 H) 3.4 (q, *J*=5.9 Hz, 2 H) 3.6 (s, 2 H) 3.8 (m, 4 H) 4.2 (q, *J*=7.0 Hz, 2 H) 6.8 (t, *J*=5.5 Hz, 1 H) 7.3-7.7 (m, 6 H) 7.9 (s, 1 H) 8.4 (s, 1 H) 8.6 (s, 2 H) 8.6 (s, 1 H) 9.5 (s, 1 H) 9.6 (s, 1 H)
HPLC/MS (30 min) retention time 11.34 min
LRMS: *m*/*z* 665 (M+)

### EXAMPLE 152

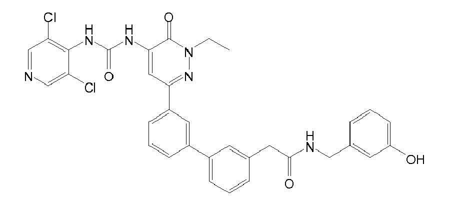

### 2-(3'-(5-(3-(3,5-dichloropyridin-4-yl)ureido)-1-ethyl-6-oxo-1,6-dihydropyridazin-3-yl)biphenyl-3-yl)-N-(3-hydroxybenzyl)acetamide

The title compound of Example 42 (200 mg, 0.37 mmol), 1-hydroxybenzotriazole hydrate (75 mg, 0.56 mmol) and N-Ethyl-N'-(3-dimethyl-aminopropyl)carbodiimide hydrochloride (106 mg, 0.55 mmol) were dissolved in 3 ml of DMF. The mixture was stirred at room temperature for 1 hour. Then 3-(aminomethyl)phenol (91 mg, 0.74 mmol) was added and the mixture was stirred at room temperature for 16 h.

The solvent was removed under reduced pressure. Purification by SP1® automated purification system gave 99 mg (41%) of the title compound.
¹H NMR (200 MHz, CHLOROFORM-D) ppm 1.3 (t, *J*=7.2 Hz, 3 H) 3.7 (s, 2 H) 4.2 (q, *J*=6.9 Hz, 2 H) 4.4 (d, *J*=5.9 Hz, 2 H) 6.2 (t, *J*=6.1 Hz, 1 H) 6.7 (m, 2 H) 6.9 (s, 1 H) 7.0-7.7 (m, 8 H) 7.9 (s, 1 H) 8.4 (s, 1 H) 8.5 (s, 2 H) 9.1 (s, 1 H) 9.5 (s, 1 H)
HPLC/MS (15 min) retention time 9.0 min
LRMS: *m*/*z* 644 (M+)

### EXAMPLE 153

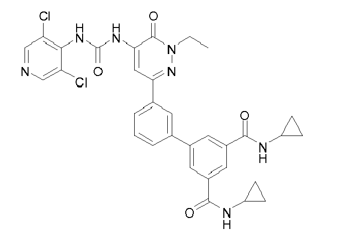

### N,N'-dicyclopropyl-3'-[5-({[(3,5-dichloropyridin-4-yl)amino]carbonyl}amino)-1-ethyl-6-oxo-1,6-dihydropyridazin-3-yl]biphenyl-3,5-dicarboxamide

Reaction of the title compound of Preparation 7 (0.2 g, 0.41 mmol) with (0.199 g, 0.54 mmol) of the title compound of Preparation 63 according to the method described in Example 1 gave 0.123 g (46 %) of the title compound.
¹H NMR (400 MHz, DMSO-*d*₆) ppm 0.43 - 0.67 (m, 4 H) 0.64 - 0.80 (m, 4 H) 1.39 (t, *J*=7.24 Hz, 3 H) 2.77 - 2.97 (m, 2 H) 4.29 (q, *J*=7.17 Hz, 2 H) 7.65 (t, *J*=7.83 Hz, 1 H) 7.83 (dd, *J*=14.28, 8.02 Hz, 2 H) 8.07 (s, 1 H) 8.21 (d, *J*=1.56 Hz, 2 H) 8.27 (s, 1 H) 8.41 (s, 1 H) 8.64 - 8.73 (m, 3 H) 9.71 (s, 1 H) 9.90 (s, 1 H)
HPLC/MS (15 min) retention time 8.66 min
LRMS: *m*/*z* 646 (M+)

### EXAMPLE 154

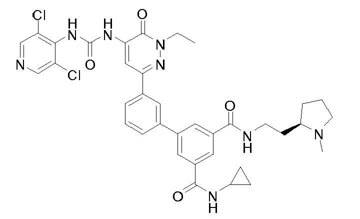

### N-cyclopropyl-3'-[5-({[(3,5-dichloropyridin-4-yl)amino]carbonyl}amino)-1-ethyl-6-oxo-1,6-dihydropyridazin-3-yl]-N'-{2-[(2R)-1-methylpyrrolidin-2-yl]ethyl}biphenyl-3,5-dicarboxamide

Reaction of the title compound of Example 147 (150 mg, 0.25 mmol) with {2-[(2R)-1-methylpyrrolidin-2-yl]ethyl}amine (97 mg, 0.49 mmol) according to the method described in Example 49 gave 21.6 mg (12%) of the title compound after purification by SP1® automated purification system.
¹H NMR (400 MHz, DMSO-*d*₆) ppm 0.57 - 0.65 (m, 2 H) 0.67 - 0.75 (m, 2 H) 1.38 (t, *J*=7.04 Hz, 3 H) 1.41 - 1.55 (m, 2 H) 1.56 - 1.70 (m, 2 H) 1.82 - 2.00 (m, 2 H) 1.99 - 2.14 (m, 2 H) 2.22 (s, 3 H) 2.84 - 2.98 (m, 1 H) 3.33 (q, 2 H) 4.27 (q, *J*=7.17 Hz, 2 H) 7.63 (t, *J*=7.63 Hz, 1 H) 7.78 - 7.83 (m, 2 H) 8.07 (s, 1 H) 8.20 (d, *J*=2.74 Hz, 2 H) 8.28 (s, 1 H) 8.36 - 8.49 (m, 2 H) 8.56 (d, *J*=3.91 Hz, 1 H) 8.64 (t, *J*=5.28 Hz, 1 H)
HPLC/MS (30 min) retention time 11.73 min
LRMS: *m*/*z* 717 (M+)

### EXAMPLE 155

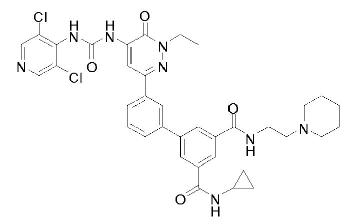

### N-cyclopropyl-3'-[5-({[(3,5-dichloropyridin-4-yl)amino]carbonyl}amino)-1-ethyl-6-oxo-1,6-dihydropyridazin-3-yl]-N'-(2-piperidin-1-ylethyl)biphenyl-3,5-dicarboxamide

Reaction of the title compound of Example 147 (150 mg, 0.25 mmol) with (2-piperidin-1-ylethyl)amine (0.1 µl, 0.49 mmol) according to the method described in Example 49 gave 88.26 mg (50%) of the title compound after purification by SP1® automated purification system.
¹H NMR (400 MHz, DMSO-*d*₆) ppm 0.55 - 0.64 (m, 2 H) 0.67 - 0.78 (m, 2 H) 1.39 (t, *J*=7.04 Hz, 4 H) 1.44 - 1.55 (m, 4 H) 2.31 - 2.43 (m, 3 H) 2.41 - 2.49 (m, 2 H) 2.79 - 2.97 (m, 1 H) 4.29 (q, *J*=7.04 Hz, 2 H) 7.65 (t, *J*=7.83 Hz, 1 H) 7.83 (dd, *J*=14.87, 7.83 Hz, 2 H) 8.08 (s, 1 H) 8.23 (d, *J*=6.26 Hz, 1 H) 8.26 - 8.32 (m, 1 H) 8.40 (s, 1 H) 8.59 - 8.74 (m, 3 H) 9.63 (s, 1 H)
HPLC/MS (15 min) retention time 6.65 min
LRMS: *m*/*z* 717 (M+)

### EXAMPLE 156

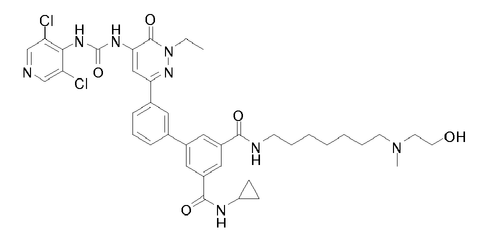

### N-cyclopropyl-3'-[5-({[(3,5-dichloropyridin-4-yl)amino]carbonyl}amino)-1-ethyl-6-oxo-1,6-dihydropyridazin-3-yl]-N'-{7-[(2-hydroxyethyl)(methyl)amino]heptyl} biphenyl-3,5-dicarboxamide

Reaction of the title compound of Example 147 (160 mg, 0.26 mmol) with 2-[(7-aminoheptyl)(methyl)amino]ethanol (99mg, 0.53 mmol) according to the method described in Example 49 gave 78.5 mg (38%) of the title compound after purification by SP1® automated purification system.
¹H NMR (400 MHz, DMSO-*d*₆) ppm 0.53 - 0.65 (m, 2 H) 0.66 - 0.76 (m, 2 H) 0.79 (none, 1 H) 1.15 - 1.33 (m, 8 H) 1.34 - 1.45 (m, 3 H) 1.49 - 1.57 (m, 2 H) 2.17 (s, 3 H) 2.33 (t, *J*=7.24 Hz, 2 H) 2.41 (t, *J*=6.46 Hz, 2 H) 2.78 - 2.94 (m, 1 H) 3.45 (t, *J*=6.26 Hz, 3 H) 4.29 (q, *J*=7.30 Hz, 2 H) 7.65 (t, *J*=7.83 Hz, 1 H) 7.83 (dd, *J*=16.43, 7.83 Hz, 2 H) 8.08 (s, 1 H) 8.21 (s, 2 H) 8.24 (s, 1 H) 8.29 (s, 1 H) 8.40 (s, 1 H) 8.68 (s, 2 H) 9.66 (s, 1 H)
HPLC/MS (15 min) retention time 6.97 min
LRMS: *m*/*z* 777 (M+)

### EXAMPLE 157

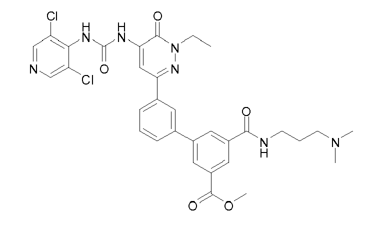

### Methyl 3'-[5-({[(3,5-dichloropyridin-4-yl)amino]carbonyl}amino)-1-ethyl-6-oxo-1,6-dihydropyridazin-3-yl]-5-({[3-(dimethylamino)propyl]amino}carbonyl)biphenyl-3-carboxylate

Reaction of the title compound of Preparation 7 (3.13g, 6.45 mmol) with (3.00 g, 7.69 mmol) of the title compound of Preparation 65 according to the method described in Example 1 gave 0.93 g (22 %) of the title compound.
HPLC/MS (30 min) retention time 13.1 min
LRMS: *m*/*z* 666 (M+)
¹H NMR (400 MHz, DMSO-*d*₆) ppm 1.39 (t, *J*=7.04 Hz, 3 H) 1.70 (q, 2 H) 2.15 (s, 6 H) 2.35 (t, *J*=7.24 Hz, 2 H) 3.33 (q, 2 H) 3.92 (s, 3 H) 4.29 (q, *J*=7.30 Hz, 2 H) 7.66 (t, *J*=7.83 Hz, 1 H) 7.77 - 7.89 (m, 2 H) 8.06 (s, 1 H) 8.24 (s, 1 H) 8.32 (s, 1 H) 8.40 (s, 2 H) 8.45 (s, 1 H) 8.68 (s, 2 H) 8.89 (t, *J*=5.48 Hz, 1 H) 9.69 (s, 1 H)

### EXAMPLE 158

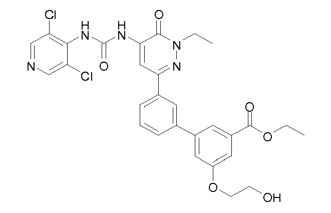

### Ethyl 3'-[5-({[(3,5-dichloropyridin-4-yl)aminolcarbonyl}amino)-1-ethyl-6-oxo-1,6-dihydropyridazin-3-yl]-5-(2-hydroxyethoxy)biphenyl-3-carboxylate

Reaction of the title compound of Preparation 7 (1.4 g, 2.9 mmol) with (1.17 g, 3.48 mmol) of the title compound of Preparation 68 according to the method described in Example 1 gave 1.12 g (63 %) of the title compound.
HPLC/MS (10 min) retention time 7.22 min
LRMS: *m*/*z* 611 (M-)

### EXAMPLE 159

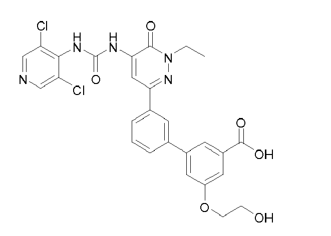

### 3'-[5-({[(3,5-dichloropyridin-4-yl)amino]carbonyl}amino)-1-ethyl-6-oxo-1,6-dihydropyridazin-3-yl]-5-(2-hydroxyethoxy)biphenyl-3-carboxylic acid

Reaction of the title compound of Example 158 (1.2g, 1.83 mmol) according to the method described in Example 26 gave 1.123 g (95%) of the title compound without further purification.
HPLC/MS (30 min) retention time 14.47 min
LRMS: *m*/*z* 585 (M+)
¹H NMR (200 MHz, DMSO-*d*₆) d ppm 1.39 (t, 3 H) 3.75 (t, *J*=4.69 Hz, 2 H) 4.13 (t, *J*=4.88 Hz, 2 H) 4.28 (q, *J*=7.16 Hz, 2 H) 7.41 - 7.51 (m, 2 H) 7.61 (t, *J*=7.61 Hz, 1 H) 7.73 - 7.85 (m, 3 H) 8.01 (s, 1 H) 8.40 (s, 1 H) 8.69 (s, 2 H)

### EXAMPLE 160

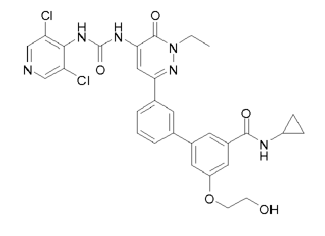

### N-cyclopropyl-3'-[5-({[(3,5-dichloropyridin-4-yl)amino]carbonyl}amino)-1-ethyl-6-oxo-1,6-dihydropyridazin-3-yl]-5-(2-hydroxyethoxy)biphenyl-3-carboxamide

Reaction of the title compound of Example 159 (150 mg, 0.25 mmol) with *N* cyclopropanamine (34.65 µl, 0.0.49 mmol) according to the method described in Example 49 gave 91.3 mg (59%) of the title compound after purification by SP1® automated purification system.
HPLC/MS (30 min) retention time 14.73 min
LRMS: *m*/*z* 624 (M+)
¹H NMR (400 MHz, DMSO-*d*₆) ppm 0.54 - 0.61 (m, 2 H) 0.67 - 0.74 (m, 2 H) 1.39 (t, *J*=7.05 Hz, 3 H) 2.81 - 2.91 (m, 1 H) 3.75 (q, *J*=5.25 Hz, 2 H) 4.12 (t, *J*=4.98 Hz, 2 H) 4.28 (q, *J*=7.33 Hz, 2 H) 4.89 (t, *J*=5.39 Hz, 1 H) 7.36 (s, 1 H) 7.41 (s, 1 H) 7.61 (t, *J*=7.88 Hz, 1 H) 7.69 (s, 1 H) 7.79 (d, *J*=9.12 Hz, 2 H) 8.02 (s, 1 H) 8.39 (s, 1 H) 8.53 (d, *J*=3.73 Hz, 1 H) 8.68 (s, 2 H) 9.67 (s, 1 H) 9.90 (s, 1 H)

### EXAMPLE 161

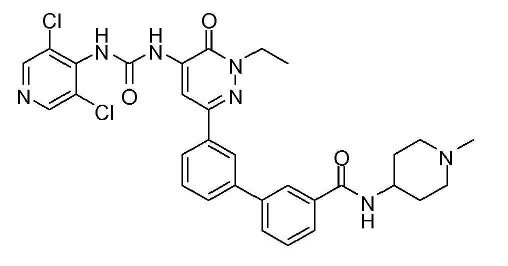

### 3'-[5-({[(3,5-Dichloropyridin-4-yl)amino]carbonyl}amino)-1-ethyl-6-oxo-1,6-dihydropyridazin-3-yl]-N-(1-methylpiperidin-4-yl)biphenyl-3-carboxamide

Reaction of the title compound of Example 48 (290 mg, 0.55 mmol) with 1-methylpiperidine-4-amine (126 mg, 1.1 mmol) according to the method described in Example 49 gave 118 mg (34%) of the title compound.
¹H NMR (200 MHz, CHLOROFORM-*d*) ppm 1.27 (t, 3 H) 1.43 - 1.79 (m, 2 H) 1.87 - 2.45 (m, 6 H) 2.67 - 3.05 (m, 2 H) 3.49 (s, 3 H) 3.81 - 4.12 (m, 1 H) 4.21 (q, 2 H) 6.07 (s, 1 H) 7.39 - 7.93 (m, 5 H) 7.93 - 8.16 (m, 2 H) 8.61 (s, 2 H) 8.73 (s, 1 H) 9.62 (s, 1 H)
HPLC/MS (15 min) retention time 6.75 min.
LRMS: *m*/*z* 620 (M+)

### EXAMPLE 162

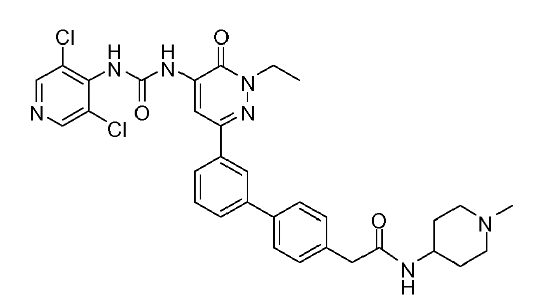

### 2-{3'-[5-({[(3,5-dichloropyridin-4-yl)amino]carbonyl}amino)-1-ethyl-6-oxo-1,6-dihydropyridazin-3-yl]biphenyl-3-yl}-N-(1-methylpiperidin-4-yl)acetamide

Reaction of the title compound of Example 40 (200 mg, 0.22 mmol) with 1-methylpiperidine-4-amine (51 mg, 0.45 mmol) according to the method described in Example 151 gave 55 mg (38%) of the title compound.
¹H NMR (300 MHz, DMSO-*d*6) ppm 1.38 (t, *J*=7.14 Hz, 3 H) 1.42 - 1.61 (m, 4 H) 1.67 - 1.80 (m, 4 H) 2.08 - 2.25 (m, 2 H) 2.27 (s, 3 H) 2.79 - 2.92 (m, 2 H) 4.27 (q, *J*=7.14 Hz, 2 H) 7.38 (d, *J*=7.97 Hz, 2 H) 7.53 - 7.66 (m, 2 H) 7.73 (t, *J*=7.28 Hz, 2 H) 7.98 (s, 1 H) 8.17 - 8.35 (m, 2 H) 8.40 (s, 1 H) 8.69 (s, 2 H) 9.72 (s, 1 H)
HPLC/MS (15 min) retention time 6.57 min.
LRMS: *m*/*z* 634 (M+)

### EXAMPLE 163

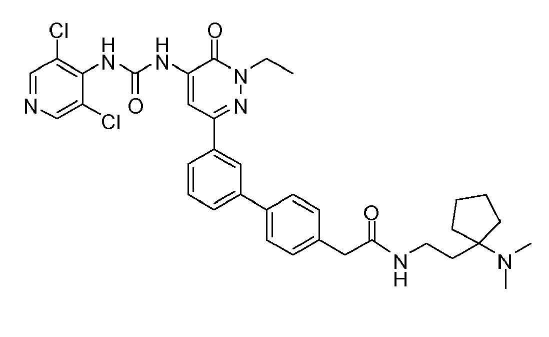

### 2-{3'-[5-({[(3,5-Dichloropyridin-4-yl)amino]carbonyl}amino)-1-ethyl-6-oxo-1,6-dihydropyridazin-3-yl]biphenyl-3-yl}-N-{2-[1-(dimethylamino)cyclopentyl]ethyl}acetamide

Reaction of the title compound of Example 40 (200 mg, 0.37 mmol) with 1-(2-aminoethyl)-N,N-dimethylcyclopentanamine (116 mg, 0.74 mmol, prepared as described by Suzuki, Takeshi et al. at *Synthetic Communications* (1998), *28(4)*, 701-712) according to the method described in Example 151 gave 60 mg (23%) of the title compound.
¹H NMR (200 MHz, CHLOROFORM-*d*) ppm 1.30 (t, *J*=7.22 Hz, 3 H) 1.49 - 1.77 (m, 6 H) 1.82 - 2.10 (m, 4 H) 2.54 (s, 6 H) 3.29-3.54 (m, 2 H) 3.61 (s, 2 H) 4.21 (q, 2 H) 7.30 - 7.61 (m, 8 H) 7.60 - 7.76 (m, 1 H) 7.94 (s, 1 H) 8.58 (s, 2 H) 9.41 (s, 1 H) 9.76 (s, 1 H)
HPLC/MS (15 min) retention time 6.80 min.
LRMS: *m*/*z* 676 (M+)

### EXAMPLE 164

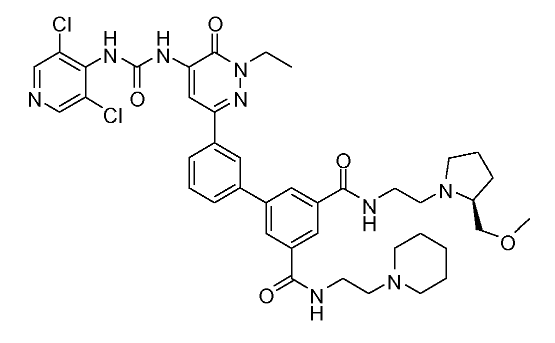

### 3'-[5-({[(3,5-Dichloropyridin-4-yl)amino]carbonyl}amino)-1-ethyl-6-oxo-1,6-dihydropyridazin-3-yl]-N-{2-[(2S)-2-(methoxymethyl)pyrrolidin-1-yl]ethyl}-N'-(2-piperidin-1-ylethyl)biphenyl-3,5-dicarboxamide

Reaction of the title compound of Example 201 (150 mg, 0.22 mmol) with (S)-2-(2-(methoxymethyl)pyrrolidin-1-yl)ethanamine (102 mg, 0.44 mmol, prepared as described by Stadtmueller, Heinz et al. at PCT Int. Appl. WO 2006021544, method 23 page 58) according to the method described in Example 151 gave 51 mg (27%) of the title compound.
¹H NMR (200 MHz, CHLOROFORM-*d*) ppm 1.21 (t, 3 H) 1.61 (s, 2 H) 1.89 (s, 5 H) 2.91 - 3.18 (m, 8 H) 3.30 (s, 3 H) 3.43 - 3.77 (m, 8 H) 3.90 (s, 4 H) 3.98 - 4.19 (m, 2 H) 7.45 (s, 2 H) 7.64 (s, 3 H) 7.94 (s, 1 H) 8.08 - 8.34 (m, 2 H) 8.41 (d, 1 H) 8.55 (d, *J*=1.56 Hz, 2 H) 8.80 (s, 1 H) 9.05 (s, 1 H) 9.44 (s, 1 H)
HPLC/MS (15 min) retention time 6.35 min.
LRMS: *m*/*z* 818 (M+)

### EXAMPLE 165

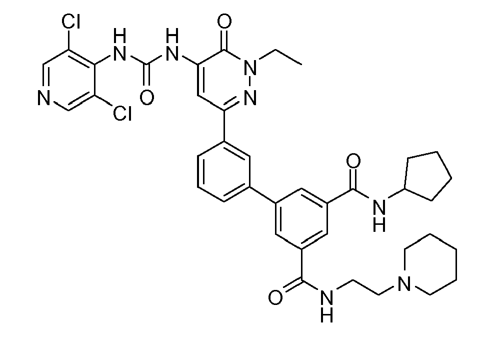

### N-Cyclopentyl-3'-[5-({[(3,5-dichloropyridin-4-yl)amino]carbonyl}amino)-1-ethyl-6-oxo-1,6-dihydropyridazin-3-yl]-N'-(2-piperidin-1-ylethyl)biphenyl-3,5-dicarboxamide

Reaction of the title compound of Example 201 (150 mg, 0.22 mmol) with cyclopentanamine (44µl, 0.45 mmol) according to the method described in Example 151 gave 51 mg (31 %) of the title compound.
¹H NMR (200 MHz, CHLOROFORM-*d*) ppm 1.06 - 1.35 (m, 3 H) 1.44 - 2.44 (m, 16 H) 2.90 - 3.14 (m, 2 H) 3.14 - 3.34 (m, 2 H) 3.70 - 3.97 (m, 2 H) 4.02 - 4.24 (m, 2 H) 4.26 - 4.54 (m, 1 H) 7.40 - 7.54 (m, 2 H) 7.55 - 7.80 (m, 3 H) 8.00 (s, 1 H) 8.16 (s, 1 H) 8.33 (s, 2 H) 8.42 (s, 1 H) 8.56 (s, 2 H) 9.12 - 9.53 (m, 2 H)
HPLC/MS (15 min) retention time 7.17 min.
LRMS: *m*/*z* 745 (M+)

### EXAMPLE 166

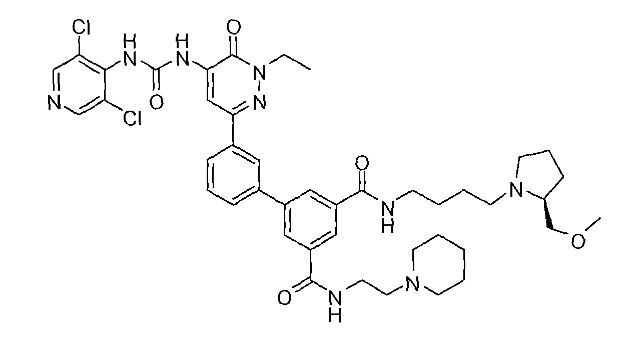

### 3'-[5-({[(3,5-Dichloropyridin-4-yl)amino]carbonyl}amino)-1-ethyl-6-oxo-1,6-dihydropyridazin-3-yl]-N-{4-[(2S)-2-(methoxymethyl)pyrrolidin-1-yl]butyl}-N'-(2-piperidin-1-ylethyl)biphenyl-3,5-dicarboxamide

Reaction of the title compound of Example 201 (150 mg, 0.22 mmol) with 4-(2-(methoxymethyl)pyrrolidin-1-yl)butan-1-amine (116 mg, 0.44 mmol, prepared in a similar manner as described by Stadtmueller, Heinz et al. at PCT Int.Appl. WO 2006021544, method 23 page 58) according to the method described in Example 151 gave 53 mg (28%) of the title compound.
¹H NMR (200 MHz, CHLOROFORM-*d*) ppm 1.14 - 1.37 (m, 3 H) 1.48 - 2.19 (m, 14 H) 2.66 - 2.92 (m, 2 H) 2.90 - 3.38 (m, 14 H) 3.42 - 3.66 (m, 2 H) 3.81 - 3.97 (m, 2 H) 4.03 - 4.29 (m, 2 H) 7.35 - 7.55 (m, 2 H) 7.56 - 7.77 (m, 4 H) 7.98 (s, 1 H) 8.20 (s, 1 H) 8.32 (s, 1 H) 8.40 (s, 1 H) 8.55 (s, 2 H) 9.23 (s, 1 H) 9.41 (s, 1 H)
HPLC/MS (15 min) retention time 5.13 min.
LRMS: *m*/*z* 846 (M+)

### EXAMPLE 167

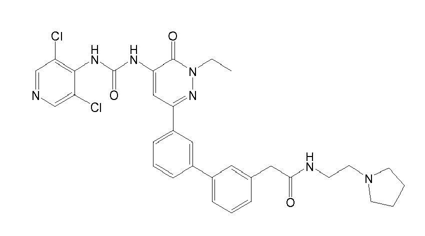

### 2-{3'-[5-({[(3,5-dichloropyridin-4-yl)amino]carbonyl}amino)-1-ethyl-6-oxo-1,6-dihydropyridazin-3-yl]biphenyl-3-yl}-N-(2-pyrrolidin-1-ylethyl)acetamide

Reaction of the title compound of Example 42 (200 mg, 0.37 mmol) with 2-(pyrrolidin-1-yl)ethanamine (93.54 µl, 0.74 mmol) according to the method described in Example 150 gave 62 mg (26%) of the title compound.
¹H NMR (200 MHz, CHLOROFORM-*d*) ppm 1.2 (t, *J*=7.2 Hz, 3 H) 2.1 (m, 4 H) 3.3 (m, 6 H) 3.7 (m, 4 H) 3.8 (q, *J*=7.2 Hz, 2 H) 7.4 (m, 4 H) 7.6 (s, 2 H) 7.8 (s, 1 H) 8.3 (s, 1 H) 8.4 (m, 1 H) 8.6 (s, 2 H) 8.7 (s, 1 H) 9.2 (s, 1 H) 10.4 (s, 1 H)
HPLC/MS (15 min) retention time 6.62 min
LRMS: *m*/*z* 634 (M+)

### EXAMPLE 168

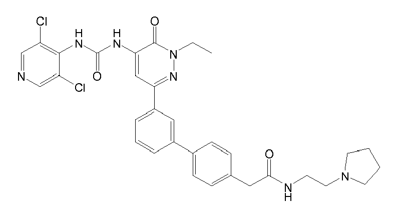

### 2-{3'-[5-({[(3,5-dichloropyridin-4-yl)amino]carbonyl}amino)-1-ethyl-6-oxo-1,6-dihydropyridazin-3-yl]biphenyl-4-yl}-N-(2-pyrrolidin-1-ylethyl)acetamide

Reaction of the title compound of Example 40 (200 mg, 0.37 mmol) with 2-(pyrrolidin-1-yl)ethanamine (93.54 µl, 0.74 mmol) according to the method described in Example 150 gave 154 mg (65%) of the title compound.
¹H NMR (200 MHz, CHLOROFORM-*d*) ppm 1.3 (t, *J*=7.0 Hz, 3 H) 2.1 (s, 4 H) 3.4 (m, 6 H) 3.7 (s, 2 H) 3.7 (m, 2 H) 4.0 (q, *J*=7.2 Hz, 2 H) 7.1-7.5 (m, 6 H) 7.8 (s, 1 H) 8.0 (s, 1 H) 8.3 (s, 1 H) 8.6 (s, 2 H) 8.8 (s, 1 H) 9.1 (s, 1 H) 11.1 (s, 1 H)
HPLC/MS (15 min) retention time 6.49 min
LRMS: *m*/*z* 634 (M+)

### EXAMPLE 169

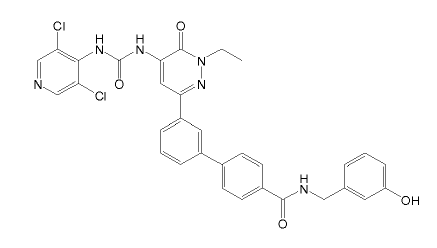

### 3'-[5-({[(3,5-dichloropyridin-4-yl)amino]carbonyl}amino)-1-ethyl-6-oxo-1,6-dihydropyridazin-3-yl]-N-(3-hydroxybenzyl)biphenyl-4-carboxamide

Reaction of the title compound of Example 26 (200 mg, 0.38 mmol) with 3-(aminomethyl)phenol (94 mg, 0.76 mmol) according to the method described in Example 150 gave 141 mg (59%) of the title compound.
¹H NMR (200 MHz, CHLOROFORM-*d*) ppm 1.5 (t, *J*=7.0 Hz, 3 H) 4.4 (q, *J*=7.4 Hz, 2 H) 4.6 (d, *J*=5.9 Hz, 2 H) 6.8 (m, 3 H) 7.1 (m, 2 H) 7.6 (m, 4 H) 7.9 (m, 3 H) 8.0 (s, 1 H) 8.5 (s, 2 H) 8.5 (s, 1 H) 9.5 (s, 1 H) 9.6 (s, 1 H)
HPLC/MS (15 min) retention time 8.67 min
LRMS: *m*/*z* 629 (M+)

### EXAMPLE 170

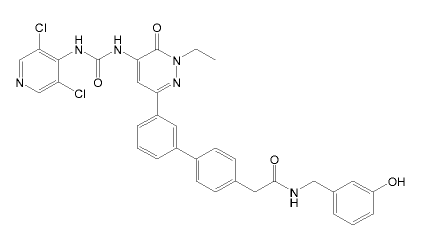

### 2-{3'-[5-({[(3,5-dichloropyridin-4-yl)amino]carbonyl}amino)-1-ethyl-6-oxo-1,6-dihydropyridazin-3-yl]biphenyl-4-yl}-N-(3-hydroxybenzyl)acetamide

Reaction of the title compound of Example 40 (200 mg, 0.37 mmol) with 3-(aminomethyl)phenol (92 mg, 0.75 mmol) according to the method described in Example 49 gave, after purification by SP1® automated purification system, 65 mg (27%) of the title compound.
¹H NMR (200 MHz, CHLOROFORM-*d*) ppm 1.4 (t, *J*=7.0 Hz, 3 H) 3.7 (s, 2 H) 4.2 (q, *J*=7.0 Hz, 2 H) 4.4 (d, *J*=6.2 Hz, 2 H) 6.1 (t, *J*=5.9 Hz, 1 H) 6.6 (m, 2 H) 6.9 (s, 1 H) 7.1 (t, *J*=7.8 Hz, 1 H) 7.3 (m, 2 H) 7.5 (m, 4 H) 7.6 (d, *J*=7.8 Hz, 1 H) 7.9 (s, 1 H) 8.4 (s, 1 H) 8.6 (s, 2 H) 9.1 (s, 1 H) 9.4 (s, 1 H)
HPLC/MS (15 min) retention time 8.99 min
LRMS: *m*/*z* 643 (M+)

### EXAMPLE 171

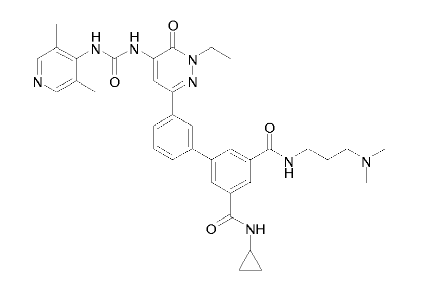

### N-cyclopropyl-N'-[3-(dimethylamino)propyl]-3'-[5-({[(3,5-dimethylpyridin-4-yl)amino]carbonyl}amino)-1-ethyl-6-oxo-1,6-dihydropyridazin-3-yl]biphenyl-3,5-dicarboxamide

Reaction of the title compound of Preparation 57 (200 mg, 0.45 mmol) with the title compound of Preparation 120 (280 mg, 0.68 mmol) according to the method described in Example 1 gave 38 mg (13%) of the title compound after purification by SP1® automated purification system.
¹H NMR (400 MHz, DMSO-*d*₆) δ ppm 0.60 (m, 2H), 0.72 (m, 2H), 1.38 (m, 3H), 1.67 (m, 2H), 2.13 (s, 3H), 2.20 (s, 3H), 2.27 (m, 2H), 2.89 (m, 1 H), 3.30 (m, 2H), 3.34 (bs, 6H), 4.28 (q, 2H), 7.42 - 7.87 (m, 3H), 8.08 - 8.42 (m, 7H), 8.70 (bs, 1H), 8.79 (bs, 1H), 9.30 (bs, 1H), 9.52 (bs, 1H).

### EXAMPLE 172

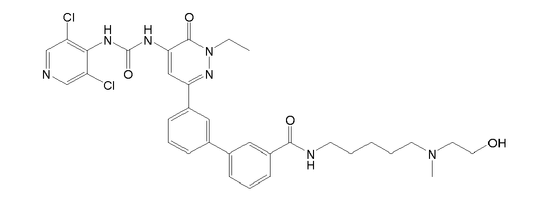

### 3'-[5-({[(3,5-dichloropyridin-4-yl)amino]carbonyl}amino)-1-ethyl-6-oxo-1,6-dihydropyridazin-3-yl]-N-{5-[(2-hydroxyethyl)(methyl)amino]pentyl}biphenyl-3-carboxamide

Reaction of the title compound of Example 48 (200 mg, 0.38 mmol) with the title compound of Preparation 72 (122 mg, 0.76 mmol) according to the method described in Example 150 gave 111 mg (44%) of the title compound.
¹H NMR (200 MHz, CHLOROFORM-*d*) ppm 1.3 (t, *J*=7.0 Hz, 3 H) 1.4-1.7 (m, 6 H) 2.2 (s, 3 H) 2.4-2.5 (m, 4 H) 3.4-3.6 (m, 5 H) 4.2 (q, *J*=7.0 Hz, 2 H) 6.3 (t, *J*=5.1 Hz, 1 H) 7.5 (m, 2 H) 7.7 (m, 3 H) 7.8 (d, *J*=7.8 Hz, 1 H) 8.0 (m, 2 H) 8.6 (s, 2 H) 8.7 (s, 1 H) 9.5 (s, 1H)
HPLC/MS (15 min) retention time 6.48 min
LRMS: *m*/*z* 666 (M+)

### EXAMPLE 173

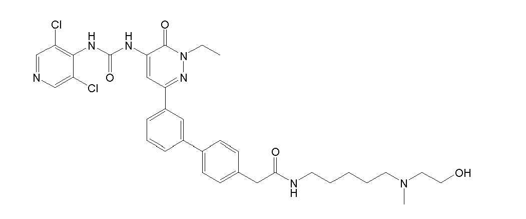

### 2-{3'-[5-({[(3,5-dichloropyridin-4-yl)amino]carbonyl}amino)-1-ethyl-6-oxo-1,6-dihydropyridazin-3-yl]biphenyl-4-yl}-N-{5-[(2-hydroxyethyl)(methyl)amino]pentyl}acetamide

Reaction of the title compound of Example 40 (200 mg, 0.37 mmol) with the title compound of Preparation 72 (119 mg, 0.74 mmol) according to the method described in Example 150 gave 113 mg (45%) of the title compound.
¹H NMR (200 MHz, CHLOROFORM-*d*) ppm 1.21-1.35 (m, Hz, 6 H) 1.4 (m, 6 H) 2.2 (s, Hz, 2 H) 2.4 (t, 2 H) 2.5 (m, 2 H) 3.2 (q, *J*=6.6 Hz, 2 H) 3.6 (q, 4 H) 4.3 (q, *J*=6.9 Hz, 2 H) 5.5 (t, *J*=6.4 Hz, 1 H) 7.3 (d, *J*=8.2 Hz, 1 H) 7.6 (m, 4 H) 7.8 (d, *J*=7.8 Hz, 1 H) 8.0 (s, 1 H) 8.6 (s, 2 H) 8.7 (s, 1 H)
HPLC/MS (15 min) retention time 6.43 min
LRMS: *m*/*z* 680 (M+)

### EXAMPLE 174

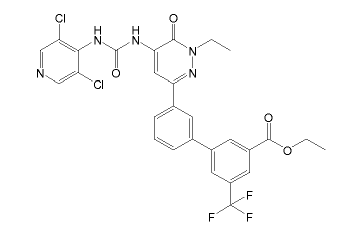

### Ethyl 3'-[5-({[(3,5-dichloropyridin-4-yl)amino]carbonyl}amino)-1-ethyl-6-oxo-1,6-dihydropyridazin-3-yl]-5-(trifluoromethyl)biphenyl-3-carboxylate

Reaction of the title compound of Preparation 7 (1.40 g, 2.90 mmol) with compound of Preparation 78 (1.20 g, 3.49 mmol) according to the method described in Example 1 gave 1.3 g (72%) of the title compound after purification by the SP1® automated purification system.
HPLC/MS (10 min) retention time 7.95 min
LRMS: *m*/*z* 620 (M+)

### EXAMPLE 175

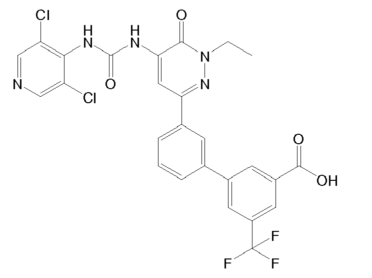

### 3'-[5-({[(3,5-dichloropyridin-4-yl)amino]carbonyl}amino)-1-ethyl-6-oxo-1,6-dihydropyridazin-3-yl]-5-(trifluoromethyl)biphenyl-3-carboxylic acid

Reaction of the title compound of Example 174 (1.30 g, 2.10 mmol) according to the method described in Example 26 with no need of the final crystallisation gave 1.21 g (98%) of the title compound.
¹H NMR (200 MHz, DMSO-*d*6) ppm 1.4 (t, *J*=7.0 Hz, 3 H) 4.3 (q, 2 H) 7.7 (m, 1 H) 7.9 (m, 2 H) 8.1 (s, 1 H) 8.2 (s, 1 H) 8.3 (s, 1 H) 8.4 (s, 1 H) 8.5 (s, 1 H) 8.7 (s, 2 H) 9.7 (s, 1 H) 9.9 (s, 1 H) 13.7 (s, 1 H)
HPLC/MS (10 min) retention time 7.48 min
LRMS: *m*/*z* 592 (M+)

### EXAMPLE 176

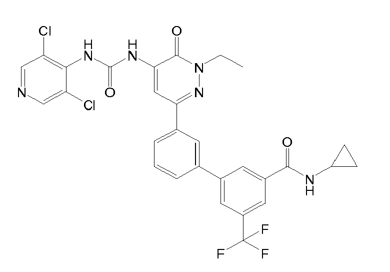

### N-cyclopropyl-3'-[5-({[(3,5-dichloropyridin-4-yl)amino]carbonyl}amino)-1-ethyl-6-oxo-1,6-dihydropyridazin-3-yl]-5-(trifluoromethyl)biphenyl-3-carboxamide

Reaction of the title compound of Example 175 (150 mg, 0.25 mmol) with cyclopropylamine (35 I, 0.50 mmol) according to the method described in Example 150 gave 99 mg (41 %) of the title compound.
¹H NMR (200 MHz, DMSO-*d*6) ppm 0.7 (m, 4 H) 1.4 (t, *J*=7.0, 3 H) 2.9 (m, 1 H) 4.3 (q, *J*=7.0 Hz, 2 H) 7.7 (t, *J*=7.8 Hz, 1 H) 7.9 (m, 2 H) 8.1 (s, 1 H) 8.2 (s, 2 H) 8.4 (s, 2 H) 8.7 (s, 2 H) 8.8 (d, *J*=4.3 Hz, 1 H) 9.7 (s, 1 H) 9.9 (s, 1 H)
HPLC/MS (15 min) retention time 9.65 min
LRMS: *m*/*z* 631 (M+)

### EXAMPLE 177

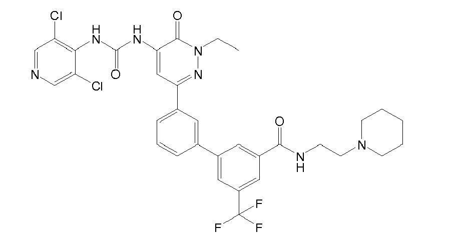

### 3'-[5-({[(3,5-dichloropyridin-4-yl)amino]carbonyl}amino)-1-ethyl-6-oxo-1,6-dihydropyridazin-3-yl]-N-(2-piperidin-1-ylethyl)-5-(trifluoromethyl)biphenyl-3-carboxamide

Reaction of the title compound of Example 175 (150 mg, 0.25 mmol) with 2-piperidin-1-yl)ethanamine (72 I, 0.51 mmol) according to the method described in Example 150 gave 40 mg (23%) of the title compound.
¹H NMR (200 MHz, DMSO-*d*6) ppm 1.4 (m, 9 H) 2.5 (m, 8 H) 4.3 (q, *J*=7.6 Hz, 2 H) 7.6 (t, *J*=7.6 Hz, 1 H) 7.8 (t, *J*=6.6 Hz, 2 H) 8.1 (s, 1 H) 8.2 (s, 2 H) 8.4 (m, 2 H) 8.7 (s, 2 H) 8.8 (t, *J*=5.1 Hz, 1 H) 9.7 (s, 1 H)
HPLC/MS (15 min) retention time 7.62 min
LRMS: *m*/*z* 702 (M+)

### EXAMPLE 178

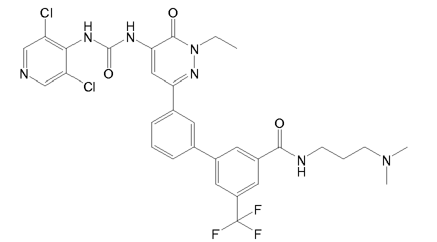

### 3'-[5-({[(3,5-dichloropyridin-4-yl)amino]carbonyl}amino)-1-ethyl-6-oxo-1,6-dihydropyridazin-3-yl]-N-[3-(dimethylamino)propyl]-5-(trifluoromethyl)biphenyl-3-carboxamide

Reaction of the title compound of Example 175 (150 mg, 0.25 mmol) with N1,N1-dimethylpropane-1,3-diamine (64 I, 0.51 mmol) according to the method described in Example 150 gave 43 mg (25%) of the title compound.
¹H NMR (200 MHz, DMSO-*d₆*) ppm 1.4 (t, *J*=7.3 Hz, 3 H) 1.7 (m, 2 H) 2.2 (s, 6 H) 2.4 (t, *J*=7.0 Hz, 2 H) 3.3 (m, 2 H) 4.3 (q, *J*=7.3 Hz, 2 H) 7.7 (t, *J*=7.8 Hz, 1 H) 7.9 (m, 7.4 Hz, 2 H) 8.1 (s, 1 H) 8.2 (s, 2 H) 8.4 (m, 1 H) 8.4 (s, 1 H) 8.7 (s, 2 H) 8.9 (t, *J*=5.5 Hz, 1 H) 9.7 (s, 1 H)
HPLC/MS (15 min) retention time 7.47 min
LRMS: *m*/*z* 676 (M+)

### EXAMPLE 179

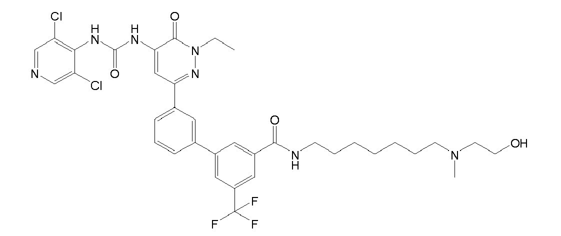

### 3'-[5-({[(3,5-dichloropyridin-4-yl)amino]carbonyl}amino)-1-ethyl-6-oxo-1,6-dihydropyridazin-3-yl]-N-{7-[(2-hydroxyethyl)(methyl)amino]heptyl}-5-(trifluoromethyl)biphenyl-3-carboxamide

Reaction of the title compound of Example 175 (150 mg, 0.25 mmol) with the title compound of Preparation 70 (95 mg, 0.50 mmol) according to the method described in Example 150 gave 56 mg (29%) of the title compound.
¹H NMR (200 MHz, DMSO-*d₆*) ppm 1.2-1.45 (m, 11 H) 1.6 (m, 2 H) 2.2 (s, 3 H) 2.4 (m, 4 H) 3.2-3.5 (m, 4 H) 4.3 (q, *J*=6.9 Hz, 2 H) 7.7 (t, *J*=7.8 Hz, 1 H) 7.9 (m, 2 H) 8.1 (s, 1 H) 8.2 (s, 2 H) 8.3 (s, 1 H) 8.4 (s, 1 H) 8.4 (s, 1 H) 8.7 (s, 2 H) 8.8 (t, *J*=5.5 Hz, 1 H) 9.6 (s, 1 H)
HPLC/MS (15 min) retention time 8.05 min
LRMS: *m*/*z* 762 (M+)

### EXAMPLE 180

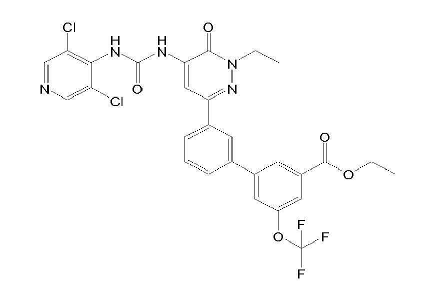

### Ethyl 3'-[5-({[(3,5-dichloropyridin-4-yl)amino]carbonyl}amino)-1-ethyl-6-oxo-1,6-dihydropyridazin-3-yl]-5-(trifluoromethoxy)biphenyl-3-carboxylate

Reaction of the title compound of Preparation 7 (1.2 g, 2.48 mmol) with compound of Preparation 78 (1.07 g, 2.98 mmol) according to the method described in Example 1 gave 770 mg (49%) of the title compound after purification by column chromatography (hexane/ethyl acetate, 2:1).
¹H NMR (200 MHz, CHLOROFORM-D) ppm 1.2-1.5 (m, 6 H) 4.3 (q, *J*=7.0 Hz, 2 H) 4.4 (q, *J*=7.3 Hz, 2 H) 7.6 (m, 3 H) 7.9 (m, 2 H) 8.1 (s, 1 H) 8.2 (s, 1 H) 8.6 (s, 2 H) 8.7 (s, 1 H) 9.1 (s, 1 H) 9.6 (s, 1 H)
HPLC/MS (10 min) retention time 8.00 min
LRMS: *m*/*z* 636 (M+)

### EXAMPLE 181

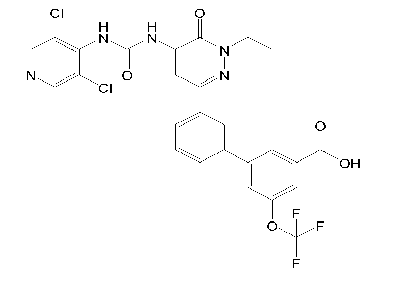

### 3'-[5-({[(3,5-dichloropyridin-4-yl)amino]carbonyl}amino)-1-ethyl-6-oxo-1,6-dihydropyridazin-3-yl]-5-(trifluoromethoxy)biphenyl-3-carboxylic acid

Reaction of the title compound of Example 180 (770 mg, 1.21 mmol) according to the method described in Example 26 with no need of the final crystallisation gave 730 mg (99%) of the title compound.
¹H NMR (200 MHz, DMSO-*d₆*) ppm 1.4 (t, *J*=7.2 Hz, 3 H) 4.3 (q, *J*=6.9 Hz, 2 H) 7.6 (m, 1 H) 7.9 (m, 3 H) 8.0 (s, 1 H) 8.1 (s, 1 H) 8.2 (s, 1 H) 8.4 (s, 1 H) 8.7 (s, 2 H) 9.7 (s, 1 H) 9.9 (s, 1 H)
HPLC/MS (10 min) retention time 7.53 min
LRMS: *m*/*z* 608 (M+)

### EXAMPLE 182

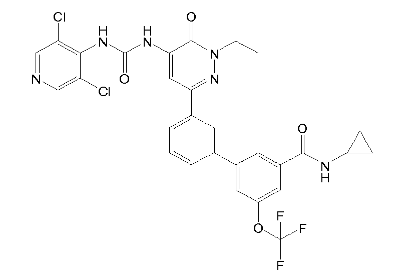

### N-cyclopropyl-3'-[5-({[(3,5-dichloropyridin-4-yl)amino]carbonyl}amino)-1-ethyl-6-oxo-1,6-dihydropyridazin-3-yl]-5-(trifluoromethoxy)biphenyl-3-carboxamide

Reaction of the title compound of Example 181 (150 mg, 0.25 mmol) with cyclopropanamine (34 l, 0.49 mmol) according to the method described in Example 150 gave 98 mg (61 %) of the title compound.
¹H NMR (200 MHz, DMSO-*d₆*) ppm 0.7 (m, 4 H) 1.4 (t, *J*=7.0 Hz, 3 H) 2.9 (m, 1 H) 4.3 (q, *J*=7.0 Hz, 2 H) 7.6 (m, 1 H) 7.8 (m, 4 H) 8.1 (s, 1 H) 8.2 (s, 1 H) 8.4 (s, 1 H) 8.7 (m, 2 H) 8.8 (d, *J*=3.9 Hz, 1 H) 9.7 (s, 1 H) 9.9 (s, 1 H)
HPLC/MS (15 min) retention time 10.07 min
LRMS: *m*/*z* 647 (M+)

### EXAMPLE 183

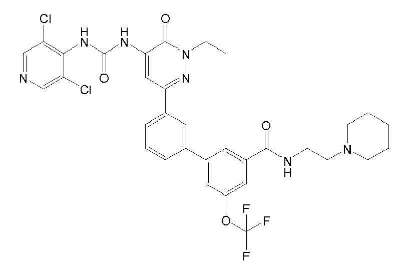

### 3'-[5-({[(3,5-dichloropyridin-4-yl)amino]carbonyl}amino)-1-ethyl-6-oxo-1,6-dihydropyridazin-3-yl]-N-(2-piperidin-1-ylethyl)-5-(trifluoromethoxy)biphenyl-3-carboxamide

Reaction of the title compound of Example 181 (150 mg, 0.25 mmol) with 2-piperidin-1-yl)ethanamine (70 I, 0.49 mmol) according to the method described in Example 150 gave 57 mg (32%) of the title compound.
¹H NMR (200 MHz, DMSO-*d₆*) ppm 1.4 (m, 9 H) 2.4 (m, 8 H) 4.3 (m, 2 H) 7.6 (m, 1 H) 7.8 (m, 2 H) 7.9 (s, 2 H) 8.1 (s, 1 H) 8.2 (s, 1 H) 8.4 (s, 1 H) 8.7 (s, 2 H) 8.8 (t, *J*=5.7 Hz, 1 H) 9.7 (s, 1 H)
HPLC/MS (15 min) retention time 7.28 min
LRMS: *m*/*z* 718 (M+)

### EXAMPLE 184

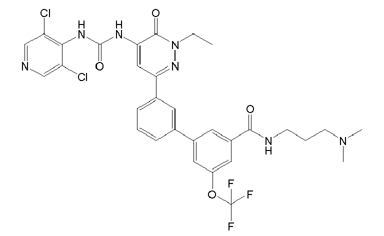

### 3'-[5-({[(3,5-dichloropyridin-4-yl)amino]carbonyl}amino)-1-ethyl-6-oxo-1,6-dihydropyridazin-3-yl]-N-[3-(dimethylamino)propyl]-5-(trifluoromethoxy)biphenyl-3-carboxamide

Reaction of the title compound of Example 181 (150 mg, 0.25 mmol) with N1,N1-dimethylpropane-1,3-diamine (62 I, 0.49 mmol) according to the method described in Example 150 gave 90 mg (53%) of the title compound.
¹H NMR (200 MHz, DMSO-*d₆*) ppm 1.4 (t, *J*=7.0 Hz, 3 H) 1.7 (m, 2 H) 2.2 (s, 6 H) 2.4 (t, *J*=7.0 Hz, 2 H) 3.3 (q, *J*=6.4 Hz, 2 H) 4.3 (q, 2 H) 7.6 (t, *J*=7.8 Hz, 1 H) 7.8 (s, 2 H) 7.9 (s, 2 H) 8.1 (s, 1 H) 8.2 (s, 1 H) 8.3 (s, 1 H) 8.4 (s, 1 H) 8.7 (s, 2 H) 8.9 (t, *J*=5.5 Hz, 1 H) 9.7 (s, 1 H)
HPLC/MS (15 min) retention time 7.20 min
LRMS: *m*/*z* 692 (M+)

### EXAMPLE 185

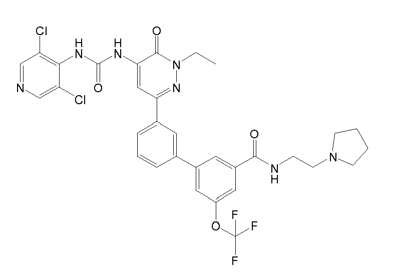

### 3'-[5-({[(3,5-dichloropyridin-4-yl)amino]carbonyl}amino)-1-ethyl-6-oxo-1,6-dihydropyridazin-3-yl]-N-(2-pyrrolidin-1-ylethyl)-5-(trifluoromethoxy)biphenyl-3-carboxamide

Reaction of the title compound of Example 181 (110 mg, 0.18 mmol) with 2-(pirrolidin-1-yl)ethanamine (41 mg, 0.36 mmol) according to the method described in Example 150 gave 74 mg (58%) of the title compound.
¹H NMR (200 MHz, DMSO-*d₆*) ppm 1.4 (m, 3 H) 1.7 (s, 4 H) 2.6 (m, 8 H) 3.4 (m, 1 H) 4.3 (m, 2 H) 7.6 (t, *J*=7.8 Hz, 1 H) 7.8 (m, 3 H) 8.1 (s, 1 H) 8.2 (s, 2 H) 8.4 (s, 1 H) 8.7 (s, 2 H) 8.9 (s, 1 H) 9.7 (s, 1 H)
HPLC/MS (15 min) retention time 7.33 min
LRMS: *m*/*z* 704 (M+)

### EXAMPLE 186

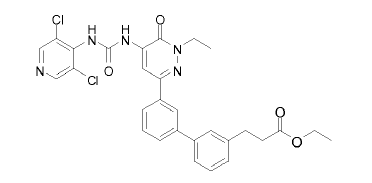

### Ethyl 3-{3'-[5-({[(3,5-dichloropyridin-4-yl)amino]carbonyl}amino)-1-ethyl-6-oxo-1,6-dihydropyridazin-3-yl]biphenyl-3-yl}propanoate

Reaction of the title compound of Preparation 7 (2 g, 4.14 mmol) with (1.76 g, 5.38 mmol) of the title compound of Preparation 82 according to the method described in Example 1 gave 0.94 g (39 %) of the title compound.
HPLC/MS (10 min) retention time 7.63 min
LRMS: *m*/*z* 579 (M-)

### EXAMPLE 187

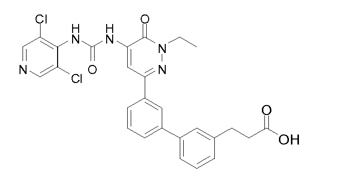

### 3-{3'-[5-({[(3,5-dichloropyridin-4-yl)amino]carbonyl}amino)-1-ethyl-6-oxo-1,6-dihydropyridazin-3-yl]biphenyl-3-yl}propanoic acid

Reaction of the title compound of Example 186 (0.94g, 1.62 mmol) according to the method described in Example 26 gave 0.832 g (93%) of the title compound without further purification.
HPLC/MS (30 min) retention time 16.71 min
LRMS: *m*/*z* 553 (M+)
1H NMR (200 MHz, DMSO-*d*₆) d ppm 1.39 (t, *J*=7.03 Hz, 3 H) 2.59 (t, *J*=7.61 Hz, 2 H) 2.91 (t, *J*=7.42 Hz, 2 H) 4.28 (q, *J*=7.29 Hz, 2 H) 7.22 - 7.32 (m, 1 H) 7.34 - 7.64 (m, 4 H) 7.69 - 7.80 (m, 2 H) 7.98 (s, 1 H) 8.39 (s, 1 H) 8.67 (s, 2 H) 9.63 (s, 1 H)

### EXAMPLE 188

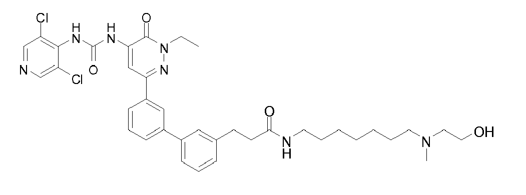

### 3-{3'-[5-({[(3,5-dichloropyridin-4-yl)amino]carbonyl}amino)-1-ethyl-6-oxo-1,6-dihydropyridazin-3-yl]biphenyl-3-yl}-N-{7-[(2-hydroxyethyl)(methyl)amino]heptyl}propanamide

Reaction of the title compound of Example 187 (150 mg, 0.27 mmol) with 2-[(7-aminoheptyl)(methyl)amino]ethanol (102 mg, 0.54 mmol) according to the method described in Example 49 gave 87 mg (44%) of the title compound after purification by SP1® automated purification system.
HPLC/MS (30 min) retention time 12.65 min
LRMS: *m*/*z* 723 (M+)
1H NMR (200 MHz, CHLOROFORM-*d*) d ppm 0.77 - 0.96 (m, 3 H) 1.17 - 1.45 (m, 13 H) 2.21 (s, 3 H) 2.35 (t, *J*=7.42 Hz, 2 H) 2.45 - 2.58 (m, 3 H) 3.04 (t, *J*=7.61 Hz, 2 H) 3.19 (q, *J*=6.64 Hz, 2 H) 3.57 (t, *J*=5.27 Hz, 2 H) 4.23 (q, 2 H) 5.41 - 5.50 (m, 1 H) 7.18 - 7.25 (m, 1 H) 7.30 - 7.66 (m, 5 H) 7.81 (d, *J*=7.81 Hz, 1 H) 7.99 (s, 1 H) 8.61 (s, 2 H) 8.71 (s, 1 H)

### EXAMPLE 189

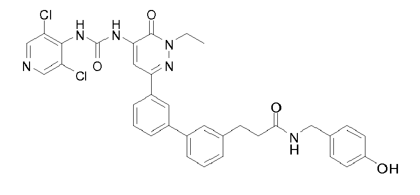

### 3-{3'-[5-({[(3,5-dichloropyridin-4-yl)amino]carbonyl}amino)-1-ethyl-6-oxo-1,6-dihydropyridazin-3-yl]biphenyl-3-yl}-N-(4-hydroxybenzyl)propanamide

Reaction of the title compound of Example 187 (150 mg, 0.27 mmol) with 4-(aminomethyl)phenol (67 mg, 0.54 mmol) according to the method described in Example 49 gave 30 mg (16%) of the title compound after purification by SP1® automated purification system.
HPLC/MS (30 min) retention time 16.12 min
LRMS: *m*/*z* 658 (M+)
1H NMR (400 MHz, DMSO-*d*₆) d ppm 1.38 (t, *J*=7.04 Hz, 3 H) 2.93 (t, *J*=7.63 Hz, 2 H) 4.12 (d, *J*=5.48 Hz, 2 H) 4.27 (q, *J*=7.30 Hz, 2 H) 6.60 - 6.65 (m, 2 H) 6.93 (d, *J*=8.61 Hz, 2 H) 7.24 (d, *J*=7.43 Hz, 1 H) 7.39 (t, *J*=7.63 Hz, 1 H) 7.47 - 7.62 (m, 3 H) 7.73 (dd, *J*=18.39, 7.83 Hz, 2 H) 7.98 (s, 1 H) 8.20 (t, *J*=5.87 Hz, 1 H) 8.39 (s, 1 H) 8.67 (s, 2 H) 9.23 (s, 1 H) 9.62 (s, 1 H) 9.81 - 10.01 (m, 1 H)

### EXAMPLE 190

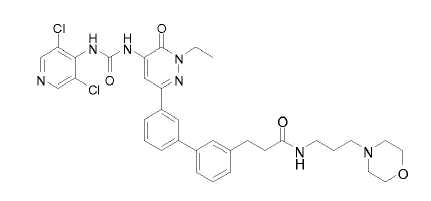

### 3-{3'-[5-({[(3,5-dichloropyridin-4-yl)amino]carbonyl}amino)-1-ethyl-6-oxo-1,6-dihydropyridazin-3-yl]biphenyl-3-yl}-N-(3-morpholin-4-ylpropyl)propanamide

Reaction of the title compound of Example 187 (150 mg, 0.27 mmol) with (3-morpholin-4-ylpropyl)amine (80.9 µl, 0.54 mmol) according to the method described in Example 49 gave 87 mg (47%) of the title compound after purification by SP1® automated purification system.
HPLC/MS (30 min) retention time 12.30 min
LRMS: *m*/*z* 679 (M+)
1H NMR (200 MHz, CHLOROFORM-*d*) d ppm 0.78 - 0.95 (m, 1 H) 1.21 - 1.73 (m, 4 H) 2.25 - 2.38 (m, 6 H) 2.48 (t, *J*=7.42 Hz, 2 H) 3.05 (t, *J*=7.61 Hz, 2 H) 3.31 (q, *J*=5.86 Hz, 2 H) 3.54 - 3.66 (m, 4 H) 4.24 (q, *J*=7.42 Hz, 2 H) 6.86 (t, *J*=4.88 Hz, 1 H) 7.17 - 7.24 (m, 1 H) 7.30 - 7.66 (m, 5 H) 7.80 (d, *J*=7.81 Hz, 1 H) 8.00 (s, 1 H) 8.62 (s, 2 H) 8.73 (s, 1 H) 9.30 (s, 1 H) 9.64 (s, 1 H)

### EXAMPLE 191

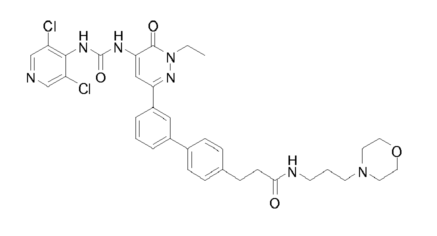

### 3-{3'-[5-({[(3,5-dichloropyridin-4-yl)amino]carbonyl}amino)-1-ethyl-6-oxo-1,6-dihydropyridazin-3-yl]biphenyl-4-yl}-N-(3-morpholin-4-ylpropyl)propanamide

Reaction of the title compound of Example 148 (150 mg, 0.27 mmol) with (3-morpholin-4-ylpropyl)amine (80 µl, 0.55 mmol) according to the method described in Example 49 gave 44 mg (24%) of the title compound after purification by SP1® automated purification system.
HPLC/MS (30 min) retention time 11.63 min
LRMS: *m*/*z* 679 (M+)
1H NMR (400 MHz, CHLOROFORM-*d*) d ppm 1.27 - 1.32 (m, 3 H) 2.33 - 2.40 (m, 5 H) 2.47 (t, *J*=7.63 Hz, 2 H) 3.02 (t, *J*=7.43 Hz, 2 H) 3.34 (q, *J*=5.87 Hz, 2 H) 3.60 - 3.66 (m, 3 H) 4.25 (q, *J*=7.30 Hz, 2 H) 6.87 (t, *J*=4.70 Hz, 1 H) 7.29 (s, 1 H) 7.46 - 7.55 (m, 3 H) 7.60 (d, *J*=7.43 Hz, 1 H) 7.80 (d, *J*=7.83 Hz, 1 H) 8.00 (s, 1 H) 8.60 - 8.63 (m, 2 H) 8.71 (s, 1 H) 9.04 (s, 1 H) 9.52 (s, 1 H)

### EXAMPLE 192

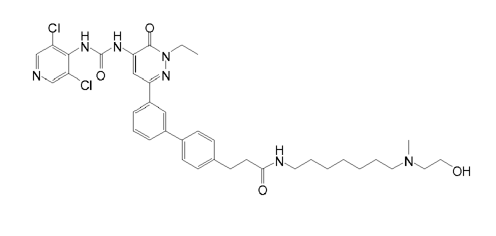

### 3-{3'-[5-({[(3,5-dichloropyridin-4-yl)amino]carbonyl}amino)-1-ethyl-6-oxo-1,6-dihydropyridazin-3-yl]biphenyl-4-yl}-N-{7-[(2-hydroxyethyl)(methyl)amino]heptyl}propanamide

Reaction of the title compound of Example 148 (150 mg, 0.27 mmol) with 2-[(7-aminoheptyl)(methyl)amino]ethanol (102 mg, 0.54 mmol) according to the method described in Example 49 gave 43 mg (22%) of the title compound after purification by SP1® automated purification system.
HPLC/MS (30 min) retention time 12.3 min
LRMS: *m*/*z* 722 (M+)
1H NMR (200 MHz, CHLOROFORM-*d*) d ppm 1.17 - 1.32 (m, 12 H) 1.36 - 1.52 (m, 4 H) 2.22 (s, 3 H) 2.36 (t, 2 H) 2.44 - 2.56 (m, 4 H) 3.01 (t, *J*=7.61 Hz, 2 H) 3.22 (q, 2 H) 3.45 - 3.51 (m, 1 H) 3.53 - 3.62 (m, 2 H) 5.45 (q, 2 H) 7.30 (s, 1 H) 7.43 - 7.65 (m, 4 H) 7.80 (d, *J*=7.42 Hz, 1 H) 8.00 (s, 1 H) 8.61 (s, 2 H) 8.73 (s, 1 H)

### EXAMPLE 193

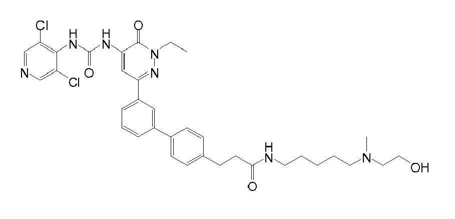

### 3-{3'-[5-({[(3,5-dichloropyridin-4-yl)amino]carbonyl}amino)-1-ethyl-6-oxo-1,6-dihydropyridazin-3-yl]biphenyl-4-yl}-N-{5-[(2-hydroxyethyl)(methyl)amino]pentyl}propanamide

Reaction of the title compound of Example 148 (150 mg, 0.27 mmol) with 2-[(5-aminopentyl)(methyl)amino]ethanol (87 mg, 0.54 mmol) according to the method described in Example 49 gave 41.6 mg (22%) of the title compound after purification by SP1® automated purification system.
HPLC/MS (30 min) retention time 11.8 min
LRMS: *m*/*z* 695 (M+)
1H NMR (200 MHz, DMSO-*d*₆) d ppm 1.05 - 1.45 (m, 12 H) 2.11 (s, 3 H) 2.18 - 2.44 (m, 6 H) 2.85 (t, *J*=7.22 Hz, 2 H) 2.95 - 3.09 (m, 2 H) 4.27 (q, *J*=7.16 Hz, 2 H) 7.31 (d, *J*=7.81 Hz, 2 H) 7.54 - 7.64 (m, 3 H) 7.67 - 7.85 (m, 3 H) 7.96 (s, 1 H) 8.40 (s, 1 H) 8.68 (s, 2 H) 9.63 (s, 1 H)

### EXAMPLE 194

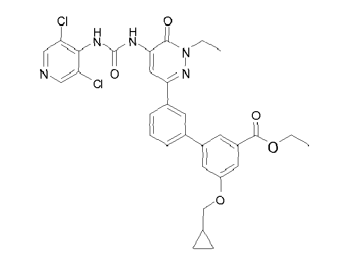

### Ethyl 5-(cyclopropylmethoxy)-3'-[5-({[(3,5-dichloropyridin-4-yl)amino]carbonyl}amino)-1-ethyl-6-oxo-1,6-dihydropyridazin-3-yl]biphenyl-3-carboxylate

Reaction of the title compound of Preparation 7 (0.85 g, 1.74 mmol) with (0.927 g, 2.44 mmol) of the title compound of Preparation 74 according to the method described in Example 1 gave 0.89 g (76 %) of the title compound.
HPLC/MS (9 min) retention time 7.9 min
LRMS: *m*/*z* 623 (M+)
1H NMR (300 MHz, CHLOROFORM-*d*) d ppm 0.36 - 0.43 (m, 2 H) 0.64 - 0.73 (m, 2 H) 1.27 - 1.31 (m, 2 H) 1.41 (t, 3 H) 1.64 (s, 2 H) 3.88 - 3.96 (m, 2 H) 4.21 - 4.29 (m, 1 H) 4.40 (q, 2 H) 7.33 - 7.38 (m, 1 H) 7.48 - 7.57 (m, 2 H) 7.65 (d, *J*=8.24 Hz, 1 H) 7.79 - 7.93 (m, 2 H) 8.05 - 8.08 (m, 1 H) 8.62 (s, 1 H) 8.76 (s, 1 H) 9.26 (s, 1H) 9.69 (s, 1 H)

### EXAMPLE 195

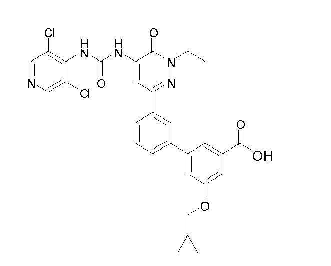

### 5-(cyclopropylmethoxy)-3'-[5-({[(3,5-dichloropyridin-4-yl)amino]carbonyl}amino)-1-ethyl-6-oxo-1 ,6-dihydropyridazin-3-yl]biphenyl-3-carboxylic acid

Reaction of the title compound of Example 194 (0.89g, 1.43 mmol) according to the method described in Example 26 gave 0.657 g (66%) of the title compound without further purification.
HPLC/MS (9 min) retention time 7.52 min
LRMS: *m*/*z* 593 (M-)
1H NMR (200 MHz, DMSO-*d*₆) d ppm 0.31 - 0.42 (m, 2 H) 0.52 - 0.65 (m, 2 H) 1.17 - 1.30 (m, 1 H) 1.39 (t, *J*=7.22 Hz, 3 H) 3.96 (d, *J*=7.03 Hz, 2 H) 4.28 (q, *J*=7.16 Hz, 2 H) 7.41 - 7.51 (m, 2 H) 7.60 (t, *J*=7.61 Hz, 1 H) 7.75 - 7.86 (m, 3 H) 7.97 - 8.10 (m, 1 H) 8.40 (s, 1 H) 8.69 (s, 2 H) 9.71 (s, 1 H) 9.91 (s, 1 H) 13.14 (s, 1 H)

### EXAMPLE 196

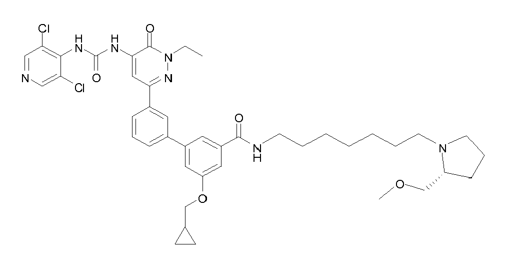

### 5-(cyclopropylmethoxy)-3'-[5-({[(3,5-dichloropyridin-4-yl)amino]carbonyl}amino)-1-ethyl-6-oxo-1,6-dihydropyridazin-3-yl]-N-{7-[(2R)-2-(methoxymethyl)pyrrolidin-1-yl]heptyl}biphenyl-3-carboxamide

Reaction of the title compound of Example 195 (160 mg, 0.23 mmol) with {7-[(2R)-2-(methoxymethyl)pyrrolidin-1-yl]heptyl}amine (160 mg, 0.46 mmol) according to the method described in Example 49 gave 69 mg (37%) of the title compound after purification by SP1® automated purification system.
HPLC/MS (30 min) retention time 14.92 min
LRMS: *m*/*z* 805 (M+)
1H NMR (200 MHz, CHLOROFORM-*d*) d ppm 0.34 - 0.42 (m, 2 H) 0.61 - 0.73 (m, 2 H) 1.24 - 1.31 (m, 4 H) 1.55 - 1.68 (m, 4 H) 1.76 - 2.08 (m, 12 H) 2.33 - 2.53 (m, 2 H) 2.80 - 3.06 (m, 2 H) 3.34 (s, 3 H) 3.36 - 3.62 (m, 5 H) 3.91 (d, *J*=7.03 Hz, 2 H) 4.25 (q, 2 H) 6.35 - 6.42 (m, 1 H) 7.29 - 7.33 (m, 1 H) 7.47 - 7.55 (m, 2 H) 7.59 - 7.66 (m, 1 H) 7.82 (d, *J*=7.81 Hz, 1 H) 8.00 - 8.05 (m, 1 H) 8.60 (s, 2 H) 8.69 (s, 1 H) 9.56 (s, 1 H)

### EXAMPLE 197

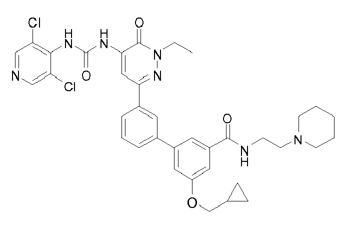

### 5-(cyclopropylmethoxy)-3'-[5-({[(3,5-dichloropyridin-4-yl)amino]carbonyl}amino)-1-ethyl-6-oxo-1,6-dihydropyridazin-3-yl]-N-(2-piperidin-1-ylethyl)biphenyl-3-carboxamide

Reaction of the title compound of Example 195 (160 mg, 0.23 mmol) with (2-piperidin-1-ylethyl)amine (66.5 µl, 0.46 mmol) according to the method described in Example 49 gave 104.87 mg (65%) of the title compound after purification by SP1® automated purification system.
HPLC/MS (30 min) retention time 14.15 min
LRMS: *m*/*z* 705 (M+)
1H NMR (200 MHz, DMSO-*d*₆) d ppm 0.31 - 0.43 (m, 2 H) 0.51 - 0.67 (m, 2 H) 1.15 - 1.59 (m, 10 H) 2.39 - 2.46 (m, 4 H) 3.95 (d, *J*=7.03 Hz, 2 H) 4.27 (q, 2 H) 7.38 (d, *J*=5.47 Hz, 2 H) 7.54 - 7.86 (m, 4 H) 8.02 (s, 1 H) 8.20 (s, 1 H) 8.39 (s, 1 H) 8.54 (s, 1 H) 8.69 (s, 2 H) 9.70 (s, 1 H)

### EXAMPLE 198

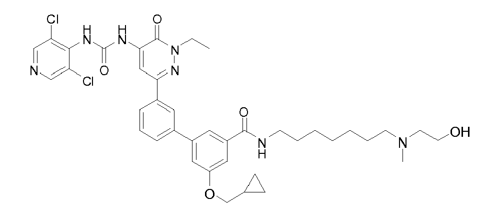

### 5-(cyclopropylmethoxy)-3'-[5-({[(3,5-dichloropyridin-4-yl)amino]carbonyl}amino)-1-ethyl-6-oxo-1,6-dihydropyridazin-3-yl]-N-{7-[(2-hydroxyethyl)(methyl)amino]heptyl}biphenyl-3-carboxamide

Reaction of the title compound of Example 195 (160 mg, 0.23 mmol) with 2-[(7-aminoheptyl)(methyl)amino]ethanol (86.1 mg, 0.46 mmol) according to the method described in Example 49 gave 104.87 mg (65%) of the title compound after purification by SP1® automated purification system.
HPLC/MS (30 min) retention time 14.77 min
LRMS: *m*/*z* 765 (M+)
1H NMR (200 MHz, DMSO-*d*₆) d ppm 0.30 - 0.43 (m, 2 H) 0.52 - 0.66 (m, 2 H) 1.14 - 1.62 (m, 14 H) 2.23 - 2.44 (m, 4 H) 3.95 (d, *J*=7.03 Hz, 2 H) 4.29 (q, *J*=6.77 Hz, 2 H) 7.38 (d, *J*=9.37 Hz, 2 H) 7.54 - 7.85 (m, 4 H) 8.02 (s, 1 H) 8.40 (s, 1 H) 8.54 (t, *J*=5.08 Hz, 1 H) 8.67 (s, 2 H) 9.62 (s, 1 H)

### EXAMPLE 199

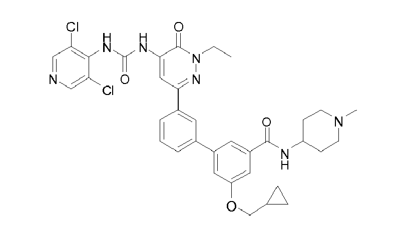

### 5-(cyclopropylmethoxy)-3'-[5-({[(3,5-dichloropyridin-4-yl)amino]carbonyl}amino)-1-ethyl-6-oxo-1,6-dihydropyridazin-3-yl]-N-(1-methylpiperidin-4-yl)biphenyl-3-carboxamide

Reaction of the title compound of Example 195 (160 mg, 0.23 mmol) with 1-methylpiperidin-4-amine (52.2 mg, 0.46 mmol) according to the method described in Example 49 gave 111.9 mg (71%) of the title compound after purification by SP1® automated purification system.
HPLC/MS (30 min) retention time 13.9 min
LRMS: *m*/*z* 691 (M+)
1H NMR (200 MHz, DMSO-*d*₆) d ppm 0.32 - 0.43 (m, 2 H) 0.53 - 0.69 (m, 2 H) 1.19 -1.34 (m,1 H) 1.34 - 1.50 (m, 3 H) 1.54 - 1.90 (m, 4 H) 1.96 - 2.15 (m, 2 H) 2.22 (s, 3 H) 2.77 - 2.91 (m, 2 H) 3.95 (d, *J*=7.03 Hz, 2 H) 4.22 - 4.39 (m, 2 H) 7.39 (d, *J*=10.54 Hz, 2 H) 7.56 - 7.91 (m, 4 H) 8.02 (s, 1 H) 8.30 - 8.49 (m, 2 H) 8.69 (s, 2 H) 9.70 (s, 1 H)

### EXAMPLE 200

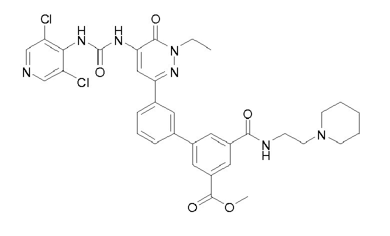

### Methyl 3'-[5-({[(3,5-dichloropyridin-4-yl)amino]carbonyl}amino)-1-ethyl-6-oxo-1,6-dihydropyridazin-3-yl]-5-{[(2-piperidin-1-ylethyl)amino]carbonyl}biphenyl-3-carboxylate

Reaction of the title compound of Preparation 7 (2 g, 4.14 mmol) with (2.43 g, 4.96 mmol) of the title compound of Preparation 84 according to the method described in Example 1 gave 2.42 g (70%) of the title compound after purification by chromatography (hexane 100% to ethyl acetate/MeOH (80:20) 1000%).
HPLC/MS (30 min) retention time 11.77 min
LRMS: *m*/*z* 693 (M+)
1H NMR (400 MHz, DMSO-*d*₆) d ppm 1.34 - 1.42 (m, 5 H) 1.44 - 1.53 (m, 4 H) 2.34 - 2.48 (m, 7 H) 3.38 - 3.46 (m, 3 H) 3.92 (s, 3 H) 4.29 (q, *J*=6.78 Hz, 2 H) 7.66 (t, *J*=7.63 Hz, 1 H) 7.84 (d, *J*=8.22 Hz, 1 H) 8.06 (s, 1 H) 8.32 (s, 1 H) 8.40 (d, *J*=4.70 Hz, 2 H) 8.44 (s, 1 H) 8.67 (s, 1 H) 8.77 (t, 1 H) 9.66 (s, 1 H) 9.92 (s, 1 H)

### EXAMPLE 201

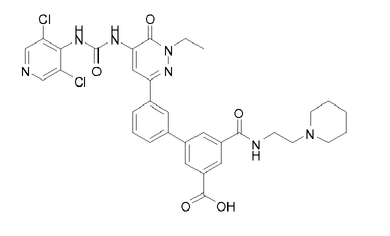

### 3'-[5-({[(3,5-Dichloropyridin-4-yl)amino]carbonyl}amino)-1-ethyl-6-oxo-1,6-dihydropyridazin-3-yl]-5-{[(2-piperidin-1-ylethyl)amino]carbonyl}biphenyl-3-carboxylic acid

Reaction of the title compound of Example 200 (2.30 g, 3.32 mmol) according to the method described in Example 26 gave 1.40 g (61 %) of the title compound.
HPLC/MS (30 min) retention time 10.73 min
LRMS: *m*/*z* 679 (M+)
1H NMR (400 MHz, DMSO-*d*₆) d ppm 1.19 - 1.26 (m, 2 H) 1.38 (t, *J*=7.26 Hz, 3 H) 1.53 - 1.61 (m, 4 H) 2.63 - 2.70 (m, 3 H) 2.71 - 2.77 (m, 2 H) 4.27 (q, *J*=7.05 Hz, 1 H) 7.23 - 7.39 (m, 1 H) 7.60 (t, *J*=7.88 Hz, 1 H) 7.71 - 7.87 (m, 2 H) 7.99 (s, 1 H) 8.22 (s, 1 H) 8.26 (s, 1 H) 8.37 (s, 1 H) 8.44 (s, 1 H) 8.67 - 8.71 (m, 2 H) 8.92 - 9.04 (m, 1 H) 9.69 (s, 1 H) 9.92 (s, 1 H)

### EXAMPLE 202

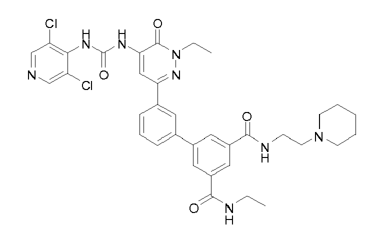

### 3'-[5-({[(3,5-dichloropyridin-4-yl)amino]carbonyl}amino)-1-ethyl-6-oxo-1,6-dihydropyridazin-3-yl]-N-ethyl-N'-(2-piperidin-1-ylethyl)biphenyl-3,5-dicarboxamide

Reaction of the title compound of Example 201 (150 mg, 0.22 mmol) with ethanamine (220 µl, 0.44 mmol) according to the method described in Example 49 gave 32.4 mg (20%) of the title compound after purification by SP1® automated purification system.
HPLC/MS (30 min) retention time 11.17 min
LRMS: *m*/*z* 706 (M+)
1H NMR (400 MHz, DMSO-*d*₆) d ppm 1.15 (t, *J*=7.26 Hz, 3 H) 1.31 - 1.42 (m, 4 H) 1.44 - 1.54 (m, 3 H) 2.31 - 2.42 (m, 3 H) 2.45 (t, *J*=7.05 Hz, 2 H) 2.74 (s, 2 H) 2.89 (s, 2 H) 4.27 (q, *J*=7.05 Hz, 2 H) 7.65 (t, *J*=7.67 Hz, 1 H) 7.83 (dd, *J*=13.89, 7.67 Hz, 2 H) 7.96 (s, 1 H) 8.09 (s, 1 H) 8.26 (d, *J*=4.56 Hz, 2 H) 8.33 (s, 1 H) 8.39 - 8.49 (m, 2 H) 8.67 (t, *J*=5.39 Hz, 1 H) 8.73 (t, *J*=4.98 Hz, 1 H)

### EXAMPLE 203

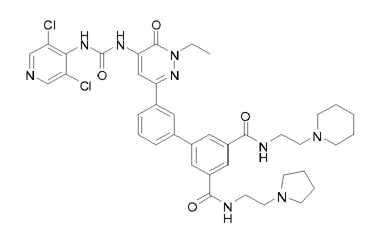

### 3'-[5-({[(3,5-dichloropyridin-4-yl)amino]carbonyl}amino)-1-ethyl-6-oxo-1,6-dihydropyridazin-3-yl]-N-(2-piperidin-1-ylethyl)-N'-(2-pyrrolidin-1-ylethyl)biphenyl-3,5-dicarboxamide

Reaction of the title compound of Example 201 (150 mg, 0.22 mmol) with ethanamine (60 µl, 0.44 mmol) according to the method described in Example 49 gave 42.7 mg (25%) of the title compound after purification by SP1® automated purification system.
HPLC/MS (30 min) retention time 8.42 min
LRMS: *m*/*z* 775 (M+)
1H NMR (400 MHz, DMSO-*d*₆) d ppm 1.34 - 1.42 (m, 4 H) 1.45 - 1.54 (m, 3 H) 1.64 - 1.73 (m, 3 H) 2.36 - 2.43 (m, 3 H) 2.46 (t, *J*=6.63 Hz, 2 H) 2.51 - 2.54 (m, 2 H) 2.63 (t, *J*=7.05 Hz, 2 H) 3.37 - 3.47 (m, 5 H) 4.29 (q, *J*=7.33 Hz, 2 H) 7.66 (t, *J*=7.67 Hz, 1 H) 7.84 (dd, *J*=15.96, 8.09 Hz, 2 H) 8.08 (s, 1 H) 8.21 - 8.28 (m, 3 H) 8.33 (s, 1 H) 8.40 (s, 1 H) 8.66 - 8.68 (m, 2 H) 8.74 (t, *J*=5.60 Hz, 1 H) 9.68 (s, 1 H)

### EXAMPLE 204

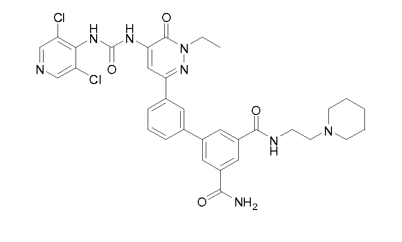

### 3'-[5-({[(3,5-dichloropyridin-4-yl)amino]carbonyl}amino)-1-ethyl-6-oxo-1,6-dihydropyridazin-3-yl]-N-(2-piperidin-1-ylethyl)biphenyl-3,5-dicarboxamide

Reaction of the title compound of Example 201 (150 mg, 0.18 mmol) with ammonium (45.2 µl, 0.36 mmol) according to the method described in Example 49 gave 66.3 mg (53%) of the title compound after purification by SP1® automated purification system.
HPLC/MS (30 min) retention time 10.13 min
LRMS: *m*/*z* 678 (M+)
1H NMR (400 MHz, DMSO-*d*₆) d ppm 1.35 - 1.42 (m, 5 H) 1.45 - 1.54 (m, 4 H) 2.40 (s, 3 H) 2.43 - 2.53 (m, 6 H) 4.29 (q, *J*=6.91 Hz, 2 H) 7.54 (s, 1 H) 7.66 (t, *J*=7.67 Hz, 1 H) 7.84 (dd, *J*=19.49, 7.46 Hz, 2 H) 8.10 (s, 1 H) 8.22 (s, 1 H) 8.25 (s, 1 H) 8.31 (s, 1 H) 8.36 (s, 1 H) 8.40 (s, 1 H) 8.68 (s, 1 H) 9.68 (s, 1 H)

### EXAMPLE 205

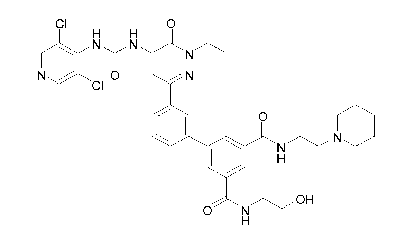

### 3'-[5-({[(3,5-dichloropyridin-4-yl)amino]carbonyl}amino)-1-ethyl-6-oxo-1,6-dihydropyridazin-3-yl]-N-(2-hydroxyethyl)-N'-(2-piperidin-1-ylethyl)biphenyl-3,5-dicarboxamide

Reaction of the title compound of Example 201 (150 mg, 0.18 mmol) with 2-aminoethanol (22.4 mg, 0.37 mmol) according to the method described in Example 49 gave 67 mg (49%) of the title compound after purification by SP1® automated purification system.
HPLC/MS (30 min) retention time 10.12 min
LRMS: *m*/*z* 722 (M+)
1H NMR (400 MHz, DMSO-*d*₆) d ppm 1.39 (t, *J*=7.26 Hz, 3 H) 1.45 - 1.53 (m, 4 H) 2.35 - 2.42 (m, 4 H) 2.46 (t, *J*=7.05 Hz, 2 H) 2.49 - 2.53 (m, 2 H) 3.38 - 3.45 (m, 4 H) 3.55 (q, *J*=5.81 Hz, 2 H) 4.29 (q, *J*=7.05 Hz, 2 H) 4.76 (t, *J*=5.18 Hz, 1 H) 7.66 (t, *J*=7.67 Hz, 1 H) 7.84 (dd, *J*=20.32, 7.88 Hz, 2 H) 8.09 (s, 1 H) 8.25 (s, 1 H) 8.29 (s, 1 H) 8.34 (s, 1 H) 8.40 (s, 1 H) 8.63 - 8.67 (m, 1 H) 8.68 (s, 2 H) 8.71 (t, *J*=5.60 Hz, 1 H) 9.68 (s, 1 H) 9.91 (s, 1 H)

### EXAMPLE 206

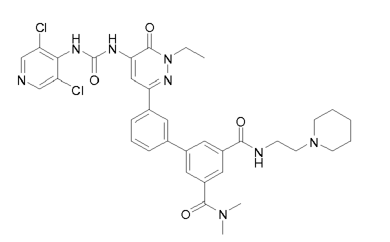

### 3'-[5-({[(3,5-dichloropyridin-4-yl)amino]carbonyl}amino)-1-ethyl-6-oxo-1,6-dihydropyridazin-3-yl]-N,N-dimethyl-N'-(2-piperidin-1-ylethyl)biphenyl-3,5-dicarboxamide

Reaction of the title compound of Example 201 (150 mg, 0.18 mmol) with N-methylmethanamine (183.5 µl, 0.37 mmol) according to the method described in Example 49 gave 34 mg (26%) of the title compound after purification by SP1® automated purification system.
HPLC/MS (30 min) retention time 10.80 min
LRMS: *m*/*z* 706 (M+)
1H NMR (400 MHz, DMSO-*d*₆) d ppm 1.25 - 1.34 (m, 5 H) 1.36 - 1.44 (m, 4 H) 2.21 - 2.27 (m, 4 H) 2.31 (t, *J*=6.65 Hz, 2 H) 2.78 (s, 3 H) 2.85 (s, 3 H) 3.20 (q, 2 H) 4.04 (q, *J*=6.65 Hz, 2 H) 7.21 (t, *J*=7.43 Hz, 1 H) 7.35 - 7.44 (m, 4 H) 7.60 (s, 1 H) 7.73 (s, 1 H) 7.93 (s, 1 H) 8.14 (t, *J*=5.09 Hz, 1 H) 8.18 (s, 2 H) 9.11 (s, 1 H) 9.34 (s, 1 H)

### EXAMPLE 207

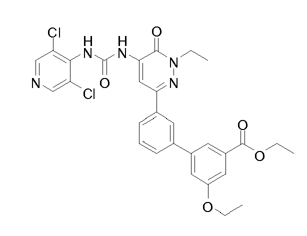

### Ethyl 3'-[5-({[(3,5-dichloropyridin-4-yl)amino]carbonyl}amino)-1-ethyl-6-oxo-1,6-dihydropyridazin-3-yl]-5-ethoxybiphenyl-3-carboxylate

Reaction of the title compound of Preparation 7 (0.98 g, 2.03 mmol) with (0.97 g, 3.03 mmol) of the title compound of Preparation 80 according to the method described in Example 1 gave 0.94 g (39 %) of the title compound after purification by chromatography (hexane 100% to ethyl acetate 50%).
HPLC/MS (10 min) retention time 7.86 min
LRMS: *m*/*z* 597 (M+)

### EXAMPLE 208

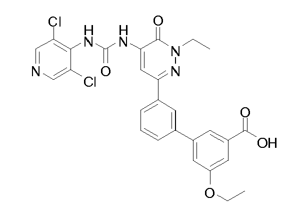

### 3'-[5-({[(3,5-dichloropyridin-4-yl)amino]carbonyl}amino)-1-ethyl-6-oxo-1,6-dihydropyridazin-3-yl]-5-ethoxybiphenyl-3-carboxylic acid

Reaction of the title compound of Example 207 (0.95g, 1.45 mmol) according to the method described in Example 26 gave 0.304 g (69%) of the title compound without further purification.
HPLC/MS (30 min) retention time 17.38 min
LRMS: *m*/*z* 569 (M+)
1H NMR (400 MHz, DMSO-*d*₆) d ppm 1.38 (q, *J*=7.04 Hz, 6 H) 4.17 (q, *J*=6.91 Hz, 2 H) 4.28 (q, *J*=7.30 Hz, 2 H) 7.45 (s, 2 H) 7.61 (t, *J*=7.63 Hz, 1 H) 7.75 - 7.83 (m, 3 H) 8.00 (s, 1 H) 8.39 (s, 1 H) 8.69 (s, 2 H) 9.70 (s, 1 H) 9.91 (s, 1 H)

### EXAMPLE 209

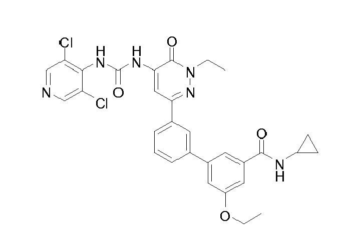

### N-cyclopropyl-3'-[5-({[(3,5-dichloropyridin-4-yl)amino]carbonyl}amino)-1-ethyl-6-oxo-1,6-dihydropyridazin-3-yl]-5-ethoxybiphenyl-3-carboxamide

Reaction of the title compound of Example 208 (160 mg, 0.24 mmol) with cyclopropanamine (33.3 µl, 0.48 mmol) according to the method described in Example 49 gave 101.6 mg (70%) of the title compound after purification by SP1® automated purification system.
HPLC/MS (30 min) retention time 17.17 min
LRMS: *m*/*z* 608 (M+)
1H NMR (200 MHz, DMSO-*d*₆) d ppm 0.54 - 0.63 (m, 2 H) 0.66 - 0.77 (m, 2 H) 1.33 - 1.45 (m, 6 H) 2.80 - 2.92 (m, 1 H) 4.08 - 4.37 (m, 4 H) 7.36 (d, *J*=8.98 Hz, 2 H) 7.55 - 7.87 (m, 4 H) 8.02 (s, 1 H) 8.40 (s, 1 H) 8.52 (d, *J*=3.90 Hz, 1 H) 8.68 (s, 2 H) 9.67 (s, 1 H) 9.91 (s, 1 H)

### EXAMPLE 210

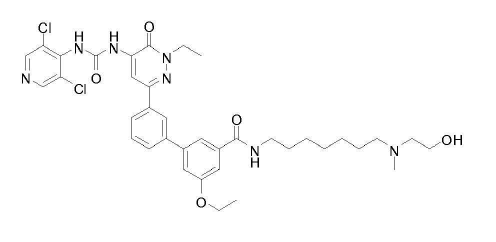

### 3'-[5-({[(3,5-dichloropyridin-4-yl)amino]carbonyl}amino)-1-ethyl-6-oxo-1,6-dihydropyridazin-3-yl]-5-ethoxy-N-{7-[(2-hydroxyethyl)(methyl)amino]heptyl}biphenyl-3-carboxamide

Reaction of the title compound of Example 208 (160 mg, 0.24 mmol) with 2-[(7-aminoheptyl)(methyl)amino]ethanol (90.3 mg, 0.48 mmol) according to the method described in Example 49 gave 73 mg (56%) of the title compound after purification by SP1® automated purification system.
HPLC/MS (30 min) retention time 13.27 min
LRMS: *m*/*z* 739 (M+)
1H NMR (200 MHz, CHLOROFORM-*d*) d ppm 1.21 - 1.66 (m, 20 H) 2.24 (s, 3 H) 2.40 (t, 2 H) 2.52 (t, 2 H) 3.45 (q, 2 H) 3.58 (t, 2 H) 4.05 - 4.32 (m, 4 H) 6.31 (t, *J*=5.66 Hz, 1 H) 7.22 - 7.24 (m, 1 H) 7.29 - 7.33 (m, 1 H) 7.48 - 7.56 (m, 2 H) 7.58 - 7.66 (m, 1 H) 7.82 (d, *J*=7.81 Hz, 1 H) 8.02 (s, 1 H) 8.61 (s, 1 H) 8.72 (s, 1 H)

### EXAMPLE 211

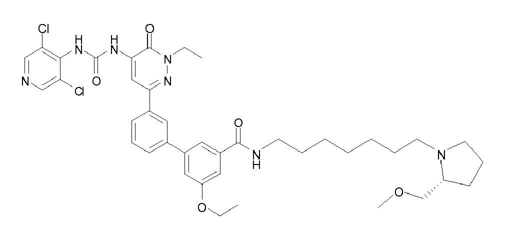

### 3'-[5-({[(3,5-dichloropyridin-4-yl)amino]carbonyl}amino)-1-ethyl-6-oxo-1,6-dihydropyridazin-3-yl]-5-ethoxy-N-{7-[(2R)-2-(methoxymethyl)pyrrolidin-1-yl]heptyl}biphenyl-3-carboxamide

Reaction of the title compound of Example 208 (195 mg, 0.29 mmol) with {7-[(2R)-2-(methoxymethyl)pyrrolidin-1-yl]heptyl}amine (133.2 mg, 0.58 mmol) according to the method described in Example 49 gave 126.9 mg (55%) of the title compound after purification by SP1® automated purification system.
HPLC/MS (30 min) retention time 14.18 min
LRMS: *m*/*z* 779 (M+)
1H NMR (200 MHz, DMSO-*d*₆) d ppm 1.26 - 1.32 (m, 4 H) 1.32 - 1.44 (m, 10 H) 1.46 - 1.87 (m, 6 H) 2.00 - 2.25 (m, 2 H) 2.68 - 2.81 (m, 1 H) 2.93 - 3.18 (m, 3 H) 3.21 (s, 3 H) 3.24 - 3.35 (m, 5 H) 4.16 (q, *J*=6.77 Hz, 2 H) 4.28 (q, *J*=7.03 Hz, 2 H) 7.34 (s, 1 H) 7.40 (s, 1 H) 7.56 - 7.84 (m, 4 H) 8.02 (s, 1 H) 8.39 (s, 1 H) 8.55 (t, *J*=5.66 Hz, 1 H) 9.69 (s, 1 H)

### EXAMPLE 212

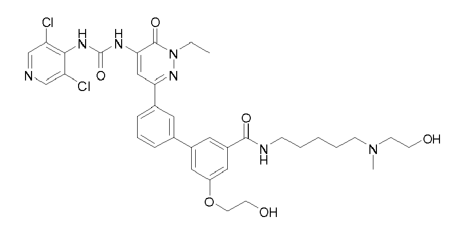

### 3'-[5-({[(3,5-dichloropyridin-4-yl)amino]carbonyl}amino)-1-ethyl-6-oxo-1,6-dihydropyridazin-3-yl]-5-(2-hydroxyethoxy)-N-{5-[(2-hydroxyethyl)(methyl)amino]pentyl}biphenyl-3-carboxamide

Reaction of the title compound of Example 159 (160 mg, 0.26 mmol) with 2-[(5-aminopentyl)(methyl)amino]ethanol (83.4 mg, 0.52 mmol) according to the method described in Example 49 gave 90.9 mg (42%) of the title compound after purification by SP1® automated purification system.
HPLC/MS (30 min) retention time 10.95 min
LRMS: *m*/*z* 727 (M+)
1H NMR (400 MHz, DMSO-*d*₆) d ppm 1.13 - 1.35 (m, 4 H) 1.36 - 1.47 (m, 4 H) 1.49 - 1.59 (m, 2 H) 2.15 (s, 3 H) 2.31 (t, *J*=7.05 Hz, 2 H) 2.37 (t, *J*=6.43 Hz, 2 H) 3.23 - 3.28 (m, 2 H) 3.41 - 3.49 (m, 2 H) 3.73 - 3.80 (m, 2 H) 4.12 (t, *J*=4.77 Hz, 2 H) 4.24 - 4.35 (m, 2 H) 4.89 (t, *J*=5.18 Hz, 2 H) 7.40 (d, *J*=28.20 Hz, 2 H) 7.61 (t, *J*=7.88 Hz, 1 H) 7.72 (s, 1 H) 7.79 (d, *J*=5.81 Hz, 2 H) 8.02 (s, 1 H) 8.40 (s, 1 H) 8.55 (s, 1 H) 8.66 (s, 1 H) 9.59 (s, 1 H) 9.88 (s, 1 H)

### EXAMPLE 213

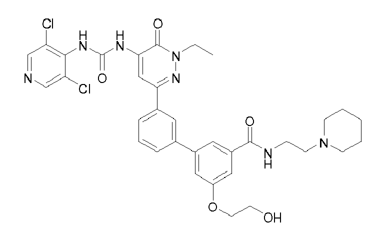

### 3'-[5-({[(3,5-dichloropyridin-4-yl)amino]carbonyl}amino)-1-ethyl-6-oxo-1,6-dihydropyridazin-3-yl]-5-(2-hydroxyethoxy)-N-(2-piperidin-1-ylethyl)biphenyl-3-carboxamide

Reaction of the title compound of Example 159 (150 mg, 0.24 mmol) with (2-piperidin-1-ylethyl)amine (69.5 µl, 0.49 mmol) according to the method described in Example 49 gave 81.7 mg (47%) of the title compound after purification by SP1® automated purification system.
HPLC/MS (30 min) retention time 10.95 min
LRMS: *m*/*z* 695 (M+)
1H NMR (400 MHz, DMSO-*d*₆) d ppm 1.39 (t, *J*=7.26 Hz, 4 H) 1.44 - 1.53 (m, 4 H) 2.33 - 2.47 (m, 5 H) 3.35 - 3.42 (m, 2 H) 3.76 (q, *J*=4.98 Hz, 2 H) 4.12 (t, *J*=4.98 Hz, 2 H) 4.28 (q, *J*=7.05 Hz, 2 H) 4.89 (t, *J*=5.39 Hz, 1 H) 7.36 (s, 1 H) 7.42 (s, 1 H) 7.61 (t, *J*=7.67 Hz, 1 H) 7.71 (s, 1 H) 7.79 (d, *J*=7.46 Hz, 2 H) 8.02 (s, 1 H) 8.39 (s, 1 H) 8.51 (t, *J*=5.60 Hz, 1 H) 8.67 (s, 1 H) 9.64 (s, 1 H) 9.92 (s, 1 H)

### EXAMPLE 214

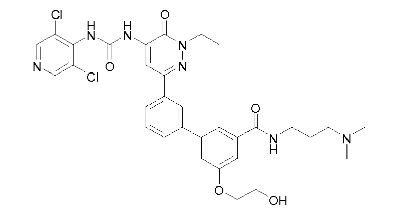

### 3'-[5-({[(3,5-dichloropyridin-4-yl)amino]carbonyl}amino)-1-ethyl-6-oxo-1,6-dihydropyridazin-3-yl]-N-[3-(dimethylamino)propyl]-5-(2-hydroxyethoxy)biphenyl-3-carboxamide

Reaction of the title compound of Example 159 (150 mg, 0.24 mmol) with N,N-dimethylpropane-1,3-diamine (61.4 µl, 0.49 mmol) according to the method described in Example 49 gave 74.6 mg (44%) of the title compound after purification by SP1® automated purification system.
HPLC/MS (30 min) retention time 10.58 min
LRMS: *m*/*z* 669 (M+)
1H NMR (400 MHz, DMSO-*d*₆) d ppm 1.39 (t, *J*=7.05 Hz, 3 H) 1.62 - 1.71 (m, 2 H) 2.14 (s, 6 H) 2.27 (t, *J*=7.05 Hz, 2 H) 3.73 - 3.79 (m, 2 H) 4.12 (t, *J*=4.98 Hz, 2 H) 4.28 (q, *J*=7.05 Hz, 2 H) 4.87 - 4.93 (m, 1 H) 7.36 (s, 1 H) 7.42 (s, 1 H) 7.61 (t, *J*=7.88 Hz, 1 H) 7.71 (s, 1 H) 7.79 (d, *J*=7.05 Hz, 2 H) 8.02 (s, 1 H) 8.62 (t, *J*=5.39 Hz, 1 H) 8.67 (s, 1 H) 9.61 (s, 1 H) 9.89 (s, 1 H)

### EXAMPLE 215

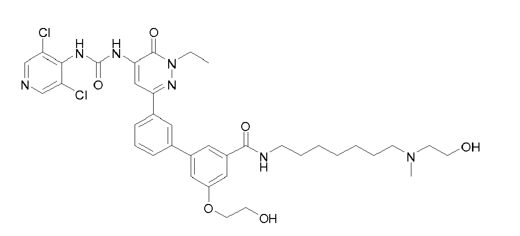

### 3'-[5-({[(3,5-dichloropyridin-4-yl)amino]carbonyl}amino)-1-ethyl-6-oxo-1,6-dihydropyridazin-3-yl]-5-(2-hydroxyethoxy)-N-{7-[(2-hydroxyethyl)(methyl)amino]heptyl}biphenyl-3-carboxamide

Reaction of the title compound of Example 159 (150 mg, 0.24 mmol) with 2-[(5-aminopentyl)(methyl)amino]ethanol (91.8 mg, 0.49 mmol) according to the method described in Example 49 gave 37.9 mg (21 %) of the title compound after purification by SP1® automated purification system.
HPLC/MS (30 min) retention time 10.58 min
LRMS: *m*/*z* 753 (M-)
1H NMR (400 MHz, DMSO-*d*₆) d ppm 1.23 - 1.33 (m, 6 H) 1.39 (t, *J*=7.04 Hz, 3 H) 1.47 - 1.57 (m, 2 H) 2.14 (s, 3 H) 2.30 (t, 2 H) 2.38 (t, *J*=6.26 Hz, 2 H) 3.32 (s, 7 H) 3.44 (t, *J*=6.46 Hz, 2 H) 3.76 (t, *J*=4.50 Hz, 2 H) 4.12 (t, *J*=5.09 Hz, 2 H) 4.28 (q, *J*=6.78 Hz, 2 H) 4.89 (s, 1 H) 7.36 (s, 1 H) 7.43 (s, 1 H) 7.61 (t, *J*=7.63 Hz, 1 H) 7.72 (s, 1 H) 7.79 (d, *J*=7.43 Hz, 2 H) 8.02 (s, 1 H) 8.40 (s, 1 H) 8.55 (t, *J*=5.48 Hz, 1 H) 8.67 (s, 1 H) 9.62 (s, 1 H)

### EXAMPLE 216

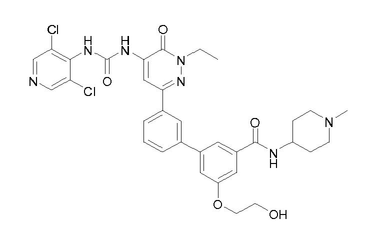

### 3'-[5-({[(3,5-dichloropyridin-4-yl)amino]carbonyl}amino)-1-ethyl-6-oxo-1,6-dihydropyridazin-3-yl]-5-(2-hydroxyethoxy)-N-(1-methylpiperidin-4-yl)biphenyl-3-carboxamide

Reaction of the title compound of Example 159 (150 mg, 0.24 mmol) with 1-methylpiperidin-4-amine (55.7 mg, 0.49 mmol) according to the method described in Example 49 gave 80.1 mg (47%) of the title compound after purification by SP1® automated purification system.
HPLC/MS (30 min) retention time 10.18 min
LRMS: *m*/*z* 681 (M+)
1H NMR (400 MHz, DMSO-*d*₆) d ppm 1.39 (t, *J*=7.24 Hz, 3 H) 1.53 - 1.65 (m, 2 H) 1.73-1.81 (m, 2H) 1.94 (t, 2H) 2.17 (s, 3H) 2.78 (d, *J*=11.74 Hz, 2 H) 3.71 - 3.80 (m, 3 H) 4.12 (t, *J*=5.09 Hz, 2 H) 4.28 (q, *J*=7.04 Hz, 2 H) 4.89 (t, *J*=5.48 Hz, 1 H) 7.36 (s, 1 H) 7.43 (s, 1 H) 7.61 (t, *J*=7.63 Hz, 1 H) 7.71 (s, 1 H) 7.79 (d, *J*=7.83 Hz, 2 H) 8.02 (s, 1 H) 8.35 (d, *J*=7.83 Hz, 1 H) 8.40 (s, 1 H) 8.67 (s, 1 H) 9.64 (s, 1 H) 9.90 (s, 1 H)

### EXAMPLE 217

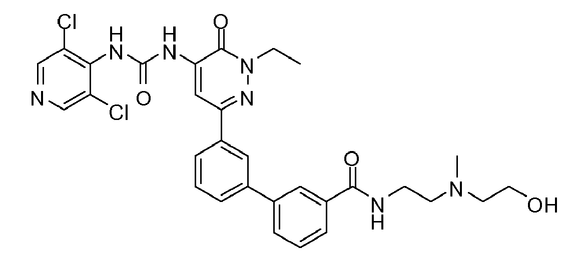

### 3'-[5-({[(3,5-Dichloropyridin-4-yl)amino]carbonyl}amino)-1-ethyl-6-oxo-1,6-dihydropyridazin-3-yl]-N-{2-[(2-hydroxyethyl)(methyl)amino]ethyl}biphenyl-3-carboxamide

Reaction of the title compound of Example 48 (244 mg, 0.47 mmol) with 2-((2-aminoethyl)(methyl)amino)ethanol (110 mg, 0.93 mmol) according to the method described in Example 49 gave 28 mg (10%) of the title compound.
¹H NMR (200 MHz, CHLOROFORM-*d*) ppm 1.26 (t, *J*=7.03 Hz, 3 H) 2.85 (s, 3 H) 3.06 - 3.25 (m, 2 H) 3.27 - 3.43 (m, 2 H) 3.78 - 4.00 (m, 4 H) 4.08 (q, *J*=7.29 Hz, 2 H) 7.35 - 7.99 (m, 7 H) 8.03 (s, 1 H) 8.33 (s, 1 H) 8.40 - 8.50 (m, 1 H) 8.58 (s, 2 H) 8.60 - 8.75 (m, 1 H) 9.25 (s, 1 H)
HPLC/MS (15 min) retention time 6.69 min.
LRMS: *m*/*z* 624 (M+)

### EXAMPLE 218

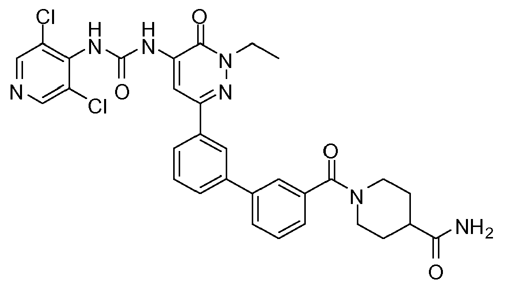

### 1-({3'-[5-({[(3,5-Dichloropyridin-4-yl)amino]carbonyl}amino)-1-ethyl-6-oxo-1,6-dihydropyridazin-3-yl]biphenyl-3-yl}carbonyl)piperidine-4-carboxamide

Reaction of the title compound of Example 48 (100 mg, 0.19 mmol) with piperidine-4-carboxamide (49 mg, 0.38 mmol) according to the method described in Example 49 gave 53 mg (43%) of the title compound.
¹H NMR (200 MHz, DMSO-*d*₆) ppm 1.38 (t, *J*=7.22 Hz, 3 H) 1.50 - 1.57 (m, 4 H) 1.69 - 1.78 (m, 4 H) 2.35 - 2.40 (m, 1 H) 4.28 (q, *J*=6.51 Hz, 2 H) 6.82 (s, 1 H) 7.28 (s, 1 H) 7.40 (d, *J*=7.81 Hz, 1 H) 7.47 - 7.71 (m, 3 H) 7.71 - 7.90 (m, 3 H) 8.02 (s, 1 H) 8.40 (s, 1 H) 8.69 (s, 2 H) 9.68 (s, 1 H) 9.90 (s, 1 H)
HPLC/MS (15 min) retention time 7.93 min.
LRMS: *m*/*z* 634 (M+)

### EXAMPLE 219

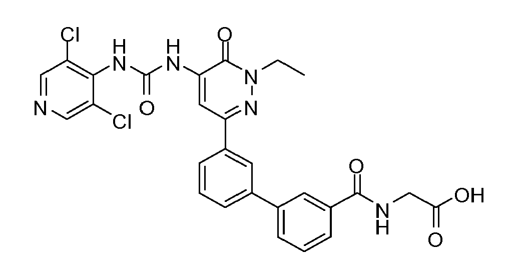

### N-({3'-[5-({[(3,5-Dichloropyridin-4-yl)amino]carbonyl}amino)-1-ethyl-6-oxo-1,6-dihydropyridazin-3-yl]biphenyl-3-yl}carbonyl)glycine

Reaction of the title compound of Preparation 87 (70 mg, 0.09 mmol) with lithium hydroxide (20 mg, 0.48 mmol) in a mixture 1:1 water/THF at room temperature under stirring for 4h gave 15 mg (27%) of the title compound as a solid after acidification with 2N HCI.
¹H NMR (200 MHz, DMSO-*d*₆) ppm 1.38 (t, *J*=7.22 Hz, 3 H) 3.86 (d, *J*=5.47 Hz, 2 H) 4.28 (q, *J*=7.42 Hz, 2 H) 7.64 (dd, *J*=7.81, 3.90 Hz, 3 H) 7.74 - 7.97 (m, *J*=17.18 Hz, 4 H) 8.05 (s, 1 H) 8.17 (s, 1 H) 8.40 (s, 1 H) 8.68 (s, 2 H) 8.73 - 8.88 (m, 2 H)
HPLC/MS (15 min) retention time 8.44 min.
LRMS: *m*/*z* 581 (M+)

### EXAMPLE 220

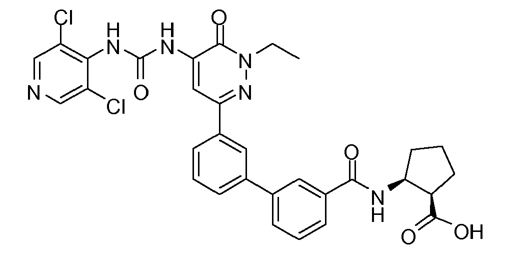

### (1R,2S)-2-[({3'-[5-({[(3,5-Dichloropyridin-4-yl)amino]carbonyl}amino)-1-ethyl-6-oxo-1,6-dihydropyridazin-3-yl]biphenyl-3-yl}carbonyl)amino]cyclopentanecarboxylic acid

Reaction of the title compound of Example 48 (100 mg, 0.19 mmol) with (1 S,2R)-2-aminocyclopentanecarboxylic acid (49 mg, 0.38 mmol) according to the method described in Example 49 gave 20 mg (16%) of the title compound.
¹H NMR (200 MHz, CHLOROFORM-*d*) ppm 1.04 - 1.35 (m, 3 H) 1.42 - 2.25 (m, 6 H) 2.92 - 3.27 (m, 1 H) 4.14 (q, *J*=7.68 Hz, 2 H) 4.54 - 4.97 (m, 1 H) 7.35 (d, *J*=8.59 Hz, 1 H) 7.49 (t, *J*=7.61 Hz, 2 H) 7.57 - 7.76 (m, 2 H) 7.78 - 8.02 (m, 5 H) 8.61 (s, 2 H) 9.20 (s, 1 H) 9.63 (s, 1 H)
HPLC/MS (15 min) retention time 8.74 min.
LRMS: *m*/*z* 635 (M+)

### EXAMPLE 221

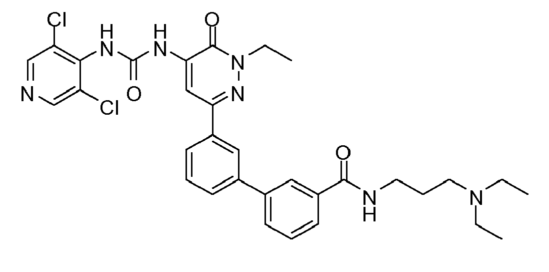

### 3'-[5-({[(3,5-Dichloropyridin-4-yl)amino]carbonyl}amino)-1-ethyl-6-oxo-1,6-dihydropyridazin-3-yl]-N-[3-(diethylamino)propyl]biphenyl-3-carboxamide

Reaction of the title compound of Example 48 (150 mg, 0.29 mmol) with N¹,N¹-diethylpropane-1,3-diamine (75 mg, 0.58 mmol) according to the method described in Example 49 gave 105 mg (58%) of the title compound.
¹H NMR (200 MHz, CHLOROFORM-*d*) ppm 1.04 - 1.47 (m, 8 H) 1.99 - 2.41 (m, 2 H) 2.99 - 3.23 (m, 4 H) 3.31 (t, *J*=7.42 Hz, 2 H) 3.54 - 3.81 (m, 2 H) 3.84 - 4.12 (m, 2 H) 7.35 - 7.74 (m, 5 H) 7.82 (s, 1 H) 7.93 (d, *J*=7.81 Hz, 1 H) 8.02 (s, 1 H) 8.28 (s, 1 H) 8.47 (t, *J*=5.66 Hz, 1 H) 8.58 (s, 2 H) 9.31 (s, 1 H) 10.26 (s, 1 H)
HPLC/MS (15 min) retention time 7.01 min.
LRMS: *m*/*z* 636 (M+)

### EXAMPLE 222

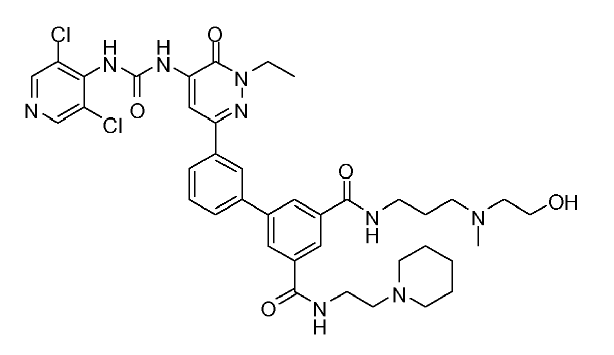

### 3'-[5-({[(3,5-Dichloropyridin-4-yl)amino]carbonyl}amino)-1-ethyl-6-oxo-1,6-dihydropyridazin-3-yl]-N-{3-[(2-hydroxyethyl)(methyl)amino]propyl}-N'-(2-piperidin-1-ylethyl)biphenyl-3,5-dicarboxamide

Reaction of the title compound of Example 201 (150 mg, 0.22 mmol) with 2-((3-aminopropyl)(methyl)amino)ethanol (83 mg, 0.44 mmol, prepared in a similar manner as described by Stadtmueller, Heinz et al. at PCT Int.Appl. WO 2006021544, method 23 page 58) according to the method described in Example 151 gave 46 mg (25%) of the title compound.
¹H NMR (200 MHz, CHLOROFORM-*d*) ppm 1.11 - 1.43 (m, 3 H) 1.47 - 1.69 (m, 2 H) 1.88 (d, *J*=1.56 Hz, 4 H) 2.11 - 2.46 (m, 3 H) 2.51 (s, 3 H) 2.69 - 2.93 (m, 4 H) 2.94 - 3.17 (m, 4 H) 3.17 - 3.35 (m, 2 H) 3.49 - 3.69 (m, *J*=3.51 Hz, 2 H) 3.73 - 4.00 (m, 4 H) 4.02 - 4.28 (m, 2 H) 7.47 (s, 1 H) 7.65 (s, 2 H) 7.94 (s, 1 H) 8.12 (s, 1 H) 8.33 (s, 4 H) 8.55 (s, 2 H) 8.80 (s, 1 H) 9.03 (s, 1 H) 9.33 (s, 1 H)
HPLC/MS (15 min) retention time 8.18 min.
LRMS: *m*/*z* 792 (M+)

### EXAMPLE 223

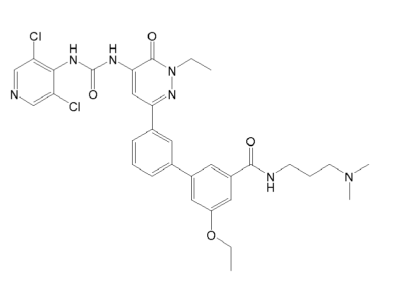

### 3'-[5-({[(3,5-dichloropyridin-4-yl)amino]carbonyl}amino)-1-ethyl-6-oxo-1,6-dihydropyridazin-3-yl]-N-[3-(dimethylamino)propyl]-5-ethoxybiphenyl-3-carboxamide

Reaction of the title compound of Example 208 (150 mg, 0.26 mmol) with N1,N1-dimethylpropane-1,3-diamine (67 I, 0.53 mmol) according to the method described in Example 150 gave 113 mg (66%) of the title compound.
¹H NMR (200 MHz, DMSO-*d₆*) ppm 1.4 (m, 6 H) 1.7 (m, 2 H) 2.1 (s, 6 H) 2.3 (t, *J*=7.0 Hz, 2 H) 4.2 (q, *J*=6.9 Hz, 2 H) 4.3 (q, *J*=7.4 Hz, 2 H) 7.3 (s, 1 H) 7.4 (s, 1 H) 7.6 (m, 1 H) 7.7 (s, 1 H) 7.8 (s, 1 H) 7.8 (s, 1 H) 8.0 (s, 1 H) 8.4 (s, 1 H) 8.6 (t, *J*=5.7 Hz, 1 H) 8.7 (s, 2 H) 9.6 (s, 1 H) 9.9 (s, 1 H)
HPLC/MS (15 min) retention time 6.93 min
LRMS: *m*/*z* 652 (M+)

### EXAMPLE 224

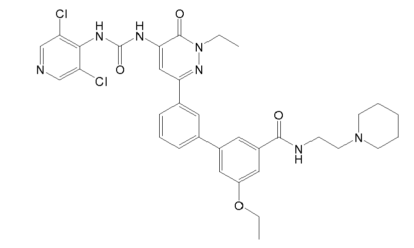

### 3'-[5-({[(3,5-dichloropyridin-4-yl)amino]carbonyl}amino)-1-ethyl-6-oxo-1,6-dihydropyridazin-3-yl]-5-ethoxy-N-(2-piperidin-1-ylethyl)biphenyl-3-carboxamide

Reaction of the title compound of Example 208 (150 mg, 0.26 mmol) with 2-(piperidin-1-yl)ethanamine (75 I, 0.53 mmol) according to the method described in Example 150 gave 117 mg (65%) of the title compound.
¹H NMR (200 MHz, DMSO-*d₆*) ppm 1.4 (m, 12 H) 4.2 (m, 6 H) 4.3 (q, *J*=7.0 Hz, 2 H) 7.3 (s, 1 H) 7.4 (s, 1 H) 7.6 (m, 1 H) 7.7 (s, 1 H) 7.8 (s, 1 H) 7.8 (s, 1 H) 8.0 (s, 1 H) 8.4 (s, 1 H) 8.5 (t, *J*=5.5 Hz, 1 H) 8.7 (s, 2 H) 9.7 (s, 1 H) 9.9 (s, 1 H)
HPLC/MS (15 min) retention time 5.80 min
LRMS: *m*/*z* 678 (M+)

### EXAMPLE 225

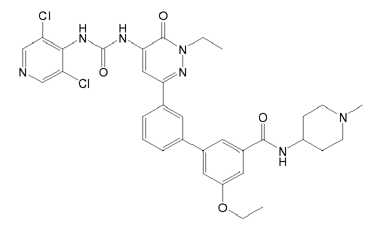

### 3'-[5-({[(3,5-dichloropyridin-4-yl)amino]carbonyl}amino)-1-ethyl-6-oxo-1,6-dihydropyridazin-3-yl]-5-ethoxy-N-(1-methylpiperidin-4-yl)biphenyl-3-carboxamide

Reaction of the title compound of Example 208 (160 mg, 0.28 mmol) with 1-methylpiperidin-4-amine (60 mg, 0.53 mmol) according to the method described in Example 150 gave 130 mg (70%) of the title compound.
¹H NMR (200 MHz, DMSO-*d₆*) ppm 1.4 (m, 6 H) 1.7 (m, 4 H) 2.0 (m, 2 H) 2.2 (s, 3 H) 2.8 (m, 2 H) 3.8 (m, 1 H) 4.2 (q, *J*=7.0 Hz, 2 H) 4.3 (q, *J*=6.9 Hz, 2 H) 7.3 (s, 1 H) 7.4 (s, 1 H) 7.6 (m, 1 H) 7.7 (s, 1 H) 7.8 (s, 1 H) 7.8 (s, 1 H) 8.0 (s, 1 H) 8.3 (s, 1 H) 8.4 (s, 1 H) 8.4 (s, 1 H) 8.7 (s, 2 H)
HPLC/MS (15 min) retention time 7.30 min
LRMS: *m*/*z* 664 (M+)

### EXAMPLE 226

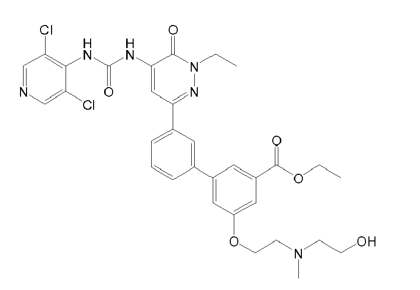

### Ethyl 3'-[5-({[(3,5-dichloropyridin-4-yl)amino]carbonyl}amino)-1-ethyl-6-oxo-1,6-dihydropyridazin-3-yl]-5-{2-[(2-hydroxyethyl)(methyl)amino]ethoxy}biphenyl-3-carboxylate

Reaction of the title compound of Preparation 7 (330 mg, 0.68 mmol) with compound of Preparation 90 (300 mg, 0.76 mmol) according to the method described in Example 1 gave 49 mg (11%) of the title compound after purification by the SP1® automated purification system.
HPLC/MS (15 min) retention time 6.95 min
LRMS: *m*/*z* 669 (M+)

### EXAMPLE 227

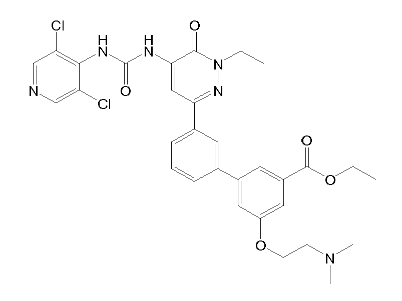

### Ethyl 3'-[5-({[(3,5-dichloropyridin-4-yl)amino]carbonyl}amino)-1-ethyl-6-oxo-1,6-dihydropyridazin-3-yl]-5-[2-(dimethylamino)ethoxy]biphenyl-3-carboxylate

Reaction of the title compound of Preparation 7 (300 mg, 0.62 mmol) with compound of Preparation 92 (270 mg, 0.74 mmol) according to the method described in Example 1 gave 131 mg (33%) of the title compound after purification by the SP1® automated purification system.
¹H NMR (200 MHz, DMSO-*d₆*) ppm 1.3 (m, 6 H) 2.2 (m, 6 H) 2.7 (m, 2 H) 4.3 (m, 6 H) 7.6 (m, 3 H) 7.8 (m, 3 H) 8.0 (s, 1 H) 8.4 (s, 1 H) 8.7 (s, 2 H) 9.7 (s, 1 H) 9.9 (s, 1 H)
HPLC/MS (15 min) retention time 7.02 min
LRMS: *m*/*z* 639 (M+)

### EXAMPLE 228

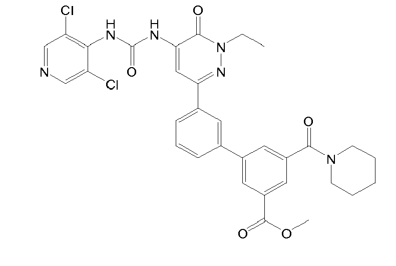

### Methyl 3'-[5-({[(3,5-dichloropyridin-4-yl)amino]carbonyl}amino)-1-ethyl-6-oxo-1,6-dihydropyridazin-3-yl]-5-(piperidin-1-ylcarbonyl)biphenyl-3-carboxylate

Reaction of the title compound of Preparation 104 (200 mg, 0.24 mmol) with piperidine (48 mg, 0.36 mmol) according to the method described in Example 150 gave 30 mg (19%) of the title compound.
¹H NMR (200 MHz, DMSO-*d6*) ppm 1.5 (m, 9 H) 2.1 (m, 2 H) 3.6 (m, 2 H) 3.9 (s, 3 H) 4.3 (m, 2 H) 7.7 (m, 1 H) 7.9 (m, 5 H) 8.3 (s, 1 H) 8.4 (s, 1 H) 8.7 (s, 2 H) 9.7 (s, 1 H) 9.9 (m, 1 H)
HPLC/MS (15 min) retention time 9.50 min
LRMS: *m*/*z* 649 (M+)

### EXAMPLE 229

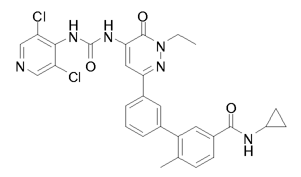

### N-cyclopropyl-3'-[5-({[(3,5-dichloropyridin-4-yl)amino]carbonyl}amino)-1-ethyl-6-oxo-1,6-dihydropyridazin-3-yl]-6-methylbiphenyl-3-carboxamide

Reaction of the title compound of Preparation 7 (300 mg, 0.62 mmol) with 0.28 g of the title compound of Preparation 92 according to the method described in Example 1 gave 192.6 mg (54%) of the title compound.
¹H NMR (200 MHz, DMSO-*d*₆) ppm 0.40 - 0.76 (m, 4 H) 1.36 (t, 3 H) 2.26 (s, 3 H) 2.63 - 3.05 (m, 1 H) 4.26 (q, *J*=7.03 Hz, 2 H) 7.33 - 7.51 (m, 2 H) 7.59 (t, *J*=7.61 Hz, 1 H) 7.71 (d, *J*=1.56 Hz, 2 H) 7.78 (t, *J*=7.42 Hz, 2 H) 8.38 (s, 1 H) 8.41 (d, *J*=4.69 Hz, 1 H) 8.68 (s, 2 H) 9.68 (s, 1 H) 9.89 (s, 1 H)
HPLC/MS (15 min) retention time 9.23 min.
LRMS: *m*/*z* 575 (M-)

### EXAMPLE 230

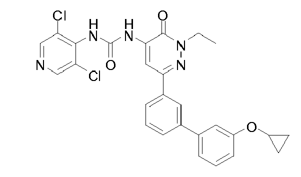

### N-{6-[3'-(cyclopropyloxy)biphenyl-3-yl]-2-ethyl-3-oxo-2,3-dihydropyridazin-4-yl}-N'-(3,5-dichloropyridin-4-yl)urea

Reaction of the title compound of Preparation 7 (250 mg, 0.52 mmol) with 0.202 g (0.78 mmol) of 2-[3-(cyclopropyloxy)phenyl]-4,4,5,5-tetramethyl-1,3,2-dioxaborolane (prepared as described in WO 2008077639) according to the method described in Example 1 gave 147.9 mg (52%) of the title compound.
¹H NMR (400 MHz, DMSO-*d*₆) ppm 0.63 - 0.73 (m, 2 H) 0.75 - 0.88 (m, 2 H) 1.39 (t, *J*=7.24 Hz, 3 H) 3.76 - 4.04 (m, 1 H) 4.27 (q, *J*=7.04 Hz, 2 H) 7.09 (dd, *J*=7.63, 2.15 Hz, 1 H) 7.27 (d, *J*=8.22 Hz, 1 H) 7.31 (d, *J*=1.96 Hz, 1 H) 7.41 (t, *J*=7.83 Hz, 1 H) 7.57 (t, *J*=7.83 Hz, 1 H) 7.72 (d, *J*=8.22 Hz, 1 H) 7.77 (d, *J*=7.83 Hz, 1 H) 7.98 (s, 1 H) 8.40 (s, 1 H) 8.64 (s, 2 H) 9.57 (s, 1 H) 9.82 (s, 1 H)
HPLC/MS (15 min) retention time 10.66 min.
LRMS: *m*/*z* 536 (M+)

### EXAMPLE 231

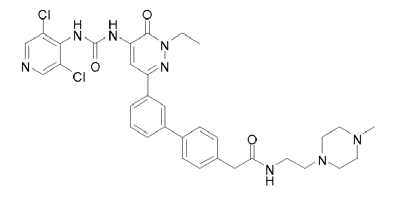

### 2-{3'-[5-({[(3,5-dichloropyridin-4-yl)amino]carbonyl}amino)-1-ethyl-6-oxo-1,6-dihydropyridazin-3-yl]biphenyl-4-yl}-N-[2-(4-methylpiperazin-1-yl)ethyl]acetamide

Reaction of the title compound of Example 40 (200 mg, 0.37 mmol) with [2-(4-methylpiperazin-1-yl)ethyl]amine (106 mg, 0.74 mmol) according to the method described in Example 49 gave 188 mg (76%) of the title compound.
¹H NMR (400 MHz, DMSO-*d*₆) ppm 1.38 (t, *J*=7.04 Hz, 3 H) 2.11 (s, 3 H) 2.33 (t, *J*=6.85 Hz, 8 H) 3.09 - 3.21 (m, 2 H) 4.27 (q, *J*=7.43 Hz, 2 H) 7.38 (d, *J*=8.22 Hz, 2 H) 7.53 - 7.65 (m, 3 H) 7.67 - 7.77 (m, 2 H) 7.93 (t, *J*=5.67 Hz, 1 H) 7.98 (s, 1 H) 8.22 (s, 1 H) 8.40 (s, 1 H) 8.69 (s, 2 H) 9.67 (s, 1 H)
HPLC/MS (15 min) retention time 6.62 min
LRMS: *m*/*z* 663 (M+)

### EXAMPLE 232

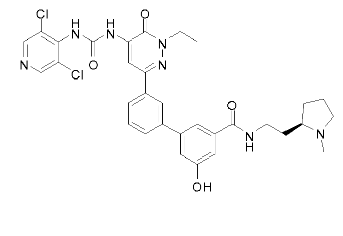

### 3'-[5-({[(3,5-dichloropyridin-4-yl)amino]carbonyl}amino)-1-ethyl-6-oxo-1,6-dihydropyridazin-3-yl]-5-hydroxy-N-{2-[(2R)-1-methylpyrrolidin-2-yl]ethyl}biphenyl-3-carboxamide

Reaction of the title compound of Example 112 (150 mg, 0.28 mmol) with {2-[(2R)-1-methylpyrrolidin-2-yl]ethyllamine (109 mg, 0.56 mmol) according to the method described in Example 49 gave 30 mg (16%) of the title compound.
¹H NMR (400 MHz, DMSO-*d*₆) ppm 1.39 (t, *J*=7.24 Hz, 3 H) 1.50 (dd, *J*=13.30, 7.43 Hz, 2 H) 1.59- 1.73 (m, 2 H) 1.75-2.00 (m, 3 H) 2.02-2.16 (m, 2 H) 2.22 (s, 3 H) 3.25 - 3.41 (m, 2 H) 4.26 (q, *J*=7.17 Hz, 2 H) 7.17 (s, 1 H) 7.24 (s, 1 H) 7.52 (s, 1 H) 7.57 (t, *J*=7.63 Hz, 1 H) 7.68 (d, *J*=8.22 Hz, 1 H) 7.76 (d, *J*=7.83 Hz, 1 H) 7.96 (s, 1 H) 8.26 - 8.33 (m, 1 H) 8.42 (s, 1 H) 8.45 (s, 2 H) 9.55 (s, 1 H)
HPLC/MS (30 min) retention time 11.2 min
LRMS: *m*/*z* 650 (M+)

### EXAMPLE 233

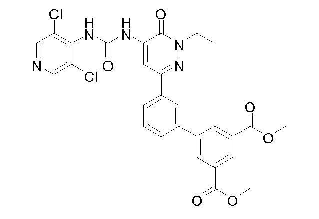

### Dimethyl 3'-[5-({[(3,5-dichloropyridin-4-yl)amino]carbonyl}amino)-1-ethyl-6-oxo-1,6-dihydropyridazin-3-yl]biphenyl-3,5-dicarboxylate

Reaction of the title compound of Preparation 7 (2.40 g, 4.97 mmol) with 2.07 g (6.46 mmol) of the title compound of Preparation 93 according to the method described in Example 1 gave 2.28 mg (Purity: 85%; Yeld:65%) of the title compound after triturating with methanol
¹H NMR (400 MHz, DMSO-*d*₆) ppm 1.39 (t, *J*=7.24 Hz, 3 H) 3.93 (s, 6 H) 4.28 (q, *J*=7.30 Hz, 2 H) 7.66 (t, *J*=7.63 Hz, 1 H) 7.85 (t, *J*=8.80 Hz, 2 H) 8.05 (s, 1 H) 8.40 (s, 1 H) 8.44 (s, 2 H) 8.50 (s, 1 H) 8.67 (s, 1 H) 9.68 (s, 1 H) 9.90 (s, 1 H)
HPLC/MS (30 min) retention time 19.9 min.
LRMS: *m*/*z* 596 (M+)

### EXAMPLE 234

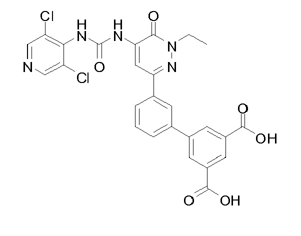

### 3'-[5-({[(3,5-dichloropyridin-4-yl)amino]carbonyl}amino)-1-ethyl-6-oxo-1,6-dihydropyridazin-3-yl]biphenyl-3,5-dicarboxylic acid

Reaction of the title compound of Example 233 (2.28 g, 3.82 mmol) with lithium hydroxide (1.6 g, 38.23 mmol) in a mixture 1:1 water/THF (40ml/40ml) at room temperature under stirring for 3h30min gave 2.16 g (Purity: 94%; Yeld:93%) of the title compound as a solid after acidification with 2N HCI and acetonitril trituration.
¹H NMR (400 MHz, DMSO-*d*₆) ppm 1.39 (t, *J*=7.04 Hz, 3 H) 4.29 (q, *J*=7.56 Hz, 2 H) 7.65 (t, *J*=7.83 Hz, 1 H) 7.83 (t, *J*=7.04 Hz, 2 H) 8.05 (s, 1 H) 8.40 (s, 2 H) 8.49 (s, 1 H) 8.69 (s, 2 H) 9.71 (s, 1 H) 9.92 (s, 1 H) 13.43 (s, 2 H)
HPLC/MS (10 min) retention time 6.55 min.
LRMS: *m*/*z* 568 (M+)

### EXAMPLE 235

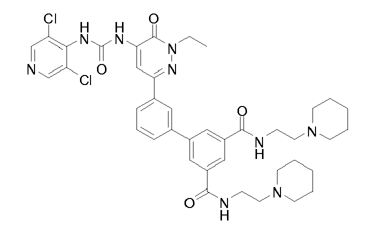

### 3'-[5-({[(3,5-dichloropyridin-4-yl)amino]carbonyl}amino)-1-ethyl-6-oxo-1,6-dihydropyridazin-3-yl]-N,N'-bis(2-piperidin-1-ylethyl)biphenyl-3,5-dicarboxamide

The title compound of Example 234 (150 mg, 0.26 mmol) and (2-piperidin-1-ylethyl)amine (190 µg, 1.32 mmol) were added to a solution of 1-hydroxybenzotriazole hydrate (107 mg, 0.79 mmol) and N-Ethyl-N'-(3-dimethyl-aminopropyl)carbodiimide hydrochloride (152 mg, 0.79 mmol) in dimethylformamide (7 ml). The mixture was stirred at room temperature for 18 h.

The solvent was removed under reduced pressure and the residue dissolved in 4% sodium bicarbonate solution. The aqueous phase was extracted three times with ethyl acetate. The combined organic layers were washed with water and brine, and dried over anhydrous sodium sulphate.

The solvent was removed in vacuum and the residue was purified by the SP1® automated purification system to give 69 mg (33%) of the title compound.
¹H NMR (400 MHz, DMSO-*d*₆) ppm 1.39 (t, *J*=7.24 Hz, 5 H) 1.43 - 1.55 (m, 6 H) 2.47 (d, *J*=7.04 Hz, 2 H) 2.48 - 2.52 (m, 12 H) 3.23 - 3.58 (m, 4 H) 4.29 (q, *J*=7.17 Hz, 2 H) 7.66 (t, *J*=7.83 Hz, 1 H) 7.77 - 7.89 (m, 2 H) 8.08 (t, *J*=1.57 Hz, 1 H) 8.20 - 8.26 (m, 3 H) 8.32 (t, *J*=1.57 Hz, 1 H) 8.40 (s, 1 H) 8.68 (s, 2 H)
HPLC/MS (15 min) retention time 5.08 min
LRMS: *m*/*z* 788 (M+)

### EXAMPLE 236

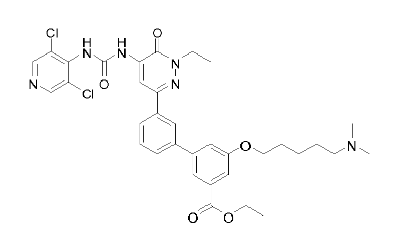

### Ethyl 3'-[5-({[(3,5-dichloropyridin-4-yl)amino]carbonyl}amino)-1-ethyl-6-oxo-1,6-dihydropyridazin-3-yl]-5-{[5-(dimethylamino)pentyl]oxy}biphenyl-3-carboxylate

Reaction of the title compound of Preparation 7 (0.25 g, 0.52 mmol) with (251.7 mg, 0.62 mmol) of the title compound of Preparation 96 according to the method described in Example 1 gave 226.3 mg (64%) of the title compound
HPLC/MS (10 min) retention time 6.18 min
LRMS: *m*/*z* 682 (M+)

### EXAMPLE 237

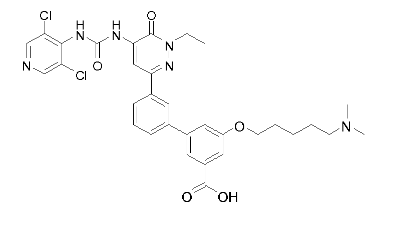

### 3'-[5-({[(3,5-dichloropyridin-4-yl)amino]carbonyl}amino)-1-ethyl-6-oxo-1,6-dihydropyridazin-3-yl]-5-{[5-(dimethylamino)pentyl]oxy}biphenyl-3-carboxylic acid

Reaction of the title compound of Example 236 (226.3 mg, 0.33 mmol) according to the method described in Example 26 gave 100.3 mg (46%) of the title compound without further purification.
HPLC/MS (10 min) retention time 5.53 min
LRMS: *m*/*z* 654 (M+)

### EXAMPLE 238

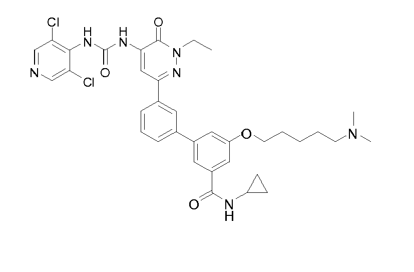

### N-cyclopropyl-3'-[5-({[(3,5-dichloropyridin-4-yl)amino]carbonyl}amino)-1-ethyl-6-oxo-1,6-dihydropyridazin-3-yl]-5-{[5-(dimethylamino)pentyl]oxy}biphenyl-3-carboxamide

Reaction of the title compound of Example 237 (100.3 mg, 0.15 mmol) with cyclopropanamine (21.8 µl, 0.31 mmol) according to the method described in Example 49 gave 9.6 mg (9%) of the title compound after purification by SP1® automated purification system.
HPLC/MS (30 min) retention time 11.78 min
LRMS: *m*/*z* 693 (M+)
1H NMR (400 MHz, DMSO-*d*₆) d ppm 0.55 - 0.61 (m, 2 H) 0.67 - 0.73 (m, 2 H) 1.39 (t, *J*=7.05 Hz, 3 H) 1.70 - 1.81 (m, 2 H) 1.71 - 1.80 (m, 2 H) 1.72 - 1.80 (m, 2 H) 2.13 (s, 6 H) 2.23 (t, *J*=6.84 Hz, 2 H) 2.82 - 2.89 (m, 1 H) 4.09 (t, *J*=6.22 Hz, 2 H) 4.28 (q, *J*=7.19 Hz, 2 H) 7.34 (s, 1 H) 7.38 (s, 1 H) 7.60 (t, *J*=7.67 Hz, 1 H) 7.68 (s, 1 H) 7.79 (d, *J*=6.63 Hz, 2 H) 8.01 (s, 1 H) 8.26 (s, 1 H) 8.40 (s, 1 H) 8.52 (d, *J*=4.15 Hz, 1 H) 8.67 (s, 2 H) 9.61 (s, 1 H)

### EXAMPLE 239

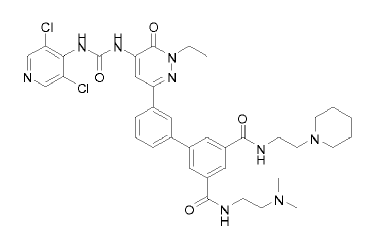

### 3'-[5-({[(3,5-dichloropyridin-4-yl)amino]carbonyl}amino)-1-ethyl-6-oxo-1,6-dihydropyridazin-3-yl]-N-[2-(dimethylamino)ethyl]-N'-(2-piperidin-1-ylethyl)biphenyl-3,5-dicarboxamide

Reaction of the title compound of Example 201 (150 mg, 0.22 mmol) with N,N-dimethylethane-1,2-diamine (480 µl, 0.44 mmol) according to the method described in

Example 49 gave 34 mg (20%) of the title compound after purification by SP1® automated purification system.
HPLC/MS (30 min) retention time 8.05 min
LRMS: *m*/*z* 749 (M+)
1H NMR (400 MHz, DMSO-*d*₆) d ppm 1.39 (t, *J*=7.05 Hz, 3 H) 1.46 - 1.53 (m, 4 H) 2.17 - 2.20 (m, 6 H) 2.35 - 2.48 (m, 7 H) 3.35 - 3.45 (m, 8 H) 4.29 (q, *J*=7.05 Hz, 2 H) 7.66 (t, *J*=7.67 Hz, 1 H) 7.84 (dd, *J*=15.76, 7.88 Hz, 2 H) 8.07 - 8.09 (m, 1 H) 8.23 - 8.26 (m, 2 H) 8.29 - 8.33 (m, 1 H) 8.40 (s, 1 H) 8.63 - 8.71 (m, 3 H) 9.68 (s, 1 H)

### EXAMPLE 240

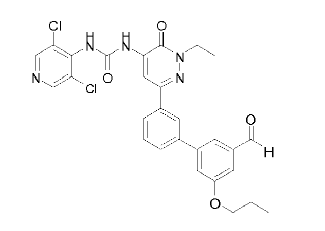

### N-(3,5-dichloropyridin-4-yl)-N'-[2-ethyl-6-(3'-formyl-5'-propoxybiphenyl-3-yl)-3-oxo-2,3-dihydropyridazin-4-yl]urea

Reaction of the title compound of Preparation 7 (0.5 g, 1.03 mmol) with (301.4 mg, 1.45 mmol) of (3-formyl-5-propoxyphenyl)boronic acid according to the method described in Example 1 gave 472.2 mg (73%) of the title compound.

### EXAMPLE 241

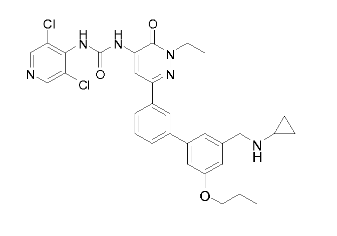

### N-(6-{3'-[(cyclopropylamino)methyl]-5'-propoxybiphenyl-3-yl}-2-ethyl-3-oxo-2,3-dihydropyridazin-4-yl)-N'-(3,5-dichloropyridin-4-yl)urea

150 mg (0.24 mmols) the title compound of Example 240 was dissolved in 10 mL of methanol and 10 ml of tetrahydrofuran. 124 I (2.17 mmols) of acetic acid and 144.6 I(0.29 mmols) of cyclopropanamine were added and the mixture stirred at room temperature for 2h. Then NaBH3CN was added and the reaction mixture stirred at room temperature overnight. The solvent was concentrated and the residue partitioned between water and ethyl ether. The organic phase was washed with NaHCO3 4%, water and brine, dried over magnesium sulphate. Evaporation gave 41.5 mg (54%) of the title compound after purification by SP1® automated purification system.
HPLC/MS (30 min) retention time 13.42 min
LRMS: *m*/*z* 607 (M+)
1H NMR (400 MHz, DMSO-*d*₆) d ppm 0.70 - 0.76 (m, 2 H) 0.83 - 0.88 (m, 2 H) 1.00 (t, *J*=7.46 Hz, 3 H) 1.25 (t, *J*=7.26 Hz, 3 H) 1.70-1.84 (m, 3 H) 2.45 (s, 1 H) 3.88-3.99 (m, 3 H) 4.07-4.19 (m, 4 H) 6.94 (s, 1 H) 7.00 (d, 2 H) 7.41 - 7.52 (m, 2 H) 7.61 (d, *J*=7.46 Hz, 1 H) 7.82 (s, 1 H) 8.44 (s, 1 H) 8.57 (s, 2 H) 8.65 (s, 1 H) 9.32 (s, 1 H)

### EXAMPLE 242

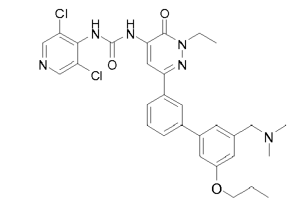

### N-(3,5-dichloropyridin-4-yl)-N'-(6-{3'-[(dimethylamino)methyl]-5'-propoxybiphenyl-3-yl}-2-ethyl-3-oxo-2,3-dihydropyridazin-4-yl)urea

Reaction of the title compound of Example 240 (150 mg, 0.24 mmol) with methylmethanamine (144.59 µl, 0.29 mmol) according to the method described in Example 241 gave 39.8 mg (55%) of the title compound after purification by SP1® automated purification system.
HPLC/MS (30 min) retention time 13.12 min
LRMS: *m*/*z* 596 (M+)
¹H NMR (400 MHz, DMSO-*d*₆) ppm 0.99 (t, *J*=7.43 Hz, 3 H) 1.38 (t, *J*=7.04 Hz, 3 H) 1.69 - 1.80 (m, 2 H) 2.15 - 2.20 (m, 6 H) 3.41 - 3.46 (m, 2 H) 3.99 (t, *J*=6.65 Hz, 2 H) 4.28 (q, *J*=7.30 Hz, 2 H) 6.89 (s, 1 H) 7.08 (s, 1 H) 7.15 (s, 1 H) 7.57 (t, *J*=7.63 Hz, 1 H) 7.74 (dd, *J*=13.30, 7.83 Hz, 2 H) 7.96 (s, 1 H) 8.39 (s, 1 H) 8.68 (s, 2 H) 9.67 (s, 1 H) 9.90 (s, 1 H)

### EXAMPLE 243

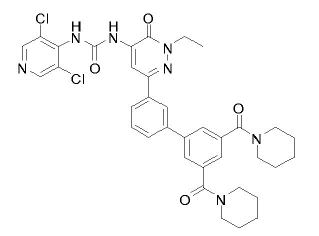

### N-{6-[3',5'-bis(piperidin-1-ylcarbonyl)biphenyl-3-yl]-2-ethyl-3-oxo-2,3-dihydropyridazin-4-yl}-N'-(3,5-dichloropyridin-4-yl)urea

Reaction of the title compound of Example 234 (150 mg, 0.26 mmol) with piperidine (130 µg, 1.32 mmol) according to the method described in Example 235 gave 54.5 mg (29%) of the title compound.
¹H NMR (400 MHz, DMSO-*d*₆) ppm 1.38 (t, *J*=7.04 Hz, 3 H) 1.45 - 1.65 (m, 12 H) 3.32 (s, 8 H) 4.28 (q, *J*=7.30 Hz, 2 H) 7.34 (s, 1 H) 7.62 (t, *J*=7.83 Hz, 1 H) 7.73 (s, 2 H) 7.82 (t, *J*=9.19 Hz, 2 H) 8.03 (s, 1 H) 8.43 (s, 1 H) 8.68 (s, 2 H) 9.66 (s, 1 H) 9.86 (s, 1 H)
HPLC/MS (15 min) retention time 9.25 min
LRMS: *m*/*z* 702 (M+)

### EXAMPLE 244

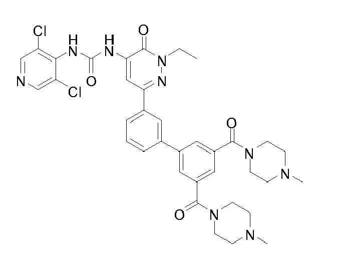

### N-(6-{3',5'-bis[(4-methylpiperazin-1-yl)carbonyl]biphenyl-3-yl}-2-ethyl-3-oxo-2,3-dihydropyridazin-4-yl)-N'-(3,5-dichloropyridin-4-yl)urea

Reaction of the title compound of Example 234 (150 mg, 0.26 mmol) with 1-methylpiperazine (146 µg, 1.32 mmol) according to the method described in Example 235 gave 96.4 mg (50%) of the title compound.
¹H NMR (400 MHz, DMSO-*d*₆) ppm 1.39 (t, *J*=7.24 Hz, 3 H) 2.17 (s, 6 H) 2.22 - 2.44 (m, 8 H) 3.37 (s, 4 H) 3.63 (s, 4 H) 4.28 (q, *J*=7.04 Hz, 2 H) 7.36 (s, 1 H) 7.62 (t, *J*=7.83 Hz, 1 H) 7.75 (d, *J*=1.56 Hz, 2 H) 7.81 (t, *J*=8.22 Hz, 2 H) 8.03 (s, 1 H) 8.24 (s, 1 H) 8.41 (s, 1 H) 8.68 (s, 2 H)
HPLC/MS (15 min) retention time 4.40 min
LRMS: *m*/*z* 732 (M+)

### EXAMPLE 245

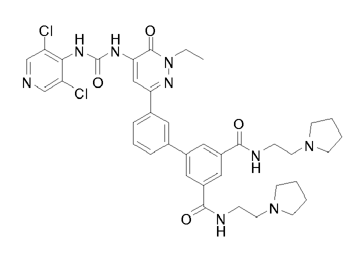

### 3'-[5-({[(3,5-dichloropyridin-4-yl)amino]carbonyl}amino)-1-ethyl-6-oxo-1,6-dihydropyridazin-3-yl]-N,N'-bis(2-pyrrolidin-1-ylethyl)biphenyl-3,5-dicarboxamide

Reaction of the title compound of Example 234 (150 mg, 0.26 mmol) with (2-pyrrolidin-1-ylethyl)amine (166 µg, 1.32 mmol) according to the method described in Example 235 gave 75.2 mg (38%) of the title compound.
¹H NMR (400 MHz, DMSO-*d*₆) ppm 1.39 (t, *J*=7.04 Hz, 3 H) 1.58 - 1.78 (m, 8 H) 2.52 - 2.58 (m, 8 H) 2.66 (t, *J*=6.85 Hz, 4 H) 3.44 (q, *J*=6.65 Hz, 4 H) 4.29 (q, *J*=7.04 Hz, 2 H) 7.66 (t, *J*=7.63 Hz, 1 H) 7.84 (dd, *J*=15.06, 8.02 Hz, 2 H) 8.08 (s, 1 H) 8.20 (s, 2 H) 8.27 (d, *J*=1.57 Hz, 2 H) 8.34 (s, 1 H) 8.40 (s, 1 H) 8.68 (s, 2 H) 8.75 (t, *J*=5.67 Hz, 2 H) 9.70 (s, 1 H)
HPLC/MS (15 min) retention time 4.93 min
LRMS: *m*/*z* 760 (M+)

### EXAMPLE 246

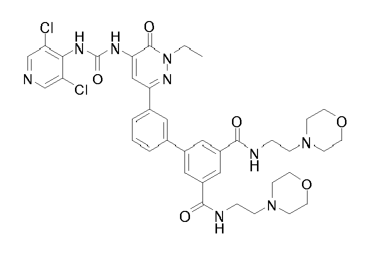

### 3'-[5-({[(3,5-dichloropyridin-4-yl)amino]carbonyl}amino)-1-ethyl-6-oxo-1,6-dihydropyridazin-3-yl]-N,N'-bis(2-morpholin-4-ylethyl)biphenyl-3,5-dicarboxamide

Reaction of the title compound of Example 234 (150 mg, 0.26 mmol) with (2-morpholin-4-ylethyl)amine (172 µg, 1.32 mmol) according to the method described in Example 235 gave 91.5 mg (44%) of the title compound.
¹H NMR (400 MHz, DMSO-*d*₆) ppm 1.39 (t, *J*=7.24 Hz, 3 H) 2.31 - 2.55 (m, 8 H) 3.35 - 3.47 (m, 4 H) 3.53 - 3.68 (m, 8 H) 4.29 (q, *J*=7.04 Hz, 2 H) 7.66 (t, *J*=7.83 Hz, 1 H) 7.84 (dd, *J*=15.26, 8.22 Hz, 2 H) 8.08 (s, 1 H) 8.23 (s, 1 H) 8.25 (d, *J*=1.96 Hz, 2 H) 8.32 (t, *J*=1.57 Hz, 1 H) 8.40 (s, 1 H) 8.63 - 8.73 (m, 3 H)
HPLC/MS (15 min) retention time 4.80 min
LRMS: *m*/*z* 792 (M+)

### EXAMPLE 247

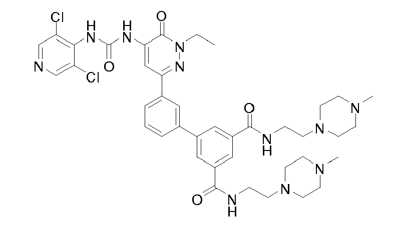

### 3'-[5-({[(3,5-dichloropyridin-4-yl)amino]carbonyl}amino)-1-ethyl-6-oxo-1,6-dihydropyridazin-3-yl]-N,N'-bis[2-(4-methylpiperazin-1-yl)ethyl]biphenyl-3,5-dicarboxamide

Reaction of the title compound of Example 234 (150 mg, 0.26 mmol) with [2-(4-methylpiperazin-1-yl)ethyl]amine (151 mg, 1.06 mmol) according to the method described in Example 235 gave 89.7 mg (42%) of the title compound.
¹H NMR (400 MHz, DMSO-*d*₆) ppm 1.40 (s, 3 H) 2.13 (s, 6 H) 2.25 - 2.36 (m, 8 H) 2.41 - 2.46 (m, 8 H) 2.50 - 2.54 (m, 4 H) 3.41 (q, *J*=6.52 Hz, 4 H) 4.28 (q, *J*=7.30 Hz, 2 H) 7.63 (t, *J*=7.83 Hz, 1 H) 7.76 - 7.88 (m, 2 H) 8.05 (s, 1 H) 8.20 (s, 4 H) 8.27 (s, 1 H) 8.36 - 8.44 (m, 3 H) 8.60 (s, 2 H)
HPLC/MS (15 min) retention time 4.83 min
LRMS: *m*/*z* 818 (M+)

### EXAMPLE 248

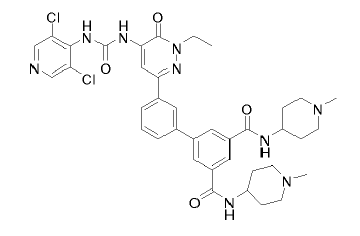

### 3'-[5-({[(3,5-dichloropyridin-4-yl)amino]carbonyl}amino)-1-ethyl-6-oxo-1,6-dihydropyridazin-3-yl]-N,N'-bis(1-methylpiperidin-4-yl)biphenyl-3,5-dicarboxamide

Reaction of the title compound of Example 234 (150 mg, 0.26 mmol) with 1-methylpiperidin-4-amine (120 mg, 1.06 mmol) according to the method described in Example 235 gave 91.3 mg (45%) of the title compound.
¹H NMR (400 MHz, DMSO-*d*₆) ppm 1.40 (t, *J*=7.24 Hz, 3 H) 1.56 - 1.71 (m, 4 H) 1.75 - 1.87 (m, 4 H) 1.95 - 2.07 (m, 4 H) 2.19 (s, 6 H) 2.70 - 2.85 (m, 4 H) 3.67 - 3.88 (m, 2 H) 4.28 (q, *J*=7.04 Hz, 2 H) 7.63 (t, *J*=7.83 Hz, 1 H) 7.80 (t, *J*=7.43 Hz, 2 H) 8.06 (s, 1 H) 8.19 (s, 4 H) 8.23 - 8.29 (m, 3 H) 8.38 (s, 1 H) 8.61 (s, 2 H)
HPLC/MS (15 min) retention time 4.83 min
LRMS: *m*/*z* 762 (M+)

### EXAMPLE 249

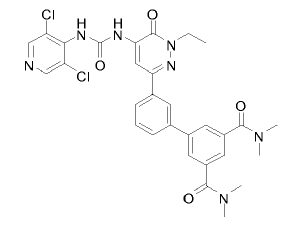

### 3'-[5-({[(3,5-dichloropyridin-4-yl)amino]carbonyl}amino)-1-ethyl-6-oxo-1,6-dihydropyridazin-3-yl]-N,N,N',N'-tetramethylbiphenyl-3,5-dicarboxamide

Reaction of the title compound of Example 234 (150 mg, 0.26 mmol) with dimethylamine (660 µg, 1.32 mmol) according to the method described in Example 235 gave 75.4 mg (46%) of the title compound.
¹H NMR (400 MHz, DMSO-*d*₆) ppm 1.38 (t, *J*=7.26 Hz, 3 H) 2.96 (s, 6 H) 3.01 (s, 6 H) 4.28 (q, *J*=7.05 Hz, 2 H) 7.42 (s, 1 H) 7.61 (t, *J*=7.88 Hz, 1 H) 7.77 (s, 2 H) 7.78 - 7.84 (m, 2 H) 8.03 (s, 1 H) 8.41 (s, 1 H) 8.66 (s, 2 H) 9.61 (s, 1 H) 9.91 (s, 1 H)
HPLC/MS (15 min) retention time 7.77 min
LRMS: *m*/*z* 622 (M+)

### EXAMPLE 250

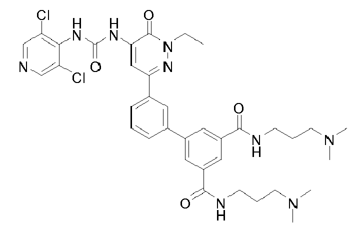

### 3'-[5-({[(3,5-dichloropyridin-4-yl)amino]carbonyl}amino)-1-ethyl-6-oxo-1,6-dihydropyridazin-3-yl]-N,N'-bis[3-(dimethylamino)propyl]biphenyl-3,5-dicarboxamide

Reaction of the title compound of Example 234 (150 mg, 0.26 mmol) with N,N-dimethylpropane-1,3-diamine (166 µg, 1.32 mmol) according to the method described in Example 235 gave 58.7 mg (29%) of the title compound.
¹H NMR (400 MHz, DMSO-*d*₆) ppm 1.39 (t, *J*=7.26 Hz, 3 H) 1.58 - 1.78 (m, 4 H) 2.14 (s, 12 H) 2.28 (t, *J*=7.05 Hz, 4 H) 4.29 (q, *J*=7.46 Hz, 2 H) 7.66 (t, *J*=7.88 Hz, 1 H) 7.84 (dd, *J*=16.79, 8.09 Hz, 2 H) 8.08 (s, 1 H) 8.24 (d, *J*=1.66 Hz, 2 H) 8.32 (s, 1 H) 8.40 (s, 1 H) 8.66 (s, 2 H) 8.77 (t, *J*=5.60 Hz, 2 H) 9.64 (s, 1 H) 9.88 (s, 1 H)
HPLC/MS (15 min) retention time 4.87 min
LRMS: *m*/*z* 734 (M+)

### EXAMPLE 251

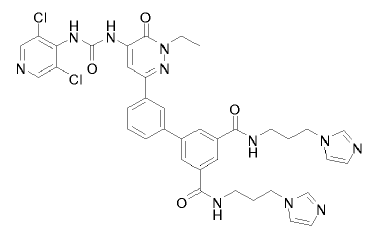

### 3'-[5-({[(3,5-dichloropyridin-4-yl)amino]carbonyl}amino)-1-ethyl-6-oxo-1,6-dihydropyridazin-3-yl]-N,N'-bis[3-(1H-imidazol-1-yl)propyl]biphenyl-3,5-dicarboxamide

Reaction of the title compound of Example 234 (150 mg, 0.26 mmol) with [3-(1H-imidazol-1-yl)propyl]amine (159 µg, 1.32 mmol) according to the method described in Example 235 gave 97.4 mg (47%) of the title compound.
¹H NMR (400 MHz, DMSO-*d*₆) ppm 1.38 (t, *J*=7.24 Hz, 3 H) 1.81 - 2.07 (m, 4 H) 3.28 (q, *J*=6.39 Hz, 4 H) 4.04 (t, *J*=6.85 Hz, 4 H) 4.28 (q, *J*=7.04 Hz, 2 H) 6.89 (s, 2 H) 7.21 (s, 2 H) 7.63 - 7.73 (m, 3 H) 7.82 (d, *J*=7.83 Hz, 1 H) 7.87 (d, *J*=7.43 Hz, 1 H) 8.10 (s, 1 H) 8.26 (d, *J*=1.96 Hz, 4 H) 8.34 (s, 1 H) 8.40 (s, 1 H) 8.67 (s, 2 H) 8.78 (t, *J*=5.48 Hz, 2 H)
HPLC/MS (15 min) retention time 5.13 min
LRMS: *m*/*z* 782 (M+)

### EXAMPLE 252

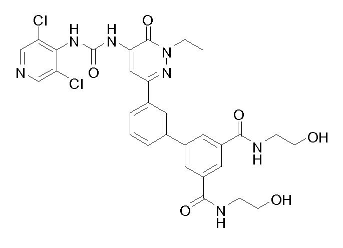

### 3'-[5-({[(3,5-dichloropyridin-4-yl)amino]carbonyl}amino)-1-ethyl-6-oxo-1,6-dihydropyridazin-3-yl]-N,N'-bis(2-hydroxyethyl)biphenyl-3,5-dicarboxamide

Reaction of the title compound of Example 234 (150 mg, 0.26 mmol) with 2-aminoethanol (79 µg, 1.32 mmol) according to the method described in Example 235 gave 19.5 mg (11%) of the title compound.
¹H NMR (400 MHz, DMSO-*d*₆) ppm 1.39 (t, *J*=7.24 Hz, 3 H) 3.37 (q, *J*=6.13 Hz, 4 H) 3.54 (q, *J*=6.00 Hz, 4 H) 4.29 (q, *J*=6.65 Hz, 2 H) 4.74 (t, *J*=5.67 Hz, 2 H) 7.66 (t, J=7.83 Hz, 1 H) 7.81 (d, *J*=7.43 Hz, 1 H) 7.87 (d, *J*=8.22 Hz, 1 H) 8.09 (t, *J*=1.76 Hz, 1 H) 8.28 (d, *J*=1.56 Hz, 2 H) 8.36 (t, *J*=1.57 Hz, 1 H) 8.39 (s, 1 H) 8.62 - 8.73 (m, 4 H) 9.68 (s, 1 H)
HPLC/MS (15 min) retention time 7.20 min
LRMS: *m*/*z* 654 (M+)

### EXAMPLE 253

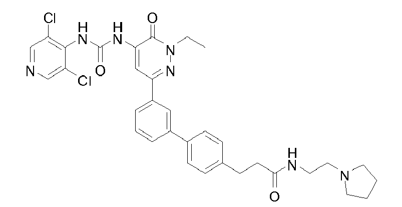

### 3-{3'-[5-({[(3,5-dichloropyridin-4-yl)amino]carbonyl}amino)-1-ethyl-6-oxo-1,6-dihydropyridazin-3-yl]biphenyl-4-yl}-N-(2-pyrrolidin-1-ylethyl)propanamide

The title compound of Example 148 (152 mg, 0.27 mmol), 1-hydroxybenzotriazole hydrate (65 mg, 0.48 mmol) and N-ethyl-N'-(3-dimethyl-aminopropyl)carbodiimide hydrochloride (92 mg, 0.48 mmol) were suspended in 5 ml of DMF. The mixture was stirred at room temperature for 30 min. 2-(Pyrrolidin-yl)ethanamine (80 µl, 0.64 mmol) was added and the mixture was stirred at 50°C for 4 h. The solvent was removed under reduced pressure. Purification by SP1® automated purification system gave 57 mg (0.088 mmol, 32%) of the title compound.
1H NMR (200 MHz, CHLOROFORM-*d*) ppm 9.55 (1 H, s), 8.62 (2 H, br. s.), 8.58 (2 H, s), 8.37 - 8.52 (1 H, m), 7.97 (1 H, s), 7.70 (1 H, d, *J*=7.4 Hz), 7.56 (1 H, d, *J*=7.8 Hz), 7.48 (2 H, d, *J*=7.8 Hz), 7.41 (1 H, t, *J*=7.8 Hz), 7.26 (2 H, d, *J*=8.0 Hz), 4.22 (2 H, q, *J*=7.0 Hz), 3.55 - 3.70 (2 H, m), 3.15 (6 H, br. s.), 3.00 (2 H, t, *J*=7.4 Hz), 2.60 (2 H, t, *J*=7.6 Hz), 2.00 (4 H, br. s.), 1.30 (3 H, t, *J*=7.0 Hz)
HPLC/MS (30 min) retention time 13.10 min
LRMS: *m*/*z* 648 (M+)

### EXAMPLE 254

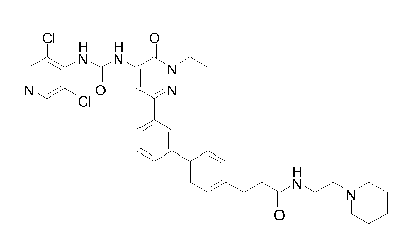

### 3-{3'-[5-({[(3,5-dichloropyridin-4-yl)amino]carbonyl}amino)-1-ethyl-6-oxo-1,6-dihydropyridazin-3-yl]biphenyl-4-yl}-N-(2-piperidin-1-ylethyl)propanamide

The title compound of Example 148 (148 mg, 0.27 mmol) was reacted with (2-piperidin-1-ylethyl)amine (0.09 ml, 0.67 mmol) according to the method of Example 253. Purification by SP1® automated purification system gave 67 mg (0.10 mmol, 37%) of the title compound.
1H NMR (200 MHz, CHLOROFORM-*d*) ppm 9.48 (1 H, s), 8.63 (2 H, s), 8.58 (2 H, s), 8.16 (1 H, br. s.), 7.98 (1 H, s), 7.70 (1 H, d, *J*=7.4 Hz), 7.57 (1 H, d, *J*=7.8 Hz), 7.49 (2 H, d, *J*=7.8 Hz), 7.42 (1 H, t, *J*=7.8 Hz), 7.28 (2 H, d, *J*=7.8 Hz), 4.23 (2 H, q, *J*=6.8 Hz), 3.60 (2 H, q, *J*=4.8 Hz), 2.73 - 3.09 (8 H, m), 2.58 (2 H, t, *J*=7.6 Hz), 1.71 - 1.88 (4 H, m), 1.54 (2 H, br. s.), 1.30 (3 H, t, *J*=7.2 Hz).
HPLC/MS (30 min) retention time 13.42 min
LRMS: *m*/*z* 662 (M+)

### EXAMPLE 255

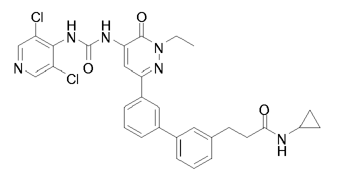

### N-cyclopropyl-3-{3'-[5-({[(3,5-dichloropyridin-4-yl)amino]carbonyl}amino)-1-ethyl-6-oxo-1,6-dihydropyridazin-3-yl]biphenyl-3-yl}propanamide

The title compound of Example 187 (149 mg, 0.27 mmol) was reacted with N-cyclopropanamine (48 mg, 0.84 mmol) according to the method of Example 253 Purification by SP1® automated purification system gave 80 mg (0.13 mmol, 49%) of the title compound.
¹H NMR (200 MHz, CHLOROFORM-*d*) ppm 9.50 (1 H, s), 8.97 (1 H, s), 8.70 (1 H, s), 8.61 (2 H, s), 8.00 (1 H, s), 7.81 (1 H, d, *J*=7.8 Hz), 7.61 (1 H, d, *J*=8.0 Hz), 7.40 - 7.55 (3 H, m), 7.36 (1 H, t, *J*=7.8 Hz), 7.16 - 7.24 (1 H, m), 5.47 (1 H, br. s.)*, 4.25 (2 H, q, *J*=6.9 Hz), 3.03 (2 H, t, *J*=7.8 Hz)**, 2.58 - 2.73 (1 H, ttd, *J*=6.9, 3.5, 3.7 Hz), 2.46 (2 H, t, *J*=7.6 Hz), 1.29 (3 H, t, *J*=7.0 Hz), 0.64 - 0.80 (2 H, m), 0.33 - 0.44 (2 H, m)***
rotamer peaks: ppm 6.21 (1 H, br. s.)*, 2.83 (0 H, t, *J*=7.4 Hz)**, 0.46 - 0.58 (1 H, m)***
HPLC/MS (30 min) retention time 18.00 min
LRMS: *m*/*z* 591 (M+)

### EXAMPLE 256

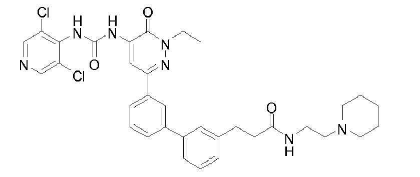

### 3-{3'-[5-({[(3,5-dichloropyridin-4-yl)amino]carbonyl}amino)-1-ethyl-6-oxo-1,6-dihydropyridazin-3-yl]biphenyl-3-yl}-N-(2-piperidin-1-ylethyl)propanamide

The title compound of Example 187 (149 mg, 0.27 mmol) was reacted with (2-piperidin-1-ylethyl)amine (0.09 ml, 0.67 mmol) according to the method of Example 253 Purification by SP1® automated purification system gave 103 mg (0.15 mmol, 57%) of the title compound.
¹H NMR (200 MHz, CHLOROFORM-*d*) ppm 9.55 (1 H, s), 8.68 (1 H, s), 8.62 (1 H, br. s.), 8.60 (2 H, s), 8.00 (1 H, s), 7.78 (1 H, d, *J*=7.8 Hz), 7.61 (1 H, d, *J*=7.4 Hz), 7.14 - 7.55 (5 H, m), 4.23 (2 H, q, *J*=7.3 Hz), 3.44 (2 H, q, *J*=5.2 Hz), 3.04 (2 H, t, *J*=7.6 Hz), 2.45 - 2.70 (8 H, m), 1.56 - 1.73 (4 H, m), 1.39 - 1.55 (2 H, m), 1.28 (3 H, t, *J*=7.0 Hz).
HPLC/MS (30 min) retention time 13.64 min
LRMS: *m*/*z* 662 (M+)

### EXAMPLE 257

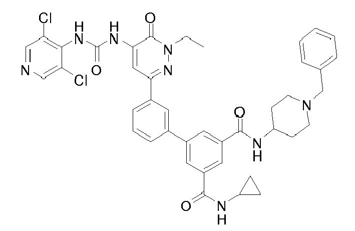

### N-(1-benzylpiperidin-4-yl)-N'-cyclopropyl-3'-[5-({[(3,5-dichloropyridin-4-yl)amino]carbonyl}amino)-1-ethyl-6-oxo-1,6-dihydropyridazin-3-yl]biphenyl-3,5-dicarboxamide

The title compound of Example 147 (150 mg, 0.25 mmol) was reacted with 1-benzylpiperidin-4-ylamine (95 mg, 0.50 mmol) according to the method of Example 253 Purification by SP1® automated purification system gave 95 mg (0.12 mmol, 49%) of the title compound.
1H NMR (200 MHz, DMSO-*d*₆) ppm (partial) 9.72 (1 H, br. s.), 8.63 - 8.76 (3 H, m), 8.53 (1 H, d, *J*=7.4 Hz), 8.41 (1 H, s), 8.28 (1 H, s), 8.22 (3 H, br. s.), 8.08 (1 H, s), 7.84 (2 H, t, *J*=7.8 Hz), 7.65 (1 H, t, *J*=7.6 Hz), 7.31 (5 H, s), 4.29 (2 H, q, J=6.8 Hz), 2.83 (3H, m), 2.04 (2 H, t, *J*=10.9 Hz), 1.72 - 1.90 (2 H, m), 1.62 (2 H, t, *J*=11.0 Hz), 1.39 (3 H, t, *J*=7.0 Hz), 0.65 - 0.79 (2 H, m), 0.61 (2 H, br. s.)
HPLC/MS (30 min) retention time 12.22 min
LRMS: *m*/*z* 779 (M+1)

### EXAMPLE 258

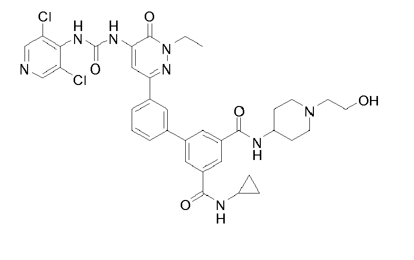

### N-cyclopropyl-3'-[5-({[(3,5-dichloropyridin-4-yl)amino]carbonyl}amino)-1-ethyl-6-oxo-1,6-dihydropyridazin-3-yl]-N'-[1-(2-hydroxyethyl)piperidin-4-yl]biphenyl-3,5-dicarboxamide

The title compound of Example 147 (145 mg, 0.24 mmol), 1-hydroxybenzotriazole hydrate (64 mg, 0.47 mmol) and N-ethyl-N'-(3-dimethyl-aminopropyl)carbodiimide hydrochloride (88 mg, 0.46 mmol) were suspended in 5ml of DMF. The mixture was stirred at room temperature for 30 min. Triethylamine (0.1 ml, 0.72 mmol) and the product from preparation 98 (110 mg, 0.51 mmol) were added and the mixture was stirred at 50 °C for 4 h. The solvent was removed under reduced pressure. Purification by SP1® automated purification system gave 95 mg (0.13 mmol, 54%).
1H NMR (200 MHz, DMSO-*d*₆) ppm (partial) 9.70 (1 H, br. s.), 8.67 (3 H, s), 8.52 (1 H, d, *J*=7.4 Hz), 8.39 (1 H, s), 8.27 (1 H, s), 8.20 (3 H, br. s.), 8.06 (1 H, s), 7.82 (2 H, t, *J*=7.6 Hz), 7.65 (1 H, d, *J*=7.8 Hz), 4.27 (2 H, q, *J*=6.9 Hz), 3.50 (2 H, t, *J*=6.2 Hz), 2.92 (2 H, d, *J*=12.1 Hz), 2.12 (2 H, t, *J*=10.7 Hz), 1.72 - 1.87 (2 H, m), 1.61 (2 H, t, *J*=10.3 Hz), 1.37 (3 H, t, *J*=7.0 Hz), 0.69 (2 H, d, *J*=6.6 Hz), 0.59 (2 H, br. s.)
HPLC retention time (30 min) 10.93 min.
LRMS 733 (M+1)

### EXAMPLE 259

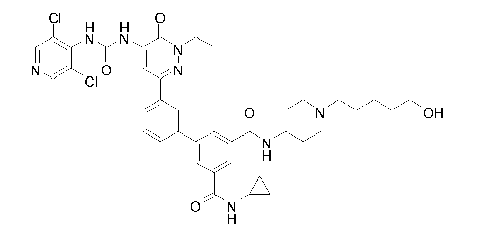

### N-cyclopropyl-3'-[5-({[(3,5-dichloropyridin-4-yl)amino]carbonyl}amino)-1-ethyl-6-oxo-1,6-dihydropyridazin-3-yl]-N'-[1-(5-hydroxypentyl)piperidin-4-yl]biphenyl-3,5-dicarboxamide

The title compound of Example 147 (120 mg, 0.20 mmol) was reacted with the product of preparation 100 (130 mg, 0.50 mmol) according to the method of Example 258 Purification by SP1® automated purification system gave 86 mg (0.11 mmol, 56%) of the title compound.
1H NMR (200 MHz, DMSO-*d*₆) ppm (partial) 9.66 (1 H, br. s.), 8.63 - 8.83 (3 H, m), 8.53 (1 H, d, *J*=7.4 Hz), 8.41 (1 H, s), 8.16 - 8.37 (4 H, m), 8.08 (1 H, br. s.), 7.84 (2 H, t, *J*=7.0 Hz), 7.65 (1 H, t, *J*=7.8 Hz), 4.29 (2 H, q, *J*=6.8 Hz), 3.38 (2 H, t, *J*=6.0 Hz), 2.86 (3 H, br. s.), 2.19 - 2.36 (2 H, m), 1.98 (2 H, t, *J*=11.0 Hz), 1.78 (2 H, br. s.), 1.60 (1 H, br. s.), 1.19 - 1.52 (7 H, m), 0.71 (2 H, d, *J*=6.2 Hz), 0.61 (2 H, br. s.)
HPLC retention time (30 min) 11.33 min.
LRMS 775 (M+1)

### EXAMPLE 260

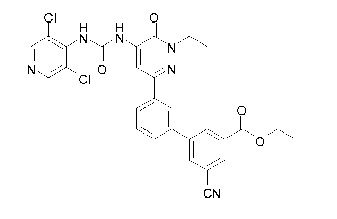

### Ethyl 5-cyano-3'-[5-({[(3,5-dichloropyridin-4-yl)amino]carbonyl}amino)-1-ethyl-6-oxo-1,6-dihydropyridazin-3-yl]biphenyl-3-carboxylate

Reaction of the title compound of Preparation 7 (850 mg, 1.76 mmol) with 635 mg of the title compound of Preparation 103 according to the method described in Example 1 gave 1.00 g (100%) of the title compound.
¹H NMR (400 MHz, DMSO-*d*₆) ppm 1.20 - 1.55 (m, 6 H) 4.29 (q, *J*=7.17 Hz, 2 H) 4.38 (q, *J*=6.91 Hz, 2 H) 7.53 (s, 2 H) 7.63 - 7.70 (m, 1 H) 7.88 (d, *J*=7.43 Hz, 2 H) 8.10 (s, 1 H) 8.34 (s, 1 H) 8.42 (s, 1 H) 8.46 (s, 1 H) 8.54 (s, 1 H) 8.69 (s, 2 H) 9.64 - 9.76 (m, 1 H) 9.89 (s, 1 H)
HPLC/MS (15 min) retention time 10.17 min.
LRMS: *m*/*z* 577 (M-)

### EXAMPLE 261

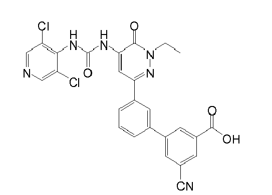

### 5-cyano-3'-[5-({[(3,5-dichloropyridin-4-yl)amino]carbonyl}amino)-1-ethyl-6-oxo-1,6-dihydropyridazin-3-yl]biphenyl-3-carboxylic acid

Reaction of the title compound of Example 260 (1.00 g, 1.77 mmol) according to the method described in Example 26 gave 0.87 g (Purity: 91; Yield: 81%) of the title compound after triturating with acetonitrile.
HPLC/MS (30 min) retention time 6.83 min
LRMS: *m*/*z* 549 (M+)

### EXAMPLE 262

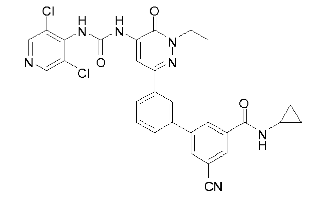

### 5-cyano-N-cyclopropyl-3'-[5-({[(3,5-dichloropyridin-4-yl)amino]carbonyl}amino)-1-ethyl-6-oxo-1,6-dihydropyridazin-3-yl]biphenyl-3-carboxamide

Reaction of the title compound of Example 261 (200 mg, 0.36 mmol) with N cyclopropanamine (50.6 µg, 0.73 mmol) according to the method described in Example 49 gave 73.9 mg (34%) of the title compound.
¹H NMR (400 MHz, DMSO-*d*₆) ppm 0.52 - 0.66 (m, 2 H) 0.67 - 0.83 (m, 2 H) 1.39 (t, *J*=7.24 Hz, 3 H) 2.82 - 3.05 (m, 1 H) 4.29 (q, *J*=6.91 Hz, 2 H) 7.65 (t, *J*=7.83 Hz, 1 H) 7.86 (t, *J*=7.43 Hz, 2 H) 8.11 (s, 1 H) 8.23 (s, 1 H) 8.33 - 8.43 (m, 3 H) 8.68 (s, 2 H) 8.75 (d, *J*=4.30 Hz, 1 H) 9.66 (s, 1 H) 9.91 (s, 1 H)
HPLC/MS (30 min) retention time 9.15 min
LRMS: *m*/*z* 588 (M+)

### EXAMPLE 263

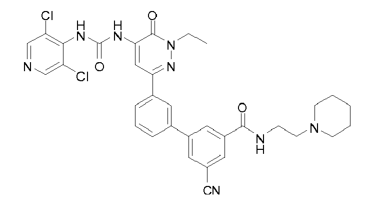

### 5-cyano-3'-[5-({[(3,5-dichloropyridin-4-yl)amino]carbonyl}amino)-1-ethyl-6-oxo-1,6-dihydropyridazin-3-yl]-N-(2-piperidin-1-ylethyl)biphenyl-3-carboxamide

Reaction of the title compound of Example 261 (200 mg, 0.36 mmol) with (2-piperidin-1-ylethyl)amine (103.7 µg, 0.73 mmol) according to the method described in Example 49 gave 61.03 mg (25%) of the title compound.
¹H NMR (400 MHz, DMSO-*d*₆) ppm 1.39 (t, *J*=7.24 Hz, 5 H) 1.44 - 1.56 (m, 4 H) 2.35 - 2.41 (m, 3 H) 2.42 - 2.49 (m, 3 H) 3.35 - 3.47 (m, 2 H) 4.29 (q, *J*=7.17 Hz, 2 H) 7.66 (t, *J*=7.83 Hz, 1 H) 7.86 (t, *J*=8.41 Hz, 2 H) 8.11 (s, 1 H) 8.17 - 8.27 (m, 2 H) 8.36 - 8.44 (m, 3 H) 8.68 (s, 2 H) 8.74 (t, *J*=6.06 Hz, 1 H)
HPLC/MS (30 min) retention time 6.75 min
LRMS: *m*/*z* 659 (M+)

### EXAMPLE 264

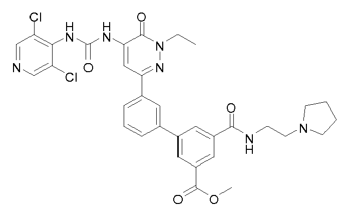

### Methyl 3'-[5-({[(3,5-dichloropyridin-4-yl)amino]carbonyl}amino)-1-ethyl-6-oxo-1,6-dihydropyridazin-3-yl]-5-{[(2-pyrrolidin-1-ylethyl)amino]carbonyl}biphenyl-3-carboxylate

Reaction of the title compound of Preparation 104 (200 mg, 0.22 mmol) with (2-pyrrolidin-1-ylethyl)amine (83 µg, 0.66 mmol) according to the method described in Example 49 gave 27.5 mg (18%) of the title compound.
¹H NMR (400 MHz, DMSO-*d*₆) ppm 1.39 (t, *J*=7.26 Hz, 3 H) 1.58 - 1.73 (m, 4 H) 2.61 (t, *J*=6.84 Hz, 2 H) 3.36 - 3.53 (m, 2 H) 3.92 (s, 3 H) 4.13 - 4.40 (m, 3 H) 7.66 (t, *J*=7.67 Hz, 1 H) 7.84 (d, *J*=7.88 Hz, 2 H) 8.07 (s, 1 H) 8.25 (s, 2 H) 8.33 (s, 1 H) 8.41 (s, 2 H) 8.45 (s, 1 H) 8.67 (s, 2 H) 8.83 (t, *J*=5.81 Hz, 1 H) 9.64 (s, 1 H)
HPLC/MS (30 min) retention time 6.82 min
LRMS: *m*/*z* 678 (M+)

### EXAMPLE 265

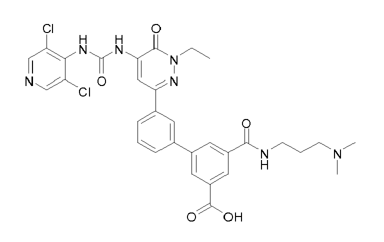

### 3'-[5-({[(3,5-dichloropyridin-4-yl)amino]carbonyl}amino)-1-ethyl-6-oxo-1,6-dihydropyridazin-3-yl]-5-({[3-(dimethylamino)propyl]amino}carbonyl)biphenyl-3-carboxylic acid

Reaction of the title compound of Example 157 (930 mg, 1.39 mmol) according to the method described in Example 26 gave 1.16 g (99%) of the title compound.
¹H NMR (400 MHz, DMSO-*d*₆) ppm 1.39 (t, *J*=7.04 Hz, 3 H) 1.86 - 1.97 (m, 2 H) 2.70 (s, 6 H) 3.02 (t, 2 H) 3.36 - 3.45 (m, 2 H) 4.29 (q, *J*=7.04 Hz, 2 H) 7.66 (t, *J*=7.83 Hz, 1 H) 7.84 (d, *J*=7.83 Hz, 2 H) 8.06 (s, 1 H) 8.33 (s, 1 H) 8.39 (s, 2 H) 8.47 (s, 1 H) 8.69 (s, 2 H) 8.98 (t, *J*=5.48 Hz, 1 H) 9.72 (s, 1 H) 9.94 (br. s., 1 H)
HPLC/MS (30 min) retention time 11.76 min
LRMS: *m*/*z* 652 (M+)

### EXAMPLE 266

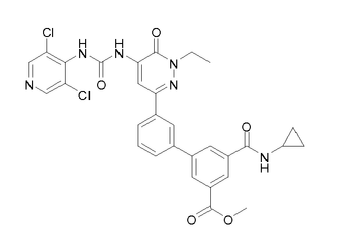

### Methyl 5-[(cyclopropylamino)carbonyl]-3'-[5-({[(3,5-dichloropyridin-4-yl)amino]carbonyl}amino)-1-ethyl-6-oxo-1,6-dihydropyridazin-3-yl]biphenyl-3-carboxylate

Reaction of the title compound of Preparation 7 (1.1 g, 2.28 mmol) with (1.2 g, 3.48 mmol) of the title compound of Preparation 60 according to the method described in Example 1 gave 0.075 g (71 %) of the title compound.
¹H NMR (400 MHz, DMSO-*d*₆) ppm 0.62 (br. s., 2 H) 0.72 (d, *J*=5.81 Hz, 2 H) 1.39 (t, *J*=6.84 Hz, 3 H) 2.86 - 2.95 (m, 1 H) 3.92 (s, 3 H) 4.29 (q, 2 H) 7.66 (t, *J*=7.46 Hz, 1 H) 7.84 (d, *J*=7.46 Hz, 2 H) 8.06 (br. s., 1 H) 8.32 (br. s., 1 H) 8.35 - 8.47 (m, 3 H) 8.69 (s, 2 H) 8.78 (br. s., 1 H) 9.71 (br. s., 1 H) 9.90 (br. s., 1 H)
HPLC/MS (30 min) retention time 18.46 min
LRMS: *m*/*z* 621 (M+)

### EXAMPLE 267

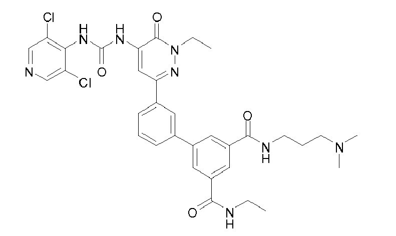

### 3'-[5-({[(3,5-dichloropyridin-4-yl)amino]carbonyl}amino)-1-ethyl-6-oxo-1,6-dihydropyridazin-3-yl]-N-[3-(dimethylamino)propyl]-N'-ethylbiphenyl-3,5-dicarboxamide

Reaction of the title compound of Example 265 (175 mg, 0.25 mmol) with ethanamine (268 µl, 0.54 mmol, 2M THF) according to the method described in Example 49 gave 26 mg (15%) of the title compound after purification by SP1® automated purification system.
¹H NMR (400 MHz, DMSO-*d*₆) ppm 1.15 (t, *J*=7.04 Hz, 3 H) 1.40 (t, *J*=7.04 Hz, 3 H) 1.69 (quin, *J*=6.95 Hz, 2 H) 2.16 (s, 6 H) 2.31 (t, *J*=6.85 Hz, 2 H) 3.28 - 3.40 (m, 4 H) 4.28 (q, *J*=7.04 Hz, 2 H) 7.63 (t, *J*=7.63 Hz, 1 H) 7.81 (t, *J*=6.85 Hz, 2 H) 8.06 (s, 1 H) 8.20 (d, *J*=5.09 Hz, 2 H) 8.29 (s, 1 H) 8.38 (s, 1 H) 8.47 (br. s., 1 H) 8.55 (br. s., 1 H) 8.62 (s, 2 H) 9.52 (br. s., 1 H)
HPLC/MS (30 min) retention time 11.99 min
LRMS: *m*/*z* 679 (M+)

### EXAMPLE 268

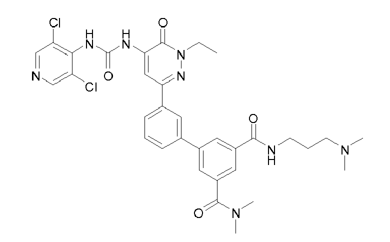

### 3'-[5-({[(3,5-dichloropyridin-4-yl)amino]carbonyl}amino)-1-ethyl-6-oxo-1,6-dihydropyridazin-3-yl]-N'-[3-(dimethylamino)propyl]-N,N-dimethylbiphenyl-3,5-dicarboxamide

Reaction of the title compound of Example 265 (200 mg, 0.28 mmol) with dimethylamine (307 µl, 0.61 mmol, 2M THF) according to the method described in Example 49 gave 70 mg (36%) of the title compound after purification by SP1® automated purification system.
¹H NMR (400 MHz, DMSO-*d*₆) ppm 1.37 (t, *J*=7.04 Hz, 3 H) 1.68 (quin, *J*=7.04 Hz, 2 H) 2.17 (s, 6 H) 2.28 - 2.37 (m, 2 H) 2.93 (s, 3 H) 3.01 (s, 3 H) 3.25 - 3.34 (m, 2 H) 4.27 (q, *J*=7.04 Hz, 2 H) 7.62 (t, *J*=7.83 Hz, 1 H) 7.77 - 7.84 (m, 4 H) 7.85 (s, 1 H) 8.18 (br. s., 2 H) 8.38 (s, 1 H) 8.66 (s, 2 H) 8.68 - 8.75 (m, 1 H) 9.66 (br. s., 1 H)
HPLC/MS (30 min) retention time 11.64 min
LRMS: *m*/*z* 679 (M+)

### EXAMPLE 269

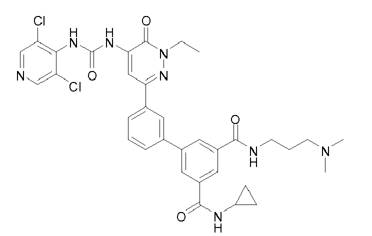

### N-cyclopropyl-3'-[5-({[(3,5-dichloropyridin-4-yl)amino]carbonyl}amino)-1-ethyl-6-oxo-1,6-dihydropyridazin-3-yl]-N'-[3-(dimethylamino)propyl]biphenyl-3,5-dicarboxamide

Reaction of the title compound of Example 265 (200 mg, 0.28 mmol) with cyclopropanamine (43 µl, 0.62 mmol) according to the method described in Example 49 gave 45 mg (23%) of the title compound after purification by SP1® automated purification system.
¹H NMR (400 MHz, DMSO-*d*₆) ppm 0.60 (br. s., 2 H) 0.70 (d, *J*=6.65 Hz, 2 H) 1.40 (t, *J*=7.04 Hz, 3 H) 1.69 (quin, *J*=6.85 Hz, 2 H) 2.16 (s, 6 H) 2.31 (t, *J*=6.85 Hz, 2 H) 2.85 - 2.94 (m, 1 H) 3.29 - 3.37 (m, 2 H) 4.28 (q, *J*=7.04 Hz, 2 H) 7.62 (t, *J*=7.83 Hz, 1 H) 7.79 (m, 2 H) 8.05 (s, 1 H) 8.15 (br. s., 1 H) 8.19 (d, *J*=3.13 Hz, 2 H) 8.26 (s, 1 H) 8.38 (s, 1 H) 8.48 (br. s., 1 H) 8.57 (br. s., 1 H) 8.63 (s, 2 H) 9.55 (br. s., 1 H)
HPLC/MS (30 min) retention time 12.18 min
LRMS: *m*/*z* 691 (M+)

### EXAMPLE 270

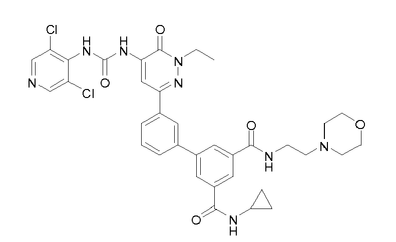

### N-cyclopropyl-3'-[5-({[(3,5-dichloropyridin-4-yl)amino]carbonyl}amino)-1-ethyl-6-oxo-1,6-dihydropyridazin-3-yl]-N'-(2-morpholin-4-ylethyl)biphenyl-3,5-dicarboxamide

Reaction of the title compound of Example 147 (150 mg, 0.23 mmol) with 2-morpholinoethanamine (61 mg, 0.47 mmol) according to the method described in Example 49 gave 140 mg (83%) of the title compound after purification by SP1® automated purification system.
¹H NMR (400 MHz, DMSO-*d*₆) ppm 0.56 - 0.63 (m, 2 H) 0.68 - 0.75 (m, 2 H) 1.39 (t, *J*=7.05 Hz, 3 H) 2.42 (br. s., 2 H) 2.44 - 2.50 (m, 4 H) 2.89 (td, *J*=7.15, 3.94 Hz, 1 H) 3.42 (q, *J*=6.22 Hz, 2 H) 3.56 (t, *J*=4.15 Hz, 4 H) 4.29 (q, *J*=7.05 Hz, 2 H) 7.65 (t, *J*=7.67 Hz, 1 H) 7.78 - 7.88 (m, 2 H) 8.08 (s, 1 H) 8.23 (d, *J*=5.39 Hz, 2 H) 8.30 (s, 2 H) 8.41 (s, 1 H) 8.67 (s, 2 H) 8.68 - 8.73 (m, 1 H)
HPLC/MS (30 min) retention time 11.91 min
LRMS: *m*/*z* 719 (M+)

### EXAMPLE 271

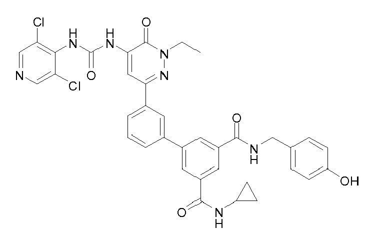

### N-cyclopropyl-3'-[5-({[(3,5-dichloropyridin-4-yl)amino]carbonyl}amino)-1-ethyl-6-oxo-1,6-dihydropyridazin-3-yl]-N'-(4-hydroxybenzyl)biphenyl-3,5-dicarboxamide

Reaction of the title compound of Example 147 (150 mg, 0.23 mmol) with 4-(aminomethyl)phenol (58 mg, 0.47 mmol) according to the method described in Example 49 gave 150 mg (90%) of the title compound after purification by SP1® automated purification system.
¹H NMR (400 MHz, DMSO-*d*₆) ppm 0.56 - 0.63 (m, 2 H) 0.68 - 0.76 (m, 2 H) 1.39 (t, *J*=7.26 Hz, 3 H) 2.89 (td, *J*=7.26, 3.73 Hz, 1 H) 4.29 (q, *J*=7.05 Hz, 2 H) 4.36 - 4.44 (m, 2 H) 6.71 (d, *J*=8.71 Hz, 2 H) 7.15 (d, J=8.29 Hz, 2 H) 7.65 (t, *J*=7.67 Hz, 1 H) 7.77 - 7.90 (m, 2 H) 8.09 (s, 1 H) 8.22 (s, 1 H) 8.29 (s, 1 H) 8.34 (s, 1 H) 8.40 (s, 1 H) 8.66 - 8.68 (m, 1 H) 8.69 (s, 2 H) 9.16 (t, *J*=5.81 Hz, 1 H) 9.29 (br. s., 1 H) 9.70 (br. s., 1 H)
HPLC/MS (30 min) retention time 16.64 min
LRMS: *m*/*z* 712 (M+)

### EXAMPLE 272

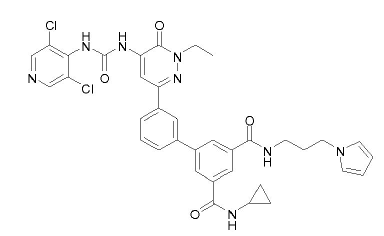

### N-cyclopropyl-3'-[5-({[(3,5-dichloropyridin-4-yl)amino]carbonyl}amino)-1-ethyl-6-oxo-1,6-dihydropyridazin-3-yl]-N'-[3-(1H-pyrrol-1-yl)propyl]biphenyl-3,5-dicarboxamide

Reaction of the title compound of Example 147 (150 mg, 0.23 mmol) with 3-(1H-imidazol-1-yl)propan-1-amine (59 mg, 0.47 mmol) according to the method described in Example 49 gave 140 mg (83%) of the title compound after purification by SP1® automated purification system.
¹H NMR (400 MHz, DMSO-*d*₆) ppm 0.57 - 0.64 (m, 2 H) 0.67 - 0.78 (m, 2 H) 1.39 (t, *J*=7.04 Hz, 3 H) 1.99 (qd, *J*=6.72, 6.46 Hz, 2 H) 2.89 (td, *J*=7.04, 3.52 Hz, 1 H) 3.25 - 3.32 (m, 2 H) 4.03 (t, *J*=6.65 Hz, 2 H) 4.29 (q, *J*=7.04 Hz, 2 H) 6.89 (s, 1 H) 7.21 (s, 1 H) 7.62 - 7.71 (m, 2 H) 7.84 (dd, *J*=15.65, 7.83 Hz, 2 H) 8.09 (s, 1 H) 8.18 - 8.27 (m, 2 H) 8.31 (s, 1 H) 8.40 (s, 1 H) 8.65 - 8.72 (m, 3 H) 8.75 (t, *J*=5.09 Hz, 1 H) 9.68 (br. s., 2 H)
HPLC/MS (30 min) retention time 11.98 min
LRMS: *m*/*z* 714 (M+)

### EXAMPLE 273

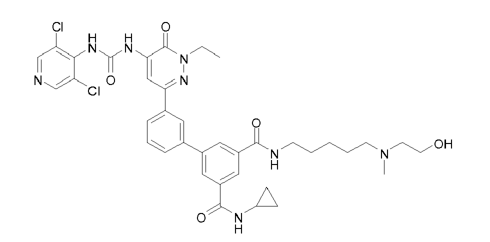

### N-cyclopropyl-3'-[5-({[(3,5-dichloropyridin-4-yl)amino]carbonyl}amino)-1-ethyl-6-oxo-1,6-dihydropyridazin-3-yl]-N'-{5-[(2-hydroxyethyl)(methyl)amino]pentyl}biphenyl-3,5-dicarboxamide

Reaction of the title compound of Example 147 (130 mg, 0.20 mmol) with 2-((5-aminopentyl)(methyl)amino)ethanol (65 mg, 0.41 mmol) according to the method described in Example 49 gave 50 mg (33%) of the title compound after purification by SP1® automated purification system.
¹H NMR (400 MHz, DMSO-*d*₆) ppm 0.53 - 0.64 (m, 2 H) 0.67 - 0.76 (m, 2 H) 1.26 - 1.37 (m, 2 H) 1.39 (t, *J*=7.24 Hz, 3 H) 1.42 - 1.48 (m, 2 H) 1.55 (dt, *J*=14.09, 7.04 Hz, 2 H) 2.20 (s, 3 H) 2.37 (t, *J*=7.24 Hz, 2 H) 2.44 (t, *J*=6.26 Hz, 2 H) 2.89 (td, *J*=7.14, 3.72 Hz, 1 H) 3.25 - 3.34 (m, 2 H) 3.47 (t, *J*=6.26 Hz, 2 H) 4.29 (q, *J*=6.65 Hz, 2 H) 7.65 (t, *J*=7.63 Hz, 1 H) 7.83 (dd, *J*=16.04, 7.83 Hz, 2 H) 8.08 (s, 1 H) 8.19 - 8.27 (m, 2 H) 8.30 (s, 1 H) 8.40 (s, 1 H) 8.68 (s, 3 H) 9.68 (br. s., 1 H)
HPLC/MS (30 min) retention time 12.12 min
LRMS: *m*/*z* 749 (M+)

### EXAMPLE 274

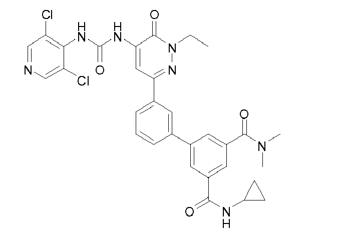

### N'-cyclopropyl-3'-[5-({[(3,5-dichloropyridin-4-yl)amino]carbonyl}amino)-1-ethyl-6-oxo-1,6-dihydropyridazin-3-yl]-N,N-dimethylbiphenyl-3,5-dicarboxamide

Reaction of the title compound of Example 147 (150 mg, 0.24 mmol) with dimethylamine (242 µl, 0.48 mmol, 2M THF) according to the method described in Example 49 gave 100 mg (65%) of the title compound after purification by SP1® automated purification system.
¹H NMR (400 MHz, DMSO-*d*₆) ppm 0.55 - 0.65 (m, 2 H) 0.66 - 0.74 (m, 2 H) 1.39 (t, *J*=7.24 Hz, 3 H) 2.88 (dt, *J*=7.14, 3.67 Hz, 1 H) 2.94 (br. s., 3 H) 3.02 (br. s., 3 H) 4.28 (q, *J*=7.04 Hz, 2 H) 7.63 (t, *J*=7.63 Hz, 1 H) 7.80 - 7.87 (m, 4 H) 8.05 (s, 1 H) 8.17 (s, 1 H) 8.40 (s, 1 H) 8.64 (d, *J*=3.91 Hz, 1 H) 8.69 (s, 2 H) 9.68 (br. s., 1 H) 9.90 (br. s., 1 H)
HPLC/MS (30 min) retention time 15.96 min
LRMS: *m*/*z* 634 (M+)

### EXAMPLE 275

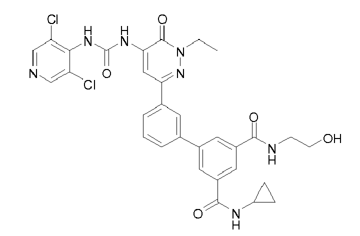

### N-cyclopropyl-3'-[5-({[(3,5-dichloropyridin-4-yl)amino]carbonyl}amino)-1-ethyl-6-oxo-1,6-dihydropyridazin-3-yl]-N'-(2-hydroxyethyl)biphenyl-3,5-dicarboxamide

Reaction of the title compound of Example 147 (150 mg, 0.24 mmol) with 2-amino ethanol (30 mg, 0.49 mmol) according to the method described in Example 49 gave 80 mg (51%) of the title compound after purification by SP1® automated purification system.
¹H NMR (400 MHz, DMSO-*d*₆) ppm 0.56 - 0.65 (m, 2 H) 0.68 - 0.76 (m, 2 H) 1.39 (t, *J*=7.24 Hz, 3 H) 2.85 - 2.94 (m, 1 H) 3.38 (q, 2 H) 3.54 (q, *J*=6.00 Hz, 2 H) 4.29 (q, *J*=7.04 Hz, 2 H) 4.75 (t, *J*=5.28 Hz, 1 H) 7.65 (t, *J*=7.63 Hz, 1 H) 7.81 (d, *J*=7.83 Hz, 1 H) 7.86 (d, *J*=7.83 Hz, 1 H) 8.09 (s, 1 H) 8.22 (s, 1 H) 8.27 (s, 1 H) 8.31 (s, 1 H) 8.40 (s, 1 H) 8.68 (s, 3 H) 9.68 (br. s., 1 H) 9.87 (br. s., 1 H)
HPLC/MS (30 min) retention time 15.03 min
LRMS: *m*/*z* 650 (M+)

### EXAMPLE 276

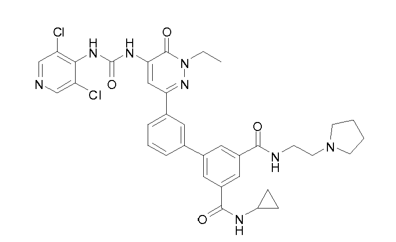

### N-cyclopropyl-3'-[5-({[(3,5-dichloropyridin-4-yl)amino]carbonyl}amino)-1-ethyl-6-oxo-1,6-dihydropyridazin-3-yl]-N'-(2-pyrrolidin-1-ylethyl)biphenyl-3,5-dicarboxamide

Reaction of the title compound of Example 147 (140 mg, 0.23 mmol) with 2-(pyrrolidin-1-yl)ethanamine (52 mg, 0.45 mmol) according to the method described in Example 49 gave 90 mg (56%) of the title compound after purification by SP1® automated purification system.
¹H NMR (400 MHz, DMSO-*d*₆) ppm 0.56 - 0.65 (m, 2 H) 0.66 - 0.78 (m, 2 H) 1.39 (t, *J*=7.24 Hz, 3 H) 1.69 (br. s., 4 H) 2.56 (br. s., 4 H) 2.66 (t, *J*=6.85 Hz, 2 H) 2.89 (td, *J*=7.14, 3.72 Hz, 1 H) 3.44 (q, 2 H) 4.29 (q, *J*=7.04 Hz, 2 H) 7.66 (t, *J*=7.83 Hz, 1 H) 7.82 (d, *J*=7.83 Hz, 1 H) 7.86 (d, *J*=7.43 Hz, 1 H) 8.08 (s, 1 H) 8.20 (s, 1 H) 8.22 (s, 1 H) 8.25 (s, 1 H) 8.31 (s, 1 H) 8.40 (s, 1 H) 8.69 (s, 2 H) 8.73 (t, *J*=5.48 Hz, 1 H) 9.70 (br. s., 1 H)
HPLC/MS (30 min) retention time 11.86 min
LRMS: *m*/*z* 703 (M+)

### EXAMPLE 277

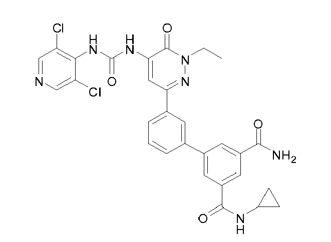

### N-cyclopropyl-3'-[5-({[(3,5-dichloropyridin-4-yl)amino]carbonyl}amino)-1-ethyl-6-oxo-1,6-dihydropyridazin-3-yl]biphenyl-3,5-dicarboxamide

Reaction of the title compound of Example 147 (150 mg, 0.24 mmol) with ammonia (44 µl 0.72 mmol) according to the method described in Example 49 gave 80 mg (54%) of the title compound after purification by SP1® automated purification system.
¹H NMR (400 MHz, DMSO-*d*₆) ppm 0.57 - 0.66 (m, 2 H) 0.67 - 0.77 (m, 2 H) 1.39 (t, *J*=7.24 Hz, 3 H) 2.85 - 2.93 (m, 1 H) 4.29 (q, *J*=7.04 Hz, 2 H) 7.53 (br. s., 1 H) 7.65 (t, *J*=7.63 Hz, 1 H) 7.82 (d, *J*=8.22 Hz, 1 H) 7.86 (d, *J*=7.83 Hz, 1 H) 8.09 (s, 1 H) 8.17 - 8.26 (m, 2 H) 8.30 (s, 1 H) 8.33 (s, 1 H) 8.40 (s, 1 H) 8.67 (d, *J*=3.91 Hz, 1 H) 8.69 (s, 2 H) 9.70 (br. s., 1 H) 9.90 (br. s., 1 H)
HPLC/MS (30 min) retention time 15.48 min
LRMS: *m*/*z* 606 (M+)

### EXAMPLE 278

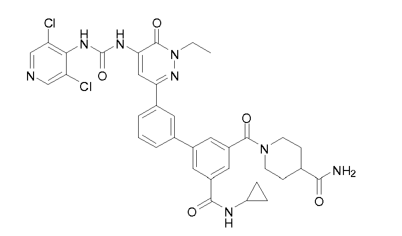

### 1-({5-[(cyclopropylamino)carbonyl]-3'-[5-({[(3,5-dichloropyridin-4-yl)amino]carbonyl}amino)-1-ethyl-6-oxo-1,6-dihydropyridazin-3-yl]biphenyl-3-yl}carbonyl)piperidine-4-carboxamide

Reaction of the title compound of Example 147 (140 mg, 0.23 mmol) with piperidine-4-carboxamide (58 mg , 0.45 mmol) according to the method described in Example 49 gave 80 mg (49%) of the title compound after purification by SP1® automated purification system.
¹H NMR (400 MHz, DMSO-*d*₆) ppm 0.56 - 0.64 (m, 2 H) 0.67 - 0.78 (m, 2 H) 1.39 (t, *J*=7.24 Hz, 3 H) 1.45 - 1.90 (m, 4 H) 2.30 - 2.44 (m, 1 H) 2.79 - 2.95 (m, 2 H) 3.10 (br. s., 1 H) 3.60 (br. s., 1 H) 4.28 (q, *J*=7.04 Hz, 2 H) 4.47 (br. s., 1 H) 6.81 (br. s., 1 H) 7.29 (br. s., 1 H) 7.64 (t, *J*=7.63 Hz, 1 H) 7.76 - 7.88 (m, 3 H) 8.06 (s, 1 H) 8.18 (s, 1 H) 8.40 (s, 1 H) 8.67 (d, *J*=3.91 Hz, 1 H) 8.69 (s, 2 H) 9.68 (br. s., 1 H) 9.86 (br. s., 1 H)
HPLC/MS (30 min) retention time 14.53 min
LRMS: *m*/*z* 717 (M+)

### EXAMPLE 279

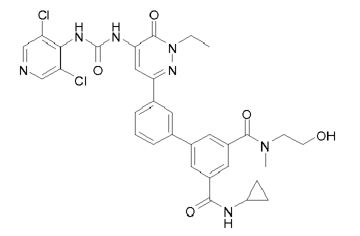

### N'-cyclopropyl-3'-[5-({[(3,5-dichloropyridin-4-yl)amino]carbonyl}amino)-1-ethyl-6-oxo-1,6-dihydropyridazin-3-yl]-N-(2-hydroxyethyl)-N-methylbiphenyl-3,5-dicarboxamide

Reaction of the title compound of Example 147 (150 mg, 0.24 mmol) with 2-(methylamino)ethanol (36 mg, 0.48 mmol) according to the method described in Example 49 gave 35 mg (21%) of the title compound after purification by SP1® automated purification system.
¹H NMR (400 MHz, DMSO-*d*₆) ppm 0.55 - 0.64 (m, 2 H) 0.66 - 0.77 (m, 2 H) 1.39 (t, *J*=7.26 Hz, 3 H) 2.84 - 2.93 (m, 1 H) 2.94 - 3.05 (s, 3 H) 3.39 - 3.41 (m, 2H) 3.43 - 3.57 (m, 2 H) 4.28 (q, *J*=7.05 Hz, 2 H) 4.81 (br. s., 1 H) 7.63 (t, *J*=7.67 Hz, 1 H) 7.78 - 7.91 (m, 4 H) 8.06 (s, 1 H) 8.16 (br. s., 1 H) 8.41 (s, 1 H) 8.64 (br. s., 1 H) 8.66 (s, 2 H) 9.64 (br. s., 2 H)
HPLC/MS (30 min) retention time 14.92 min
LRMS: *m*/*z* 664 (M+)

### EXAMPLE 280

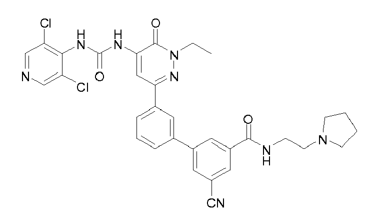

### 5-cyano-3'-[5-({[(3,5-dichloropyridin-4-yl)amino]carbonyl}amino)-1-ethyl-6-oxo-1,6-dihydropyridazin-3-yl]-N-(2-pyrrolidin-1-ylethyl)biphenyl-3-carboxamide

Reaction of the title compound of Example 261 (150 mg, 0.25 mmol) with 2-(pyrrolidin-1-yl)ethanamine (56 mg, 0.49 mmol) according to the method described in Example 49 gave 39 mg (25%) of the title compound after purification by SP1® automated purification system.
¹H NMR (400 MHz, DMSO-*d*₆) ppm 1.39 (t, *J*=7.05 Hz, 3 H) 1.62 - 1.75 (m, 4 H) 2.52 - 2.58 (m, 4 H) 2.64 (t, *J*=6.84 Hz, 2 H) 3.38 - 3.48 (m, 2 H) 4.29 (q, *J*=7.05 Hz, 2 H) 7.66 (t, *J*=7.67 Hz, 1 H) 7.87 (t, *J*=8.09 Hz, 2 H) 8.11 (s, 1 H) 8.26 (s, 2 H) 8.37 - 8.42 (m, 2 H) 8.43 (s, 1 H) 8.68 (s, 2 H) 8.83 (t, *J*=5.39 Hz, 1 H)
HPLC/MS (30 min) retention time 12.25 min
LRMS: *m*/*z* 645 (M+)

### EXAMPLE 281

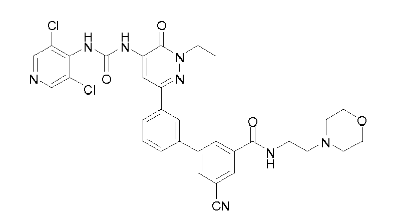

### 5-cyano-3'-[5-({[(3,5-dichloropyridin-4-yl)amino]carbonyl}amino)-1-ethyl-6-oxo-1,6-dihydropyridazin-3-yl]-N-(2-morpholin-4-ylethyl)biphenyl-3-carboxamide

Reaction of the title compound of Example 261 (150 mg, 0.25 mmol) with 2-morpholinoethanamine (64 mg, 0.49 mmol) according to the method described in Example 49 gave 64 mg (38%) of the title compound after purification by SP1® automated purification system.
¹H NMR (400 MHz, DMSO-*d*₆) ppm 1.39 (t, *J*=7.26 Hz, 3 H) 2.39 - 2.45 (m, 4 H) 2.46 - 2.49 (m, 2 H) 3.43 (q, *J*=6.50 Hz, 2 H) 3.52 - 3.63 (m, 4 H) 4.29 (q, *J*=7.19 Hz, 2 H) 7.66 (t, *J*=7.67 Hz, 1 H) 7.86 (t, *J*=7.88 Hz, 2 H) 8.11 (s, 1 H) 8.24 (s, 1 H) 8.26 (s, 1 H) 8.37 - 8.44 (m, 3 H) 8.68 (s, 2 H) 8.77 (t, *J*=5.39 Hz, 1 H)
HPLC/MS (30 min) retention time 12.24 min
LRMS: *m*/*z* 661 (M+)

### EXAMPLE 282

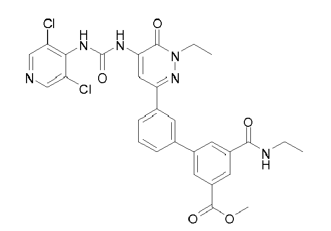

### Methyl 3'-[5-({[(3,5-dichloropyridin-4-yl)amino]carbonyl}amino)-1-ethyl-6-oxo-1,6-dihydropyridazin-3-yl]-5-[(ethylamino)carbonyl]biphenyl-3-carboxylate

Reaction of the title compound of Preparation 104 (648 mg, 0.71 mmol) with ethanamine (890 µl, 0.48 mmol, 2M THF) according to the method described in Example 49 gave 330 mg (76%) of the title compound after purification by SP1® automated purification system.
¹H NMR (400 MHz, DMSO-*d*₆) ppm 1.15 (t, *J*=7.26 Hz, 3 H) 1.39 (t, *J*=7.26 Hz, 3 H) 3.35 - 3.39 (m, 2 H) 3.92 (s, 3 H) 4.29 (q, *J*=7.05 Hz, 2 H) 7.66 (t, *J*=7.67 Hz, 1 H) 7.80 - 7.89 (m, 2 H) 8.07 (s, 1 H) 8.32 (s, 1 H) 8.40 (s, 2 H) 8.46 (s, 1 H) 8.69 (s, 2 H) 8.82 (t, *J*=5.39 Hz, 1 H) 9.70 (br. s., 1 H) 9.90 (br. s., 1 H)
HPLC/MS (30 min) retention time 17.90 min
LRMS: *m*/*z* 609 (M+)

### EXAMPLE 283

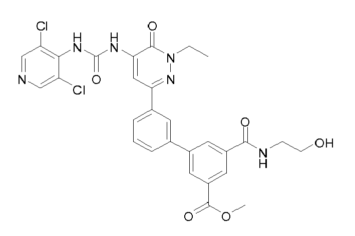

### Methyl 3'-[5-({[(3,5-dichloropyridin-4-yl)amino]carbonyl}amino)-1-ethyl-6-oxo-1,6-dihydropyridazin-3-yl]-5-{[(2-hydroxyethyl)amino]carbonyl}biphenyl-3-carboxylate

Reaction of the title compound of Preparation 104 (200 mg, 0.22 mmol) with 2-amino ethanol (33 µl, 0.55 mmol) according to the method described in Example 49 gave 36 mg (26%) of the title compound after purification by SP1® automated purification system.
¹H NMR (400 MHz, DMSO-*d*₆) ppm 1.38 (t, 3 H) 3.36 - 3.42 (m, 2 H) 3.54 (q, *J*=5.61 Hz, 2 H) 3.92 (s, 3 H) 4.29 (q, *J*=7.04 Hz, 2 H) 4.76 (t, *J*=5.48 Hz, 1 H) 7.66 (t, *J*=7.63 Hz, 1 H) 7.85 (t, *J*=6.85 Hz, 2 H) 8.07 (s, 1 H) 8.33 (s, 1 H) 8.40 (s, 1 H) 8.43 (s, 1 H) 8.47 (s, 1 H) 8.68 (s, 2 H) 8.83 (t, *J*=5.28 Hz, 1 H) 9.69 (br. s., 1 H) 9.90 (br. s., 1 H)
HPLC/MS (30 min) retention time 16.63 min
LRMS: *m*/*z* 625 (M+)

### EXAMPLE 284

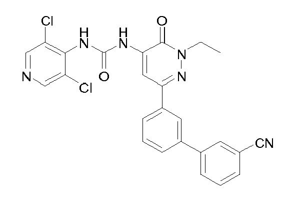

### N-[6-(3'-cyanobiphenyl-3-yl)-2-ethyl-3-oxo-2,3-dihydropyridazin-4-yl]-N'-(3,5-dichloropyridin-4-yl)urea

Reaction of the title compound of Preparation 7 (150 mg, 0.31 mmol) with 3-cyanophenylboronic acid (60 mg, 0.37 mmol) according to the method described in Example 1 gave 32 mg (20%) of the title compound after purification by chromatography (hexane/acetone 85:15 - 90:10).
¹H NMR (400 MHz, DMSO-*d*₆) ppm 1.40 (t, 3 H), 4.29 (q, 2 H), 7.62 - 8.18 (m, 8 H), 8.39 (s, 1 H), 8.67 (s, 2 H), 9.65 (bs), 9.86 (bs)
LRMS: *m*/*z* 505 (M+)

### EXAMPLE 285

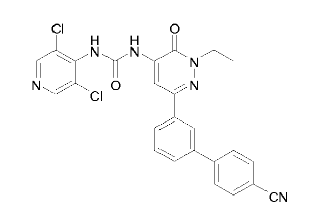

### N-[6-(4'-cyanobiphenyl-3-yl)-2-ethyl-3-oxo-2,3-dihydropyridazin-4-yl]-N'-(3,5-dichloropyridin-4-yl)urea

Reaction of the title compound of Preparation 7 (150 mg, 0.31 mmol) with 4-cyanophenylboronic acid (60 mg, 0.37 mmol) according to the method described in Example 1 gave 24 mg (15%) of the title compound after purification by chromatography (hexane/acetone 75:25).
¹H NMR (400 MHz, DMSO-*d*₆) ppm 1.40 (t, 3 H), 4.29 (q, 2 H), 7.64 - 8.06 (m, 8 H), 8.40 (s, 1 H), 8.68 (s, 2 H), 9.66 (bs), 9.88 (bs)
LRMS: *m*/*z* 505 (M+)

### EXAMPLE 286

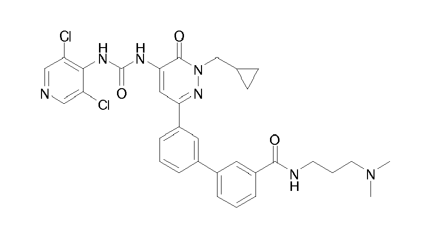

### 3'-[1-(cyclopropylmethyl)-5-({[(3,5-dichloropyridin-4-yl)amino]carbonyl}amino)-6-oxo-1,6-dihydropyridazin-3-yl]-N-[3-(dimethylamino)propyl]biphenyl-3-carboxamide

Reaction of the title compound of Preparation 110 (150 mg, 0.31 mmol) with the title compound of Preparation 36 (60 mg, 0.37 mmol) according to the method described in Example 1 gave 24 mg (15%) of the title compound after purification by chromatography (hexane/acetone 75:25).
¹H NMR (400 MHz, DMSO-*d*₆) δ ppm 0.52 (m, 4H), 1.38 (m, 1 H), 1.69 (q, 2H), 2.17 (s, 6H), 2.32 (t, 2H), 3.32 (m, 2H), 4.13 (d, 2H), 7.58 (m, 2H), 7.80 (m, 5H), 8.05 (bs, 2H), 8.40 (s, 1H), 8.46 (s, 1H), 8.64 (s, 2H), 9.57 (s, 1H).
LRMS: *m*/*z* 634 (M+)

### EXAMPLE 287

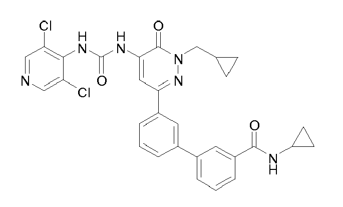

### N-cyclopropyl-3'-[1-(cyclopropylmethyl)-5-({[(3,5-dichloropyridin-4-yl)amino]carbonyl}amino)-6-oxo-1,6-dihydropyridazin-3-yl]biphenyl-3-carboxamide

Reaction of the title compound of Preparation 110 (290 mg, 0.56 mmol) with N-cyclopropyl-3-(4,4,5,5-tetramethyl-1,3,2-dioxaborolan-2-yl)benzamide (240 mg, 0.84 mmol) according to the method described in Example 1 gave 146 mg (44%) of the title compound after purification by chromatography (CH₂Cl₂- MeOH 2.5%).
¹H NMR (400 MHz, DMSO-*d*₆) δ ppm 0.58 (m, 8H), 1.38 (m, 1H), 2.87 (m, 1H), 4.13 (d, 2H), 7.60 (m, 2H), 7.82 (m, 4H), 8.09 (d, 2H), 8.41 (s, 1H), 8.56 (s, 1H), 8.69 (s, 2H), 9.72 (s, 1H), 9.89 (s, 1H).
LRMS: *m*/*z* 589 (M+)

### EXAMPLE 288

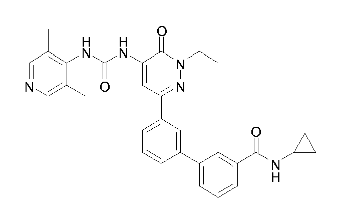

### N-cyclopropyl-3'-[5-({[(3,5-dimethylpyridin-4-yl)amino]carbonyl}amino)-1-ethyl-6-oxo-1,6-dihydropyridazin-3-yl]biphenyl-3-carboxamide

Reaction of the title compound of Preparation 57 (200 mg, 0.45 mmol) with N-cyclopropyl-3-(4,4,5,5-tetramethyl-1,3,2-dioxaborolan-2-yl)benzamide (190 mg, 0.68 mmol) according to the method described in Example 1 gave 219 mg (93%) of the title compound after purification by chromatography (CH₂Cl₂- MeOH 4%).
¹H NMR (400 MHz, DMSO-*d*₆) δ ppm 1.37 (m, 9H), 2.20 (s, 6H), 2.30 (s, 6H), 3.14 (m, 2H), 3.43 (s, 2H), 4.26 (m, 2H), 7.34 - 7.94 (m, 9H), 8.28 (s, 2H), 8.42 (s, 1H), 9.29 (s, 1H), 9.49 (s, 1H).
LRMS: *m*/*z* 523 (M+)

### EXAMPLE 289

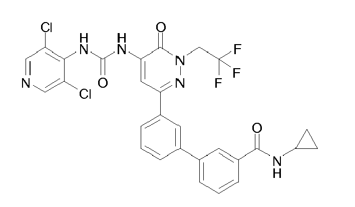

### N-cyclopropyl-3'-[5-({[(3,5-dichloropyridin-4-yl)amino]carbonyl}amino)-6-oxo-1-(2,2,2-trifluoroethyl)-1,6-dihydropyridazin-3-yl]biphenyl-3-carboxamide

Reaction of the title compound of Preparation 111 (300 mg, 0.56 mmol) with N-cyclopropyl-3-(4,4,5,5-tetramethyl-1,3,2-dioxaborolan-2-yl)benzamide (240 mg, 0.84 mmol) according to the method described in Example 1 gave 145 mg (42%) of the title compound after purification by SP1® automated purification system (20-100% H2O/ACN:MeOH (1:1)).
¹H NMR (400 MHz, DMSO-*d*₆) δ ppm 0.62 (m, 2H), 0.71 (m, 2H), 2.89 (m, 1H), 5.04 (q, 2H), 7.57 (m, 2H), 7.79 (m, 5H), 8.02 (t, 1H), 8.09 (t, 1H), 8.13 (s, 2H), 8.41 (bs, 1H), 8.62 (s, 1H).
LRMS: *m*/*z* 617 (M+)

### EXAMPLE 290

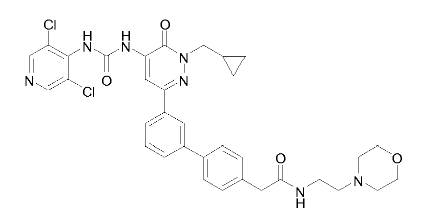

### 2-{3'-[1-(cyclopropylmethyl)-5-({[(3,5-dichloropyridin-4-yl)amino]carbonyl}amino)-6-oxo-1,6-dihydropyridazin-3-yl]biphenyl-4-yl}-N-(2-morpholin-4-ylethyl)acetamide

Reaction of the title compound of Preparation 110 (300 mg, 0.56 mmol) with the title compound of Preparation 113 (380 mg, 1.02 mmol) according to the method described in Example 1 gave 220 mg (53%) of the title compound purification by chromatography (CH₂Cl₂-MeOH 9:1).
¹H NMR (400 MHz, DMSO-*d*₆) δ pm 0.32 (m, 4H), 1.20 (m, 7H), 2.12 (m, 6H), 2.99 (m, 2H), 3.29 (s, 2H), 3.90 (d, 2H), 7.20 (d, 2H), 7.82 - 7.46 (m, 7H), 8.02 (m, 2H), 8.36 (s, 1H).
LRMS: *m*/*z* 676 (M+)

### EXAMPLE 291

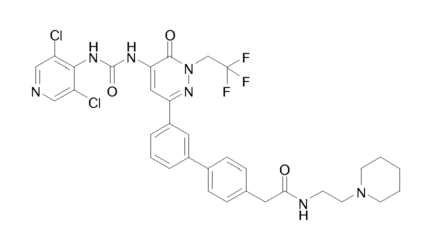

### 2-{3'-[5-({[(3,5-dichloropyridin-4-yl)amino]carbonyl}amino)-6-oxo-1-(2,2,2-trifluoroethyl)-1,6-dihydropyridazin-3-yl]biphenyl-4-yl}-N-(2-piperidin-1-ylethyl)acetamide

Reaction of the title compound of Preparation 111 (300 mg, 0.56 mmol) with the title compound of Preparation 113 (380 mg, 1.02 mmol) according to the method described in Example 1 gave 220 mg (53%) of the title compound purification by chromatography (CH₂Cl₂-MeOH 9:1).
¹H NMR (400 MHz, DMSO-*d*₆) δ ppm 0.32 (m, 4H), 1.20 (m, 7H), 2.12 (m, 6H), 2.99 (m, 2H), 3.29 (s, 2H), 3.90 (d, 2H), 7.20 (d, 2H), 7.82 -7.46 (m, 7H), 8.02 (m, 2H), 8.36 (s, 1H).
LRMS: *m*/*z* 702 (M+)

### EXAMPLE 292

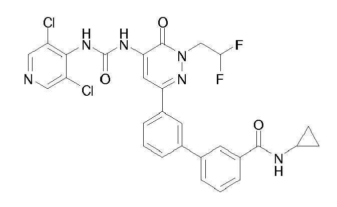

### N-cyclopropyl-3'-[5-({[(3,5-dichloropyridin-4-yl)amino]carbonyl}amino)-1-(2,2-difluoroethyl)-6-oxo-1,6-dihydropyridazin-3-yl]biphenyl-3-carboxamide

Reaction of the title compound of Preparation 112 (200 mg, 0.39 mmol) with N-cyclopropyl-3-(4,4,5,5-tetramethyl-1,3,2-dioxaborolan-2-yl)benzamide (170 mg, 0.58 mmol) according to the method described in Example 1 gave 48 mg (21 %) of the title compound after purification by SP1® automated purification system (20-100% H2O/ACN:MeOH (1:1)).
¹H NMR (400 MHz, DMSO-*d*₆) δ ppm 0.62 (m, 4H), 2.85 (m, 1H), 4.63 (td, 2H), 6.52 (tt, 1H), 7.58 (m, 2H), 7.81 (m, 5H), 8.02 (bs, 1H), 8.09 (bs, 1H), 8.14 (s, 2H), 8.40 (s, 1H), 8.57 (s, 1H).
LRMS: *m*/*z* 598 (M+)

### EXAMPLE 293

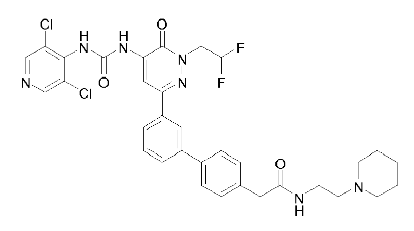

### 2-{3'-[5-({[(3,5-dichloropyridin-4-yl)amino]carbonyl}amino)-1-(2,2-difluoroethyl)-6-oxo-1,6-dihydropyridazin-3-yl]biphenyl-4-yl}-N-(2-piperidin-1-ylethyl)acetamide

Reaction of the title compound of Preparation 112 (240 mg, 0.46 mmol) with the title compound of Preparation 114 (260 mg, 0.69 mmol) according to the method described in Example 1 gave 92 mg (29%) of the title compound after purification by SP1® automated purification system (20-100% H2O/ACN:MeOH (1:1)).
¹H NMR (400 MHz, DMSO-*d*₆) δ ppm 1.38 (m, 6H), 2.28 (m, 6H), 3.14 (q, 2H), 3.45 (s, 2H), 4.63 (td, 2H), 6.51 (tt, 1H), 7.36 (m, 2H), 7.72 (m, 6H), 7.97 (m, 2H), 8.16 (s, 2H), 8.59 (s, 1H).
LRMS: *m*/*z* 684 (M+)

### EXAMPLE 294

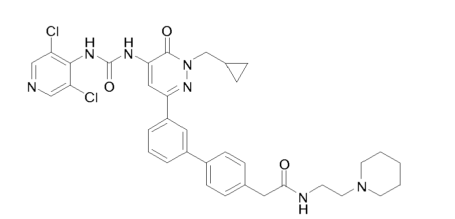

### 2-{3'-[1-(cyclopropylmethyl)-5-({[(3,5-dichloropyridin-4-yl)amino]carbonyl}amino)-6-oxo-1,6-dihydropyridazin-3-yl]biphenyl-4-yl}-N-(2-piperidin-1-ylethyl)acetamide

Reaction of the title compound of Preparation 110 (300 mg, 0.59 mmol) with the title compound of Preparation 114 (330 mg, 0.88 mmol) according to the method described in Example 1 gave 179 mg (45%) of the title compound after purification by SP1® automated purification system (20-100% H2O/ACN:MeOH (1:1)).
¹H NMR (400 MHz, DMSO-*d*₆) δ pm 0.32 (m, 4H), 1.20 (m, 7H), 2.12 (m, 6H), 2.99 (m, 2H), 3.29 (s, 2H), 3.90 (d, 2H), 7.20 (d, 2H), 7.82 - 7.46 (m, 7H), 8.02 (m, 2H), 8.36 (s, 1H).
LRMS: *m*/*z* 674 (M+)

### EXAMPLE 295

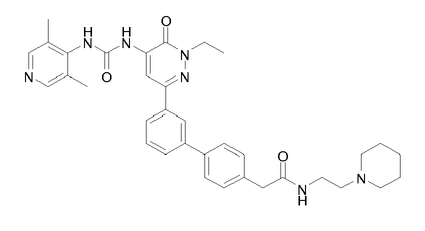

### 2-{3'-[5-({[(3,5-dimethylpyridin-4-yl)amino]carbonyl}amino)-1-ethyl-6-oxo-1,6-dihydropyridazin-3-yl]biphenyl-4-yl}-N-(2-piperidin-1-ylethyl)acetamide

Reaction of the title compound of Preparation 57 (300 mg, 0.68 mmol) with the title compound of Preparation 114 (380 mg, 1.02 mmol) according to the method described in Example 1 gave 220 mg (53%) of the title compound after purification by chromatography (CH₂Cl₂- MeOH 9:1).
¹H NMR (400 MHz, DMSO-*d*₆) δ ppm 1.37 (m, 9H), 2.20 (s, 6H), 2.30 (s, 6H), 3.14 (q, 2H), 3.43 (s, 2H), 4.26 (q, 2H), 7.34 - 7.94 (m, 9H), 8.28 (s, 2H), 8.42 (s, 1H), 9.29 (s, 1H), 9.49 (s, 1H).
LRMS: *m*/*z* 608 (M+)

### EXAMPLE 296

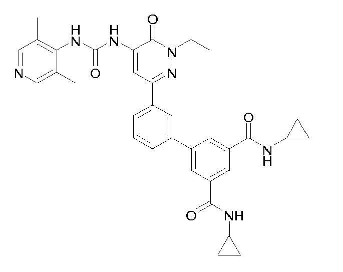

### N,N'-dicyclopropyl-3'-[5-({[(3,5-dimethylpyridin-4-yl)amino]carbonyl}amino)-1-ethyl-6-oxo-1,6-dihydropyridazin-3-yl]biphenyl-3,5-dicarboxamide

Reaction of the title compound of Preparation 57 (160 mg, 0.36 mmol) with the title compound of Preparation 63 (200 mg, 0.54 mmol) according to the method described in Example 1 gave 136 mg (63%) of the title compound after purification by chromatography (CH₂Cl₂- MeOH 9:1).
¹H NMR (400 MHz, DMSO-*d*₆) δ ppm 0.59 (m, 4H), 0.71 (m, 4H), 1.38 (t, 3H), 2.20 (bs, 6H), 2.88 (m, 2H), 4.28 (q, 2H), 7.61-7.86 (m, 3H), 8.06-8.42 (m, 7H), 8.69 (m, 2H), 9.30 (bs, 1H), 9.52 (bs, 1H).
LRMS: *m*/*z* 606 (M+)

### EXAMPLE 297

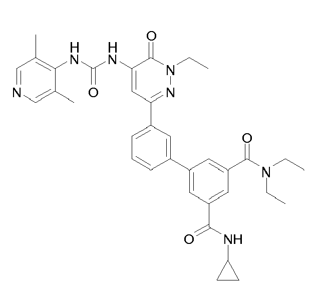

### N'-cyclopropyl-3'-[5-({[(3,5-dimethylpyridin-4-yl)amino]carbonyl}amino)-1-ethyl-6-oxo-1,6-dihydropyridazin-3-yl]-N,N-diethylbiphenyl-3,5-dicarboxamide

Reaction of the title compound of Preparation 57 (600 mg, 1.35 mmol) with the title compound of Preparation 116 (780 mg, 2.03 mmol) according to the method described in Example 1 gave 520 mg (62%) of the title compound after purification by chromatography (hexane - acetone 60:40).
¹H NMR (400 MHz, DMSO-*d*₆) δ ppm 0.59 (m, 2H), 0.71 (m, 2H), 1.06 (m, 3H), 1.16 (m, 3H), 1.38 (t, 3H), 2.20 (s, 6H), 2.89 (m, 1H), 3.20 (m, 2H), 3.46 (m, 2H), 4.27 (q, 2H), 7.59 - 7.84 (m, 5H), 8.05 - 8.43 (m, 5H), 8.67 (bs, 1H), 9.29 (bs, 1H), 9.52 (bs, 1H).
LRMS: *m*/*z* 622 (M+)

### EXAMPLE 298

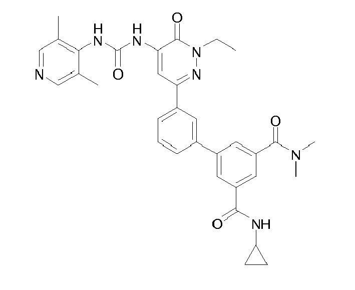

### N'-cyclopropyl-3'-[5-({[(3,5-dimethylpyridin-4-yl)amino]carbonyl}amino)-1-ethyl-6-oxo-1,6-dihydropyridazin-3-yl]-N,N-dimethylbiphenyl-3,5-dicarboxamide

Reaction of the title compound of Preparation 57 (360 mg, 0.82 mmol) with the title compound of Preparation 116 (440 mg, 1.23 mmol) according to the method described in Example 1 gave 81 mg (17%) of the title compound after purification by chromatography (hexane - acetone 60:40).
¹H NMR (400 MHz, DMSO-*d*₆) δ ppm 0.60 (m, 2H), 0.70 (m, 2H), 1.38 (t, 3H), 2.20 (s, 6H), 2.89 (m, 1H), 2.94 (s, 3H), 3.02 (s, 3H), 4.28 (q, 2H), 7.60 - 7.84 (m, 5H), 8.05 - 8.42 (m, 5H), 8.65 (bs, 1H), 9.29 (bs, 1H), 9.52 (bs, 1H).
LRMS: *m*/*z* 594 (M+)

### PHARMACOLOGICAL ACTIVITY

### PDE4 Assay Procedure

Measurement of Phosphodiesterase Activity was done using a [3H] cAMP Scintillation Proximity Assay (SPA) (GE Healthcare). Compounds to be tested were disolved in DMSO at a stock concentration of 1mM and serial dilutions were prepared in 50% DMSO to determine IC50s.

The reactions were conducted in 96-well plates (Corning, Ref.3604) at room temperature, in 0.1 ml of reaction buffer containing (final concentrations): 50 mM Tris-HCl, pH 7.5, 8.3 mM MgCl₂, 1.7 mM EGTA, 30 nM [3H] cAMP (approximately 150000 dpm/well) with or without the inhibitors. The reaction was initiated by adding yeast extract of recombinant PDE4 enzyme.

Plates were shaken for 1 hr at room temperature and the incubation was terminated by adding 50 µl (0.5 mg /well) of SPA yttrium silicate beads (RPNQ 0150; GE Healthcare) in the presence of zinc sulfate. Plates were stored overnight in the dark and read on a TRILUX microtiter plate reader (Perkin Elmer).

The results are shown in Table 1.

| **Examples** | **IC₅₀ (nM)** |
|---|---|
| 004 | 0.237 |
| 026 | 0.098 |
| 017 | 0.047 |
| 021 | 0.127 |
| 022 | 0.133 |
| 051 | 0.104 |
| 126 | 0.076 |
| 127 | 0.173 |
| 117 | 0.049 |
| 118 | 0.100 |
| 119 | 0.061 |
| 120 | 0.031 |
| 122 | 0.056 |
| 124 | 0.037 |
| 138 | 0.391 |

It can be seen from Table 1 that the compounds of formula (I) are very potent inhibitors of phosphodiesterase 4 (PDE 4). Preferred (3-oxo)pyridazin-4-ylurea derivatives of the invention possess an IC₅₀ value for the inhibition of PDE4 (determined as defined above) of less than 10 nM, preferably less than 1 nM, more preferably less than 0.5 nM being most preferably less than 0.1 nM. The compounds are also capable of blocking the production of some pro-inflammatory cytokines such as, for example, TNF .

Thus, they can be used in the treatment of allergic, inflammatory and immunological diseases, as well as those diseases or conditions where the blockade of pro-inflammatory cytokines or the selective inhibition of PDE 4 could be of benefit. These disease states include asthma, chronic obstructive pulmonary disease, allergic rhinitis, rheumatoid arthritis, osteoarthritis, osteoporosis, bone-formation disorders, glomerulonephritis, multiple sclerosis, ankylosing spondylitis, Graves ophtalmopathy, myasthenia gravis, diabetes insipidus, graft rejection, gastrointestinal disorders such as irritable bowel disease, ulcerative colitis or Crohn disease, septic shock, adult distress respiratory syndrome, and skin diseases such as atopic dermatitis, contact dermatitis, acute dermatomyositis and psoriasis. They can also be used as improvers of cerebrovascular function as well as in the treatment of other CNS related diseases such as dementia, Alzheimer's disease, depression, and as nootropic agents.

Like other PDE4 inhibitors (see references above) the compounds of the invention can also be used for blocking, after preventive and/or curative treatment, the erosive and ulcerogenic effects induced by a variety of etiological agents, such as antiinflammatory drugs (steroidal or non-steroidal antiinflammatory agents), stress, ammonia, ethanol and concentrated acids.

They can be used alone or in combination with antacids and/or antisecretory drugs in the preventive and/or curative treatment of gastrointestinal pathologies like drug-induced ulcers, peptic ulcers, H. Pylori-related ulcers, esophagitis and gastro-esophageal reflux disease.

They can also be used in the treatment of pathological situations where damage to the cells or tissues is produced through conditions like anoxia or the production of an excess of free radicals. Examples of such beneficial effects are the protection of cardiac tissue after coronary artery occlusion or the prolongation of cell and tissue viability when the compounds of the invention are added to preserving solutions intended for storage of transplant organs or fluids such as blood or sperm. They are also of benefit on tissue repair and wound healing.

The compounds of the present invention can also be used in combination with other drugs known to be effective in the treatment of these diseases for example (a) β2-adrenergic agonists, (b) anti-cholinergics, (c) anti-allergic agents, (d) ant-iinflammatory agents, (e) immunosuppressants, and (f) anti-infectives; for simultaneous, separate or sequential use in the treatment of the human or animal body.

Accordingly, another embodiment of the invention is the use of the compounds of formula (I) in the manufacture of a medicament for treatment or prevention of pathological conditions, diseases and disorders known to be susceptible of amelioration by inhibition of PDE4, as well as a method for treating a subject afflicted with a pathological condition or disease susceptible to amelioration by inhibition of PDE4, which comprises administering to said subject an effective amount of a compound of formula (I).

The present invention also provides pharmaceutical compositions which comprise, as an active ingredient, at least a (3-oxo)pyridazin-4-ylurea derivative of formula (I) or a pharmaceutically acceptable salt thereof in association with a pharmaceutically acceptable excipient such as a carrier or diluent. The active ingredient may comprise 0.001 % to 99% by weight, preferably 0.01 % to 90% by weight, of the composition depending upon the nature of the formulation and whether further dilution is to be made prior to application. Preferably the compositions are made up in a form suitable for oral, inhalation, topical, nasal, rectal, percutaneous or injectable administration.

The pharmaceutically acceptable excipients which are admixed with the active compound or salts of such compound, to form the compositions of this invention are well-known per se and the actual excipients used depend inter alia on the intended method of administering the compositions.

Compositions for oral administration may take the form of tablets, retard tablets, sublingual tablets, capsules, inhalation aerosols, inhalation solutions, dry powder inhalation, or liquid preparations, such as mixtures, elixirs, syrups or suspensions, all containing the compound of the invention; such preparations may be made by methods well-known in the art.

The diluents which may be used in the preparation of the compositions include those liquid and solid diluents which are compatible with the active ingredient, together with colouring or flavouring agents, if desired. Tablets or capsules may conveniently contain between 0.01-3000 mg, more preferably 0.5-1000 mg of active ingredient or the equivalent amount of a pharmaceutically acceptable salt thereof.

The liquid composition adapted for oral use may be in the form of solutions or suspensions. The solutions may be aqueous solutions of a soluble salt or other derivative of the active compound in association with, for example, sucrose to form a syrup. The suspensions may comprise an insoluble active compound of the invention or a pharmaceutically acceptable salt thereof in association with water, together with a suspending agent or flavouring agent.

Compositions for parenteral injection may be prepared from soluble salts, which may or may not be freeze-dried and which may be dissolved in pyrogen free aqueous media or other appropriate parenteral injection fluid.

Compositions for topical administration may take the form of ointments, creams or lotions, all containing the compound of the invention; such preparations may be made by methods well-known in the art.

Effective doses are normally in the range of 0.01-3000 mg, more preferably 0.5-1000 mg of active ingredient or the equivalent amount of a pharmaceutically acceptable salt thereof per day. Daily dosage may be administered in one or more treatments, preferably from 1 to 4 treatments, per day.

The pharmaceutical formulations may conveniently be presented in unit dosage form and may be prepared by any of the methods well known in the art of pharmacy.

Formulations of the present invention suitable for oral administration may be presented as discrete units such as capsules, cachets or tablets each containing a predetermined amount of the active ingredient; as a powder or granules; as a solution or a suspension in an aqueous liquid or a non-aqueous liquid; or as an oil- in-water liquid emulsion or a water-in-oil liquid emulsion. The active ingredient may also be presented as a bolus, electuary or paste.

A syrup formulation will generally consist of a suspension or solution of the compound or salt in a liquid carrier for example, ethanol, peanut oil, olive oil, glycerine or water with flavouring or colouring agent.

Where the composition is in the form of a tablet, any pharmaceutical carrier routinely used for preparing solid formulations may be used. Examples of such carriers include magnesium stearate, talc, gelatine, acacia, stearic acid, starch, lactose and sucrose.

A tablet may be made by compression or moulding, optionally with one or more accessory ingredients. Compressed tablets may be prepared by compressing in a suitable machine the active ingredient in a free-flowing form such as a powder or granules, optionally mixed with a binder, lubricant, inert diluent, lubricating, surface active or dispersing agent.

Moulded tablets may be made by moulding in a suitable machine a mixture of the powdered compound moistened with an inert liquid diluent. The tablets may optionally be coated or scored and may be formulated so as to provide slow or controlled release of the active ingredient therein.

Where the composition is in the form of a capsule, any routine encapsulation is suitable, for example using the aforementioned carriers in a hard gelatine capsule. Where the composition is in the form of a soft gelatine capsule any pharmaceutical carrier routinely used for preparing dispersions or suspensions may be considered, for example aqueous gums, celluloses, silicates or oils, and are incorporated in a soft gelatine capsule.

Dry powder compositions for topical delivery to the lung by inhalation may, for example, be presented in capsules and cartridges of for example gelatine or blisters of for example laminated aluminium foil, for use in an inhaler or insufflator. Formulations generally contain a powder mix for inhalation of the compound of the invention and a suitable powder base (carrier substance) such as lactose or starch. Use of lactose is preferred. Each capsule or cartridge may generally contain between 0.001-50 mg, more preferably 0.01-5 mg of active ingredient or the equivalent amount of a pharmaceutically acceptable salt thereof. Alternatively, the active ingredient (s) may be presented without excipients.

Packaging of the formulation may be suitable for unit dose or multi-dose delivery. In the case of multi- dose delivery, the formulation can be pre-metered or metered in use. Dry powder inhalers are thus classified into three groups: (a) single dose, (b) multiple unit dose and (c) multi dose devices.

For inhalers of the first type, single doses have been weighed by the manufacturer into small containers, which are mostly hard gelatine capsules. A capsule has to be taken from a separate box or container and inserted into a receptacle area of the inhaler. Next, the capsule has to be opened or perforated with pins or cutting blades in order to allow part of the inspiratory air stream to pass through the capsule for powder entrainment or to discharge the powder from the capsule through these perforations by means of centrifugal force during inhalation. After inhalation, the emptied capsule has to be removed from the inhaler again. Mostly, disassembling of the inhaler is necessary for inserting and removing the capsule, which is an operation that can be difficult and burdensome for some patients.

Other drawbacks related to the use of hard gelatine capsules for inhalation powders are (a) poor protection against moisture uptake from the ambient air, (b) problems with opening or perforation after the capsules have been exposed previously to extreme relative humidity, which causes fragmentation or indenture, and (c) possible inhalation of capsule fragments. Moreover, for a number of capsule inhalers, incomplete expulsion has been reported (e. g. Nielsen et al, 1997).

Some capsule inhalers have a magazine from which individual capsules can be transferred to a receiving chamber, in which perforation and emptying takes place, as described in WO 92/03175. Other capsule inhalers have revolving magazines with capsule chambers that can be brought in line with the air conduit for dose discharge (e. g. WO91/02558 and GB 2242134). They comprise the type of multiple unit dose inhalers together with blister inhalers, which have a limited number of unit doses in supply on a disk or on a strip.

Blister inhalers provide better moisture protection of the medicament than capsule inhalers. Access to the powder is obtained by perforating the cover as well as the blister foil, or by peeling off the cover foil. When a blister strip is used instead of a disk, the number of doses can be increased, but it is inconvenient for the patient to replace an empty strip. Therefore, such devices are often disposable with the incorporated dose system, including the technique used to transport the strip and open the blister pockets.

Multi-dose inhalers do not contain pre-measured quantities of the powder formulation. They consist of a relatively large container and a dose measuring principle that has to be operated by the patient. The container bears multiple doses that are isolated individually from the bulk of powder by volumetric displacement. Various dose measuring principles exist, including rotatable membranes (Ex. EP0069715) or disks (Ex. GB 2041763; EP 0424790; DE 4239402 and EP 0674533), rotatable cylinders (Ex. EP 0166294; GB 2165159 and WO 92/09322) and rotatable frustums (Ex. WO 92/00771), all having cavities which have to be filled with powder from the container. Other multi dose devices have measuring slides (Ex. US 5201308 and WO 97/00703) or measuring plungers with a local or circumferential recess to displace a certain volume of powder from the container to a delivery chamber or an air conduit (Ex. EP 0505321, WO 92/04068 and WO 92/04928), or measuring slides such as the Genuiar® (formerly known as Novolizer SD2FL), which is described the following patent applications Nos: WO97/000703, WO03/000325 and WO03/061742.

Reproducible dose measuring is one of the major concerns for multi dose inhaler devices.

The powder formulation has to exhibit good and stable flow properties, because filling of the dose measuring cups or cavities is mostly under the influence of the force of gravity.

For reloaded single dose and multiple unit dose inhalers, the dose measuring accuracy and reproducibility can be guaranteed by the manufacturer. Multi dose inhalers on the other hand, can contain a much higher number of doses, whereas the number of handlings to prime a dose is generally lower.

Because the inspiratory air stream in multi-dose devices is often straight across the dose measuring cavity, and because the massive and rigid dose measuring systems of multi dose inhalers can not be agitated by this inspiratory air stream, the powder mass is simply entrained from the cavity and little de-agglomeration is obtained during discharge.

Consequently, separate disintegration means are necessary. However in practice, they are not always part of the inhaler design. Because of the high number of doses in multi-dose devices, powder adhesion onto the inner walls of the air conduits and the de-agglomeration means must be minimized and/or regular cleaning of these parts must be possible, without affecting the residual doses in the device. Some multi dose inhalers have disposable drug containers that can be replaced after the prescribed number of doses has been taken (Ex. WO 97/000703). For such semi-permanent multi dose inhalers with disposable drug containers, the requirements to prevent drug accumulation are even more strict.

Apart from applications through dry powder inhalers the compositions of the invention can be administered in aerosols which operate via propellant gases or by means of so-called atomisers, via which solutions of pharmacologically-active substances can be sprayed under high pressure so that a mist of inhalable particles results. The advantage of these atomisers is that the use of propellant gases can be completely dispensed with. Such atomiser is the respimat® which is described, for example, in PCT Patent Applications Nos. W0 91/14468 and WO 97/12687, reference here is being made to the contents thereof.

Spray compositions for topical delivery to the lung by inhalation may for example be formulated as aqueous solutions or suspensions or as aerosols delivered from pressurised packs, such as a metered dose inhaler, with the use of a suitable liquefied propellant. Aerosol compositions suitable for inhalation can be either a suspension or a solution and generally contain the active ingredient (s) and a suitable propellant such as a fluorocarbon or hydrogen-containing chlorofluorocarbon or mixtures thereof, particularly hydrofluoroalkanes, e. g. dichlorodifluoromethane, trichlorofluoromethane, dichlorotetrafluoroethane, especially 1,1, 1, 2-tetrafluoroethane, 1,1, 1,2, 3,3, 3-heptafluoro-n-propane or a mixture thereof. Carbon dioxide or other suitable gas may also be used as propellant.

The aerosol composition may be excipient free or may optionally contain additional formulation excipients well known in the art such as surfactants eg oleic acid or lecithin and cosolvens eg ethanol. Pressurised formulations will generally be retained in a canister (eg an aluminium canister) closed with a valve (eg a metering valve) and fitted into an actuator provided with a mouthpiece.

Medicaments for administration by inhalation desirably have a controlled particle size. The optimum particle size for inhalation into the bronchial system is usually 1-10µ, preferably 2-5µ. Particles having a size above 20µ are generally too large when inhaled to reach the small airways. To achieve these particle sizes the particles of the active ingredient as produced may be size reduced by conventional means eg by micronisation. The desired fraction may be separated out by air classification or sieving. Preferably, the particles will be crystalline.

Achieving high dose reproducibility with micronised powders is difficult because of their poor flowability and extreme agglomeration tendency. To improve the efficiency of dry powder compositions, the particles should be large while in the inhaler, but small when discharged into the respiratory tract. Thus, an excipient such as lactose or glucose is generally employed. The particle size of the excipient will usually be much greater than the inhaled medicament within the present invention. When the excipient is lactose it will typically be present as milled lactose, preferably crystalline alpha lactose monohydrate. Pressurized aerosol compositions will generally be filled into canisters fitted with a valve, especially a metering valve. Canisters may optionally be coated with a plastics material e. g. a fluorocarbon polymer as described in W096/32150. Canisters will be fitted into an actuator adapted for buccal delivery.

Typical compositions for nasal delivery include those mentioned above for inhalation and further include non-pressurized compositions in the form of a solution or suspension in an inert vehicle such as water optionally in combination with conventional excipients such as buffers, anti-microbials, tonicity modifying agents and viscosity modifying agents which may be administered by nasal pump.

Typical dermal and transdermal formulations comprise a conventional aqueous or non-aqueous vehicle, for example a cream, ointment, lotion or paste or are in the form of a medicated plaster, patch or membrane.

Preferably the composition is in unit dosage form, for example a tablet, capsule or metered aerosol dose, so that the patient may administer a single dose.

The amount of each active which is required to achieve a therapeutic effect will, of course, vary with the particular active, the route of administration, the subject under treatment, and the particular disorder or disease being treated.

The compounds, salts and/or pharmaceutical compositions according to the invention may also be used in combination with another therapeutically active agent, for example a β2-adrenoreceptor agonist, an anti-cholinergic agent, an anti-allergic, an anti-inflammatory agent, an immunosuppressant, or an anti-infective agent.

The amount of each active which is required to achieve a therapeutic effect will, of course, vary with the particular active, the route of administration, the subject under treatment, and the particular disorder or disease being treated.

The active ingredients may be administered from 1 to 6 times a day, sufficient to exhibit the desired activity. Preferably, the active ingredients are administered once or twice a day, most preferably once a day.

Examples of suitable β2-agonists that can be combined with PDE4 inhibitors are terbutaline sulphate, eformoterol fumarate, formoterol fumarate, bambuterol, procaterol hydrochloride, sibenadet hydrochloride, mabuterol hydrochloride, albuterol sulphate, salbutamol sulphate, salmeterol xinafoate, carmoterol hydrochloride, (R)-albuterol hydrochloride, Levalbuterol hydrochloride; Levosalbutamol hydrochloride; (-)-Salbutamol hydrochloride, (R,R)-Formoterol tartrate; Arformoterol tartrate, Bedoradrine sulphate, Indacaterol, Trantinterol hydrochloride, AZD-3199, GSK-159802; GSK-597901, GSK-678007, GSK-642444;GSK-961081; AR-C98955AA, Milveterol hydrochloride, BI-1744-CL, and compounds described in international patent applications Nos. WO2007/124898, WO2006/122788A1, WO2008/046598 and WO2008095720

Examples of suitable corticosteroids and glucocorticoids that can be combined with PDE4 inhibitors are prednisolone, methylprednisolone, dexamethasone, dexamethasone cipecilate, naflocort, deflazacort, halopredone acetate, budesonide, beclomethasone dipropionate, hydrocortisone, triamcinolone acetonide, fluocinolone acetonide, fluocinonide, clocortolone pivalate, methylprednisolone aceponate, dexamethasone palmitoate, tipredane, hydrocortisone aceponate, prednicarbate, alclometasone dipropionate, halometasone, methylprednisolone suleptanate, mometasone furoate, rimexolone, prednisolone farnesylate, ciclesonide, butixocort propionate, RPR-106541, deprodone propionate, fluticasone propionate, fluticasone furoate, halobetasol propionate, loteprednol etabonate, betamethasone butyrate propionate, flunisolide, prednisone, dexamethasone sodium phosphate, triamcinolone, betamethasone 17-valerate, betamethasone, betamethasone dipropionate, hydrocortisone acetate, hydrocortisone sodium succinate, prednisolone sodium phosphate and hydrocortisone probutate.

Examples of suitable M3 antagonists (anticholinergics) that can be combined with PDE4 inhibitors are tiotropium salts, oxitropium salts, flutropium salts, ipratropium salts, glycopyrronium salts, trospium salts, revatropate, espatropate, 3-[2-Hydroxy-2,2-bis(2-thienyl)acetoxy]-1-(3-phenoxypropyl)-1-azoniabicyclo[2.2.2]octane salts (in particular aclidinium salts, more preferably aclidinium bromide), 1-(2-Phenylethyl)-3-(9H-xanthen-9-ylcarbonyloxy)-1-azoniabicyclo[2.2.2]octane salts, 2-oxo-1,2,3,4-tetrahydroquinazoline-3-carboxylic acid endo-8-methyl-8-azabicyclo[3.2.1]oct-3-yl ester salts (DAU-5884), 3-(4-Benzylpiperazin-1-yl)-1-cyclobutyl-1-hydroxy-1-phenylpropan-2-one (NPC-14695), N-[1-(6-Aminopyridin-2-ylmethyl)piperidin-4-yl]-2(R)-[3,3-difluoro-1(R)-cyclopentyl]-2-hydroxy-2-phenylacetamide (J-104135), 2(R)-Cyclopentyl-2-hydroxy-N-[1-[4(S)-methylhexyl]piperidin-4-yl]-2-phenylacetamide (J-106366), 2(R)-Cyclopentyl-2-hydroxy-N-[1-(4-methyl-3-pentenyl)-4-piperidinyl]-2-phenylacetamide (J-104129), 1-[4-(2-Aminoethyl)piperidin-1-yl]-2(R)-[3,3-difluorocyclopent-1(R)-yl]-2-hydroxy-2-phenylethan-1-one (Banyu-280634), N-[N-[2-[N-[1-(Cyclohexylmethyl)piperidin-3(R)-ylmethyl]carbamoyl]ethyl]carbamoylmethyl]-3,3,3-triphenylpropionamide (Banyu CPTP), 2(R)-Cyclopentyl-2-hydroxy-2-phenylacetic acid 4-(3-azabicyclo[3.1.0]hex-3-yl)-2-butynyl ester (Ranbaxy 364057), UCB-101333, Merck's OrM3, 7-endo-(2-hydroxy-2,2-diphenylacetoxy)-9,9-dimethyl-3-oxa-9-azoniatricyclo[3.3.1.0(2,4)]nonane salts, 7-(2,2-diphenylpropionyloxy)-7,9,9-trimethyl-3-oxa-9-azoniatricyclo[3.3.1.0*2,4*]nonane salts, 7-hydroxy-7,9,9-trimethyl-3-oxa-9-azoniatricyclo[3.3.1.0*2,4*]nonane 9-methyl-9H-fluorene-9-carboxylic acid ester salts, all of them optionally in the form of their racemates, their enantiomers, their diastereomers and mixtures thereof, and optionally in the form of their pharmacologically-compatible acid addition salts. Among the salts chlorides, bromides, iodides and methanesulphonates are preferred.

Examples of suitable anti-allergic agents that can be combined with PDE4 inhibitors are anti-histamines (e.g. Methapyrilene, Mequitazine,Azelastine hydrochloride,Acrivastine, Emedastine difumarate; Emedastine fumarate, Loratadine, Cyproheptadine hydrochloride, Diphenhydramine hydrochloride, Doxepin hydrochloride, Promethazine hydrochloride, Levocabastine hydrochloride, Desloratadine, Cinnarizine, Setastine hydrochloride, Mizolastine, Ebastine, Cetirizine hydrochloride,Epinastine hydrochloride, Olopatadine hydrochloride, Bepotastine besilate,Triprolidine hydrochloride, Rupatadine fumarate, Fexofenadine hydrochloride, Levocetirizine dihydrochloride, Ketotifen, Azatadine maleate, Dimethindene maleate, Clemastine fumarate, Alcaftadine, Bilastine, Vapitadine hydrochloride, AZD-1744, GSK-1004723D, GSK-835726, SUN-1334H), CRTH antagonists (e.g. Ramatroban, AMG-009, OC-000459), or mast cell stabilizers (e.g. nedocromil, pemirolast potassium, chromoglycate sodium, suplatast tosilate)

Other possible combinations include, for example, a combination comprising a PDE4 inhibitor with another anti-inflammatory agent such as a non-steroidal anti-inflammatory drug (NSAID) such as a leukotriene antagonist (e.g. lbudilast, Pranlukast hydrate, Zafirlukast, Montelukast, Tipelukast), a FLAP inhibitor, a lipoxygenase inhibitor, a cyclooxygenase inhibitor (e.g. diclofenac, ibuprofen, celecoxib); an elastase inhibitor, a Syk kinase inhibitor or another PDE inhibitor (e.g. theophylline),.

Examples for suitable immunosupressants that can be combined with PDE4 inhibitors are picremolimus, tacromilus, cyclosporine A, leflunomide, methotrexate, anti-TNF agents and compounds described in International patent applications Nos. PCT/EP2008/006573 and WO2008/077639.

Examples for suitable anti-infectives that can be combined with PDE4 inhibitors are mupiricin, retapamulin, clotrimazole, ketoconazole and terbinafine.

Particularly preferred pharmaceutical composition according to the invention comprise a salt of formula (I) and a therapeutically effective amount of one or more additional therapeutic agents selected from the group consisting of mometasone furoate, ciclesonide, budesonide, fluticasone propionate, fluticasone furoate, betamethasone valerate, clobetasol propionate, tiotropium salts, glycopyrronium salts, 3-[2-Hydroxy-2,2-bis(2-thienyl)acetoxy]-1-(3-phenoxypropyl)-1-azoniabicyclo[2.2.2]octane salts (in particular aclidinium salts, preferably aclidinium bromide), 1-(2-Phenylethyl)-3-(9H-xanthen-9-ylcarbonyloxy)-1-azoniabicyclo[2.2.2]octane salts, formoterol, salmeterol, indacaterol, carmoterol, compounds described in International patent applications Nos. PCT/EP2008/006573 and WO2008/077639, methapyrilene, cetirizine, loratadine, ebastine, desloratadine, fexofenadine, azelastine, levocabastine, olopatadine, Montelukast, picremolimus, tacromilus, mupiricin, retapamulin, clotrimazole, ketoconazole, terbinafine.

Thus, in one aspect of the invention, the composition comprises a compound of formula (I) and a corticosteroid. Particularly preferred corticosteroids are those selected from the group consisting of mometasone furoate, ciclesonide, budesonide, fluticasone furoate, fluticasone propionate, betamethasone valerate and clobetasol propionate.

In another aspect of the invention, the composition comprises a compound of formula (I) and an anticholinergic agent. Particulary preferred anticholinergic agents are those selected from the group consisting of tiotropium salts, glycopirronium salts, 3-[2-Hydroxy-2,2-bis(2-thienyl)acetoxy]-1-(3-phenoxypropyl)-1-azoniabicyclo[2.2.2]octane salts and 1-(2-Phenylethyl)-3-(9H-xanthen-9-ylcarbonyloxy)-1-azoniabicyclo[2.2.2]octane salts. The composition may further comprise a corticosteroid selected from the group consisting of mometasone furoate, ciclesonide, budesonide, fluticasone furoate and fluticasone propionate.

In a still other aspect of the invention, the composition comprises a compound of formula (I) and a suitable β2-agonist. Particularly preferred β2-agonists are those selected from the group consisting of formoterol, salmeterol, indacaterol, carmoterol and compound described in International patent applications Nos. WO2007/124898, WO2006/122788A1, WO2008/046598 and WO2008095720. The composition may further comprise a corticosteroid selected from the group consisting of mometasone furoate, ciclesonide, budesonide, fluticasone furoate, and fluticasone propionate. In addition to the salt of the invention and to the β2-agonist, the composition may further comprise an anticholinergic agent selected from the group consisting of tiotropium salts, glycopyrronium salts, 3-[2-Hydroxy-2,2-bis(2-thienyl)acetoxy]-1-(3-phenoxypropyl)-1-azoniabicyclo[2.2.2] octane salts and 1-(2-Phenylethyl)-3-(9H-xanthen-9-ylcarbonyloxy)-1-azoniabicyclo[2.2.2] octane salts.

The compounds of formula (I) and the combinations of the invention may be used in the treatment of respiratory, skin and inflammatory diseases, wherein the use of a PDE4 inhibitor is expected to have a beneficial effect, for example asthma, chronic obstructive pulmonary disease, allergic rhinitis, rheumatoid arthritis, multiple sclerosis, atopic dermatitis, psoriasis, inflammatory bowel disease.

The active compounds in the combination may be administered together in the same pharmaceutical composition or in different compositions intended for separate, simultaneous, concomitant or sequential administration by the same or a different route.

It is contemplated that all active agents would be administered at the same time, or very close in time. Alternatively, one or two actives could be taken in the morning and the other (s) later in the day. Or in another scenario, one or two actives could be taken twice daily and the other (s) once daily, either at the same time as one of the twice-a-day dosing occurred, or separately. Preferably at least two, and more preferably all, of the actives would be taken together at the same time. Preferably, at least two, and more preferably all actives would be administered as an admixture.

The active substance compositions according to the invention are preferably administered in the form of compositions for inhalation delivered with the help of inhalers, especially dry powder inhalers; however, any other form of nasal, topical, parenteral or oral application is possible. Here, the application of inhaled compositions embodies the preferred application form, especially in the therapy of obstructive lung diseases or for the treatment of asthma.

Additional suitable carriers for formulations of the active salts of the present invention can be found in Remington: The Science and Practice of Pharmacy, 20th Edition, Lippincott Williams & Wilkins, Philadelphia, Pa., 2000. The following non-limiting examples illustrate representative pharmaceutical compositions of the invention.

When combinations of actives are used, it is contemplated that all active agents would be administered at the same time, or very close in time. Alternatively, one or two actives could be taken in the morning and the other (s) later in the day. Or in another scenario, one or two actives could be taken twice daily and the other (s) once daily, either at the same time as one of the twice-a-day dosing occurred, or separately. Preferably at least two, and more preferably all, of the actives would be taken together at the same time. Preferably, at least two, and more preferably all actives would be administered as an admixture.

The following preparations forms are cited as formulation examples:

### COMPOSITION EXAMPLE 1

50,000 capsules, each containing 100 mg of N-{6-[3-(Benzyloxy)phenyl]-2-ethyl-3-oxo-2,3-dihydropyridazin-4-yl}-N'-(3,5-dichloropyridin-4-yl)urea (active ingredient), were prepared according to the following formulation:

| | |
|---|---|
| Active ingredient | 5 Kg |
| Lactose monohydrate | 10 Kg |
| Colloidal silicon dioxide | 0.1 Kg |
| Corn starch | 1 Kg |
| Magnesium stearate | 0.2 Kg |

### Procedure

The above ingredients were sieved through a 60 mesh sieve, and were loaded into a suitable mixer and filled into 50,000 gelatine capsules.

### COMPOSITION EXAMPLE 2

50,000 tablets, each containing 50 mg of N-{6-[3-(Benzyloxy)phenyl]-2-ethyl-3-oxo-2,3-dihydropyridazin-4-yl}-N'-(3,5-dichloropyridin-4-yl)urea (active ingredient), were prepared from the following formulation:

| | |
|---|---|
| Active ingredient | 2.5 Kg |
| Microcrystalline cellulose | 1.95 Kg |
| Spray dried lactose | 9.95 Kg |
| Carboxymethyl starch | 0.4 Kg |
| Sodium stearyl fumarate | 0.1 Kg |
| Colloidal silicon dioxide | 0.1 Kg |

### Procedure

All the powders were passed through a screen with an aperture of 0.6 mm, then mixed in a suitable mixer for 20 minutes and compressed into 300 mg tablets using 9 mm disc and flat bevelled punches. The disintegration time of the tablets was about 3 minutes.

## Claims

1. A compound of formula (I): 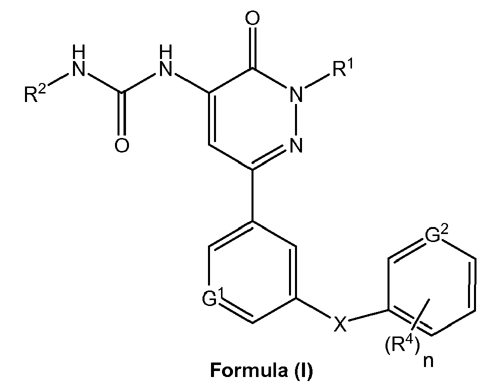 wherein:
• R¹ is selected from the group consisting of a -(C₁₋₄ alkyl)-(C₃₋₅ cycloalkyl) group and a C₁₋₄ alkyl group optionally substituted by one or more halogen atoms,
• R² is selected from the group consisting of a C₅₋₁₀ aryl group and a 5 to 10- membered heteroaryl group containing at least one heteroatom selected from N, S and O, wherein the aryl and the heteroaryl group are optionally substituted by one or more substituents selected from halogen atoms, C₁₋₄ alkyl groups and C₁₋₄.alkoxy groups,
• X is selected from the group consisting of a direct bond, -O-, -O-CH₂-, -S-, and - S(O)₂-,
• G¹ is selected from the group consisting of a nitrogen atom and a -CH= group,
• G² is selected from the group consisting of a nitrogen atom and a -CR³= group,
• R³ and R⁴ independently are selected from the group consisting of a hydrogen atom, a halogen atom, a hydroxy group, a cyano group, a linear or branched C₁₋₄ alkyl group, a linear or branched C₁₋₄ hydroxyalkyl group, a methylsulphonyl group, a sulphonyloxy group, a trifluoromethyl group, a trifluoromethoxy group, a cyclopropyloxy group, a formyl group, a -CH₂N(CH₃)₂ group, -O-(CH₂)₍₁₋₃₎-R^{b}, -O-(CH₂)_{(1- 5)}NR^{a}R^{b}, -(CH₂)₍₀₋₄₎-C(O)OR^{b}, -(CH₂)₍₀₋₄₎-C(O)NR^{a}R^{b}, -(CH₂)₍₀₋₂₎-CO-NH-(CH₂)₍₁₋₃₎-R^{b} and -(CH₂)₍₀₋₂)-CO-NH-(CH₂)₍₁₋₈₎-NR^{a}R^{b} wherein
○ R^{a} represents a hydrogen atom or a C₁₋₄ alkyl group,
○ R^{b} represents a hydrogen atom, a C₁₋₄ alkyl group, a C₁₋₄ carboxyalkyl group, a C₁₋₂ hydroxyalkyl group, a carbamoyl group, a monocyclic or polycyclicC₃₋₁₀ cycloalkyl group, a monocyclic or polycyclic unsaturated non-aromatic C₃₋₁₀ carbocyclic group, a monocylic or polycyclic C₅₋₁₀ aryl group, a monocylic or polycyclic 5- to 10- membered heteroaryl group or a monocyclic or polycyclic 5- to 10- membered heterocyclic group, which cycloalkyl, unsaturated non-aromatic carboycyclic, aryl, heteroaryl and heterocyclic groups are optionally substituted by one or more substituents selected from the group consisting of halogen atoms, hydroxy groups, benzyl groups, C₁₋₂ alkyl groups, C₁₋₆ hydroxyalkyl groups, C₁₋₄ dialkylamino groups or carboxy groups, or
○ R^{a} and R^{b} together with the nitrogen atom to which they are attached form a 4 to 6 membered saturated or unsaturated heterocyclic ring optionally containing one additional heteroatom selected from N and O, and optionally substituted with a C₁₋₄ alkyl group; a carbamoyl group or a C₁₋₂alkoxy(C₁₋₂)alkyl group;
n is integer from 1 to 3or pharmaceutically acceptable salts and N-oxides thereof.
or pharmaceutically acceptable salts and N-oxides thereof.

2. A compound according to claim 1, wherein R¹ represents a C₁₋₄ alkyl group optionally substituted with one or more halogen atoms.

3. A compound according to claim 2 wherein R¹ represents a methyl or ethyl group.

4. A compound according to claim 3, wherein R¹ represents an ethyl group

5. A compound according to any one of claims 1 to 4, wherein R² represents a 5 to 10 membered N- containing heteroaryl group optionally substituted by one or more substituents selected from halogen atoms and C₁₋₄ alkyl groups.

6. A compound according to claim 5, wherein R² is a pyridyl group optionally substituted by one or two substituents selected from fluorine atoms, chlorine atoms and methyl groups.

7. A compound according to claim 6, wherein R² is a pyridyl group substituted by two substituents selected from chlorine atoms and methyl groups.

8. A compound according to claim 7, wherein R² is a pyridyl group substituted by two chlorine atoms.

9. A compound according to any one of claims 1-8, wherein X is selected from the group consisting of a direct bond, -O-, -S-, and -S(O)₂.

10. A compound according to claim 9, wherein X is a direct bond.

11. A compound according to any one of claims 1 to 10, wherein G¹ represents a -CH= group.

12. A compound according to any one of claims 1 to 11, wherein G² represents a -CR³= group.

13. A compound according to any one of claims 1-12, wherein R³ and R⁴ independently are selected from the group consisting of hydrogen atom, fluorine atom, chlorine atom, hydroxy group, cyano group, a methyl group, a hydroxyethyl group, a trifluoromethoxy group, -O-(CH₂)₍₁₋₃₎-R^{b}, -O-(CH₂)₍₂₋₅₎NR^{a}R^{b}, -(CH₂)₍₀₋₂₎-C(O)OR^{b}, - (CH₂)₍₀₋₂₎-C(O)NR^{a}R^{b}, -(CH₂)₍₀₋₂₎-CO-NH-(CH₂)₍₁₋₃₎-R^{b} and -(CH₂)₍₀₋₂₎-CO-NH-(CH₂)₍₂₋₇₎-NR^{a}R^{b}, wherein:
○ R^{b} represents a hydrogen atom, a C₁₋₄ alkyl group, a C₁₋₂ hydroxyalkyl group, a C₃₋₅ cycloalkyl group, a phenyl group, a pirrolidin-2-yl group, a piperidin-4-yl group, werein the cyclic groups optionally are substituted by one or two substituents selected from the group consisting of chlorine atoms, hydroxy groups, benzyl group, C₁₋₂ alkyl groups, C₂₋₅ hydroxyalkyl groups and C₁₋₂ dialkylamino groups, or
○ R^{a} and R^{b} together with the nitrogen atom to which they are attached from a 5 to 6 membered saturated or non-saturated heterocyclic ring optionally containing one additional heteroatom selected from N and O, and optionally substituted with a C₁₋₂ alkyl group, a carbamoyl group or a C₁₋₂alkoxy(C₁₋₂)alkyl group,

14. A compound according to claim 13, wherein R³ and R⁴ independently are selected from the group consisting of hydrogen atom, fluorine atom, hydroxy group, cyano group, a trifluoromethoxy group, -O-(CH₂)₍₁₋₃₎-R^{b}, -O-(CH₂)₍₂₋₄₎NR^{a}R^{b}, -(CH₂)₍₀₋₁₎-C(O)OR^{b}, -(CH₂)₍₀₋₁₎-C(O)NR^{a}R^{b}, -(CH₂)₍₀₋₁₎-CO-NH-(CH₂)₍₁₋₃₎-R^{b} and -(CH₂)₍₀₋₂₎-CO-NH-(CH₂)₍₂₋₇₎-NR^{a}R^{b}, wherein:
○ R^{b} represents a hydrogen atom, a C₁₋₄ alkyl group, a C₁₋₂ hydroxyalkyl group, a C₃₋₅ cycloalkyl group, a phenyl group, a pirrolidin-2-yl group, a piperidin-4-yl group, werein the cyclic groups optionally are substituted by one or more substituents selected from the group consisting of halogen atoms, hydroxy groups, benzyl groups, C₁₋₂ alkyl groups, C₁₋₅ hydroxyalkyl groups, C₁₋₂ dialkylamino groups or carboxy groups, or
○ R^{a} and R^{b} together with the nitrogen atom to which they are attached from a 5 to 6 membered saturated or non-saturated heterocyclic ring optionally containing one additional heteroatom selected from N and O, and optionally substituted with a C₁₋₂ alkyl group, a carbamoyl group or a C₁₋₂alkoxy(C₁₋₂)alkyl

15. A compound according to claim 14, wherein R³ and R⁴ independently are selected from the group consisting of -O-(CH₂)₍₁₋₂₎-R^{b}, -O-(CH₂)₍₂₋₅₎NR^{a}R^{b}, -C(O)OR^{b}, - C(O)NR^{a}R^{b}, -CO-NH-(CH₂)₍₁₋₃₎-R^{b} and -CO-NH-(CH₂)₍₂₋₄₎-NR^{a}R^{b}, wherein
○ R^{a} represents a hydrogen atom, a methyl group or an ethyl group,
○ R^{b} represents a hydrogen atom, a methyl group, a hydroxyethyl group, a cyclopropyl group, a phenyl group, a pirrolidin-2-yl group or a piperidin-4-yl group, werein the cyclic groups optionally are substituted by one substituent selected from the group consisting of hydroxy group, a methyl groups, a benzyl group and a hydroxyethyl group, or
○ R^{a} and R^{b} together with the nitrogen atom to which they are attached from a 5 or 6 membered saturated or non-saturated heterocyclic ring optionally containing one additional heteroatom selected from N and O, and optionally substituted with a methyl group, cabamoyl or a methoxyethyl group,

16. A compound according to claim 15, wherein n has a value of 1

17. A compound according to any one of claims 1 to 16, wherein R¹ represents an ethyl group, R² is a pyridyl group substituted by two chlorine atoms, X is a direct bond, G¹ represents a -CH= group, G² represents a -CR³= group, n has a value of 1, R³ and R⁴ independently are selected from the group consisting of -O-(CH₂)₍₁₋₂₎-R^{b}, -O-(CH₂)₍₂₋₅₎NR^{a}R^{b}, -C(O)OR^{b}, -C(O)NR^{a}R^{b}, -CO-NH-(CH₂)₍₁₋₃₎-R^{b} and -CO-NH-(CH₂)₍₂₋₄₎-NR^{a}R^{b}, wherein
○ R^{a} represents a hydrogen atom, a methyl group or an ethyl group,
○ R^{b} represents a hydrogen atom, a methyl group, a hydroxyethyl group, a cyclopropyl group, a phenyl group, a pirrolidin-2-yl group or a piperidin-4-yl group, werein the cyclic groups optionally are substituted by one substituent selected from the group consisting of hydroxy group, a methyl groups, a benzyl group and a hydroxyethyl group, or
○ R^{a} and R^{b} together with the nitrogen atom to which they are attached from a 5 or 6 membered saturated or non-saturated heterocyclic ring optionally containing one additional heteroatom selected from N and O, and optionally substituted with a methyl group, a carbamoyl or a methoxyethyl group.

18. A compound according to claim 1, wherein:
R¹ represents an C₁-C₂ alkyl group which is unsubstituted or substituted by 1 or 2 halogen atoms or a -(C₁₋₂ alkyl)-(cyclopropyl) group,
R² represents a pyridyl group which is unsubstituted or substituted by two substituents independently selected from halogen atoms and methyl groups,
X represents a direct bond, -O-, -O-CH₂-, -S-, or -S(O)₂-,
G¹ represents a nitrogen atom or a -CH= group,
G² represents a nitrogen atom or a -CR³= group,
R³ and R⁴ are independently selected from a hydrogen atom, a fluorine atom, a chlorine atom, hydroxy group, a cyano group, a methyl group, a linear C₁₋₃ hydroxyalkyl group, a methylsulphonyl group, a sulphonyloxy group, a trifluoromethyl group, a trifluoromethoxy group, a cyclopropyloxy group, a formyl group, a -CH₂N(CH₃)₂ group, -O-(CH₂)₍₁₋₃)-R^{b}, -O-(CH₂)₍₂₋₅₎NR^{a}R^{b}, -(CH₂)₍₀₋₂₎-C(O)OR^{b}, -(CH₂)₍₀₋₂₎-C(O)NR^{a}R^{b}, -(CH₂)₍₀₋₂₎-CO-NH-(CH₂)₍₁₋₂₎-R^{b} and -(CH₂)₍₀₋₂₎-CO-NH-(CH₂)₍₂₋₇₎-NR^{a}R^{b} wherein
○ R^{a} represents a hydrogen atom or a methyl or ethyl group,
○ R^{b} represents a hydrogen atom, a C₁₋₄ alkyl group, a -CH₂COOH group, a C₁₋₂ hydroxyalkyl group, a carbamoyl group, a pyrrolidin-2-yl group, a piperidin-4-yl group, an adamantyl group, a pyridinyl group, a phenyl group, which pyrrolidin-2-yl, a piperidin-4-yl, adamantyl, pyridinyl and phenyl groups are optionally substituted by one or two substituents selected from chlorine atoms, hydroxy groups, benzyl groups, methyl groups, hydroxyethyl groups and carboxy groups, or
○ R^{a} and R^{b} together with the nitrogen atom to which they are attached form a 5 or 6 membered saturated or unsaturated heterocyclic ring optionally containing one additional heteroatom selected from N and O, and optionally substituted with a methyl group; a carbamoyl group or a - CH₂OCH₃ group; and
n is 1 or 2.

19. A compound according to claim 1 which is one of:
N-(3,5-Dichloropyridin-4-yl)-N'-[2-ethyl-6-(3'-hydroxybiphenyl-3-yl)-3-oxo-2,3-dihydropyridazin-4-yl]urea
N-(3,5-Dichloropyridin-4-yl)-N'-[2-ethyl-6-(4'-hydroxybiphenyl-3-yl)-3-oxo-2,3-dihydropyridazin-4-yl]urea
N-(3,5-Dichloropyridin-4-yl)-N'-[2-ethyl-6-(2'-hydroxybiphenyl-3-yl)-3-oxo-2,3-dihydropyridazin-4-yl]urea
N-(3,5-Dichloropyridin-4-yl)-N'-[2-ethyl-6-(5'-fluoro-2'-hydroxybiphenyl-3-yl)-3-oxo-2,3-dihydropyridazin-4-yl]urea
N-(3,5-Dichloropyridin-4-yl)-N'-[2-ethyl-6-(2'-fluoro-4'-hydroxybiphenyl-3-yl)-3-oxo-2,3-dihydropyridazin-4-yl]urea
N-(3,5-Dichloropyridin-4-yl)-N'-[2-ethyl-6-(3'-fluoro-4'-hydroxybiphenyl-3-yl)-3-oxo-2,3-dihydropyridazin-4-yl]urea
N-(3,5-Dichloropyridin-4-yl)-N'-[2-ethyl-6-(2'-hydroxy-5'-methylbiphenyl-3-yl)-3-oxo-2,3-dihydropyridazin-4-yl]urea
N-(3,5-Dichloropyridin-4-yl)-N'-[2-ethyl-6-(2'-fluoro-3'-hydroxybiphenyl-3-yl)-3-oxo-2,3-dihydropyridazin-4-yl]urea
N-(3,5-Dichloropyridin-4-yl)-N'-[2-ethyl-6-(4'-hydroxy-2'-methylbiphenyl-3-yl)-3-oxo-2,3-dihydropyridazin-4-yl]urea
N-(3,5-Dichloropyridin-4-yl)-N'-{2-ethyl-6-[4'-(hydroxymethyl)biphenyl-3-yl]-3-oxo-2,3-dihydropyridazin-4-yl}urea
N-(3,5-Dichloropyridin-4-yl)-N'-[2-ethyl-6-(4'-fluoro-2'-hydroxybiphenyl-3-yl)-3-oxo-2,3-dihydropyridazin-4-yl]urea
N-(6-Biphenyl-3-yl-2-ethyl-3-oxo-2,3-dihydropyridazin-4-yl)-N'-(3,5-dichloropyridin-4-yl)urea
N-(3,5-Dichloropyridin-4-yl)-N'-{2-ethyl-6-[4'-(1-hydroxy-1-methylethyl)biphenyl-3-yl]-3-oxo-2,3-dihydropyridazin-4-yl}urea
N-(3,5-Dichloropyridin-4-yl)-N'-{2-ethyl-6-[3'-(hydroxymethyl)biphenyl-3-yl]-3-oxo-2,3-dihydropyridazin-4-yl}urea
3'-[5-({[(3,5-Dichloropyridin-4-yl)amino]carbonyl}amino)-1-ethyl-6-oxo-1,6-dihydropyridazin-3-yl]-4-hydroxybiphenyl-3-carboxamide
3'-[5-({[(3,5-Dichloropyridin-4-yl)amino]carbonyl}amino)-1-ethyl-6-oxo-1,6-dihydropyridazin-3-yl]biphenyl-3-carboxamide
3'-[5-({[(3,5-Dichloropyridin-4-yl)amino]carbonyl}amino)-1-ethyl-6-oxo-1,6-dihydropyridazin-3-yl]-N-[2-(dimethylamino)ethyl]biphenyl-3-carboxamide
N-(3,5-Dichloropyridin-4-yl)-N'-{2-ethyl-6-[5-(3-hydroxyphenyl)pyridin-3-yl]-3-oxo-2,3-dihydropyridazin-4-yl}urea
2-{3'-[5-({[(3,5-Dichloropyridin-4-yl)amino]carbonyl}amino)-1-ethyl-6-oxo-1,6-dihydropyridazin-3-yl]biphenyl-3-yl}acetamide
3-{5-[5-({[(3,5-Dichloropyridin-4-yl)amino]carbonyl}amino)-1-ethyl-6-oxo-1,6-dihydropyridazin-3-yl]pyridin-3-yl}benzamide
N-Cyclopropyl-3'-[5-({[(3,5-dichloropyridin-4-yl)amino]carbonyl}amino)-1-ethyl-6-oxo-1,6-dihydropyridazin-3-yl]biphenyl-3-carboxamide
3'-[5-({[(3,5-Dichloropyridin-4-yl)amino]carbonyl}amino)-1-ethyl-6-oxo-1,6-dihydropyridazin-3-yl]-N-[3-(dimethylamino)propyl]biphenyl-3-carboxamide
3'-[5-({[(3,5-Dichloropyridin-4-yl)amino]carbonyl}amino)-1-ethyl-6-oxo-1,6-dihydropyridazin-3-yl]-6-hydroxybiphenyl-3-carboxamide
3'-[5-({[(3,5-Dichloropyridin-4-yl)amino]carbonyl}amino)-1-ethyl-6-oxo-1,6-dihydropyridazin-3-yl]-N-[2-(dimethylamino)ethyl]-3-hydroxybiphenyl-4-carboxamide
Ethyl 3'-[5-({[(3,5-dichloropyridin-4-yl)amino]carbonyl}amino)-1-ethyl-6-oxo-1,6-dihydropyridazin-3-yl]biphenyl-4-carboxylate
3'-[5-({[(3,5-Dichloropyridin-4-yl)amino]carbonyl}amino)-1-ethyl-6-oxo-1,6-dihydropyridazin-3-yl]biphenyl-4-carboxylic acid
Methyl 5-{3-[5-({[(3,5-dichloropyridin-4-yl)amino]carbonyl}amino)-1-ethyl-6-oxo-1,6-dihydropyridazin-3-yl]phenyl}nicotinate
5-{3-[5-({[(3,5-Dichloropyridin-4-yl)amino]carbonyl}amino)-1-ethyl-6-oxo-1,6-dihydropyridazin-3-yl]phenyl}nicotinic acid
Methyl 3'-[5-({[(3,5-dichloropyridin-4-yl)amino]carbonyl}amino)-1-ethyl-6-oxo-1,6-dihydropyridazin-3-yl]-4-fluorobiphenyl-3-carboxylate
3'-[5-({[(3,5-Dichloropyridin-4-yl)amino]carbonyl}amino)-1-ethyl-6-oxo-1,6-dihydropyridazin-3-yl]-4-fluorobiphenyl-3-carboxylic acid
Ethyl 3'-[5-({[(3,5-dichloropyridin-4-yl)amino]carbonyl}amino)-1-ethyl-6-oxo-1,6-dihydropyridazin-3-yl]biphenyl-2-carboxylate
3'-[5-({[(3,5-Dichloropyridin-4-yl)amino]carbonyl}amino)-1-ethyl-6-oxo-1,6-dihydropyridazin-3-yl]biphenyl-2-carboxylic acid
Methyl 3'-(5-(3-(3,5-dichloropyridin-4-yl)ureido)-1-ethyl-6-oxo-1,6-dihydropyridazin-3-yl)-6-fluorobiphenyl-3-carboxylate
3'-[5-({[(3,5-Dichloropyridin-4-yl)amino]carbonyl}amino)-1-ethyl-6-oxo-1,6-dihydropyridazin-3-yl]-6-fluorobiphenyl-3-carboxylic acid
Methyl 3'-(5-(3-(3,5-dichloropyridin-4-yl)ureido)-1-ethyl-6-oxo-1,6-dihydropyridazin-3-yl)-6-methoxybiphenyl-3-carboxylate
3'-[5-({[(3,5-Dichloropyridin-4-yl)amino]carbonyl}amino)-1-ethyl-6-oxo-1,6-dihydropyridazin-3-yl]-6-methoxybiphenyl-3-carboxylic acid
Methyl 3'-[5-({[(3,5-dichloropyridin-4-yl)amino]carbonyl}amino)-1-ethyl-6-oxo-1,6-dihydropyridazin-3-yl]-4-hydroxybiphenyl-3-carboxylate
3'-[5-({[(3,5-Dichloropyridin-4-yl)amino]carbonyl}amino)-1-ethyl-6-oxo-1,6-dihydropyridazin-3-yl]-4-hydroxybiphenyl-3-carboxylic acid
Ethyl {3'-[5-({[(3,5-dichloropyridin-4-yl)amino]carbonyl}amino)-1-ethyl-6-oxo-1,6-dihydropyridazin-3-yl]biphenyl-4-yl}acetate
{3'-[5-({[(3,5-Dichloropyridin-4-yl)amino]carbonyl}amino)-1-ethyl-6-oxo-1,6-dihydropyridazin-3-yl]biphenyl-4-yl}aceticacid
Methyl {3'-[5-({[(3,5-dichloropyridin-4-yl)amino]carbonyl}amino)-1-ethyl-6-oxo-1,6-dihydropyridazin-3-yl]biphenyl-3-yl}acetate
{3'-[5-({[(3,5-Dichloropyridin-4-yl)amino]carbonyl}amino)-1-ethyl-6-oxo-1,6-dihydropyridazin-3-yl]biphenyl-3-yl}acetic acid
Ethyl 4-{5-[5-({[(3,5-dichloropyridin-4-yl)amino]carbonyl}amino)-1-ethyl-6-oxo-1,6-dihydropyridazin-3-yl]pyridin-3-yl}benzoate
4-{5-[5-({[(3,5-Dichloropyridin-4-yl)amino]carbonyl}amino)-1-ethyl-6-oxo-1,6-dihydropyridazin-3-yl]pyridin-3-yl}benzoic acid
Methyl 3'-[5-({[(3,5-dichloropyridin-4-yl)amino]carbonyl}amino)-1-ethyl-6-oxo-1,6-dihydropyridazin-3-yl]-6-hydroxybiphenyl-3-carboxylate
3'-[5-({[(3,5-Dichloropyridin-4-yl)amino]carbonyl}amino)-1-ethyl-6-oxo-1,6-dihydropyridazin-3-yl]-6-hydroxybiphenyl-3-carboxylic acid
Ethyl 3'-[5-({[(3,5-dichloropyridin-4-yl)amino]carbonyl}amino)-1-ethyl-6-oxo-1,6-dihydropyridazin-3-yl]biphenyl-3-carboxylate
3'-[5-({[(3,5-Dichloropyridin-4-yl)amino]carbonyl}amino)-1-ethyl-6-oxo-1,6-dihydropyridazin-3-yl]biphenyl-3-carboxylic acid
3'-[5-({[(3,5-Dichloropyridin-4-yl)amino]carbonyl}amino)-1-ethyl-6-oxo-1,6-dihydropyridazin-3-yl]-N-(2-morpholin-4-ylethyl)biphenyl-3-carboxamide
3'-[5-({[(3,5-Dichloropyridin-4-yl)amino]carbonyl}amino)-1-ethyl-6-oxo-1,6-dihydropyridazin-3-yl]-N-[3-(dimethylamino)propyl]biphenyl-4-carboxamide
3'-[5-({[(3,5-Dichloropyridin-4-yl)amino]carbonyl}amino)-1-ethyl-6-oxo-1,6-dihydropyridazin-3-yl]-N-(2-piperidin-1-ylethyl)biphenyl-3-carboxamide
3'-[5-({[(3,5-Dichloropyridin-4-yl)amino]carbonyl}amino)-1-ethyl-6-oxo-1,6-dihydropyridazin-3-yl]-N-[2-(methylamino)ethyl]biphenyl-3-carboxamide
N-(3,5-Dichloropyridin-4-yl)-N'-{2-ethyl-6-[3-(5-methoxypyridin-3-yl)phenyl]-3-oxo-2,3-dihydropyridazin-4-yl}urea
N-(3,5-Dichloropyridin-4-yl)-N'-{2-ethyl-6-[3-(5-hydroxypyridin-3-yl)phenyl]-3-oxo-2,3-dihydropyridazin-4-yl}urea
N-(3,5-Dichloropyridin-4-yl)-N'-{2-ethyl-6-[3-(6-methoxypyridin-3-yl)phenyl]-3-oxo-2,3-dihydropyridazin-4-yl}urea
N-(3,5-Dichloropyridin-4-yl)-N'-{2-ethyl-6-[3-(6-hydroxypyridin-3-yl)phenyl]-3-oxo-2,3-dihydropyridazin-4-yl}urea
N-[6-(5'-Chloro-2'-methoxybiphenyl-3-yl)-2-ethyl-3-oxo-2,3-dihydropyridazin-4-yl]-N'-(3,5-dichloropyridin-4-yl)urea
N-[6-(5'-Chloro-2'-hydroxybiphenyl-3-yl)-2-ethyl-3-oxo-2,3-dihydropyridazin-4-yl]-N'-(3,5-dichloropyridin-4-yl)urea
N-(3,5-Dichloropyridin-4-yl)-N'-[2-ethyl-6-(2'-fluoro-5'-methoxybiphenyl-3-yl)-3-oxo-2,3-dihydropyridazin-4-yl]urea
N-(3,5-Dichloropyridin-4-yl)-N'-[2-ethyl-6-(2'-fluoro-5'-hydroxybiphenyl-3-yl)-3-oxo-2,3-dihydropyridazin-4-yl]urea
N-[6-(2'-Chloro-4'-methoxybiphenyl-3-yl)-2-ethyl-3-oxo-2,3-dihydropyridazin-4-yl]-N'-(3,5-dichloropyridin-4-yl)urea
N-[6-(2'-Chloro-4'-hydroxybiphenyl-3-yl)-2-ethyl-3-oxo-2,3-dihydropyridazin-4-yl]-N'-(3,5-dichloropyridin-4-yl)urea
N-[6-(3'-Chloro-4'-methoxybiphenyl-3-yl)-2-ethyl-3-oxo-2,3-dihydropyridazin-4-yl]-N'-(3,5-dichloropyridin-4-yl)urea
N-[6-(3'-Chloro-4'-hydroxybiphenyl-3-yl)-2-ethyl-3-oxo-2,3-dihydropyridazin-4-yl]-N'-(3,5-dichloropyridin-4-yl)urea
N-(3,5-Dichloropyridin-4-yl)-N'-{2-ethyl-3-oxo-6-[3'-(3-pyrrolidin-1-ylpropoxy)biphenyl-3-yl]-2,3-dihydropyridazin-4-yl}urea
N-(3,5-Dichloropyridin-4-yl)-N'-{2-ethyl-6-[4'-(2-hydroxyethyl)biphenyl-3-yl]-3-oxo-2,3-dihydropyridazin-4-yl}urea
N-(3,5-Dichloropyridin-4-yl)-N'-{2-ethyl-6-[3-(3-methoxyphenoxy)phenyl]-3-oxo-2,3-dihydropyridazin-4-yl}urea
N-(3,5-Dichloropyridin-4-yl)-N'-{2-ethyl-6-[3-(3-hydroxyphenoxy)phenyl]-3-oxo-2,3-dihydropyridazin-4-yl}urea
N-(3,5-Dichloropyridin-4-yl)-N'-{2-ethyl-6-[3-(2-methoxyphenoxy)phenyl]-3-oxo-2,3-dihydropyridazin-4-yl}urea
N-(3,5-Dichloropyridin-4-yl)-N'-{2-ethyl-6-[3-(2-hydroxyphenoxy)phenyl]-3-oxo-2,3-dihydropyridazin-4-yl}urea
N-(3,5-Dichloropyridin-4-yl)-N'-{2-ethyl-6-[3-(4-methoxyphenoxy)phenyl]-3-oxo-2,3-dihydropyridazin-4-yl}urea
N-(3,5-Dichloropyridin-4-yl)-N'-{2-ethyl-6-[3-(4-hydroxyphenoxy)phenyl]-3-oxo-2,3-dihydropyridazin-4-yl}urea
N-(3,5-Dichloropyridin-4-yl)-N'-(2-ethyl-6-{3-[(3-methoxyphenyl)thio]phenyl}-3-oxo-2,3-dihydropyridazin-4-yl)urea
N-(3,5-Dichloropyridin-4-yl)-N'-(2-ethyl-6-{3-[(3-hydroxyphenyl)thio]phenyl}-3-oxo-2,3-dihydropyridazin-4-yl)urea
N-(3,5-Dichloropyridin-4-yl)-N'-(2-ethyl-6-{3-[(2-methoxyphenyl)thio]phenyl}-3-oxo-2,3-dihydropyridazin-4-yl)urea
N-(3,5-Dichloropyridin-4-yl)-N'-(2-ethyl-6-{3-[(2-hydroxyphenyl)thio]phenyl}-3-oxo-2,3-dihydropyridazin-4-yl)urea
N-(3,5-Dichloropyridin-4-yl)-N'-(2-ethyl-6-{3-[(4-methoxyphenyl)thio]phenyl}-3-oxo-2,3-dihydropyridazin-4-yl)urea
N-(3,5-Dichloropyridin-4-yl)-N'-(2-ethyl-6-{3-[(4-hydroxyphenyl)thio]phenyl}-3-oxo-2,3-dihydropyridazin-4-yl)urea
N-(3,5-Dichloropyridin-4-yl)-N'-(2-ethyl-6-{3-[(3-methoxyphenyl)sulfonyl]phenyl}-3-oxo-2,3-dihydropyridazin-4-yl)urea
N-(3,5-Dichloropyridin-4-yl)-N'-(2-ethyl-6-{3-[(3-hydroxyphenyl)sulfonyl]phenyl}-3-oxo-2,3-dihydropyridazin-4-yl)urea
N-(3,5-Dichloropyridin-4-yl)-N'-(2-ethyl-6-{3-[(2-methoxyphenyl)sulfonyl]phenyl}-3-oxo-2,3-dihydropyridazin-4-yl)urea
N-(3,5-Dichloropyridin-4-yl)-N'-(2-ethyl-6-{3-[(2-hydroxyphenyl)sulfonyl]phenyl}-3-oxo-2,3-dihydropyridazin-4-yl)urea
N-(3,5-Dichloropyridin-4-yl)-N'-(2-ethyl-6-{3-[(4-methoxyphenyl)sulfonyl]phenyl}-3-oxo-2,3-dihydropyridazin-4-yl)urea
N-(3,5-Dichloropyridin-4-yl)-N'-(2-ethyl-6-{3-[(4-hydroxyphenyl)sulfonyl]phenyl}-3-oxo-2,3-dihydropyridazin-4-yl)urea
N-{6-[3-(Benzyloxy)phenyl]-2-ethyl-3-oxo-2,3-dihydropyridazin-4-yl}-N'-(3,5-dichloropyridin-4-yl)urea
3'-[5-({[(3,5-Dichloropyridin-4-yl)amino]carbonyl}amino)-1-ethyl-6-oxo-1,6-dihydropyridazin-3-yl]biphenyl-3-yl hydrogen sulfate
N-(3,5-Dichloropyridin-4-yl)-N'-{2-ethyl-6-[3-(4-methoxypyridin-3-yl)phenyl]-3-oxo-2,3-dihydropyridazin-4-yl}urea
3'-[5-({[(3,5-Dichloropyridin-4-yl)amino]carbonyl}amino)-1-ethyl-6-oxo-1,6-dihydropyridazin-3-yl]-N-(2-hydroxyethyl)biphenyl-3-carboxamide
3'-[5-({[(3,5-Dichloropyridin-4-yl)amino]carbonyl}amino)-1-ethyl-6-oxo-1,6-dihydropyridazin-3-yl]-N-[2-(4-methylpiperazin-1-yl)ethyl]biphenyl-3-carboxamide
3'-[5-({[(3,5-Dichloropyridin-4-yl)amino]carbonyl}amino)-1-ethyl-6-oxo-1,6-dihydropyridazin-3-yl]-N-(pyridin-3-ylmethyl)biphenyl-3-carboxamide
N-Benzyl-3'-[5-({[(3,5-dichloropyridin-4-yl)amino]carbonyl}amino)-1-ethyl-6-oxo-1,6-dihydropyridazin-3-yl]biphenyl-3-carboxamide
3'-[5-({[(3,5-Dichloropyridin-4-yl)amino]carbonyl}amino)-1-ethyl-6-oxo-1,6-dihydropyridazin-3-yl]-N-(3-morpholin-4-ylpropyl)biphenyl-3-carboxamide
3'-[5-({[(3,5-Dichloropyridin-4-yl)amino]carbonyl}amino)-1-ethyl-6-oxo-1,6-dihydropyridazin-3-yl]-N-[3-(1H-imidazol-1-yl)propyl]biphenyl-3-carboxamide
N-1-Adamantyl-3'-[5-({[(3,5-dichloropyridin-4-yl)amino]carbonyl}amino)-1-ethyl-6-oxo-1,6-dihydropyridazin-3-yl]biphenyl-3-carboxamide
N-(3-Chlorobenzyl)-3'-[5-({[(3,5-dichloropyridin-4-yl)amino]carbonyl}amino)-1-ethyl-6-oxo-1,6-dihydropyridazin-3-yl]biphenyl-3-carboxamide
N-(1-Adamantylmethyl)-3'-[5-({[(3,5-dichloropyridin-4-yl)amino]carbonyl}amino)-1-ethyl-6-oxo-1,6-dihydropyridazin-3-yl]biphenyl-3-carboxamide
3'-[5-({[(3,5-Dichloropyridin-4-yl)amino]carbonyl}amino)-1-ethyl-6-oxo-1,6-dihydropyridazin-3-yl]-N-(2-pyrrolidin-1-ylethyl)biphenyl-3-carboxamide
3'-[5-({[(3,5-Dichloropyridin-4-yl)amino]carbonyl}amino)-1-ethyl-6-oxo-1,6-dihydropyridazin-3-yl]-N-(2-morpholin-4-ylethyl)biphenyl-4-carboxamide
N-(3,4-Dichlorobenzyl)-3'-[5-({[(3,5-dichloropyridin-4-yl)amino]carbonyl}amino)-1-ethyl-6-oxo-1,6-dihydropyridazin-3-yl]biphenyl-3-carboxamide
3'-[5-({[(3,5-Dichloropyridin-4-yl)amino]carbonyl}amino)-1-ethyl-6-oxo-1,6-dihydropyridazin-3-yl]-N-(2-phenylethyl)biphenyl-3-carboxamide
N-Cyclopentyl-3'-[5-({[(3,5-dichloropyridin-4-yl)amino]carbonyl}amino)-1-ethyl-6-oxo-1,6-dihydropyridazin-3-yl]biphenyl-3-carboxamide
3'-[5-({[(3,5-Dichloropyridin-4-yl)amino]carbonyl}amino)-1-ethyl-6-oxo-1,6-dihydropyridazin-3-yl]-N-(2-pyridin-3-ylethyl)biphenyl-3-carboxamide
3'-[5-({[(3,5-Dichloropyridin-4-yl)amino]carbonyl}amino)-1-ethyl-6-oxo-1,6-dihydropyridazin-3-yl]-N-[2-(1-methylpyrrolidin-2-yl)ethyl]biphenyl-3-carboxamide
N-(3,5-Dichloropyridin-4-yl)-N'-{2-ethyl-3-oxo-6-[3'-(pyrrolidin-1-ylcarbonyl)biphenyl-3-yl]-2,3-dihydropyridazin-4-yl}urea
3'-[5-({[(3,5-Dichloropyridin-4-yl)amino]carbonyl}amino)-1-ethyl-6-oxo-1,6-dihydropyridazin-3-yl]-N-(2-pyridin-2-ylethyl)biphenyl-3-carboxamide
N-(3,5-Dichloropyridin-4-yl)-N'-{2-ethyl-3-oxo-6-[3'-(piperidin-1-ylcarbonyl)biphenyl-3-yl]-2,3-dihydropyridazin-4-yl}urea
N-Cyclobutyl-3'-[5-({[(3,5-dichloropyridin-4-yl)amino]carbonyl}amino)-1-ethyl-6-oxo-1,6-dihydropyridazin-3-yl]biphenyl-3-carboxamide
3'-[5-({[(3,5-Dichloropyridin-4-yl)amino]carbonyl}amino)-1-ethyl-6-oxo-1,6-dihydropyridazin-3-yl]-N-[2-(dimethylamino)ethyl]biphenyl-4-carboxamide
Methyl 3'-[5-({[(3,5-dichloropyridin-4-yl)amino]carbonyl}amino)-1-ethyl-6-oxo-1,6-dihydropyridazin-3-yl]-3-hydroxybiphenyl-4-carboxylate
3'-[5-({[(3,5-Dichloropyridin-4-yl)amino]carbonyl}amino)-1-ethyl-6-oxo-1,6-dihydropyridazin-3-yl]-3-hydroxybiphenyl-4-carboxylic acid
Methyl 3'-[5-({[(3,5-dichloropyridin-4-yl)amino]carbonyl}amino)-1-ethyl-6-oxo-1,6-dihydropyridazin-3-yl]-5-hydroxybiphenyl-3-carboxylate
3'-[5-({[(3,5-Dichloropyridin-4-yl)amino]carbonyl}amino)-1-ethyl-6-oxo-1,6-dihydropyridazin-3-yl]-5-hydroxybiphenyl-3-carboxylic acid
N-Cyclopropyl-3'-[5-({[(3,5-dichloropyridin-4-yl)amino]carbonyl}amino)-1-ethyl-6-oxo-1,6-dihydropyridazin-3-yl]biphenyl-4-carboxamide
N-Cyclopropyl-3'-[5-({[(3,5-dichloro-1-oxidopyridin-4-yl)amino]carbonyl}amino)-1-ethyl-6-oxo-1,6-dihydropyridazin-3-yl]biphenyl-3-carboxamide
3'-[5-({[(3,5-Dichloropyridin-4-yl)amino]carbonyl}amino)-1-ethyl-6-oxo-1,6-dihydropyridazin-3-yl]-N-(2-piperidin-1-ylethyl)biphenyl-4-carboxamide
2-{3'-[5-({[(3,5-Dichloropyridin-4-yl)amino]carbonyl}amino)-1-ethyl-6-oxo-1,6-dihydropyridazin-3-yl]biphenyl-4-yl}-N-(2-morpholin-4-ylethyl)acetamide
2-{3'-[5-({[(3,5-Dichloropyridin-4-yl)amino]carbonyl}amino)-1-ethyl-6-oxo-1,6-dihydropyridazin-3-yl]biphenyl-4-yl}-N-(2-piperidin-1-ylethyl)acetamide
2-{3'-[5-({[(3,5-Dichloropyridin-4-yl)amino]carbonyl}amino)-1-ethyl-6-oxo-1,6-dihydropyridazin-3-yl]biphenyl-3-yl}-N-(2-piperidin-1-ylethyl)acetamide
2-{3'-[5-({[(3,5-Dichloropyridin-4-yl)amino]carbonyl}amino)-1-ethyl-6-oxo-1,6-dihydropyridazin-3-yl]biphenyl-3-yl}-N-(2-morpholin-4-ylethyl)acetamide
3'-[5-({[(3,5-Dichloropyridin-4-yl)amino]carbonyl}amino)-1-ethyl-6-oxo-1,6-dihydropyridazin-3-yl]-N-{7-[(2-hydroxyethyl)(methyl)amino]heptyl}biphenyl-3-carboxamide
3'-[5-({[(3,5-Dichloropyridin-4-yl)amino]carbonyl}amino)-1-ethyl-6-oxo-1,6-dihydropyridazin-3-yl]-N-{7-[(2-hydroxyethyl)(methyl)amino]heptyl}biphenyl-4-carboxamide
2-{3'-[5-({[(3,5-Dichloropyridin-4-yl)amino]carbonyl}amino)-1-ethyl-6-oxo-1,6-dihydropyridazin-3-yl]biphenyl-4-yl}-N-{7-[(2-hydroxyethyl)(methyl)amino] heptyl}acetamide
2-{3'-[5-({[(3,5-Dichloropyridin-4-yl)amino]carbonyl}amino)-1-ethyl-6-oxo-1,6-dihydropyridazin-3-yl]biphenyl-3-yl}-N-{7-[(2-hydroxyethyl)(methyl)amino] heptyl}acetamide
3'-[5-({[(3,5-Dichloropyridin-4-yl)amino]carbonyl}amino)-1-ethyl-6-oxo-1,6-dihydropyridazin-3-yl]-5-hydroxy-N-{7-[(2-hydroxyethyl)(methyl)amino]heptyl}biphenyl-3-carboxamide
N-Cyclopropyl-3'-[5-({[(3,5-dichloropyridin-4-yl)amino]carbonyl}amino)-1-ethyl-6-oxo-1,6-dihydropyridazin-3-yl]-5-hydroxybiphenyl-3-carboxamide
N-Cyclopropyl-2-{3'-[5-({[(3,5-dichloropyridin-4-yl)amino]carbonyl}amino)-1-ethyl-6-oxo-1,6-dihydropyridazin-3-yl]biphenyl-3-yl}acetamide
N-Cyclopropyl-2-{3'-[5-({[(3,5-dichloropyridin-4-yl)amino]carbonyl}amino)-1-ethyl-6-oxo-1,6-dihydropyridazin-3-yl]biphenyl-4-yl}acetamide
N-Cyclopropyl-3'-[5-({[(3,5-dichloropyridin-4-yl)amino]carbonyl}amino)-1-ethyl-6-oxo-1,6-dihydropyridazin-3-yl]-5-fluoro-6-methylbiphenyl-3-carboxamide
N-(Cyclopropylmethyl)-3'-[5-({[(3,5-dichloropyridin-4-yl)amino]carbonyl}amino)-1-ethyl-6-oxo-1,6-dihydropyridazin-3-yl]biphenyl-3-carboxamide
3'-[5-({[(3,5-Dichloropyridin-4-yl)amino]carbonyl}amino)-1-ethyl-6-oxo-1,6-dihydropyridazin-3-yl]-N-propylbiphenyl-3-carboxamide
3'-[5-({[(3,5-Dichloropyridin-4-yl)amino]carbonyl}amino)-1-ethyl-6-oxo-1,6-dihydropyridazin-3-yl]-N-ethylbiphenyl-3-carboxamide
N-Butyl-3'-[5-({[(3,5-dichloropyridin-4-yl)amino]carbonyl}amino)-1-ethyl-6-oxo-1,6-dihydropyridazin-3-yl]biphenyl-3-carboxamide
3'-[5-({[(3,5-Dichloropyridin-4-yl)amino]carbonyl}amino)-1-ethyl-6-oxo-1,6-dihydropyridazin-3-yl]-N-(3-hydroxybenzyl)biphenyl-3-carboxamide
3'-[5-({[(3,5-dichloropyridin-4-yl)amino]carbonyl}amino)-1-ethyl-6-oxo-1,6-dihydropyridazin-3-yl]-N-pyridin-3-ylbiphenyl-3-carboxamide
N-(3,5-Dichloropyridin-4-yl)-N'-(2-ethyl-6-{3'-[(4-methylpiperazin-1-yl)carbonyl]biphenyl-3-yl}-3-oxo-2,3-dihydropyridazin-4-yl)urea
N-Cyclopropyl-5-{3-[5-({[(3,5-dichloropyridin-4-yl)amino]carbonyl}amino)-1-ethyl-6-oxo-1,6-dihydropyridazin-3-yl]phenyl}nicotinamide
3'-[5-({[(3,5-Dichloropyridin-4-yl)amino]carbonyl}amino)-1-ethyl-6-oxo-1,6-dihydropyridazin-3-yl]-N-(4-hydroxybenzyl)biphenyl-3-carboxamide
N-(3,5-Dichloropyridin-4-yl)-N'-[2-ethyl-6-(3'-methoxybiphenyl-3-yl)-3-oxo-2,3-dihydropyridazin-4-yl]urea
3'-[5-({[(3,5-Dichloropyridin-4-yl)amino]carbonyl}amino)-1-ethyl-6-oxo-1,6-dihydropyridazin-3-yl]biphenyl-4-carboxamide
3'-[5-({[(3,5-Dichloropyridin-4-yl)amino]carbonyl}amino)-1-ethyl-6-oxo-1,6-dihydropyridazin-3-yl]-2-methylbiphenyl-4-carboxamide
N-(3,5-Dichloropyridin-4-yl)-N'-{2-ethyl-6-[2'-(methylsulfonyl)biphenyl-3-yl]-3-oxo-2,3-dihydropyridazin-4-yl}urea
N-(3,5-Dichloropyridin-4-yl)-N'-{2-ethyl-6-[3'-(methylsulfonyl)biphenyl-3-yl]-3-oxo-2,3-dihydropyridazin-4-yl}urea
N-(3,5-Dichloropyridin-4-yl)-N'-{2-ethyl-6-[4'-(methylsulfonyl)biphenyl-3-yl]-3-oxo-2,3-dihydropyridazin-4-yl}urea
N-(3,5-Dimethylpyridin-4-yl)-N'-[2-ethyl-6-(3'-hydroxybiphenyl-3-yl)-3-oxo-2,3-dihydropyridazin-4-yl]urea
Methyl 5-[(cyclopropylamino)carbonyl]-3'-[5-({[(3,5-dichloropyridin-4-yl)amino]carbonyl}amino)-1-ethyl-6-oxo-1,6-dihydropyridazin-3-yl]biphenyl-3-carboxylate
5-[(Cyclopropylamino)carbonyl]-3'-[5-({[(3,5-dichloropyridin-4-yl)amino]carbonyl}amino)-1-ethyl-6-oxo-1,6-dihydropyridazin-3-yl]biphenyl-3-carboxylic acid
3-{3'-[5-({[(3,5-Dichloropyridin-4-yl)amino]carbonyl}amino)-1-ethyl-6-oxo-1,6-dihydropyridazin-3-yl]biphenyl-4-yl}propanoic acid
N-Cyclopropyl-3-{3'-[5-({[(3,5-dichloropyridin-4-yl)amino]carbonyl}amino)-1-ethyl-6-oxo-1,6-dihydropyridazin-3-yl]biphenyl-4-yl}propanamide
2-{3'-[5-({[(3,5-Dichloropyridin-4-yl)amino]carbonyl}amino)-1-ethyl-6-oxo-1,6-dihydropyridazin-3-yl]biphenyl-3-yl}-N-(3-morpholin-4-ylpropyl)acetamide
2-{3'-[5-({[(3,5-Dichloropyridin-4-yl)amino]carbonyl}amino)-1-ethyl-6-oxo-1,6-dihydropyridazin-3-yl]biphenyl-4-yl}-N-(3-morpholin-4-ylpropyl)acetamide
2-{3'-[5-({[(3,5-Dichloropyridin-4-yl)amino]carbonyl}amino)-1-ethyl-6-oxo-1,6-dihydropyridazin-3-yl]biphenyl-3-yl}-N-(3-hydroxybenzyl)acetamide
N,N'-Dicyclopropyl-3'-[5-({[(3,5-dichloropyridin-4-yl)amino]carbonyl}amino)-1-ethyl-6-oxo-1,6-dihydropyridazin-3-yl]biphenyl-3,5-dicarboxamide
N-Cyclopropyl-3'-[5-({[(3,5-dichloropyridin-4-yl)amino]carbonyl}amino)-1-ethyl-6-oxo-1,6-dihydropyridazin-3-yl]-N'-{2-[(2R)-1-methylpyrrolidin-2-yl]ethyl}biphenyl-3,5-dicarboxamide
N-Cyclopropyl-3'-[5-({[(3,5-dichloropyridin-4-yl)amino]carbonyl}amino)-1-ethyl-6-oxo-1,6-dihydropyridazin-3-yl]-N'-(2-piperidin-1-ylethyl)biphenyl-3,5-dicarboxamide
N-Cyclopropyl-3'-[5-({[(3,5-dichloropyridin-4-yl)amino]carbonyl}amino)-1-ethyl-6-oxo-1,6-dihydropyridazin-3-yl]-N'-{7-[(2-hydroxyethyl)(methyl)amino]heptyl}biphenyl-3,5-dicarboxamide
Methyl 3'-[5-({[(3,5-dichloropyridin-4-yl)amino]carbonyl}amino)-1-ethyl-6-oxo-1,6-dihydropyridazin-3-yl]-5-({[3-(dimethylamino)propyl]amino}carbonyl)biphenyl-3-carboxylate
Ethyl 3'-[5-({[(3,5-dichloropyridin-4-yl)amino]carbonyl}amino)-1-ethyl-6-oxo-1,6-dihydropyridazin-3-yl]-5-(2-hydroxyethoxy)biphenyl-3-carboxylate
3'-[5-({[(3,5-Dichloropyridin-4-yl)amino]carbonyl}amino)-1-ethyl-6-oxo-1,6-dihydropyridazin-3-yl]-5-(2-hydroxyethoxy)biphenyl-3-carboxylic acid
N-Cyclopropyl-3'-[5-({[(3,5-dichloropyridin-4-yl)amino]carbonyl}amino)-1-ethyl-6-oxo-1,6-dihydropyridazin-3-yl]-5-(2-hydroxyethoxy)biphenyl-3-carboxamide
3'-[5-({[(3,5-Dichloropyridin-4-yl)amino]carbonyl}amino)-1-ethyl-6-oxo-1,6-dihydropyridazin-3-yl]-N-(1-methylpiperidin-4-yl)biphenyl-3-carboxamide
2-{3'-[5-({[(3,5-Dichloropyridin-4-yl)amino]carbonyl}amino)-1-ethyl-6-oxo-1,6-dihydropyridazin-3-yl]biphenyl-4-yl}-N-(1-methylpiperidin-4-yl)acetamide
2-{3'-[5-({[(3,5-Dichloropyridin-4-yl)amino]carbonyl}amino)-1-ethyl-6-oxo-1,6-dihydropyridazin-3-yl]biphenyl-4-yl}-N-{2-[1-(dimethylamino)cyclopentyl]ethyl} acetamide
3'-[5-({[(3,5-Dichloropyridin-4-yl)amino]carbonyl}amino)-1-ethyl-6-oxo-1,6-dihydropyridazin-3-yl]-N-{2-[(2S)-2-(methoxymethyl)pyrrolidin-1-yl]ethyl}-N'-(2-piperidin-1-ylethyl)biphenyl-3,5-dicarboxamide
N-Cyclopentyl-3'-[5-({[(3,5-dichloropyridin-4-yl)amino]carbonyl}amino)-1-ethyl-6-oxo-1,6-dihydropyridazin-3-yl]-N'-(2-piperidin-1-ylethyl)biphenyl-3,5-dicarboxamide
3'-[5-({[(3,5-Dichloropyridin-4-yl)amino]carbonyl}amino)-1-ethyl-6-oxo-1,6-dihydropyridazin-3-yl]-N-{4-[(2S)-2-(methoxymethyl)pyrrolidin-1-yl]butyl}-N'-(2-piperidin-1-ylethyl)biphenyl-3,5-dicarboxamide
2-{3'-[5-({[(3,5-Dichloropyridin-4-yl)amino]carbonyl}amino)-1-ethyl-6-oxo-1,6-dihydropyridazin-3-yl]biphenyl-3-yl}-N-(2-pyrrolidin-1-ylethyl)acetamide
2-{3'-[5-({[(3,5-Dichloropyridin-4-yl)amino]carbonyl}amino)-1-ethyl-6-oxo-1,6-dihydropyridazin-3-yl]biphenyl-4-yl}-N-(2-pyrrolidin-1-ylethyl)acetamide
3'-[5-({[(3,5-Dichloropyridin-4-yl)amino]carbonyl}amino)-1-ethyl-6-oxo-1,6-dihydropyridazin-3-yl]-N-(3-hydroxybenzyl)biphenyl-4-carboxamide
2-{3'-[5-({[(3,5-Dichloropyridin-4-yl)amino]carbonyl}amino)-1-ethyl-6-oxo-1,6-dihydropyridazin-3-yl]biphenyl-4-yll-N-(3-hydroxybenzyl)acetamide
N-Cyclopropyl-N'-[3-(dimethylamino)propyl]-3'-[5-({[(3,5-dimethylpyridin-4-yl)amino]carbonyl}amino)-1-ethyl-6-oxo-1,6-dihydropyridazin-3-yl]biphenyl-3,5-dicarboxamide
3'-[5-({[(3,5-Dichloropyridin-4-yl)amino]carbonyl}amino)-1-ethyl-6-oxo-1,6-dihydropyridazin-3-yl]-N-{5-[(2-hydroxyethyl)(methyl)amino]pentyl}biphenyl-3-carboxamide
2-{3'-[5-({[(3,5-Dichloropyridin-4-yl)amino]carbonyl}amino)-1-ethyl-6-oxo-1,6-dihydropyridazin-3-yl]biphenyl-4-yl}-N-{5-[(2-hydroxyethyl)(methyl)amino]pentyl} acetamide
Ethyl 3'-[5-({[(3,5-dichloropyridin-4-yl)amino]carbonyl}amino)-1-ethyl-6-oxo-1,6-dihydropyridazin-3-yl]-5-(trifluoromethyl)biphenyl-3-carboxylate
3'-[5-({[(3,5-Dichloropyridin-4-yl)amino]carbonyl}amino)-1-ethyl-6-oxo-1,6-dihydropyridazin-3-yl]-5-(trifluoromethyl)biphenyl-3-carboxylic acid
N-Cyclopropyl-3'-[5-({[(3,5-dichloropyridin-4-yl)amino]carbonyl}amino)-1-ethyl-6-oxo-1,6-dihydropyridazin-3-yl]-5-(trifluoromethyl)biphenyl-3-carboxamide
3'-[5-({[(3,5-dichloropyridin-4-yl)amino]carbonyl}amino)-1-ethyl-6-oxo-1,6-dihydropyridazin-3-yl]-N-(2-piperidin-1-ylethyl)-5-(trifluoromethyl)biphenyl-3-carboxamide
3'-[5-({[(3,5-dichloropyridin-4-yl)amino]carbonyl}amino)-1-ethyl-6-oxo-1,6-dihydropyridazin-3-yl]-N-[3-(dimethylamino)propyl]-5-(trifluoromethyl)biphenyl-3-carboxamide
3'-[5-({[(3,5-dichloropyridin-4-yl)amino]carbonyl}amino)-1-ethyl-6-oxo-1,6-dihydropyridazin-3-yl]-N-{7-[(2-hydroxyethyl)(methyl)amino]heptyl}-5-(trifluoromethyl)biphenyl-3-carboxamide
ethyl 3'-[5-({[(3,5-dichloropyridin-4-yl)amino]carbonyl}amino)-1-ethyl-6-oxo-1,6-dihydropyridazin-3-yl]-5-(trifluoromethoxy)biphenyl-3-carboxylate
3'-[5-({[(3,5-dichloropyridin-4-yl)amino]carbonyl}amino)-1-ethyl-6-oxo-1,6-dihydropyridazin-3-yl]-5-(trifluoromethoxy)biphenyl-3-carboxylic acid
N-cyclopropyl-3'-[5-({[(3,5-dichloropyridin-4-yl)amino]carbonyl}amino)-1-ethyl-6-oxo-1,6-dihydropyridazin-3-yl]-5-(trifluoromethoxy)biphenyl-3-carboxamide
3'-[5-({[(3,5-dichloropyridin-4-yl)amino]carbonyl}amino)-1-ethyl-6-oxo-1,6-dihydropyridazin-3-yl]-N-(2-piperidin-1-ylethyl)-5-(trifluoromethoxy)biphenyl-3-carboxamide
3'-[5-({[(3,5-dichloropyridin-4-yl)amino]carbonyl}amino)-1-ethyl-6-oxo-1,6-dihydropyridazin-3-yl]-N-[3-(dimethylamino)propyl]-5-(trifluoromethoxy)biphenyl-3-carboxamide
3'-[5-({[(3,5-dichloropyridin-4-yl)amino]carbonyl}amino)-1-ethyl-6-oxo-1,6-dihydropyridazin-3-yl]-N-(2-pyrrolidin-1-ylethyl)-5-(trifluoromethoxy)biphenyl-3-carboxamide
ethyl 3-{3'-[5-({[(3,5-dichloropyridin-4-yl)amino]carbonyl}amino)-1-ethyl-6-oxo-1,6-dihydropyridazin-3-yl]biphenyl-3-yl}propanoate
3-{3'-[5-({[(3,5-dichloropyridin-4-yl)amino]carbonyl}amino)-1-ethyl-6-oxo-1,6-dihydropyridazin-3-yl]biphenyl-3-yl}propanoic acid
3-{3'-[5-({[(3,5-dichloropyridin-4-yl)amino]carbonyl}amino)-1-ethyl-6-oxo-1,6-dihydropyridazin-3-yl]biphenyl-3-yl}-N-{7-[(2-hydroxyethyl)(methyl)amino]heptyl}propanamide
3-{3'-[5-({[(3,5-dichloropyridin-4-yl)amino]carbonyl}amino)-1-ethyl-6-oxo-1,6-dihydropyridazin-3-yl]biphenyl-3-yl}-N-(4-hydroxybenzyl)propanamide
3-{3'-[5-({[(3,5-dichloropyridin-4-yl)amino]carbonyl}amino)-1-ethyl-6-oxo-1,6-dihydropyridazin-3-yl]biphenyl-3-yl}-N-(3-morpholin-4-ylpropyl)propanamide
3-{3'-[5-({[(3,5-dichloropyridin-4-yl)amino]carbonyl}amino)-1-ethyl-6-oxo-1,6-dihydropyridazin-3-yl]biphenyl-4-yl}-N-(3-morpholin-4-ylpropyl)propanamide
3-{3'-[5-({[(3,5-dichloropyridin-4-yl)amino]carbonyl}amino)-1-ethyl-6-oxo-1,6-dihydropyridazin-3-yl]biphenyl-4-yl}-N-{7-[(2-hydroxyethyl)(methyl)amino]heptyl}propanamide
3-{3'-[5-({[(3,5-dichloropyridin-4-yl)amino]carbonyl}amino)-1-ethyl-6-oxo-1,6-dihydropyridazin-3-yl]biphenyl-4-yl}-N-{5-[(2-hydroxyethyl)(methyl)amino]pentyl}propanamide
ethyl 5-(cyclopropylmethoxy)-3'-[5-({[(3,5-dichloropyridin-4-yl)amino]carbonyl}amino)-1-ethyl-6-oxo-1,6-dihydropyridazin-3-yl]biphenyl-3-carboxylate
5-(cyclopropylmethoxy)-3'-[5-({[(3,5-dichloropyridin-4-yl)amino]carbonyl}amino)-1-ethyl-6-oxo-1,6-dihydropyridazin-3-yl]biphenyl-3-carboxylic acid
5-(cyclopropylmethoxy)-3'-[5-({[(3,5-dichloropyridin-4-yl)amino]carbonyl}amino)-1-ethyl-6-oxo-1,6-dihydropyridazin-3-yl]-N-{7-[(2R)-2-(methoxymethyl)pyrrolidin-1-yl]heptyl}biphenyl-3-carboxamide
5-(cyclopropylmethoxy)-3'-[5-({[(3,5-dichloropyridin-4-yl)amino]carbonyl}amino)-1-ethyl-6-oxo-1,6-dihydropyridazin-3-yl]-N-(2-piperidin-1-ylethyl)biphenyl-3-carboxamide
5-(cyclopropylmethoxy)-3'-[5-({[(3,5-dichloropyridin-4-yl)amino]carbonyl}amino)-1-ethyl-6-oxo-1,6-dihydropyridazin-3-yl]-N-{7-[(2-hydroxyethyl)(methyl)amino]heptyl}biphenyl-3-carboxamide
5-(cyclopropylmethoxy)-3'-[5-({[(3,5-dichloropyridin-4-yl)amino]carbonyl}amino)-1-ethyl-6-oxo-1,6-dihydropyridazin-3-yl]-N-(1-methylpiperidin-4-yl)biphenyl-3-carboxamide
methyl 3'-[5-({[(3,5-dichloropyridin-4-yl)amino]carbonyl}amino)-1-ethyl-6-oxo-1,6-dihydropyridazin-3-yl]-5-{[(2-piperidin-1-ylethyl)amino]carbonyl}biphenyl-3-carboxylate
3'-[5-({[(3,5-dichloropyridin-4-yl)amino]carbonyl}amino)-1-ethyl-6-oxo-1,6-dihydropyridazin-3-yl]-5-{[(2-piperidin-1-ylethyl)amino]carbonyl}biphenyl-3-carboxylic acid
3'-[5-({[(3,5-dichloropyridin-4-yl)amino]carbonyl}amino)-1-ethyl-6-oxo-1,6-dihydropyridazin-3-yl]-N-ethyl-N'-(2-piperidin-1-ylethyl)biphenyl-3,5-dicarboxamide
3'-[5-({[(3,5-dichloropyridin-4-yl)amino]carbonyl}amino)-1-ethyl-6-oxo-1,6-dihydropyridazin-3-yl]-N-(2-piperidin-1-ylethyl)-N'-(2-pyrrolidin-1-ylethyl)biphenyl-3,5-dicarboxamide
3'-[5-({[(3,5-dichloropyridin-4-yl)amino]carbonyl}amino)-1-ethyl-6-oxo-1,6-dihydropyridazin-3-yl]-N-(2-piperidin-1-ylethyl)biphenyl-3,5-dicarboxamide
3'-[5-({[(3,5-dichloropyridin-4-yl)amino]carbonyl}amino)-1-ethyl-6-oxo-1,6-dihydropyridazin-3-yl]-N-(2-hydroxyethyl)-N'-(2-piperidin-1-ylethyl)biphenyl-3,5-dicarboxamide
3'-[5-({[(3,5-dichloropyridin-4-yl)amino]carbonyl}amino)-1-ethyl-6-oxo-1,6-dihydropyridazin-3-yl]-N,N-dimethyl-N'-(2-piperidin-1-ylethyl)biphenyl-3,5-dicarboxamide
ethyl 3'-[5-({[(3,5-dichloropyridin-4-yl)amino]carbonyl}amino)-1-ethyl-6-oxo-1,6-dihydropyridazin-3-yl]-5-ethoxybiphenyl-3-carboxylate
3'-[5-({[(3,5-dichloropyridin-4-yl)amino]carbonyl}amino)-1-ethyl-6-oxo-1,6-dihydropyridazin-3-yl]-5-ethoxybiphenyl-3-carboxylic acid
N-cyclopropyl-3'-[5-({[(3,5-dichloropyridin-4-yl)amino]carbonyl}amino)-1-ethyl-6-oxo-1,6-dihydropyridazin-3-yl]-5-ethoxybiphenyl-3-carboxamide
3'-[5-({[(3,5-dichloropyridin-4-yl)amino]carbonyl}amino)-1-ethyl-6-oxo-1,6-dihydropyridazin-3-yl]-5-ethoxy-N-{7-[(2-hydroxyethyl)(methyl)amino]heptyl}biphenyl-3-carboxamide
3'-[5-({[(3,5-dichloropyridin-4-yl)amino]carbonyl}amino)-1-ethyl-6-oxo-1,6-dihydropyridazin-3-yl]-5-ethoxy-N-{7-[(2R)-2-(methoxymethyl)pyrrolidin-1-yl]heptyl}biphenyl-3-carboxamide
3'-[5-({[(3,5-dichloropyridin-4-yl)amino]carbonyl}amino)-1-ethyl-6-oxo-1,6-dihydropyridazin-3-yl]-5-(2-hydroxyethoxy)-N-{5-[(2-hydroxyethyl)(methyl)amino]pentyl}biphenyl-3-carboxamide
3'-[5-({[(3,5-dichloropyridin-4-yl)amino]carbonyl}amino)-1-ethyl-6-oxo-1,6-dihydropyridazin-3-yl]-5-(2-hydroxyethoxy)-N-(2-piperidin-1-ylethyl)biphenyl-3-carboxamide
3'-[5-({[(3,5-dichloropyridin-4-yl)amino]carbonyl}amino)-1-ethyl-6-oxo-1,6-dihydropyridazin-3-yl]-N-[3-(dimethylamino)propyl]-5-(2-hydroxyethoxy)biphenyl-3-carboxamide
3'-[5-({[(3,5-dichloropyridin-4-yl)amino]carbonyl}amino)-1-ethyl-6-oxo-1,6-dihydropyridazin-3-yl]-5-(2-hydroxyethoxy)-N-{7-[(2-hydroxyethyl)(methyl)amino]heptyl}biphenyl-3-carboxamide
3'-[5-({[(3,5-dichloropyridin-4-yl)amino]carbonyl}amino)-1-ethyl-6-oxo-1,6-dihydropyridazin-3-yl]-5-(2-hydroxyethoxy)-N-(1-methylpiperidin-4-yl)biphenyl-3-carboxamide
3'-[5-({[(3,5-dichloropyridin-4-yl)amino]carbonyl}amino)-1-ethyl-6-oxo-1,6-dihydropyridazin-3-yl]-N-{2-[(2-hydroxyethyl)(methyl)amino]ethyl}biphenyl-3-carboxamide
1-({3'-[5-({[(3,5-dichloropyridin-4-yl)amino]carbonyl}amino)-1-ethyl-6-oxo-1,6-dihydropyridazin-3-yl]biphenyl-3-yl}carbonyl)piperidine-4-carboxamide
N-({3'-[5-({[(3,5-dichloropyridin-4-yl)amino]carbonyl}amino)-1-ethyl-6-oxo-1,6-dihydropyridazin-3-yl]biphenyl-3-yllcarbonyl)glycine
(1R,2S)-2-[({3'-[5-({[(3,5-dichloropyridin-4-yl)amino]carbonyl}amino)-1-ethyl-6-oxo-1,6-dihydropyridazin-3-yl]biphenyl-3-yl}carbonyl)amino]cyclopentanecarboxylic acid
3'-[5-({[(3,5-dichloropyridin-4-yl)amino]carbonyl}amino)-1-ethyl-6-oxo-1,6-dihydropyridazin-3-yl]-N-[3-(diethylamino)propyl]biphenyl-3-carboxamide
3'-[5-({[(3,5-dichloropyridin-4-yl)amino]carbonyl}amino)-1-ethyl-6-oxo-1,6-dihydropyridazin-3-yl]-N-{3-[(2-hydroxyethyl)(methyl)amino]propyl}-N'-(2-piperidin-1-ylethyl)biphenyl-3,5-dicarboxamide
3'-[5-({[(3,5-dichloropyridin-4-yl)amino]carbonyl}amino)-1-ethyl-6-oxo-1,6-dihydropyridazin-3-yl]-N-[3-(dimethylamino)propyl]-5-ethoxybiphenyl-3-carboxamide
3'-[5-({[(3,5-dichloropyridin-4-yl)amino]carbonyl}amino)-1-ethyl-6-oxo-1,6-dihydropyridazin-3-yl]-5-ethoxy-N-(2-piperidin-1-ylethyl)biphenyl-3-carboxamide
3'-[5-({[(3,5-dichloropyridin-4-yl)amino]carbonyl}amino)-1-ethyl-6-oxo-1,6-dihydropyridazin-3-yl]-5-ethoxy-N-(1-methylpiperidin-4-yl)biphenyl-3-carboxamide
ethyl 3'-[5-({[(3,5-dichloropyridin-4-yl)amino]carbonyl}amino)-1-ethyl-6-oxo-1,6-dihydropyridazin-3-yl]-5-{2-[(2-hydroxyethyl)(methyl)amino]ethoxy}biphenyl-3-carboxylate
ethyl 3'-[5-({[(3,5-dichloropyridin-4-yl)amino]carbonyl}amino)-1-ethyl-6-oxo-1,6-dihydropyridazin-3-yl]-5-[2-(dimethylamino)ethoxy]biphenyl-3-carboxylate
methyl 3'-[5-({[(3,5-dichloropyridin-4-yl)amino]carbonyl}amino)-1-ethyl-6-oxo-1,6-dihydropyridazin-3-yl]-5-(piperidin-1-ylcarbonyl)biphenyl-3-carboxylate
N-cyclopropyl-3'-[5-({[(3,5-dichloropyridin-4-yl)amino]carbonyl}amino)-1-ethyl-6-oxo-1,6-dihydropyridazin-3-yl]-6-methylbiphenyl-3-carboxamide
N-{6-[3'-(cyclopropyloxy)biphenyl-3-yl]-2-ethyl-3-oxo-2,3-dihydropyridazin-4-yl}-N'-(3,5-dichloropyridin-4-yl)urea
2-{3'-[5-({[(3,5-dichloropyridin-4-yl)amino]carbonyl}amino)-1-ethyl-6-oxo-1,6-dihydropyridazin-3-yl]biphenyl-4-yl}-N-[2-(4-methylpiperazin-1-yl)ethyl]acetamide
3'-[5-({[(3,5-dichloropyridin-4-yl)amino]carbonyl}amino)-1-ethyl-6-oxo-1,6-dihydropyridazin-3-yl]-5-hydroxy-N-{2-[(2R)-1-methylpyrrolidin-2-yl]ethyl}biphenyl-3-carboxamide
dimethyl 3'-[5-({[(3,5-dichloropyridin-4-yl)amino]carbonyl}amino)-1-ethyl-6-oxo-1,6-dihydropyridazin-3-yl]biphenyl-3,5-dicarboxylate
3'-[5-({[(3,5-dichloropyridin-4-yl)amino]carbonyl}amino)-1-ethyl-6-oxo-1,6-dihydropyridazin-3-yl]biphenyl-3,5-dicarboxylic acid
3'-[5-({[(3,5-dichloropyridin-4-yl)amino]carbonyl}amino)-1-ethyl-6-oxo-1,6-dihydropyridazin-3-yl]-N,N'-bis(2-piperidin-1-ylethyl)biphenyl-3,5-dicarboxamide
ethyl 3'-[5-({[(3,5-dichloropyridin-4-yl)amino]carbonyl}amino)-1-ethyl-6-oxo-1,6-dihydropyridazin-3-yl]-5-{[5-(dimethylamino)pentyl]oxy}biphenyl-3-carboxylate
3'-[5-({[(3,5-dichloropyridin-4-yl)amino]carbonyl}amino)-1-ethyl-6-oxo-1,6-dihydropyridazin-3-yl]-5-{[5-(dimethylamino)pentyl]oxy}biphenyl-3-carboxylicacid
N-cyclopropyl-3'-[5-({[(3,5-dichloropyridin-4-yl)amino]carbonyl}amino)-1-ethyl-6-oxo-1,6-dihydropyridazin-3-yl]-5-{[5-(dimethylamino)pentyl]oxy}biphenyl-3-carboxamide
3'-[5-({[(3,5-dichloropyridin-4-yl)amino]carbonyl}amino)-1-ethyl-6-oxo-1,6-dihydropyridazin-3-yl]-N-[2-(dimethylamino)ethyl]-N'-(2-piperidin-1-ylethyl)biphenyl-3,5-dicarboxamide
N-(3,5-dichloropyridin-4-yl)-N'-[2-ethyl-6-(3'-formyl-5'-propoxybiphenyl-3-yl)-3-oxo-2,3-dihydropyridazin-4-yl]urea
N-(6-{3'-[(cyclopropylamino)methyl]-5'-propoxybiphenyl-3-yl}-2-ethyl-3-oxo-2,3-dihydropyridazin-4-yl)-N'-(3,5-dichloropyridin-4-yl)urea
N-(3,5-dichloropyridin-4-yl)-N'-(6-{3'-[(dimethylamino)methyl]-5'-propoxybiphenyl-3-yl}-2-ethyl-3-oxo-2,3-dihydropyridazin-4-yl)urea
N-{6-[3',5'-bis(piperidin-1-ylcarbonyl)biphenyl-3-yl]-2-ethyl-3-oxo-2,3-dihydropyridazin-4-yl}-N'-(3,5-dichloropyridin-4-yl)urea
N-(6-{3',5'-bis[(4-methylpiperazin-1-yl)carbonyl]biphenyl-3-yl}-2-ethyl-3-oxo-2,3-dihydropyridazin-4-yl)-N'-(3,5-dichloropyridin-4-yl)urea
3'-[5-({[(3,5-dichloropyridin-4-yl)amino]carbonyl}amino)-1-ethyl-6-oxo-1,6-dihydropyridazin-3-yl]-N,N'-bis(2-pyrrolidin-1-ylethyl)biphenyl-3,5-dicarboxamide
3'-[5-({[(3,5-dichloropyridin-4-yl)amino]carbonyl}amino)-1-ethyl-6-oxo-1,6-dihydropyridazin-3-yl]-N,N'-bis(2-morpholin-4-ylethyl)biphenyl-3,5-dicarboxamide
3'-[5-({[(3,5-dichloropyridin-4-yl)amino]carbonyl}amino)-1-ethyl-6-oxo-1,6-dihydropyridazin-3-yl]-N,N'-bis[2-(4-methylpiperazin-1-yl)ethyl]biphenyl-3,5-dicarboxamide
3'-[5-({[(3,5-dichloropyridin-4-yl)amino]carbonyl}amino)-1-ethyl-6-oxo-1,6-dihydropyridazin-3-yl]-N,N'-bis(1-methylpiperidin-4-yl)biphenyl-3,5-dicarboxamide
3'-[5-({[(3,5-dichioropyridin-4-yl)amino]carbonyl}amino)-1-ethyl-6-oxo-1,6-dihydropyridazin-3-yl]-N,N,N',N'-tetramethylbiphenyl-3,5-dicarboxamide
3'-[5-({[(3,5-dichloropyridin-4-yl)amino]carbonyl}amino)-1-ethyl-6-oxo-1,6-dihydropyridazin-3-yl]-N,N'-bis[3-(dimethylamino)propyl]biphenyl-3,5-dicarboxamide
3'-[5-({[(3,5-dichloropyridin-4-yl)amino]carbonyl}amino)-1-ethyl-6-oxo-1,6-dihydropyridazin-3-yl]-N,N'-bis[3-(1H-imidazol-1-yl)propyl]biphenyl-3,5-dicarboxamide
3'-[5-({[(3,5-dichloropyridin-4-yl)amino]carbonyl}amino)-1-ethyl-6-oxo-1,6-dihydropyridazin-3-yl]-N,N'-bis(2-hydroxyethyl)biphenyl-3,5-dicarboxamide
3-{3'-[5-({[(3,5-dichloropyridin-4-yl)amino]carbonyl}amino)-1-ethyl-6-oxo-1,6-dihydropyridazin-3-yl]biphenyl-4-yl}-N-(2-pyrrolidin-1-ylethyl)propanamide
3-{3'-[5-({[(3,5-dichloropyridin-4-yl)amino]carbonyl}amino)-1-ethyl-6-oxo-1,6-dihydropyridazin-3-yl]biphenyl-4-yl}-N-(2-piperidin-1-ylethyl)propanamide
N-cyclopropyl-3-{3'-[5-({[(3,5-dichloropyridin-4-yl)amino]carbonyl}amino)-1-ethyl-6-oxo-1,6-dihydropyridazin-3-yl]biphenyl-3-yl}propanamide
3-{3'-[5-({[(3,5-dichloropyridin-4-yl)amino]carbonyl}amino)-1-ethyl-6-oxo-1,6-dihydropyridazin-3-yl]biphenyl-3-yl}-N-(2-piperidin-1-ylethyl)propanamide
N-(1-benzylpiperidin-4-yl)-N'-cyclopropyl-3'-[5-({[(3,5-dichloropyridin-4-yl)amino]carbonyl}amino)-1-ethyl-6-oxo-1,6-dihydropyridazin-3-yl]biphenyl-3,5-dicarboxamide
N-cyclopropyl-3'-[5-({[(3,5-dichloropyridin-4-yl)amino]carbonyl}amino)-1-ethyl-6-oxo-1,6-dihydropyridazin-3-yl]-N'-[1-(2-hydroxyethyl)piperidin-4-yl]biphenyl-3,5-dicarboxamide
N-cyclopropyl-3'-[5-({[(3,5-dichloropyridin-4-yl)amino]carbonyl}amino)-1-ethyl-6-oxo-1,6-dihydropyridazin-3-yl]-N'-[1-(5-hydroxypentyl)piperidin-4-yl]biphenyl-3,5-dicarboxamide
ethyl 5-cyano-3'-[5-({[(3,5-dichloropyridin-4-yl)amino]carbonyl}amino)-1-ethyl-6-oxo-1,6-dihydropyridazin-3-yl]biphenyl-3-carboxylate
5-cyano-3'-[5-({[(3,5-dichloropyridin-4-yl)amino]carbonyl}amino)-1-ethyl-6-oxo-1,6-dihydropyridazin-3-yl]biphenyl-3-carboxylic acid
5-cyano-N-cyclopropyl-3'-[5-({[(3,5-dichloropyridin-4-yl)amino]carbonyl}amino)-1-ethyl-6-oxo-1,6-dihydropyridazin-3-yl]biphenyl-3-carboxamide
5-cyano-3'-[5-({[(3,5-dichloropyridin-4-yl)amino]carbonyl}amino)-1-ethyl-6-oxo-1,6-dihydropyridazin-3-yl]-N-(2-piperidin-1-ylethyl)biphenyl-3-carboxamide
methyl 3'-[5-({[(3,5-dichloropyridin-4-yl)amino]carbonyl}amino)-1-ethyl-6-oxo-1,6-dihydropyridazin-3-yl]-5-{[(2-pyrrolidin-1-ylethyl)amino]carbonyl}biphenyl-3-carboxylate
3'-[5-({[(3,5-dichloropyridin-4-yl)amino]carbonyl}amino)-1-ethyl-6-oxo-1,6-dihydropyridazin-3-yl]-5-({[3-(dimethylamino)propyl]amino}carbonyl)biphenyl-3-carboxylic acid
methyl 5-[(cyclopropylamino)carbonyl]-3'-[5-({[(3,5-dichloropyridin-4-yl)amino]carbonyl}amino)-1-ethyl-6-oxo-1,6-dihydropyridazin-3-yl]biphenyl-3-carboxylate
3'-[5-({[(3,5-dichloropyridin-4-yl)amino]carbonyl}amino)-1-ethyl-6-oxo-1,6-dihydropyridazin-3-yl]-N-[3-(dimethylamino)propyl]-N'-ethylbiphenyl-3,5-dicarboxamide
3'-[5-({[(3,5-dichloropyridin-4-yl)amino]carbonyl}amino)-1-ethyl-6-oxo-1,6-dihydropyridazin-3-yl]-N'-[3-(dimethylamino)propyl]-N,N-dimethylbiphenyl-3,5-dicarboxamide
N-cyclopropyl-3'-[5-({[(3,5-dichloropyridin-4-yl)amino]carbonyl}amino)-1-ethyl-6-oxo-1,6-dihydropyridazin-3-yl]-N'-[3-(dimethylamino)propyl]biphenyl-3,5-dicarboxamide
N-cyclopropyl-3'-[5-({[(3,5-dichloropyridin-4-yl)amino]carbonyl}amino)-1-ethyl-6-oxo-1,6-dihydropyridazin-3-yl]-N'-(2-morpholin-4-ylethyl)biphenyl-3,5-dicarboxamide
N-cyclopropyl-3'-[5-({[(3,5-dichloropyridin-4-yl)amino]carbonyl}amino)-1-ethyl-6-oxo-1,6-dihydropyridazin-3-yl]-N'-(4-hydroxybenzyl)biphenyl-3,5-dicarboxamide
N-cyclopropyl-3'-[5-({[(3,5-dichloropyridin-4-yl)amino]carbonyl}amino)-1-ethyl-6-oxo-1,6-dihydropyridazin-3-yl]-N'-[3-(1H-imidazol-1-yl)propyl]biphenyl-3,5-dicarboxamide
N-cyclopropyl-3'-[5-({[(3,5-dichloropyridin-4-yl)amino]carbonyl}amino)-1-ethyl-6-oxo-1,6-dihydropyridazin-3-yl]-N'-{5-[(2-hydroxyethyl)(methyl)amino]pentyl}biphenyl-3,5-dicarboxamide
N'-cyclopropyl-3'-[5-({[(3,5-dichloropyridin-4-yl)amino]carbonyl}amino)-1-ethyl-6-oxo-1,6-dihydropyridazin-3-yl]-N,N-dimethylbiphenyl-3,5-dicarboxamide
N-cyclopropyl-3'-[5-({[(3,5-dichloropyridin-4-yl)amino]carbonyl}amino)-1-ethyl-6-oxo-1,6-dihydropyridazin-3-yl]-N'-(2-hydroxyethyl)biphenyl-3,5-dicarboxamide
N-cyclopropyl-3'-[5-({[(3,5-dichloropyridin-4-yl)amino]carbonyl}amino)-1-ethyl-6-oxo-1,6-dihydropyridazin-3-yl]-N'-(2-pyrrolidin-1-ylethyl)biphenyl-3,5-dicarboxamide
N-cyclopropyl-3'-[5-({[(3,5-dichloropyridin-4-yl)amino]carbonyl}amino)-1-ethyl-6-oxo-1,6-dihydropyridazin-3-yl]biphenyl-3,5-dicarboxamide
1-({5-[(cyclopropylamino)carbonyl]-3'-[5-({[(3,5-dichloropyridin-4-yl)amino]carbonyl}amino)-1-ethyl-6-oxo-1,6-dihydropyridazin-3-yl]biphenyl-3-yl}carbonyl)piperidine-4-carboxamide
N'-cyclopropyl-3'-[5-({[(3,5-dichloropyridin-4-yl)amino]carbonyl}amino)-1-ethyl-6-oxo-1,6-dihydropyridazin-3-yl]-N-(2-hydroxyethyl)-N-methylbiphenyl-3,5-dicarboxamide
5-cyano-3'-[5-({[(3,5-dichloropyridin-4-yl)amino]carbonyl}amino)-1-ethyl-6-oxo-1,6-dihydropyridazin-3-yl]-N-(2-pyrrolidin-1-ylethyl)biphenyl-3-carboxamide
5-cyano-3'-[5-({[(3,5-dichloropyridin-4-yl)amino]carbonyl}amino)-1-ethyl-6-oxo-1,6-dihydropyridazin-3-yl]-N-(2-morpholin-4-ylethyl)biphenyl-3-carboxamide
methyl 3'-[5-({[(3,5-dichloropyridin-4-yl)amino]carbonyl}amino)-1-ethyl-6-oxo-1,6-dihydropyridazin-3-yl]-5-[(ethylamino)carbonyl]biphenyl-3-carboxylate
methyl 3'-[5-({[(3,5-dichloropyridin-4-yl)amino]carbonyl}amino)-1-ethyl-6-oxo-1,6-dihydropyridazin-3-yl]-5-{[(2-hydroxyethyl)amino]carbonyl}biphenyl-3-carboxylate
N-[6-(3'-cyanobiphenyl-3-yl)-2-ethyl-3-oxo-2,3-dihydropyridazin-4-yl]-N'-(3,5-dichloropyridin-4-yl)urea
N-[6-(4'-cyanobiphenyl-3-yl)-2-ethyl-3-oxo-2,3-dihydropyridazin-4-yl]-N'-(3,5-dichloropyridin-4-yl)urea
3'-[1-(cyclopropylmethyl)-5-({[(3,5-dichloropyridin-4-yl)amino]carbonyl}amino)-6-oxo-1,6-dihydropyridazin-3-yl]-N-[3-(dimethylamino)propyl]biphenyl-3-carboxamide
N-cyclopropyl-3'-[1-(cyclopropylmethyl)-5-({[(3,5-dichloropyridin-4-yl)amino]carbonyl}amino)-6-oxo-1,6-dihydropyridazin-3-yl]biphenyl-3-carboxamide
N-cyclopropyl-3'-[5-({[(3,5-dimethylpyridin-4-yl)amino]carbonyl}amino)-1-ethyl-6-oxo-1,6-dihydropyridazin-3-yl]biphenyl-3-carboxamide
N-cyclopropyl-3'-[5-({[(3,5-dichloropyridin-4-yl)amin0]carbonyl}amino)-6-oxo-1-(2,2,2-trifluoroethyl)-1,6-dihydropyridazin-3-yl]biphenyl-3-carboxamide
2-{3'-[1-(cyclopropylmethyl)-5-({[(3,5-dichloropyridin-4-yl)amino]carbonyl}amino)-6-oxo-1,6-dihydropyridazin-3-yl]biphenyl-4-yl}-N-(2-morpholin-4-ylethyl)acetamide
2-{3'-[5-({[(3,5-dichloropyridin-4-yl)amino]carbonyl}amino)-6-oxo-1-(2,2,2-trifluoroethyl)-1,6-dihydropyridazin-3-yl]biphenyl-4-yl}-N-(2-piperidin-1-ylethyl)acetamide
N-cyclopropyl-3'-[5-({[(3,5-dichloropyridin-4-yl)amino]carbonyl}amino)-1-(2,2-difluoroethyl)-6-oxo-1,6-dihydropyridazin-3-yl]biphenyl-3-carboxamide
2-{3'-[5-({[(3,5-dichloropyridin-4-yl)amino]carbonyl}amino)-1-(2,2-difluoroethyl)-6-oxo-1,6-dihydropyridazin-3-yl]biphenyl-4-yl}-N-(2-piperidin-1-ylethyl)acetamide
2-{3'-[1-(cyclopropylmethyl)-5-({[(3,5-dichloropyridin-4-yl)amino]carbonyl}amino)-6-oxo-1,6-dihydropyridazin-3-yl]biphenyl-4-yl}-N-(2-piperidin-1-ylethyl)acetamide
2-{3'-[5-({[(3,5-dimethylpyridin-4-yl)amino]carbonyl}amino)-1-ethyl-6-oxo-1,6-dihydropyridazin-3-yl]biphenyl-4-yl}-N-(2-piperidin-1-ylethyl)acetamide
N,N'-dicyclopropyl-3'-[5-({[(3,5-dimethylpyridin-4-yl)amino]carbonyl}amino)-1-ethyl-6-oxo-1,6-dihydropyridazin-3-yl]biphenyl-3,5-dicarboxamide
N'-cyclopropyl-3'-[5-({[(3,5-dimethylpyridin-4-yl)amino]carbonyl}amino)-1-ethyl-6-oxo-1,6-dihydropyridazin-3-yl]-N,N-diethylbiphenyl-3,5-dicarboxamide
N'-cyclopropyl-3'-[5-({[(3,5-dimethylpyridin-4-yl)amino]carbonyl}amino)-1-ethyl-6-oxo-1,6-dihydropyridazin-3-yl]-N,N-dimethylbiphenyl-3,5-dicarboxamide

20. A compound according to any one of claims 1 to 19 for use in the treatment of a pathological condition or disease susceptible to amelioration by inhibition of phosphodiesterase IV.

21. A compound according to claim 20, wherein the pathological condition or disease is selected from asthma, chronic obstructive pulmonary disease, allergic rhinitis, rheumatoid arthritis, multiple sclerosis, atopic dermatitis, psoriasis or inflammatory bowel disease.

22. A pharmaceutical composition comprising a compound as defined in any one of claims 1 to 19 in association with a pharmaceutically acceptable diluent or carrier.

23. Use of a compound as defined in any one of claims 1 to 19 for the manufacture of a medicament for the treatment of a pathological condition or disease as defined in claims 20 or 21.

24. A method for treating a subject afflicted with a pathological condition or disease as defined in claims 20 or 21, which comprises administering to said subject an effective amount of a compound as defined in any one of claims 1 to 19.

25. A combination product comprising (i) a compound according to anyone of claims 1 to 19, and (ii) one or more active ingredients selected from the group consisting of β2-adrenergic agonist, anti-cholinergic, an anti-allergic agent, an anti-inflammatory agent, an immunosuppressant, and an anti-infective agent, for simultaneous, separate or sequential use in the treatment of the human or animal body.
